# EUROPEAN PATENT SPECIFICATION

(11) **EP 2 696 815 B1**
(45) Date of publication and mention of the grant of the patent: **20.03.2019**
(21) Application number: 12771847.6
(22) Date of filing: 12.04.2012
(51) Int. Cl.: A61L 31/10, A61L 31/16

(54) **STENTS HAVING CONTROLLED ELUTION**
STENTS MIT GESTEUERTER ELUTION
ENDOPROTHÈSES PRÉSENTANT UNE ÉLUTION RÉGULÉE

(30) Priority: 13.04.2011 US 201161475190 P; 07.11.2011 US 201161556742 P; 28.12.2011 US 201161581057 P
(43) Date of publication of application: 19.02.2014
(73) Proprietor: Micell Technologies, Inc., Durham, NC 27713 (US)
(72) Inventor: MCCLAIN, James, B., Raleigh NC 27613 (US); TAYLOR, Charles, Douglas, Franklinton NC 27525 (US)
(74) Representative: Wichmann, Hendrik
(86) International application number: PCT/US2012/033367
(87) International publication number: WO 2012/142319

(56) References cited:
- WO-A1-2011/133655
- US-A1- 2004 220 660
- US-A1- 2008 138 375
- US-A1- 2009 292 351
- US-A1- 2010 198 330
- US-A1- 2010 241 220
- US-A1- 2010 256 748
- US-A1- 2011 009 953
- US-B2- 7 727 275
- JENSEN ET AL.: 'Neointimal hyperplasia after sirolimus-eluting and paclitaxel-eluting stent implantation in diabetic patients: the randomized diabetes and drug eluting stent (DiabeDES) intravascular ultrasound trial.' EUROPEAN HEART JOURNAL vol. 29, no. 22, 02 October 2008, pages 2733 - 2741, XP055128261 Retrieved from the Internet: <URL:http://eurheartj.oxfordjournals.org/co ntent/29/22/2733.full.pdf> [retrieved on 2012-07-17]

## Description

### BACKGROUND OF THE INVENTION

Drug-eluting stents are used to address the drawbacks of bare stents, namely to treat restenosis and to promote healing of the vessel after opening the blockage by PCI/stenting. Some current drug eluting stents can have physical, chemical and therapeutic legacy in the vessel over time. Others may have less legacy, but are not optimized for thickness, deployment flexibility, access to difficult lesions, and minimization of vessel wall intrusion.
US 2010/241220 A1 discloses a method for preparing a coated stent comprising the following steps: providing a stent; forming a coating comprising a pharmaceutical agent and a polymer on the stent wherein at least part of the pharmaceutical agent is in crystalline form, and wherein said coating is substantially conformal to the stent struts when the coated stent is in an expanded state.

### SUMMARY OF THE INVENTION

The present invention relates to a method of coating a stent comprising:
mounting a stent on a holder in a coating chamber that imparts a charge to the stent,
providing a first cloud of charged particles of polymer to the stents by rapidly expanding a pressurized solution of the polymer in densified 1,1,1,2,3,3-hexafluoropropane through a first orifice, wherein the polymer comprises PLGA, wherein a first polymer layer of the polymer particles is formed on the stent by electrostatic deposition,
sintering the first polymer layer at >40°C in ambient pressure,
providing a first cloud of charged sirolimus particles to the stents having an opposite charge than the charge of the stent by pulsing sirolimus particles into the chamber using a propellant in order to deposit a first agent layer on the stent, wherein at least a portion of the sirolimus particles is in crystalline form,
providing a second cloud of charged particles of the polymer and a third cloud of charged particles of the polymer to the stents by sequentially rapidly expanding the pressurized solution through
the first orifice, wherein the particles have an opposite charge than the charge of the stent, wherein a second polymer layer of the polymer particles is formed on the stent by electrostatic deposition,
sintering the second polymer layer at >40°C in ambient pressure,
providing a second cloud of charged sirolimus particles to the stents having an opposite charge than the charge of the stent by pulsing the sirolimus particles into the chamber using a propellant in order to deposit a second agent layer on the stent, wherein at least a portion of the sirolimus particles is in crystalline form,
providing a fourth cloud of charged particles of the polymer, a fifth cloud of charged particles of the polymer, and a sixth cloud of charged particles of the polymer to the stents by sequentially rapidly
expanding a pressurized solution through the first orifice, wherein the particles have an opposite charge than the charge of the stent, wherein a third polymer layer of the polymer particles is formed on the stent by electrostatic deposition, and
sintering the third polymer layer at >40°C, at 1.03 MPa (150 psi) pressurization, and with gaseous 1,1,1,2,3,3-hexafluoropropane, wherein the crystalline form sirolimus particles in the first agent layer and second agent layer remain in crystalline form throughout all steps in the method.

### DETAILED DESCRIPTION

It is desirable to have a drug-eluting stent with minimal physical, chemical and therapeutic legacy in the vessel after a proscribed period of time. This period of time is based on the effective healing of the vessel after opening the blockage by PCI/stenting (currently believed by leading clinicians to be 6-18 months).

It is also desirable to have drug-eluting stents of minimal cross-sectional thickness for (a) flexibility of deployment (b) access to small vessels and/or tortuous lesions (c) minimized intrusion into the vessel wall and blood.

Disclosed herein is a stent; and a coating on the stent; wherein the coating comprises at least one polymer and a macrolide immunosuppressive (limus) drug, wherein at least a portion of the macrolide immunosuppressive (limus) drug is in crystalline form; wherein an evaluation of the device following implantation determines that the majority of the proliferative response depicted by the magnitude of neointimal proliferation and strut coverage occurs in the first 28 days after implantation.

In some embodiments, an evaluation of the device following implantation determines that after the first 28 days following implantation, no statistically significant changes occur in the proportion of strut coverage and amount of neointimal hyperplasia at 90 and 180 days. In some embodiments, an evaluation of the device following implantation determines that substantially all post-procedure malapposition resolves by 28-day follow-up. In some embodiments, the evaluation is performed by OCT analysis. In some embodiments, an evaluation of the device following implantation showing a satisfactory healing response to the implantation of the device by histologically demonstrating low inflammation scores and complete endothelial coverage at 180 days in combination with the neointimal maturation at 28 days following implantation by OCT analysis.

Provided herein is a method comprising providing a coated stent comprising a stent; and a coating on the stent; wherein the method is defined by the claims.

In some embodiments, the method comprises determining that, after the first 28 days following implantation, no statistically significant changes occur in the proportion of strut coverage and amount of neointimal hyperplasia at 90 and 180 days. In some embodiments, the method comprises determining that substantially all post-procedure malapposition resolves by 28-day follow-up. In some embodiments, the method comprises determining that there is neointimal maturation 28 days following implantation. In some embodiments, the determining step is performed by OCT analysis. In some embodiments, the method comprises showing a satisfactory healing response to the implantation of the device by histologically demonstrating low inflammation scores and complete endothelial coverage at 180 days in combination with the neointimal maturation at 28 days following implantation by OCT analysis.

Disclosed herein is a device comprising a stent; and a coating on the stent; wherein the coating comprises at least one polymer and a macrolide immunosuppressive (limus) drug, wherein at least a portion of the macrolide immunosuppressive (limus) drug is in crystalline form; wherein the coating is cleared from the stent in about 45 to 60 days following implantation of the device in vivo, leaving a bare metal stent.

Provided herein is a device comprising a stent; and a coating on the stent; wherein the coating comprises at least one polymer and a macrolide immunosuppressive (limus) drug, wherein at least a portion of the macrolide immunosuppressive (limus) drug is in crystalline form; and wherein the polymer is fully absorbed by the tissue in at most 90 days following implantation of the device in vivo, leaving a bare metal stent.

In certain embodiments, clearance of the coating from the stent is shown by measuring the amount of drug on the stent. In certain embodiments, clearance of the coating from the stent occurs when at least one of : over 52% of the drug is no longer associated with the stent, at least 75% of the drug is no longer associated with the stent, at least 80% of the drug is no longer associated with the stent, at least 90% of the drug is no longer associated with the stent, at least 95% of the drug is no longer associated with the stent, and at least 97% of the drug is no longer associated with the stent.

In certain embodiments, the drug loading is from about 9 µg per unit stent length to about 12 µg per unit stent length. In certain embodiments, the drug loading is from 9 µg per unit stent length to 12 µg per unit stent length. In certain embodiments, the drug loading target ranges from about 75 µg to about 300 µg. In certain embodiments, drug loading target ranges from about 83 µg to about 280 µg. In certain embodiments, the drug loading target ranges from 75 µg to 300 µg. In certain embodiments, drug loading target ranges from 83 µg to 280 µg. In certain embodiments, the polymer is fully absorbed by the vessel by at most 90 days.

In certain embodiments, full absorption is when there is at least 75% absorption of the polymer by the tissue surrounding the stent, at least 80% absorption of the polymer by the tissue surrounding the stent, at least 90% absorption of the polymer by the tissue surrounding the stent, at least 95% absorption of the polymer by the tissue surrounding the stent, or 100% absorption of the polymer by the tissue surrounding the stent. In certain embodiments, full absorption is when there is no evidence of polymer in the tissue surrounding the stent after 90 days following implantation.

In certain embodiments, the coated stent is lubricious. In certain embodiments, the coated stent is hydriphilic. In certain embodiments, the stent is thin. In certain embodiments, struts of the stent are about 64 microns on average. In certain embodiments, imaging with OCT demonstrates thin, homogenous coverage of the stent with tissue 4 months after implantation with the device. In certain embodiments, imaging with OCT demonstrates >90% strut coverage with tissue 4 months after implantation with the device. In certain embodiments, imaging with OCT demonstrates >80% strut coverage with tissue 4 months after implantation with the device. In certain embodiments, imaging with OCT demonstrates no stent strut malapposition 4 months after implantation with the device. In certain embodiments, imaging with OCT demonstrates no stent strut malapposition 6 months after implantation with the device. In certain embodiments, imaging with OCT demonstrates no stent strut malapposition 8 months after implantation with the device. In certain embodiments, imaging with OCT demonstrates a low rate of stent strut malapposition 4 months after implantation with the device in a population of subjects comprising at least 5 subjects. In certain embodiments, imaging with OCT demonstrates a low rate of stent strut malapposition 6 months after implantation with the device in a population of subjects comprising at least 5 subjects. In certain embodiments, imaging with OCT demonstrates a low rate of stent strut malapposition 8 months after implantation with the device in a population of subjects comprising at least 5 subjects.

In certain embodiments, there is minimal neointimal hyperplasia 4 months after implantation with the device. In certain embodiments, there is neointimal obstruction of no more than about 5.2% on average. In certain embodiments, there is minimal neointimal hyperplasia 6 months after implantation with the device. In certain embodiments, there is minimal neointimal hyperplasia 8 months after implantation with the device.

In certain embodiments, occurrence of late stent thrombosis is reduced as compared to other drug eluting stents. In certain embodiments, there is no indication of binary restenosis at 4 months after implantation with the device. In certain embodiments, there is no indication of binary restenosis at 6 months after implantation with the device. In certain embodiments, there is no indication of binary restenosis at 8 months after implantation with the device.

In certain embodiments, there is minimal change in late stent loss between 4 and 8 months following implantation with the device. This shows sustained and effectively suppressed neointimal hyperplasia.

In certain embodiments, there is low neointimal hyperplasia by analysis of at least one of neointimal obstruction (%), neointimal volume index (mm^3/mm), and late area loss (mm^2) measured at 8 months following implantation with the device, as determined by IVUS.

In certain embodiments, the stent was coated using an RESS method. In certain embodiments, the RESS method uses a PDPDP sequence of steps to produce the coated stent. In certain embodiments, the PDPDP sequence of steps comprises Polymer single spray, sinter, Drug spray, Polymer double spray, sinter, Drug spray, Polymer triple spray, sinter. In certain embodiments, the PDPDP sequence of steps comprises a first Polymer spray, sinter, Drug spray, a second Polymer spray that is about twice as long as the first Polymer spray, sinter, Drug spray, third Polymer spray that is about three times as long as the first Polymer spray, sinter. In certain embodiments, the PDPDP sequence of steps comprises a first Polymer spray, sinter, Drug spray, a second Polymer spray that deposits about twice as much Polymer as the first Polymer spray, sinter, Drug spray, third Polymer spray deposits about three times as much Polymer as the first Polymer spray, sinter.

In certain embodiments, the Polymer comprises PLGA 50:50 having a number average molecular weight of about 15kD.

In certain embodiments, implantation of the device results in rapid, uniform neointimal coverage with no adverse vessel reaction at four months follow up, at least. In certain embodiments, implantation of the device results late lumen loss and percent (%) obstruction which show good inhibition of neointimal hyperplasia. In certain embodiments, implantation of the device results in in-stent late lumen loss at 8 months of about 0.09mm, the percent neointimal obstruction at 8 months of about 10.9%, and there are no incidences of binary restenosis or revascularizations. In certain embodiments, after 4 months of implantation of the device, no significant changes are observed in vessel volume index, plaque volume index, or lumen volume index as compared to just after implantation. In certain embodiments, neointimal obstruction at 4 months is minimal and there is no significant lumen encroachment.

In certain embodiments, a majority of the stented segment is covered with IVUS- detectable neointima as early as 4 months following implantation with the device. In certain embodiments, at least 50% of the stented segment is covered with IVUS- detectable neointima as early as 4 months following implantation with the device. In certain embodiments, at least 60% of the stented segment is covered with IVUS- detectable neointima as early as 4 months following implantation with the device. In certain embodiments, at least 70% of the stented segment is covered with IVUS- detectable neointima as early as 4 months following implantation with the device. In certain embodiments, at least 80% of the stented segment is covered with IVUS- detectable neointima as early as 4 months following implantation with the device.

In certain embodiments, OCT demonstrates good strut coverage at 4 months, 6 months and 8 months following implantation of the device. In certain embodiments, OCT demonstrates strut coverage of at least 80% of the struts on average at each of 4 months, 6 months and 8 months following implantation of the device.

In certain embodiments, the device comprises an improved safety profile as compared to drug eluting stents made by other methods. In certain embodiments, the methods comprise solvent based coating methods. In certain embodiments, substantially all of the drug is amorphous in form on the stent of the other drug eluting stents.

In certain embodiments, the device comprises a controlled, continuous, sustained release of drug over 6 months in-vivo, without an initial drug burst into the tissue surrounding the device or into the blood stream.

In certain embodiments, the device mitigates hypersensitivity, impaired healing, and abnormal vasomotor function as compared to coated stents having longer absorption times or durable polymers thereon.

In certain embodiments, the device reduces risks of DAPT non-compliance and/or interruption as compared to other drug eluting stents.

In certain embodiments, the device reduces or eliminate risks of permanent coating such as long term thrombosis risks.

In certain embodiments, complete strut coverage is shown as early as 1 month following implantation. In certain embodiments, low intimal hyperplasia is shown up to 180 days following implantation, at least. In certain embodiments, no evidence of late catch up is shown at 180 days following implantation, at least. In certain embodiments, no stent malapposition was detected through 90 days. In certain embodiments, there is no late acquired malapposition detected in the implanted device.

In certain embodiments, drug is at least one of: 50% crystalline, at least 75% crystalline, at least 90% crystalline. In certain embodiments, the drug comprises at least one polymorph of the possible polymorphs of the crystalline structures of the drug.

In certain embodiments, the polymer comprises a bioabsorbable polymer. In certain embodiments, the polymer comprises PLGA. In certain embodiments, the polymer comprises PLGA with a ratio of about 40:60 to about 60:40. In certain embodiments, the polymer comprises PLGA with a ratio of about 40:60 to about 60:40 and further comprises PLGA with a ratio of about 60:40 to about 90:10. In certain embodiments, the polymer comprises PLGA having a weight average molecular weight of about 10kD and wherein the coating further comprises PLGA having a weight average molecular weight of about 19kD. In certain embodiments, the polymer is selected from the group: PLGA, a copolymer comprising PLGA (i.e. a PLGA copolymer), a PLGA copolymer with a ratio of about 40:60 to about 60:40, a PLGA copolymer with a ratio of about 70:30 to about 90:10, a PLGA copolymer having a weight average molecular weight of about 10kD, a PLGA copolymer having a weight average molecular weight of about 19kD, PGA poly(glycolide), LPLA poly(l-lactide), DLPLA poly(dl-lactide), PCL poly(e-caprolactone) PDO, poly(dioxolane) PGA-TMC, 85/15 DLPLG p(dl-lactide-co-glycolide), 75/25 DLPL, 65/35 DLPLG, 50/50 DLPLG, TMC poly(trimethylcarbonate), poly(anhydrides) such as p(CPP:SA) poly(1,3-bis-p-(carboxyphenoxy)propane-co-sebacic acid), and a combination thereof.

In certain embodiments, the stent comprises a cobalt-chromium alloy. In certain embodiments, the stent is formed from a material comprising the following percentages by weight: about 0.05 to about 0.15 C, about 1.00 to about 2.00 Mn, about 0.04 Si, about 0.03 P, about 0.3 S, about 19.0 to about 21.0 Cr, about 9.0 to about 11.0 Ni, about 14.0 to about 16.00 W, about 3.0 Fe, and Bal. Co. In certain embodiments, the stent is formed from a material comprising at most the following percentages by weight: about 0.025 C, about 0.15 Mn, about 0.15 Si, about 0.015 P, about 0.01 S, about 19.0 to about 21.0 Cr, about 33 to about37 Ni, about 9.0 to about 10.5 Mo, about 1.0 Fe, about 1.0 Ti, and Bal. Co. In certain embodiments, the stent is formed from a material comprising a platinum chromium alloy.

In certain embodiments, the stent has a thickness of from about 50% to about 90% of a total thickness of the device. In certain embodiments, the coating has a total thickness of from about 5 µm to about 50 µm.

The device of claim 1 or 2, wherein the device has an active agent content of from about 5 µg to about 500 µg. In certain embodiments, the device has an active agent content of from about 100 µg to about 160 µg.

The macrolide immunosuppressive drug is sirolimus.

Provided herein is a method comprising providing a coated stent comprising a stent; and a coating on the stent; wherein the coating comprises at least one polymer and at least one macrolide immunosuppressive (limus) drug, wherein at least a portion of the macrolide immunosuppressive (limus) drug is in crystalline form; and wherein the coating is cleared from the stent in about 45 to 60 days following implantation of the device in vivo, leaving a bare metal stent.

Provided herein is a method comprising providing a coated stent comprising a stent; and a coating on the stent; wherein the coating comprises at least one polymer and at least one macrolide immunosuppressive (limus) drug, wherein at least a portion of the macrolide immunosuppressive (limus) drug is in crystalline form; and wherein the polymer is fully absorbed by the tissue in at most 90 days following implantation of the device in vivo, leaving a bare metal stent.

In some embodiments, the drug is present in the vessel at about 90 days following implantation, at about 180 days following implantation, and/or at about 365 days following implantation. In some embodiments, the drug is present in the vessel at 90 days following implantation. In some embodiments, the drug is present in the vessel at 180 days following implantation. In some embodiments, the drug is present in the vessel at 365 days following implantation.

Provided herein is a method of coating a stent comprising: mounting a stent on a holder in a coating chamber that imparts a charge to the stent, providing a first cloud of charged particles of polymer to the stents by rapidly expanding a pressurized solution of the polymer in densified 1,1,1,2,3,3-hexafluoropropane through a first orifice, wherein the polymer comprises PLGA, wherein a first polymer layer of the polymer particles is formed on the stent by electrostatic deposition, sintering the first polymer layer at >40 C in ambient pressure, providing a first cloud of charged sirolimus particles to the stents having an opposite charge than the charge of the stent by pulsing sirolimus particles into the chamber using a propellant in order to deposit a first agent layer on the stent, wherein at least a portion of the sirolimus particles is in crystalline form, providing a second cloud of charged particles of the polymer and a third cloud of charged particles of the polymer to the stents by sequentially rapidly expanding the pressurized solution through the first orifice, wherein the particles have an opposite charge than the charge of the stent, wherein a second polymer layer of the polymer particles is formed on the stent by electrostatic deposition, sintering the second polymer layer at >40 C in ambient pressure, providing a second cloud of charged sirolimus particles to the stents having an opposite charge than the charge of the stent by pulsing the sirolimus particles into the chamber using a propellant in order to deposit a second agent layer on the stent, wherein at least a portion of the sirolimus particles is in crystalline form, providing a fourth cloud of charged particles of the polymer, a fifth cloud of charged particles of the polymer, and a sixth cloud of charged particles of the polymer to the stents by sequentially rapidly expanding a pressurized solution through the first orifice, wherein the particles have an opposite charge than the charge of the stent, wherein a third polymer layer of the polymer particles is formed on the stent by electrostatic deposition, and sintering the third polymer layer at >40 C 1.03 MPa (150 psi) pressurization with gaseous 1,1,1,2,3,3-hexafluoropropane, wherein the crystalline form sirolimus particles in the first agent layer and second agent layer remain in crystalline form throughout all steps in the method. In some embodiments the particles have an opposite charge than the charge of the stent. In some embodiments the sintering is performed at about 100C, or at 100C.

In some embodiments, the stent on the holder is orbiting through any of the first, second, third, fourth, fifth, or sixth clouds of charged polymer particles, or through any of the first or second clouds of charged sirolimus particles.

In some embodiments, the first orifice is heated sufficiently to overcome Jould-Thompson cooling. In some embodiments, the first orifice is heated sufficiently to ensure that the compressed gas is fully vaporized on expansion from the orifice.

In some embodiments, the concentration of the solution is any of 2 w/v% (weight or mass of polymer per total volume), 4 w/v%, about 2 w/v%, about 4 w/v%, about 2 w/v% to about 4 w/v%, 2 w/v% to 4 w/v%, 2 w/v% +/- 0.5 w/v%, 2 w/v% +/- 0.25 w/v%, 2 w/v% +/- 0.1 w/v%, 4 w/v% +/- 0.5 w/v%, 4 w/v% +/- 0.25 w/v%, 4 w/v% +/- 0.1 w/v%, at least 1 w/v%, at least 1.5 w/v%, at least 2 w/v%, at least 3 w/v%, at least 4 w/v%, at most 4 w/v%, at most 5 w/v%, at most 6 w/v%, at most 7 w/v%, at most 8 w/v%, at most 9 w/v%, at most 10 w/v%, at most 11 w/v%, at most 12 w/v%, at most 13 w/v%, at most 14 w/v%, or at most 15 w/v%.

In some embodiments, the flow rate is controlled and fixed using an automated syringe pump.

In some embodiments, the charge of the polymer or active agent particles is oppositely polarized as compared to the stent and comprises a potential of any of ±1.0 kV, ±1.2 kV, ±1.3 kV, ±1.4 kV, ±1.5 kV, ±1.6 kV, ±1.7 kV, ±1.8 kV, ±1.9 kV, ±2 kV, ±3 kV, ±3.5 kV, ±4 kV, ±5 kV, from ±1.0 kV to ±2.0 kV, from ±1.2 kV to ±1.8 kV, from ±1.4 kV to ±1.6 kV, from ±0.5 kV to ±5 kV, or about ±1.5 kV.

In some embodiments, the sirolimus particles have been micronized prior to introduction into the chamber. In some embodiments, the sirolimus particles comprise a particle distribution such that at least 99% by volume of the sirolimus particles are less than 10 microns with the distribution centered at 2.75 +/- 0.5 microns. In some embodiments, the sirolimus particles comprise a particle distribution such that 80%, 85%, 90%, 95%, 99%, at least 50%, at least 75%, at least 80%, at least 85%, at least 90%, at least 95%, at least 99%, at least about 50%, at least about 75%, at least about 80%, at least about 85%, at least about 90%, at least about 95%, or at least about 99% by volume of the particles are less than 10 microns. In some embodiments, the sirolimus particles comprise a particle distribution such that at least 50% by volume of the particles are less than 3 microns, less than 5 microns, less than 7.5 microns, less than 10 microns, less than 20 microns, less than 25 microns, less than 30 microns, less than 40 microns, less than 50 microns, less than 75 microns, less than about 10 microns, less than about 15 microns, or less than about 7.5 microns. In some embodiments, the sirolimus particles have a distribution centered at 1.0 +/-0.5 microns, 1.25 +/- 0.5 microns, 1.5 +/- 0.5 microns, 1.75 +/- 0.5 microns, 2.0 +/- 0.5 microns, 2.25 +/-0.5 microns, 2.5 +/- 0.5 microns, 2.75 +/- 0.5 microns, 3.0 +/- 0.5 microns, 3.25 +/- 0.5 microns, 3.5 +/-0.5 microns, 3.75 +/- 0.5 microns, 4.0 +/- 0.5 microns, 4.25 +/- 0.5 microns, 4.5 +/- 0.5 microns, 4.75 +/-0.5 microns, 5 +/- 0.5 microns, 5.5 +/- 0.5 microns, 6 +/- 0.5 microns, 6.5 +/- 0.5 microns, 7 +/- 0.5 microns, 7.5 +/- 0.5 microns, 8 +/- 0.5 microns, 8.5 +/- 0.5 microns, 9 +/- 0.5 microns, 10 +/- 0.5 microns, 15 +/- 0.5 microns, 20 +/- 0.5 microns, 25 +/- 0.5 microns, 30 +/- 0.5 microns, 35 +/- 0.5 microns, 40 +/- 0.5 microns, 45 +/- 0.5 microns, 50 +/- 0.5 microns, about 1.0 microns, about 1.5 microns, about 2.0 microns, about 2.5 microns, about 2.75 microns, about 3.0 microns, about 3.5 microns, about 4.0 microns, about 4.5 microns, about 5 microns, about 6 microns, about 7 microns, about 8 microns, about 9 microns, about 10 microns, about 15 microns, about 20 microns, about 25 microns, about 30 microns, about 35 microns, about 40 microns, about 45 microns, or about 50 microns.

In some embodiments, the propellant comprises a noble gas. In some embodiments, the noble gas comprises argon, nitrogen or helium. In some embodiments, the propellant is pressurized to at least 0.34 MPa (50 psi), at least 0.52 MPa (75 psi), at least 0.69 MPa (100 psi), at least 1.03 MPa (150 psi), at least 1.38 MPa (200 psi), at least 1.72 MPa (250 psi), at least 2.07 MPa (300 psi), about 0.34 MPa (50 psi), about 0.52 MPa (75 psi), about 0.69 MPa (100 psi), about 1.03 MPa (150 psi), about 1.38 MPa (200 psi), about 1.72 MPa (250 psi), about 2.07 MPa (300 psi), about 2.41 MPa (350 psi), about 2.76 MPa (400 psi), about 3.10 MPa (450 psi), about 3.45 MPa (500 psi), about 3.79 MPa (550 psi), about 4.14 MPa (600 psi), 0.34 MPa (50 psi)to 3.45 MPa (500 psi), 1.38 MPa (200 psi) to 2.76 MPa (400 psi), 1.72 MPa (250 psi) to 2.41 MPa (350 psi), 0.34 MPa (50 psi), 0.52 MPa (75 psi), 0.69 MPa (100 psi), 1.03 MPa (150 psi), 1.38 MPa (200 psi), 1.72 MPa (250 psi), 2.07 MPa (300 psi), 2.41 MPa (350 psi), 2.76 MPa (400 psi), 3.10 MPa (450 psi), 3.45 MPa (500 psi), 3.79 MPa (550 psi), or 4.14 MPa (600 psi).

Provided herein is a device comprising a stent and a coating on the stent wherein the coating comprises PLGA and crystalline sirolimus and wherein the stent is made by any one of the methods described herein.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 depicts Bioabsorbability testing of 50:50 PLGA-ester end group (weight average MW ∼ 19kD) polymer coating formulations on stents by determination of pH Changes with Polymer Film Degradation in 20% Ethanol/Phosphate Buffered Saline as set forth in Example 3 described herein.
Figure 2 depicts Bioabsorbability testing of 50:50 PLGA-carboxylate end group (weight average MW ∼ 10kD) PLGA polymer coating formulations on stents by determination of pH Changes with Polymer Film Degradation in 20% Ethanol/Phosphate Buffered Saline as set forth in Example 3 described herein.
Figure 3 depicts Bioabsorbability testing of 85:15 (85% lactic acid, 15% glycolic acid) PLGA polymer coating formulations on stents by determination of pH Changes with Polymer Film Degradation in 20% Ethanol/Phosphate Buffered Saline as set forth in Example 3 described herein.
Figure 4 depicts Bioabsorbability testing of various PLGA polymer coating film formulations by determination of pH Changes with Polymer Film Degradation in 20% Ethanol/Phosphate Buffered Saline as set forth in Example 3 described herein.
Figure 5 depicts Rapamycin Elution Profile of coated stents (PLGA/Rapamycin coatings) where the elution profile was determined by a static elution media of 5% EtOH/water, pH 7.4, 37°C via UV-Vis test method as described in Example 11b of coated stents described therein.
Figure 6 depicts Rapamycin Elution Profile of coated stents (PLGA/Rapamycin coatings) where the elution profile was determined by static elution media of 5% EtOH/water, pH 7.4, 37°C via a UV-Vis test method as described in Example 11b of coated stents described therein.
Figure 7 depicts Rapamycin Elution Rates of coated stents (PLGA/Rapamycin coatings) where the static elution profile was compared with agitated elution profile by an elution media of 5% EtOH/water, pH 7.4, 37°C via a UV-Vis test method a UV-Vis test method as described in Example 11b of coated stents described therein.
Figure 8 depicts Rapamycin Elution Profile of coated stents (PLGA/Rapamycin coatings) where the elution profile by 5% EtOH/water, pH 7.4, 37°C elution buffer was compare with the elution profile using phosphate buffer saline pH 7.4, 37°C; both profiles were determined by a UV-Vis test method as described in Example 11b of coated stents described therein.
Figure 9 depicts Rapamycin Elution Profile of coated stents (PLGA/Rapamycin coatings) where the elution profile was determined by a 20% EtOH/phosphate buffered saline, pH 7.4, 37°C elution buffer and a HPLC test method as described in Example 11c described therein, wherein the elution time (x-axis) is expressed linearly.
Figure 10 depicts Rapamycin Elution Profile of coated stents (PLGA/Rapamycin coatings) where the elution profile was determined by a 20% EtOH/phosphate buffered saline, pH 7.4, 37°C elution buffer and a HPLC test method as described in Example 11c of described therein, , wherein the elution time (x-axis) is expressed in logarithmic scale (i.e., log(time)).
Figure 11 depicts Vessel wall tissue showing various elements near the lumen.
Figure 12 depicts Low-magnification cross-sections of porcine coronary artery stent implants (AS1, AS2 and Bare-metal stent control) at 28 days post-implantation as described in Example 25.
Figure 13 depicts Low-magnification cross-sections of porcine coronary artery stent implants (AS1, AS2 and Bare-metal stent control) at 90 days post-implantation as described in Example 25.
Figure 14 depicts Low-magnification cross-sections of porcine coronary artery stent implants depicting AS1 and AS2 drug depots as described in Example 25.
Figure 15 depicts Low-magnification cross-sections of porcine coronary artery AS1 stent implants at 90 days depicting drug depots as described in Example 25.
Figure 16 depicts Arterial Tissue Concentrations (y-axis) versus time (x-axis) for AS1 and AS2 stents following testing as described in Example 25.
Figure 17 depicts Fractional Sirolimus Release (y-axis) versus time (x-axis) in Arterial Tissue for AS1 and AS2 Stents following testing as described in Example 25.
Figure 18 depicts an elution profile of stents coated according to methods described in Example 26, and having coatings described therein where the test group (upper line at day 2) has an additional sintering step performed between the 2d and third polymer application to the stent in the 3d polymer layer.
Figure 19 depicts an elution profile of stents coated according to methods described in Example 27, and having coatings described therein where the test group (bottom line) has an additional 15 second spray after final sinter step of normal process (control) followed by a sinter step.
Figure 20 depicts an elution profile of stents coated according to methods described in Example 28, and having coatings described therein where the test group (bottom line) has less polymer in all powder coats of final layer (1 second less for each of 3 sprays), then sintering, and then an additional polymer spray (3 seconds) and sintering.
Figure 21 depicts an elution profile of stents coated according to methods described in Example 30, and having coatings described therein wherein the figure shows the average (or mean) percent elution of all the tested stents at each time point (middle line), expressed as % rapamycin total mass eluted (y-axis) at each time point (x-axis).
Figure 22 shows the neointimal thickness score and standard deviation recorded at each of 30 days and 90 days in both a single and overlapping (OLP) Sirolimus DES and Vision BMS stent implantation in a porcine model as described in Example 31.
Figure 23 shows the average inflammation score and standard deviation recorded at each of 30 days and 90 days in both a single and overlapping (OLP) Sirolimus DES and Vision BMS stent implantation in a porcine model as described in Example 31.
Figure 24 shows release of sirolimus from the Sirolimus DES appeared slower over the initial 14 days following implant compared to release from 14 to 45 days after implant as described in Example 34.
Figure 25 depicts the incremental Stent Sirolimus Loss Rate from 1 to 90 Days as described in Example 34.
Figure 26 shows stented artery sirolimus concentration (in ng/mg of Tissue within Stented Segments) from Example 34.
Figure 27 shows in graphical form the fractional residual drug remaining on the stent at various time points (top line at time 0) from Example 34 using the scale on the left y-axis, and the measured arterial drug concentration (bottom line at time 0) measured at various time points using the scale on the right y-axis.
Figure 28 shows a SEM visualization of a 5 micron segment of coating on a stent strut wherein the coated stent is prepared as described herein and wherein the pharmaceutical agent is at least in part crystalline within the polymer of the coating.
Figure 29 shows the Patient level in-stent LLL by follow-up group, indicating no binary restenosis and having a linear regression indicating minimal change in LLL between 4 and 8 months as tested in the study of Example 36.
Figure 30 shows a target artery and lesion of a single patient from the study of Example 36 viewed by IVUS at 8 months follow up.
Figure 31 shows a histogram of Neointimal obstruction of devices of Example 36 at 4 months follow up as tested and analyzed using IVUS.
Figure 32 shows Vessel Response in Example 36, which shows Vessel Volume Index, Plaque Volume Index, and Lumen Volume Index at baseline (at implantation) and at 4 months follow up.
Figure 33 shows the target artery and lesion of a single patient viewed under fluoroscopy prior to implantation of the device from the study of Example 36, just after implantation, and at 8 months follow up.
Figure 34 shows the average percent stenosis analyzed over the timeframe of the study as described in Example 37.
Figure 35 shows the percent area stenosis (percent area occlusion) of the coated device and uncoated device over the course of the study described in Example 3 and shows low neointimal hyperplasia and no late catch-up.
Figure 36 shows example target arteries having an embodiment coated stent implanted therein at each of 30 days, 90 days and 180 days after implantation of the device of Example 37.
Figures 37a and 37b show receiver-operating characteristic curves showing sensitivity and specificity of normalized optical density to detect fibrin in control (Fig. 37a) and treatment (Fig. 37b) groups, respectively.
Figure 38 depicts an embodiment of micronized sirolimus used in a spray coating process described in Example 39, at least.

### Definitions

As used in the present specification, the following words and phrases are generally intended to have the meanings as set forth below, except to the extent that the context in which they are used indicates otherwise.

"Substrate" as used herein, refers to any surface upon which it is desirable to deposit a coating comprising a polymer and a pharmaceutical or biological agent, wherein the coating process does not substantially modify the morphology of the pharmaceutical agent or the activity of the biological agent. Biomedical implants are of particular interest for the present invention; however the present invention is not intended to be restricted to this class of substrates. Those of skill in the art will appreciate alternate substrates that could benefit from the coating process described herein, such as pharmaceutical tablet cores, as part of an assay apparatus or as components in a diagnostic kit (e.g. a test strip).

"Biomedical implant" as used herein refers to any implant for insertion into the body of a human or animal subject, including stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, etc.

The implants may be formed from any suitable material, including polymers (including stable or inert polymers, organic polymers, organic-inorganic copolymers, inorganic polymers, and biodegradable polymers), metals, metal alloys, inorganic materials such as silicon, and composites thereof, including layered structures with a core of one material and one or more coatings of a different material. Substrates made of a conducting material facilitate electrostatic capture. However, the invention contemplates the use of electrostatic capture, as described below, in conjunction with substrate having low conductivity or which are non-conductive. To enhance electrostatic capture when a non-conductive substrate is employed, the substrate is processed for example while maintaining a strong electrical field in the vicinity of the substrate.

Subjects into which biomedical implants may be applied or inserted include both human subjects (including male and female subjects and infant, juvenile, adolescent, adult and geriatric subjects) as well as animal subjects (including pig, rabbit, mouse, dog, cat, horse, monkey, etc.) for veterinary purposes and/or medical research.

In a preferred embodiment the biomedical implant is an expandable intraluminal vascular graft or stent that can be expanded within a blood vessel by an angioplasty balloon associated with a catheter to dilate and expand the lumen of a blood vessel, such as described in US Patent No. 4,733,665 to Palmaz.

"Pharmaceutical agent" as used herein refers to any of a variety of drugs or pharmaceutical compounds that can be used as active agents to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the pharmaceutical agents for use in the invention may also comprise two or more drugs or pharmaceutical compounds. Pharmaceutical agents, include but are not limited to antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, antigens, beta-receptor blockers, non-steroidal antiinflammatory drugs [NSAIDs], cardiac glycosides, acetylsalicylic acid, virustatics, aclarubicin, acyclovir, cisplatin, actinomycin, alpha- and beta-sympatomimetics, (dimeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytabarine, dapiprazole, desogestrel, desonide, dihydralazine, diltiazem, ergot alkaloids, dimenhydrinate, dimethyl sulphoxide, dimethicone, domperidone and domperidan derivatives, dopamine, doxazosin, doxorubicin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenytoin, riluzoles, risedronate, sildenafil, topiramate, macrolide antibiotics, oestrogen and oestrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tetracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobuterol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorelbine, vinpocetine, viquidil, warfarin, xantinol nicotinate, xipamide, zafirlukast, zalcitabine, zidovudine, zolmitriptan, zolpidem, zoplicone, zotipine. See, e.g., U.S. Patent No. 6,897,205; see also U.S. Patent No. 6,838,528; U.S. Patent No. 6,497,729.

Examples of therapeutic agents employed in conjunction with the invention include, rapamycin, 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus).

As used herein, the pharmaceutical agent sirolimus may also and/or alterantively be called rapamycin, or vice versa, unless otherwise noted with regard to a particular term for nonlimiting example, 42-Epi-(tetrazolyl)rapamycin is tacrolimus as noted herein.

The pharmaceutical agents may, if desired, also be used in the form of their pharmaceutically acceptable salts or derivatives (meaning salts which retain the biological effectiveness and properties of the compounds for use in this invention and which are not biologically or otherwise undesirable), and in the case of chiral active ingredients it is possible to employ both optically active isomers and racemates or mixtures of diastereoisomers. As well, the pharmaceutical agent may include a prodrug, a hydrate, an ester, a derivative or analogs of a compound or molecule.

In some embodiments, the pharmaceutical agent is, at least in part, crystalline. As used herein, the term crystalline may include any number of the possible polymorphs of the crystalline form of the pharmaceutical agent, including for non-limiting example a single polymorph of the pharmaceutical agent, or a plurality of polymorphs of the pharmaceutical agent. The crystalline pharmaceutical agent (which may include a semi-crystalline form of the pharmaceutical agent, depending on the embodiment) may comprise a single polymorph of the possible polymorphs of the pharmaceutical agent. The crystalline pharmaceutical agent (which may include a semi-crystalline form of the pharmaceutical agent, depending on the embodiment) may comprise a plurality of polymorphs of the possible polymorphs of the crystalline pharmaceutical agent. The polymorph, in some embodiments, is a packing polymorph, which exists as a result of difference in crystal packing as compared to another polymorph of the same crystalline pharmaceutical agent. The polymorph, in some embodiments, is a conformational polymorph, which is conformer of another polymorph of the same crystalline pharmaceutical agent. The polymorph, in some embodiments, is a pseudopolymorph. The polymorph, in some embodiments, is any type of polymorph that is, the type of polymorph is not limited to only a packing polymorph, conformational polymorph, and/or a pseudopolymorph. When referring to a particular pharmaceutical agent herein which is at least in part crystalline, it is understood that any of the possible polymorphs of the pharmaceutical agent are contemplated.

A "pharmaceutically acceptable salt" may be prepared for any pharmaceutical agent having a functionality capable of forming a salt, for example an acid or base functionality. Pharmaceutically acceptable salts may be derived from organic or inorganic acids and bases. The term "pharmaceutically-acceptable salts" in these instances refers to the relatively non-toxic, inorganic and organic base addition salts of the pharmaceutical agents.

"Prodrugs" are derivative compounds derivatized by the addition of a group that endows greater solubility to the compound desired to be delivered. Once in the body, the prodrug is typically acted upon by an enzyme, e.g., an esterase, amidase, or phosphatase, to generate the active compound.

"Stability" as used herein in refers to the stability of the drug in a polymer coating deposited on a substrate in its final product form (e.g., stability of the drug in a coated stent). The term stability will define 5% or less degradation of the drug in the final product form.

"Active biological agent" as used herein refers to a substance, originally produced by living organisms, that can be used to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the active biological agents for use in the invention may also comprise two or more active biological agents or an active biological agent combined with a pharmaceutical agent, a stabilizing agent or chemical or biological entity. Although the active biological agent may have been originally produced by living organisms, those for use in the present invention may also have been synthetically prepared, or by methods combining biological isolation and synthetic modification. By way of a non-limiting example, a nucleic acid could be isolated form from a biological source, or prepared by traditional techniques, known to those skilled in the art of nucleic acid synthesis. Furthermore, the nucleic acid may be further modified to contain non-naturally occurring moieties. Non-limiting examples of active biological agents include peptides, proteins, enzymes, glycoproteins, nucleic acids (including deoxyribonucleotide or ribonucleotide polymers in either single or double stranded form, and unless otherwise limited, encompasses known analogues of natural nucleotides that hybridize to nucleic acids in a manner similar to naturally occurring nucleotides), antisense nucleic acids, fatty acids, antimicrobials, vitamins, hormones, steroids, lipids, polysaccharides, carbohydrates. They further include, but are not limited to, antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals and chemotherapeutic agents. Preferably, the active biological agent is a peptide, protein or enzyme, including derivatives and analogs of natural peptides, proteins and enzymes. The active biological agent may also be a hormone, gene therapies, RNA, siRNA, and/or cellular therapies (for non-limiting example, stem cells or T-cells).

"Active agent" as used herein refers to any pharmaceutical agent or active biological agent as described herein.

"Activity" as used herein refers to the ability of a pharmaceutical or active biological agent to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). Thus the activity of a pharmaceutical or active biological agent should be of therapeutic or prophylactic value.

"Secondary, tertiary and quaternary structure " as used herein are defined as follows. The active biological agents for use in the present invention will typically possess some degree of secondary, tertiary and/or quaternary structure, upon which the activity of the agent depends. As an illustrative, non-limiting example, proteins possess secondary, tertiary and quaternary structure. Secondary structure refers to the spatial arrangement of amino acid residues that are near one another in the linear sequence. The α-helix and the β-strand are elements of secondary structure. Tertiary structure refers to the spatial arrangement of amino acid residues that are far apart in the linear sequence and to the pattern of disulfide bonds. Proteins containing more than one polypeptide chain exhibit an additional level of structural organization. Each polypeptide chain in such a protein is called a subunit. Quaternary structure refers to the spatial arrangement of subunits and the nature of their contacts. For example hemoglobin consists of two α and two β chains. It is well known that protein function arises from its conformation or three dimensional arrangement of atoms (a stretched out polypeptide chain is devoid of activity). Thus one aspect of the present invention is to manipulate active biological agents, while being careful to maintain their conformation, so as not to lose their therapeutic activity.

"Polymer" as used herein, refers to a series of repeating monomeric units that have been cross-linked or polymerized. According to the invention, a first polymer layer is formed from polymer particles, wherein the polymer comprises PLGA. It is possible that the polymers for use in the invention may also comprise two, three, four or more different polymers. In some embodiments, of the invention only one polymer is used. In some preferred embodiments a combination of two polymers are used. Combinations of polymers can be in varying ratios, to provide coatings with differing properties. Those of skill in the art of polymer chemistry will be familiar with the different properties of polymeric compounds.

Additional polymers useful in the present invention include, for example, stable polymers, biostable polymers, durable polymers, inert polymers, organic polymers, organic-inorganic copolymers, inorganic polymers, bioabsorbable, bioresorbable, resorbable, degradable, and biodegradable polymers. These categories of polymers may, in some cases, be synonymous, and is some cases may also and/or alternatively overlap. Those of skill in the art of polymer chemistry will be familiar with the different properties of polymeric compounds.

In some embodiments, the active agent comprises a polymer. In some embodiments, the polymer comprises at least one of polyalkyl methacrylates, polyalkylene-co-vinyl acetates, polyalkylenes, polyurethanes, polyanhydrides, aliphatic
polycarbonates, polyhydroxyalkanoates, silicone containing polymers, polyalkyl siloxanes, aliphatic polyesters, polyglycolides, polylactides, polylactide-co-glycolides, poly(e-caprolactone)s, polytetrahalooalkylenes, polystyrenes, poly(phosphasones), copolymers thereof, and combinations thereof.

Examples of polymers that may be used in the present invention include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, aliphatic polyesters, polyurethanes, polystyrenes, copolymers, silicones, silicone containing polymers, polyalkyl siloxanes, polyorthoesters, polyanhydrides, copolymers of vinyl monomers, polycarbonates, polyethylenes, polypropytenes, polylactic acids, polylactides, polyglycolic acids, polyglycolides, polylactide-co-glycolides, polycaprolactones, poly(e-caprolactone)s, polyhydroxybutyrate valerates, polyacrylamides, polyethers, polyurethane dispersions, polyacrylates, acrylic latex dispersions, polyacrylic acid, polyalkyl methacrylates, polyalkylene-co-vinyl acetates, polyalkylenes, aliphatic polycarbonates polyhydroxyalkanoates, polytetrahalooalkylenes, poly(phosphasones), polytetrahalooalkylenes, poly(phosphasones), and mixtures, combinations, and copolymers thereof.

The polymers for use in the present invention may be natural or synthetic in origin, including gelatin, chitosan, dextrin, cyclodextrin, Poly(urethanes), Poly(siloxanes) or silicones, Poly(acrylates) such as [rho]oly(methyl methacrylate), poly(butyl methacrylate), and Poly(2-hydroxy ethyl methacrylate), Poly(vinyl alcohol) Poly(olefins) such as poly(ethylene), [rho]oly(isoprene), halogenated polymers such as Poly(tetrafluoroethylene) - and derivatives and copolymers such as those commonly sold as Teflon(R) products, Poly(vinylidine fluoride), Poly(vinyl acetate), Poly(vinyl pyrrolidone), Poly(acrylic acid), Polyacrylamide, Poly(ethylene- co-vinyl acetate), Poly(ethylene glycol), Poly(propylene glycol), Poly(methacrylic acid); etc.

Examples of additional polymers that may be used in the present invention include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, aliphatic polyesters, polyurethanes, polystyrenes, copolymers, silicones, silicone containing polymers, polyalkyl siloxanes, polyorthoesters, polyanhydrides, copolymers of vinyl monomers, polycarbonates, polyethylenes, polypropytenes, polylactic acids, polylactides, polyglycolic acids, polyglycolides, polycaprolactones, poly(e-caprolactone)s, polyhydroxybutyrate valerates, polyacrylamides, polyethers, polyurethane dispersions, polyacrylates, acrylic latex dispersions, polyacrylic acid, polyalkyl methacrylates, polyalkylene-co-vinyl acetates, polyalkylenes, aliphatic polycarbonates polyhydroxyalkanoates, polytetrahalooalkylenes, poly(phosphasones), polytetrahalooalkylenes, poly(phosphasones), and mixtures, combinations, and copolymers thereof.

The polymers for use in the present invention may be natural or synthetic in origin, including gelatin, chitosan, dextrin, cyclodextrin, Poly(urethanes), Poly(siloxanes) or silicones, Poly(acrylates) such as
[rho]oly(methyl methacrylate), poly(butyl methacrylate), and Poly(2-hydroxy ethyl methacrylate), Poly( vinyl alcohol) Poly(olefins) such as poly(ethylene), [rho]oly(isoprene), halogenated polymers such as Poly(tetrafluoroethylene) - and derivatives and copolymers such as those commonly sold as Teflon(R) products, Poly(vinylidine fluoride), Poly(vinyl acetate), Poly(vinyl pyrrolidone), Poly(acrylic acid), Polyacrylamide, Poly(ethylene-co-vinyl acetate), Poly(ethylene glycol), Poly(propylene glycol), Poly(methacrylic acid); etc.

Suitable additional polymers also include absorbable and/or resorbable polymers including the following, combinations, copolymers and derivatives of the following: Polylactides (PLA), Polyglycolides (PGA), Polyanhydrides, Polyorthoesters, Poly(N-(2- hydroxypropyl) methacrylamide), Poly(1-aspartamide), including the derivatives DLPLA - poly(dl-lactide); LPLA - poly(1-lactide); PDO - poly(dioxanone); PGA-TMC - poly(glycolide-co-trimethylene carbonate); PGA-LPLA - poly(1-lactide-co-glycolide); PGA-DLPLA - poly(dl-lactide-co-glycolide); LPLA-DLPLA - poly(1-lactide-co-dl-lactide); and PDO-PGA-TMC - poly(glycolide-co-trimethylene carbonate-co-dioxanone), and combinations thereof.

"Copolymer" as used herein refers to a polymer being composed of two or more different monomers. A copolymer may also and/or alternatively refer to random, block, graft, copolymers known to those of skill in the art.

"Biocompatible" as used herein, refers to any material that does not cause injury or death to the animal or induce an adverse reaction in an animal when placed in intimate contact with the animal's tissues. Adverse reactions include for example inflammation, infection, fibrotic tissue formation, cell death, or thrombosis. The terms "biocompatible " and "biocompatibility" when used herein are art-recognized and mean that the referent is neither itself toxic to a host (e.g., an animal or human), nor degrades (if it degrades) at a rate that produces byproducts (e.g., monomeric or oligomeric subunits or other byproducts) at toxic concentrations, causes inflammation or irritation, or induces an immune reaction in the host. It is not necessary that any subject composition have a purity of 100% to be deemed biocompatible. Hence, a subject composition may comprise 99%, 98%, 97%, 96%, 95%, 90% 85%, 80%, 75% or even less of biocompatible agents, e.g., including polymers and other materials and excipients described herein, and still be biocompatible.

To determine whether a polymer or other material is biocompatible, it may be necessary to conduct a toxicity analysis. Such assays are well known in the art. One example of such an assay may be performed with live carcinoma cells, such as GT3TKB tumor cells, in the following manner: the sample is degraded in 1 M NaOH at 37 degrees C. until complete degradation is observed. The solution is then neutralized with 1 M HCl. About 200 microliters of various concentrations of the degraded sample products are placed in 96-well tissue culture plates and seeded with human gastric carcinoma cells (GT3TKB) at 104/well density. The degraded sample products are incubated with the GT3TKB cells for 48 hours. The results of the assay may be plotted as % relative growth vs. concentration of degraded sample in the tissue-culture well. In addition, polymers and formulations for use in the present invention may
also be evaluated by well-known in vivo tests, such as subcutaneous implantations in rats to confirm that they do not cause significant levels of irritation or inflammation at the subcutaneous implantation sites.

The terms "bioabsorbable," "biodegradable," "bioerodible," and "bioresorbable," are art-recognized synonyms. These terms are used herein interchangeably. Bioabsorbable polymers typically differ from non-bioabsorbable polymers (i.e. durable polymers) in that the former may be absorbed (e.g.; degraded) during use. In certain embodiments, such use involves in vivo use, such as in vivo therapy, and in other certain embodiments, such use involves in vitro use. In general, degradation attributable to biodegradability involves the degradation of a bioabsorbable polymer into its component subunits, or digestion, e.g., by a biochemical process, of the polymer into smaller, non-polymeric subunits. In certain embodiments, biodegradation may occur by enzymatic mediation, degradation in the presence of water (hydrolysis)and/or other chemical species in the body, or both. The bioabsorbabilty of a polymer may be shown in-vitro as described herein or by methods known to one of skill in the art. An in-vitro test for bioabsorbability of a polymer does not require living cells or other biologic materials to show bioabsorption properties (e.g. degradation, digestion). Thus, resorbtion, resorption, absorption, absorbtion, erosion may also be used synonymously with the terms "bioabsorbable," "biodegradable," "bioerodible," and "bioresorbable." Mechanisms of degradation of a Mechanisms of degradation of a bioabsorbable polymer may include, but are not limited to, bulk degradation, surface erosion, and combinations thereof.

As used herein, the term "biodegradation" encompasses both general types of biodegradation. The degradation rate of a biodegradable polymer often depends in part on a variety of factors, including the chemical identity of the linkage responsible for any degradation, the molecular weight, crystallinity, biostability, and degree of cross-linking of such polymer, the physical characteristics (e.g., shape and size) of the implant, and the mode and location of administration. For example, the greater the molecular weight, the higher the degree of crystallinity, and/or the greater the biostability, the biodegradation of any bioabsorbable polymer is usually slower.

As used herein, the term "durable polymer" refers to a polymer that is not bioabsorbable (and/or is not bioerodable, and/or is not biodegradable, and/or is not bioresorbable) and is, thus biostable. In some embodiments, the device comprises a durable polymer. The polymer may include a cross-linked durable polymer. Example biocompatible durable polymers include, but are not limited to: polyester, aliphatic polyester, polyanhydride, polyethylene, polyorthoester, polyphosphazene, polyurethane, polycarbonate urethane, aliphatic polycarbonate, silicone, a silicone containing polymer, polyolefin, polyamide, polycaprolactam, polyamide, polyvinyl alcohol, acrylic polymer, acrylate, polystyrene, epoxy, polyethers, cellulosics, expanded polytetrafluoroethylene, phosphorylcholine, polyethyleneyerphthalate, polymethylmethavrylate, poly(ethylmethacrylate/n-butylmethacrylate), parylene C, polyethylene-co-vinyl acetate, polyalkyl methacrylates, polyalkylene-co-vinyl acetate, polyalkylene, polyalkyl siloxanes, polyhydroxyalkanoate, polyfluoroalkoxyphasphazine, poly(styrene-b-isobutylene-b-styrene), poly-butyl methacrylate, poly-byta-diene, and blends, combinations, homopolymers, condensation polymers, alternating, block, dendritic, crosslinked, and copolymers thereof. The polymer may include a thermoset material. The polymer may provide strength for the coated implantable medical device. The polymer may provide durability for the coated implantable medical device. The coatings and coating methods provided herein provide substantial protection from these by establishing a multi-layer coating which can be bioabsorbable or durable or a combination thereof, and which can both deliver active agents and provide elasticity and radial strength for the vessel in which it is delivered.

"Therapeutically desirable morphology" as used herein refers to the gross form and structure of the pharmaceutical agent, once deposited on the substrate, so as to provide for optimal conditions of ex vivo storage, in vivo preservation and/or in vivo release. Such optimal conditions may include, but are not limited to increased shelf life, increased in vivo stability, good biocompatibility, good bioavailability or modified release rates. Typically, for the present invention, the desired morphology of a pharmaceutical agent would be crystalline or semi-crystalline or amorphous, although this may vary widely depending on many factors including the nature of the pharmaceutical agent, the disease to be treated/prevented, the intended storage conditions for the substrate prior to use or the location within the body of any biomedical implant. Preferably at least 10%, 20%, 30%, 40%, 50%, 60%, 70%, 80%, 90% or 100% of the pharmaceutical agent is in crystalline or semi-crystalline form.

"Stabilizing agent" as used herein refers to any substance that maintains or enhances the stability of the biological agent. Ideally these stabilizing agents are classified as Generally Regarded As Safe (GRAS) materials by the US Food and Drug Administration (FDA). Examples of stabilizing agents include, but are not limited to carrier proteins, such as albumin, gelatin, metals or inorganic salts. Pharmaceutically acceptable excipient that may be present can further be found in the relevant literature, for example in the Handbook of Pharmaceutical Additives: An International Guide to More Than 6000 Products by Trade Name, Chemical, Function, and Manufacturer; Michael and Irene Ash (Eds.); Gower Publishing Ltd.; Aldershot, Hampshire, England, 1995.

"Compressed fluid" as used herein refers to a fluid of appreciable density (e.g., >0.2 g/cc) that is a gas at standard temperature and pressure. "Supercritical fluid", "near-critical fluid", "near-supercritical fluid", "critical fluid", "densified fluid" or "densified gas" as used herein refers to a compressed fluid under conditions wherein the temperature is at least 80% of the critical temperature of the fluid and the pressure is at least 50% of the critical pressure of the fluid, and/or a density of +50% of the critical density of the fluid.

Examples of substances that demonstrate supercritical or near critical behavior suitable for the present invention include, but are not limited to carbon dioxide, isobutylene, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfuoropropane, chloroform, trichloro-fluoromethane, dichloro-difluoromethane, dichloro-tetrafluoroethane) and mixtures thereof. Preferably, the supercritical fluid is hexafluoropropane (FC-236EA), or 1,1,1,2,3,3-hexafluoropropane. Preferably, the supercritical fluid is hexafluoropropane (FC-236EA), or 1,1,1,2,3,3-hexafluoropropane for use in PLGA polymer coatings.

"Sintering" as used herein refers to the process by which parts of the polymer or the entire polymer becomes continuous (e.g., formation of a continuous polymer film). As discussed below, the sintering process is controlled to produce a fully conformal continuous polymer (complete sintering) or to produce regions or domains of continuous coating while producing voids (discontinuities) in the polymer. As well, the sintering process is controlled such that some phase separation is obtained or maintained between polymer different polymers (e.g., polymers A and B) and/or to produce phase separation between discrete polymer particles. Through the sintering process, the adhesions properties of the coating are improved to reduce flaking of detachment of the coating from the substrate during manipulation in use. As described below, in some embodiments, the sintering process is controlled to provide incomplete sintering of the polymer. In embodiments involving incomplete sintering, a polymer is formed with continuous domains, and voids, gaps, cavities, pores, channels or, interstices that provide space for sequestering a therapeutic agent which is released under controlled conditions. Depending on the nature of the polymer, the size of polymer particles and/or other polymer properties, a compressed gas, a densified gas, a near critical fluid or a super-critical fluid may be employed. In one example, carbon dioxide is used to treat a substrate that has been coated with a polymer and a drug, using dry powder and RESS electrostatic coating processes. In another example, isobutylene is employed in the sintering process. In other examples a mixture of carbon dioxide and isobutylene is employed. In another example, 1,1,2,3,3-hexafluoropropane is employed in the sintering process.

When an amorphous material is heated to a temperature above its glass transition temperature, or when a crystalline material is heated to a temperature above a phase transition temperature, the molecules comprising the material are more mobile, which in turn means that they are more active and thus more prone to reactions such as oxidation. However, when an amorphous material is maintained at a temperature below its glass transition temperature, its molecules are substantially immobilized and thus less prone to reactions. Likewise, when a crystalline material is maintained at a temperature below its phase transition temperature, its molecules are substantially immobilized and thus less prone to reactions. Accordingly, processing drug components at mild conditions, such as the deposition and sintering conditions described herein, minimizes cross-reactions and degradation of the drug component. One type of reaction that is minimized by the processes of the invention relates to the ability to avoid conventional solvents which in turn minimizes -oxidation of drug, whether in amorphous, semi-crystalline, or crystalline form, by reducing exposure thereof to free radicals, residual solvents, protic materials, polar-protic materials, oxidation initiators, and autoxidation initiators.

"Rapid Expansion of Supercritical Solutions" or "RESS" as used herein involves the dissolution of a polymer into a compressed fluid, typically a supercritical fluid, followed by rapid expansion into a chamber at lower pressure, typically near atmospheric conditions. The rapid expansion of the supercritical fluid solution through a small opening, with its accompanying decrease in density, reduces the dissolution capacity of the fluid and results in the nucleation and growth of polymer particles. The atmosphere of the chamber is maintained in an electrically neutral state by maintaining an isolating "cloud" of gas in the chamber. Carbon dioxide, nitrogen, argon, helium, or other appropriate gas is employed to prevent electrical charge is transferred from the substrate to the surrounding environment.

"Bulk properties" properties of a coating including a pharmaceutical or a biological agent that can be enhanced through the methods of the invention include for example: adhesion, smoothness, conformality, thickness, and compositional mixing.

"Electrostatically charged" or "electrical potential" or "electrostatic capture" or "e-" as used herein refers to the collection of the spray-produced particles upon a substrate that has a different electrostatic potential than the sprayed particles. Thus, the substrate is at an attractive electronic potential with respect to the particles exiting, which results in the capture of the particles upon the substrate, i.e. the substrate and particles are oppositely charged, and the particles transport through the gaseous medium of the capture vessel onto the surface of the substrate is enhanced via electrostatic attraction. This may be achieved by charging the particles and grounding the substrate or conversely charging the substrate and grounding the particles, by charging the particles at one potential (e.g. negative charge) and charging the substrate at an opposite potential (e.g. positive charge), or by some other process, which would be easily envisaged by one of skill in the art of electrostatic capture.

"Intimate mixture" as used herein, refers to two or more materials, compounds, or substances that are uniformly distributed or dispersed together.

"Layer" as used herein refers to a material covering a surface or forming an overlying part or segment. Two different layers may have overlapping portions whereby material from one layer may be in contact with material from another layer. Contact between materials of different layers can be measured by determining a distance between the materials. For example, Raman spectroscopy may be employed in identifying materials from two layers present in close proximity to each other.

While layers defined by uniform thickness and/or regular shape are contemplated herein, several embodiments described below relate to layers having varying thickness and/or irregular shape. Material of one layer may extend into the space largely occupied by material of another layer. For example, in a coating having three layers formed in sequence as a first polymer layer, a pharmaceutical agent layer and a second polymer layer, material from the second polymer layer which is deposited last in this sequence may extend into the space largely occupied by material of the pharmaceutical agent layer whereby material from the second polymer layer may have contact with material from the pharmaceutical layer. It is also contemplated that material from the second polymer layer may extend through the entire layer largely occupied by pharmaceutical agent and contact material from the first polymer layer.

It should be noted however that contact between material from the second polymer layer (or the first polymer layer) and material from the pharmaceutical agent layer (e.g.; a pharmaceutical agent crystal particle or a portion thereof) does not necessarily imply formation of a mixture between the material from the first or second polymer layers and material from the pharmaceutical agent layer. In some embodiments, a layer may be defined by the physical three-dimensional space occupied by crystalline particles of a pharmaceutical agent (and/or biological agent). It is contemplated that such layer may or may not be continuous as physical space occupied by the crystal particles of pharmaceutical agents may be interrupted, for example, by polymer material from an adjacent polymer layer. An adjacent polymer layer may be a layer that is in physical proximity to be pharmaceutical agent particles in the pharmaceutical agent layer. Similarly, an adjacent layer may be the layer formed in a process step right before or right after the process step in which pharmaceutical agent particles are deposited to form the pharmaceutical agent layer.

As described below, material deposition and layer formation provided herein are advantageous in that the pharmaceutical agent remains largely in crystalline form during the entire process. While the polymer particles and the pharmaceutical agent particles may be in contact, the layer formation process is controlled to avoid formation of a mixture between the pharmaceutical agent particles the polymer particles during formation of a coated device.

"Laminate coating" as used herein refers to a coating made up of two or more layers of material. Means for creating a laminate coating as described herein (e.g.; a laminate coating comprising bioabsorbable polymer(s) and pharmaceutical agent) may include coating the stent with drug and polymer as described herein (e-RESS, e-DPC, compressed-gas sintering). The process comprises performing multiple and sequential coating steps (with sintering steps for polymer materials) wherein different materials may be deposited in each step, thus creating a laminated structure with a multitude of layers (at least 2 layers) including polymer layers and pharmaceutical agent layers to build the final device (e.g.; laminate coated stent).

The coating methods provided herein may be calibrated to provide a coating bias whereby the mount of polymer and pharmaceutical agent deposited in the abluminal surface of the stent (exterior surface of the stent) is greater than the amount of pharmaceutical agent and amount of polymer deposited on the luminal surface of the stent (interior surface of the stent). The resulting configuration may be desirable to provide preferential elution of the drug toward the vessel wall (luminal surface of the stent) where the therapeutic effect of anti-restenosis is desired, without providing the same antiproliferative drug(s) on the abluminal surface, where they may retard healing, which in turn is suspected to be a cause of late-stage safety problems with current DESs.

As well, the methods described herein provide a device wherein the coating on the stent is biased in favor of increased coating at the ends of the stent. For example, a stent having three portions along the length of the stent (e.g.; a central portion flanked by two end portions) may have end portions coated with increased amounts of pharmaceutical agent and/or polymer compared to the central portion.

The present invention provides numerous advantages. The invention is advantageous in that it allows for employing a platform combining layer formation methods based on compressed fluid technologies; electrostatic capture and sintering methods. The platform results in drug eluting stents having enhanced therapeutic and mechanical properties. The invention is particularly advantageous in that it employs optimized laminate polymer technology. In particular, the present invention allows the formation of discrete layers of specific drug platforms. As indicated above, the shape of a discrete layer of crystal particles may be irregular, including interruptions of said layer by material from another layer (polymer layer) positioned in space between crystalline particles of pharmaceutical agent.

Conventional processes for spray coating stents require that drug and polymer be dissolved in solvent or mutual solvent before spray coating can occur. The platform provided herein the drugs and polymers are coated on the stent framework in discrete steps, which can be carried out simultaneously or alternately. This allows discrete deposition of the active agent (e.g., a drug) within a polymer thereby allowing the placement of more than one drug on a single medical device with or without an intervening polymer layer. For example, the present platform provides a dual drug eluting stent.

Some of the advantages provided by the subject invention include employing compressed fluids (e.g., supercritical fluids, for example E-RESS based methods); solvent free deposition methodology; a platform that allows processing at lower temperatures thereby preserving the qualities of the active agent and the polymer; the ability to incorporate two, three or more drugs while minimizing deleterious effects from direct interactions between the various drugs and/or their excipients during the fabrication and/or storage of the drug eluting stents; a dry deposition; enhanced adhesion and mechanical properties of the layers on the stent framework; precision deposition and rapid batch processing; and ability to form intricate structures.

Disclosed is a multi-drug delivery platform which produces strong, resilient and flexible drug eluting stents including an anti-restenosis drug (e.g., a limus or taxol) and anti-thrombosis drug (e.g., heparin or an analog thereof) and well characterized bioabsorbable polymers. The drug eluting stents provided herein minimize potential for thrombosis, in part, by reducing or totally eliminating thrombogenic polymers and reducing or totally eliminating residual drugs that could inhibit healing.

The platform provides optimized delivery of multiple drug therapies for example for early stage treatment (restenosis) and late-stage (thrombosis).

The platform also provides an adherent coating which enables access through tortuous lesions without the risk of the coating being compromised.

Another advantage of the present platform is the ability to provide highly desirable eluting profiles.

Advantages of the invention include the ability to reduce or completely eliminate potentially thrombogenic polymers as well as possibly residual drugs that may inhibit long term healing. As well, the invention provides advantageous stents having optimized strength and resilience if coatings which in turn allows access to complex lesions and reduces or completely eliminates delamination. Laminated layers of bioabsorbable polymers allow controlled elution of one or more drugs.

The platform provided herein reduces or completely eliminates shortcoming that have been associated with conventional drug eluting stents. For example, the platform provided herein allows for much better tuning of the period of time for the active agent to elute and the period of time necessary for the polymer to resorb thereby minimizing thrombosis and other deleterious effects associate with poorly controlled drug release.

The present invention provides several advantages which overcome or attenuate the limitations of current technology for bioabsorbable stents. For example, an inherent limitation of conventional bioabsorbable polymeric materials relates to the difficulty in forming to a strong, flexible, deformable (e.g.. balloon deployable) stent with low profile. The polymers generally lack the strength of high-performance metals. The present invention overcomes these limitations by creating a laminate structure in the essentially polymeric stent. Without wishing to be bound by any specific theory or analogy, the increased strength provided by the stents of the invention can be understood by comparing the strength of plywood vs. the strength of a thin sheet of wood.

Embodiments of the invention involving a thin metallic stent-framework provide advantages including the ability to overcome the inherent elasticity of most polymers. It is generally difficult to obtain a high rate (e.g., 100%) of plastic deformation in polymers (compared to elastic deformation where the materials have some 'spring back' to the original shape). Again, without wishing to be bound by any theory, the central metal stent framework (that would be too small and weak to serve as a stent itself) would act like wires inside of a plastic, deformable stent, basically overcoming any 'elastic memory' of the polymer.

Another advantage of the present invention is the ability to create a stent with a controlled (dialed-in) drug-elution profile. Via the ability to have different materials in each layer of the laminate structure and the ability to control the location of drug(s) independently in these layers, the method enables a stent that could release drugs at very specific elution profiles, programmed sequential and/or parallel elution profiles. Also, the present invention allows controlled elution of one drug without affecting the elution of a second drug (or different doses of the same drug).

Provided herein is a device comprising a stent; and a coating on the stent; wherein the coating comprises at least one bioabsorbable polymer and at least one active agent; wherein the active agent is present in crystalline form on at least one region of an outer surface of the coating opposite the stent and wherein 50% or less of the total amount of active agent in the coating is released after 24 hours in vitro elution.

In some embodiments, in vitro elution is carried out in a 1:1 spectroscopic grade ethanol (95%) / phosphate buffer saline at pH 7.4 and 37°C; wherein the amount of active agent released is determined by measuring UV absorption. In some embodiments, UV absorption is detected at 278 nm by a diode array spectrometer.

In some embodiments, in vitro elution testing, and/or any other test method described herein is performed following the final sintering step. In some embodiments, in vitro elution testing, and/or any other test method described herein is performed prior to crimping the stent to a balloon catheter. In some embodiments, in vitro elution testing, and/or any other test method described herein is performed following sterilization. In some embodiments in vitro elution testing, and/or any other test method described herein is performed following crimping the stent to a balloon catheter. In some embodiments, in vitro elution testing, and/or any other test method described herein is performed following expansion of the stent to nominal pressure of the balloon onto which the stent has been crimped. In some embodiments, in vitro elution testing, and/or any other test method described herein is performed following expansion of the stent to the rated burst pressure of the balloon to which the stent has been crimped.

In some embodiments, presence of active agent on at least a region of the surface of the coating is determined by cluster secondary ion mass spectrometry (cluster SIMS). In some embodiments, presence of active agent on at least a region of the surface of the coating is determined by generating cluster secondary ion mass spectrometry (cluster SIMS) depth profiles. In some embodiments, presence of active agent on at least a region of the surface of the coating is determined by time of flight secondary ion mass spectrometry (TOF-SIMS). In some embodiments, presence of active agent on at least a region of the surface of the coating is determined by atomic force microscopy (AFM). In some embodiments, presence of active agent on at least a region of the surface of the coating is determined by X-ray spectroscopy. In some embodiments, presence of active agent on at least a region of the surface of the coating is determined by electronic microscopy. In some embodiments, presence of active agent on at least a region of the surface of the coating is determined by Raman spectroscopy.

In some embodiments, between 25% and 45% of the total amount of active agent in the coating is released after 24 hours in vitro elution in a 1:1 spectroscopic grade ethanol (95%) / phosphate buffer saline at pH 7.4 and 37°C; wherein the amount of the active agent released is determined by measuring UV absorption at 278 nm by a diode array spectrometer.

In some embodiments, the active agent is at least 50% crystalline. In some embodiments, the active agent is at least 75% crystalline. In some embodiments, the active agent is at least 90% crystalline.

In some embodiments, the polymer comprises a PLGA copolymer. In some embodiments, the coating comprises a first PLGA copolymer with a ratio of about 40:60 to about 60:40 and a second PLGA copolymer with a ratio of about 60:40 to about 90:10. In some embodiments, the coating comprises a first PLGA copolymer having a molecular weight of about 10kD (weight average molecular weight) and a second polymer is a PLGA copolymer having a molecular weight of about 19kD (weight average molecular weight). In some embodiments, the coating comprises a PLGA copolymer having a number average molecular weight of between about 9.5kD and about 25kD. In some embodiments, the coating comprises a PLGA copolymer having a number average molecular weight of between about 14.5kD and about 15kD. As used herein, the term "about," when referring to a copolymer ratio, means variations of any of 0.5%, 1%, 2%, 5%, 10%, 15%, 20%, 25%, 30%, and 50%, depending on the embodiment. For example, a copolymer ratio of 40:60 having a variation of 10% ranges from 35:65 to 45:55, which is a range of 10% of the total (100) about the target. As used herein, the term "about" when referring to a polymer molecular weight means variations of any of 0.5%, 1%, 2%, 5%, 10%, 15%, 20%, 25%, 30%, and 50%, depending on the embodiment. For example, a polymer molecular weight of 10kD (weight average molecular weight) having a variation of 10% ranges from 9kD to 11kD, which is a range of 10% of the target 10kD (weight average molecular weight) on either side of the target 10kD (weight average molecular weight).

In some embodiments, the bioabsorbable polymer is selected from the group PLGA, PGA poly(glycolide), LPLA poly(1-lactide), DLPLA poly(dl-lactide), PCL poly(e-caprolactone) PDO, poly(dioxolane) PGA-TMC, 85/15 DLPLG p(dl-lactide-co-glycolide), 75/25 DLPL, 65/35 DLPLG, 50/50 DLPLG, TMC poly(trimethylcarbonate), poly(anhydrides) such as p(CPP:SA) poly(1,3-bis-p-(carboxyphenoxy)propane-co-sebacic acid).

In some embodiments, the stent is formed of stainless steel material. In some embodiments, the stent is formed of a material comprising a cobalt chromium alloy. In some embodiments, the stent is formed from a material comprising the following percentages by weight: about 0.05 to about 0.15 C, about 1.00 to about 2.00 Mn, about 0.04 Si, about 0.03 P, about 0.3 S, about 19.0 to about 21.0 Cr, about 9.0 to about 11.0 Ni, about 14.0 to about 16.00 W, about 3.0 Fe, and Bal. Co. In some embodiments, the stent is formed from a material comprising at most the following percentages by weight: about 0.025 C, about 0.15 Mn, about 0.15 Si, about 0.015 P, about 0.01 S, about 19.0 to about 21.0 Cr, about 33 to about37 Ni, about 9.0 to about 10.5 Mo, about 1.0 Fe, about 1.0 Ti, and Bal. Co. In some embodiments, the stent is formed from a material comprising L605 alloy. In some embodiments, the stent is formed from a material comprising MP35N alloy. In some embodiments, the stent is formed from a material comprising the following percentages by weight: about 35 Ni, about 35Cr, about 20 Co, and about 10 Mo. In some embodiments, the stent is formed from a material comprising a cobalt chromium nickel alloy. In some embodiments, the stent is formed from a material comprising Elgiloy®/Phynox®. In some embodiments, the stent is formed from a material comprising the following percentages by weight: about 39 to about 41 Co, about 19 to about 21 Cr, about 14 to about 16 Ni, about 6 to about 8 Mo, and Balance Fe. In some embodiments, the stent is formed of a material comprising a platinum chromium alloy. In some embodiments, the stent is formed of an alloy as described in U.S. Patent 7,329,383. In some embodiments, the stent is formed of an alloy as described in U.S. Patent Application 11/780,060. In some embodiments, the stent may be formed of a material comprising stainless steel, 316L stainless steel, BioDur ® 108 (UNS S29108), 304L stainless steel, and an alloy including stainless steel and 5-60% by weight of one or more radiopaque elements such as Pt, IR, Au, W, PERSS® as described in U.S. Publication No. 2003/001830, U.S. Publication No. 2002/0144757, and U.S. Publication No. 2003/0077200, nitinol, a nickel-titanium alloy, cobalt alloys, Elgiloy®, L605 alloys, MP35N alloys, titanium, titanium alloys, Ti-6Al-4V, Ti-50Ta, Ti-10Ir, platinum, platinum alloys, niobium, niobium alloys, Nb-lZr, Co-28Cr-6Mo, tantalum, and tantalum alloys. Other examples of materials are described in U.S. Publication No. 2005/0070990, and U.S. Publication No. 2006/0153729. Other materials include elastic biocompatible metal such as superelastic or pseudo-elastic metal alloys, as described, for example in Schetsky, L. McDonald, "Shape Memory Alloys", Encyclopedia of Chemical Technology (3d Ed), John Wiley & Sons 1982, vol. 20 pp. 726-736, and U.S. Publication No. 2004/0143317. As used herein, the term "about," when referring to a weight percentage of stent material, means variations of any of 0.5%, 1%, 2%, 5%, 10%, 15%, 20%, 25%, 30%, and 50% of the total weight percent (i.e. 100%) on either side (+/-) of the weight percentage, depending on the embodiment. For example, a weight percentage of stent material of 3.0 Fe having a variation of 1% ranges from 2.0 to 4.0, which is a range of 1% of the total (100) on either side of the target 3.0.

In some embodiments, the stent has a thickness of from about 50% to about 90% of a total thickness of the device. In some embodiments, the device has a thickness of from about 20 µm to about 500 µm. In some embodiments, the stent has a thickness of from about 50 µm to about 80 µm. In some embodiments, the coating has a total thickness of from about 5 µm to about 50 µm. The coating can be conformal around the struts, isolated on the abluminal side, patterned, or otherwise optimized for the target tissue.

In some embodiments, the device has an active agent content of from about 5 µg to about 500 µg. In some embodiments, the device has an active agent content of from about 100 µg to about 160 µg. As used herein, the term "about" when referring to a device thickness or coating thickness means variations of any of 0.5%, 1%, 2%, 5%, 10%, 15%, 20%, 25%, 30%, and 50%, depending on the embodiment. For non-limiting example, a device thickness of 20 µm having a variation of 10% ranges from 18 µm to 22 µm, which is a range of 10% on either side of the target 20 µm. For non-limiting example, a coating thickness of 100 µm having a variation of 10% ranges from 90 µm to 110 µm, which is a range of 10% on either side of the target 100 µm. As used herein, the term "about" when referring to a active agent (or pharmaceutical agent) content means variations of any of 0.5%, 1%, 2%, 5%, 10%, 15%, 20%, 25%, 30%, and 50%, depending on the embodiment. For non-limiting example, a active agent content of 120 µg having a variation of 10% ranges from 108 µg to 132 µg, which is a range of 10% on either side of the target 120 µg.

In some embodiments, the active agent is selected from rapamycin, a prodrug, a derivative, an analog, a hydrate, an ester, and a salt thereof. In some embodiments, the active agent is selected from one or more of sirolimus, everolimus, zotarolimus and biolimus. In some embodiments, the active agent comprises a macrolide immunosuppressive (limus) drug. In some embodiments, the macrolide immunosuppressive drug comprises one or more of rapamycin, biolimus (biolimus A9), 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof.
In some embodiments, the pharmaceutical agent is, at least in part, crystalline. As used herein, the term crystalline may include any number of the possible polymorphs of the crystalline form of the pharmaceutical agent, including for non-limiting example a single polymorph of the pharmaceutical agent, or a plurality of polymorphs of the pharmaceutical agent. The crystalline pharmaceutical agent (which may include a semi-crystalline form of the pharmaceutical agent, depending on the embodiment) may comprise a single polymorph of the possible polymorphs of the pharmaceutical agent. The crystalline pharmaceutical agent (which may include a semi-crystalline form of the pharmaceutical agent, depending on the embodiment) may comprise a plurality of polymorphs of the possible polymorphs of the crystalline pharmaceutical agent. The polymorph, in some embodiments, is a packing polymorph, which exists as a result of difference in crystal packing as compared to another polymorph of the same crystalline pharmaceutical agent. The polymorph, in some embodiments, is a conformational polymorph, which is conformer of another polymorph of the same crystalline pharmaceutical agent. The polymorph, in some embodiments, is a pseudopolymorph. The polymorph, in some embodiments, is any type of polymorph- that is, the type of polymorph is not limited to only a packing polymorph, conformational polymorph, and/or a pseudopolymorph. When referring to a particular pharmaceutical agent herein which is at least in part crystalline, it is understood that any of the possible polymorphs of the pharmaceutical agent are contemplated.

Provided herein is a device comprising a stent; and a coating on the stent; wherein the coating comprises at least one polymer and at least one active agent; wherein the active agent is present in crystalline form on at least one region of an outer surface of the coating opposite the stent and wherein between 25% and 50% of the total amount of active agent in the coating is released after 24 hours in vitro elution.

In some embodiments, the polymer comprises a durable polymer. In some embodiments, the polymer comprises a cross-linked durable polymer. Example biocompatible durable polymers include, but are not limited to: polyester, aliphatic polyester, polyanhydride, polyethylene, polyorthoester, polyphosphazene, polyurethane, polycarbonate urethane, aliphatic polycarbonate, silicone, a silicone containing polymer, polyolefin, polyamide, polycaprolactam, polyamide, polyvinyl alcohol, acrylic polymer, acrylate, polystyrene, epoxy, polyethers, celluiosics, expanded polytetrafluoroethylene, phosphorylcholine, polyethyleneyerphthalate, polymethylmethavrylate, poly(ethylmethacrylate/n-butylmethacrylate), parylene C, polyethylene-co-vinyl acetate, polyalkyl methacrylates, polyalkylene-co-vinyl acetate, polyalkylene, polyalkyl siloxanes, polyhydroxyalkanoate, polyfluoroalkoxyphasphazine, poly(styrene-b-isobutylene-b-styrene), poly-butyl methacrylate, poly-byta-diene, and blends, combinations, homopolymers, condensation polymers, alternating, block, dendritic, crosslinked, and copolymers thereof.

In some embodiments, the polymer comprises is at least one of: a fluoropolymer, PVDF-HFP comprising vinylidene fluoride and hexafluoropropylene monomers, PC (phosphorylcholine), Polysulfone, polystyrene-b-isobutylene-b-styrene, PVP (polyvinylpyrrolidone), alkyl methacrylate, vinyl acetate, hydroxyalkyl methacrylate, and alkyl acrylate. In some embodiments, the alkyl methacrylate comprises at least one of methyl methacrylate, ethyl methacrylate, propyl methacrylate, butyl methacrylate, hexyl methacrylate, octyl methacrylate, dodecyl methacrylate, and lauryl methacrylate. In some embodiments, the alkyl acrylate comprises at least one of methyl acrylate, ethyl acrylate, propyl acrylate, butyl acrylate, hexyl acrylate, octyl acrylate, dodecyl acrylates, and lauryl acrylate.

In some embodiments, the coating comprises a plurality of polymers. In some embodiments, the polymers comprise hydrophilic, hydrophobic, and amphiphilic monomers and combinations thereof. In one embodiment, the polymer comprises at least one of a homopolymer, a copolymer and a terpolymer. The homopolymer may comprise a hydrophilic polymer constructed of a hydrophilic monomer selected from the group consisting of poly(vinylpyrrolidone) and poly(hydroxylalkyl methacrylate). The copolymer may comprise comprises a polymer constructed of hydrophilic monomers selected from the group consisting of vinyl acetate, vinylpyrrolidone and hydroxyalkyl methacrylate and hydrophobic monomers selected from the group consisting of alkyl methacrylates including methyl, ethyl, propyl, butyl, hexyl, octyl, dodecyl, and lauryl methacrylate and alkyl acrylates including methyl, ethyl, propyl, butyl, hexyl, octyl, dodecyl, and lauryl acrylate. The terpolymer may comprise a polymer constructed of hydrophilic monomers selected from the group consisting of vinyl acetate and poly(vinylpyrrolidone), and hydrophobic monomers selected from the group consisting of alkyl methacrylates including methyl, ethyl, propyl, butyl, hexyl, octyl, dodecyl, and lauryl methacrylate and alkyl acrylates including methyl, ethyl, propyl, butyl, hexyl, octyl, dodecyl, and lauryl acrylate.

In one embodiment, the polymer comprises three polymers: a terpolymer, a copolymer and a homopolymer. In one such embodiment the terpolymer has the lowest glass transition temperature (Tg), the copolymer has an intermediate Tg and the homopolymer has the highest Tg. In one embodiment the ratio of terpolymer to copolymer to homopolymer is about 40:40:20 to about 88:10:2. In another embodiment, the ratio is about 50:35:15 to about 75:20:5. In one embodiment the ratio is approximately 63:27:10. In such embodiments, the terpolymer has a Tg in the range of about 5° C. to about 25° C., a copolymer has a Tg in the range of about 25° C. to about 40° C. and a homopolymer has a Tg in the range of about 170° C. to about 180° C. In some embodiments, the polymer system comprises a terpolymer (C19) comprising the monomer subunits n-hexyl methacrylate, N-vinylpyrrolidone and vinyl acetate having a Tg of about 10° C. to about 20° C., a copolymer (C10) comprising the monomer subunits n-butyl methacrylacte and vinyl acetate having a Tg of about 30° C. to about 35° C. and a homopolymer comprising polyvinylpyrrolidone having a Tg of about 174° C. As used herein, the term "about," when referring to a polymer ratio, means variations of any of 0.5%, 1%, 2%, 5%, 10%, 15%, 20%, 25%, 30%, and 50%, depending on the embodiment. For non-limiting example, a ratio of 40:40:20 having a variation of 10% around each of the polymers (e.g. the terpolymer may be 35-45%; the copolymer may be 35-45%, and the homopolymer may be 15 to 25% of the total). As used herein, the term "about," when referring to a Tg, means variations of any of 0.5%, 1%, 2%, 5%, 10%, 15%, 20%, 25%, 30%, and 50%, depending on the embodiment. For non-limiting example, a Tg of 30° C having a variation of 10% means a range of Tg from 27° C to 33° C.

Some embodiments comprise about 63% of C19, about 27% of C10 and about 10% of polyvinyl pyrrolidone (PVP). The C10 polymer is comprised of hydrophobic n-butyl methacrylate to provide adequate hydrophobicity to accommodate the active agent and a small amount of vinyl acetate. The C19 polymer is soft relative to the C10 polymer and is synthesized from a mixture of hydrophobic n-hexyl methacrylate and hydrophilic N-vinyl pyrrolidone and vinyl acetate monomers to provide enhanced biocompatibility. Polyvinyl pyrrolidone (PVP) is a medical grade hydrophilic polymer.

In some embodiments, the polymer is not a polymer selected from: PBMA (poly n-butyl methacrylate), Parylene C, and polyethylene-co-vinyl acetate.

In some embodiments, the polymer comprises a bioabsorbable polymer. In some embodiments, the bioabsorbable polymer is selected from the group PLGA, PGA poly(glycolide), LPLA poly(l-lactide), DLPLA poly(dl-lactide), PCL poly(e-caprolactone) PDO, poly(dioxolane) PGA-TMC, 85/15 DLPLG p(dl-lactide-co-glycolide), 75/25 DLPL, 65/35 DLPLG, 50/50 DLPLG, TMC poly(trimethylcarbonate), poly(anhydrides) such as p(CPP:SA) poly(1,3-bis-p-(carboxyphenoxy)propane-co-sebacic acid).

In some embodiments, in vitro elution is carried out in a 1:1 spectroscopic grade ethanol (95%) / phosphate buffer saline at pH 7.4 and 37°C; wherein the amount of active agent released is determined by measuring UV absorption.

In some embodiments, the active agent is at least 50% crystalline. In some embodiments, the active agent is at least 75% crystalline. In some embodiments, the active agent is at least 90% crystalline.

In some embodiments, the stent is formed of stainless steel material. In some embodiments, the stent is formed of a material comprising a cobalt chromium alloy. In some embodiments, the stent is formed from a material comprising the following percentages by weight: about 0.05 to about 0.15 C, about 1.00 to about 2.00 Mn, about 0.04 Si, about 0.03 P, about 0.3 S, about 19.0 to about 21.0 Cr, about 9.0 to about 11.0 Ni, about 14.0 to about 16.00 W, about 3.0 Fe, and Bal. Co. In some embodiments, the stent is formed from a material comprising at most the following percentages by weight: about 0.025 C, about 0.15 Mn, about 0.15 Si, about 0.015 P, about 0.01 S, about 19.0 to about 21.0 Cr, about 33 to about37 Ni, about 9.0 to about 10.5 Mo, about 1.0 Fe, about 1.0 Ti, and Bal. Co. In some embodiments, the stent is formed from a material comprising L605 alloy. In some embodiments, the stent is formed from a material comprising MP35N alloy. In some embodiments, the stent is formed from a material comprising the following percentages by weight: about 35 Ni, about 35Cr, about 20 Co, and about 10 Mo. In some embodiments, the stent is formed from a material comprising a cobalt chromium nickel alloy. In some embodiments, the stent is formed from a material comprising Elgiloy®/Phynox®. In some embodiments, the stent is formed from a material comprising the following percentages by weight: about 39 to about 41 Co, about 19 to about 21 Cr, about 14 to about 16 Ni, about 6 to about 8 Mo, and Balance Fe. In some embodiments, the stent is formed of a material comprising a platinum chromium alloy. In some embodiments, the stent is formed of an alloy as described in U.S. Patent 7,329,383. In some embodiments, the stent is formed of an alloy as described in U.S. Patent Application 11/780,060. In some embodiments, the stent may be formed of a material comprising stainless steel, 316L stainless steel, BioDur ® 108 (UNS S29108), 304L stainless steel, and an alloy including stainless steel and 5-60% by weight of one or more radiopaque elements such as Pt, IR, Au, W, PERSS® as described in U.S. Publication No. 2003/001830, U.S. Publication No. 2002/0144757, and U.S. Publication No. 2003/0077200, nitinol, a nickel-titanium alloy, cobalt alloys, Elgiloy®, L605 alloys, MP35N alloys, titanium, titanium alloys, Ti-6Al-4V, Ti-50Ta, Ti-10Ir, platinum, platinum alloys, niobium, niobium alloys, Nb-lZr, Co-28Cr-6Mo, tantalum, and tantalum alloys. Other examples of materials are described in U.S. Publication No. 2005/0070990, and U.S. Publication No. 2006/0153729. Other materials include elastic biocompatible metal such as superelastic or pseudo-elastic metal alloys, as described, for example in Schetsky, L. McDonald, "Shape Memory Alloys", Encyclopedia of Chemical Technology (3d Ed), John Wiley & Sons 1982, vol. 20 pp. 726-736, and U.S. Publication No. 2004/0143317.

In some embodiments, the stent has a thickness of from about 50% to about 90% of a total thickness of the device. In some embodiments, the device has a thickness of from about 20 µm to about 500 µm. In some embodiments, the stent has a thickness of from about 50 µm to about 80 µm. In some embodiments, the coating has a total thickness of from about 5 µm to about 50 µm. The coating can be conformal around the struts, isolated on the abluminal side, patterned, or otherwise optimized for the target tissue. As used herein, the term "about" when referring to a device thickness or coating thickness means variations of any of 0.5%, 1%, 2%, 5%, 10%, 15%, 20%, 25%, 30%, and 50%, depending on the embodiment. For non-limiting example, a device thickness of 20 µm having a variation of 10% ranges from 18 µm to 22 µm, which is a range of 10% on either side of the target 20 µm. For non-limiting example, a coating thickness of 100 µm having a variation of 10% ranges from 90 µm to 110 µm, which is a range of 10% on either side of the target 100 µm.

In some embodiments, the device has a pharmaceutical agent content of from about 5 µg to about 500 µg. In some embodiments, the device has a pharmaceutical agent content of from about 100 µg to about 160 µg. As used herein, the term "about" when referring to a active agent content (or pharmaceutical agent content) means variations of any of 0.5%, 1%, 2%, 5%, 10%, 15%, 20%, 25%, 30%, and 50%, depending on the embodiment. For non-limiting example, an active agent (or pharmaceutical agent) content of 120 µg having a variation of 10% ranges from 108 µg to 132 µg, which is a range of 10% on either side of the target 120 µg.

In some embodiments, the active agent is selected from rapamycin, a prodrug, a derivative, an analog, a hydrate, an ester, and a salt thereof. In some embodiments, the active agent comprises a macrolide immunosuppressive (limus) drug. In some embodiments, the macrolide immunosuppressive drug comprises one or more of rapamycin, biolimus (biolimus A9), 40-O-(2-Hydroxyethyl)rapamycin (everolimus), 40-O-Benzyl-rapamycin, 40-0-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof.

In some embodiments, the pharmaceutical agent is, at least in part, crystalline. As used herein, the term crystalline may include any number of the possible polymorphs of the crystalline form of the pharmaceutical agent, including for non-limiting example a single polymorph of the pharmaceutical agent, or a plurality of polymorphs of the pharmaceutical agent. The crystalline pharmaceutical agent (which may include a semi-crystalline form of the pharmaceutical agent, depending on the embodiment) may comprise a single polymorph of the possible polymorphs of the pharmaceutical agent. The crystalline pharmaceutical agent (which may include a semi-crystalline form of the pharmaceutical agent, depending on the embodiment) may comprise a plurality of polymorphs of the possible polymorphs of the crystalline pharmaceutical agent. The polymorph, in some embodiments, is a packing polymorph, which exists as a result of difference in crystal packing as compared to another polymorph of the same crystalline pharmaceutical agent. The polymorph, in some embodiments, is a conformational polymorph, which is conformer of another polymorph of the same crystalline pharmaceutical agent. The polymorph, in some embodiments, is a pseudopolymorph. The polymorph, in some embodiments, is any type of polymorph- that is, the type of polymorph is not limited to only a packing polymorph, conformational polymorph, and/or a pseudopolymorph. When referring to a particular pharmaceutical agent herein which is at least in part crystalline, it is understood that any of the possible polymorphs of the pharmaceutical agent are contemplated.

Provided herein is a device comprising a stent; and a plurality of layers that form a laminate coating on said stent; wherein at least one of said layers comprises a bioabsorbable polymer and at least one of said layers comprises one or more active agents; wherein at least a portion of the active agent is in crystalline form.

Provided herein is a device comprising a stent; and a plurality of layers that form a laminate coating on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein at least a portion of the pharmaceutical agent is in crystalline form.

In some embodiments, the device has at least one pharmaceutical agent layer defined by a three-dimensional physical space occupied by crystal particles of said pharmaceutical agent and said three dimensional physical space is free of polymer. In some embodiments, at least some of the crystal particles in said three dimensional physical space defining said at least one pharmaceutical agent layer are in contact with polymer particles present in a polymer layer adjacent to said at least one pharmaceutical agent layer defined by said three-dimensional space free of polymer.

In some embodiments, the plurality of layers comprises a first polymer layer comprising a first bioabsorbable polymer and a second polymer layer comprising a second bioabsorbable polymer, wherein said at least one layer comprising said pharmaceutical agent is between said first polymer layer and said second polymer layer. In some embodiments, first and second bioabsorbable polymers are the same polymer. In some embodiments, the first and second bioabsorbable polymers are different. In some embodiments, the second polymer layer has at least one contact point with at least one particle of said pharmaceutical agent in said pharmaceutical agent layer and said second polymer layer has at least one contact point with said first polymer layer.

In some embodiments, the stent has a stent longitudinal axis; and said second polymer layer has a second polymer layer portion along said stent longitudinal wherein said second layer portion is free of contact with particles of said pharmaceutical agent. In some embodiments, the device has at least one pharmaceutical agent layer defined by a three-dimensional physical space occupied by crystal particles of said pharmaceutical agent and said three dimensional physical space is free of polymer.

The second polymer layer may have a layer portion defined along a longitudinal axis of the stent, said polymer layer portion having a thickness less than said maximum thickness of said second polymer layer; wherein said portion is free of contact with particles of said pharmaceutical agent.

The polymer layer portion may be a sub layer which, at least in part, extends along the abluminal surface of the stent along the longitudinal axis of the stent (where the longitudinal axis of the stent is the central axis of the stent along its tubular length). For example, when a coating is removed from the abluminal surface of the stent, such as when the stent is cut along its length, flattened, and the coating is removed by scraping the coating off using a scalpel, knife or other sharp tool, the coating that is removed (despite having a pattern consistent with the stent pattern) has a layer that can be shown to have the characteristics described herein. This may be shown by sampling multiple locations of the coating that is representative of the entire coating.

Alternatively, and/or additionally, since stents are generally comprised of a series of struts and voids,. The methods provided herein advantageously allow for coatings extending around each strut, the layers of coating are likewise disposed around each strut. Thus, a polymer layer portion may be a layer which, at least, extends around each strut a distance from said strut (although the distance may vary where the coating thickness on the abluminal surface is different than the coating thickness on the luminal and/or sidewalls).

In some embodiments, the stent comprises at least one strut having a strut length along said stent longitudinal axis, wherein said second layer portion extends substantially along said strut length. In some embodiments, the stent has a stent length along said stent longitudinal axis and said second layer portion extends substantially along said stent length.

In some embodiments, the stent comprises at least five struts, each strut having a strut length along said stent longitudinal axis, wherein said second layer portion extends substantially along substantially the strut length of at least two struts. In some embodiments, the stent comprises at least five struts, each strut having a strut length along said stent longitudinal axis, wherein said second layer portion extends substantially along substantially the strut length of at least three struts. In some embodiments, the stent comprises at least five struts, each strut having a strut length along said stent longitudinal axis, wherein said second layer portion extends substantially along substantially the strut length of least four struts. In some embodiments, the stent comprises at least five struts, each strut having a strut length along said stent longitudinal axis, wherein said second layer portion extends substantially along substantially the strut length of all said at least five struts. In some embodiments, the stent has a stent length along said stent longitudinal axis and said second layer portion extends substantially along said stent length.

In some embodiments, the stent has a stent length along said stent longitudinal axis and said second layer portion extends along at least 50% of said stent length. In some embodiments, the stent has a stent length along said stent longitudinal axis and said second layer portion extends along at least 75% of said stent length. In some embodiments, the stent has a stent length along said stent longitudinal axis and said second layer portion extends along at least 85% of said stent length. In some embodiments, the stent has a stent length along said stent longitudinal axis and said second layer portion extends along at least 90% of said stent length. In some embodiments, the stent has a stent length along said stent longitudinal axis and said second layer portion extends along at least 99% of said stent length.

In some embodiments, the laminate coating has a total thickness and said second polymer layer portion has a thickness of from about 0.01% to about 10% of the total thickness of said laminate coating. In some embodiments, the laminate coating has a total thickness and said horizontal second polymer layer portion has a thickness of from about 1% to about 5% of the total thickness of said laminate coating. In some embodiments, the laminate coating has a total thickness of from about 5 µm to about 50 µm and said horizontal second polymer layer portion has a thickness of from about 0.001 µm to about 5 µm. In some embodiments, the laminate coating has a total thickness of from about 10 µm to about 20 µm and said second polymer layer portion has a thickness of from about 0.01 µm to about 5 µm. As used herein, the term "about" when referring to a laminate coating thickness means variations of any of 0.5%, 1%, 2%, 5%, 10%, 15%, 20%, 25%, 30%, and 50%, depending on the embodiment. For non-limiting example, a laminate coating thickness of 20 µm having a variation of 10% ranges from 18 µm to 22 µm, which is a range of 10% on either side of the target 20 µm. For non-limiting example, a layer portion having a thickness that is 1% of the total thickness of the laminate coating and having a variation of 0.5% means the layer portion may be from 0.5% to 1.5% of the total thickness of the laminate coating thickness. The coating can be conformal around the struts, isolated on the abluminal side, patterned, or otherwise optimized for the target tissue.

In some embodiments, the laminate coating is at least 25% by volume pharmaceutical agent. In some embodiments, the laminate coating is at least 35% by volume pharmaceutical agent. In some embodiments, the laminate coating is about 50% by volume pharmaceutical agent.

In some embodiments, at least a portion of the pharmaceutical agent is present in a phase separate from one or more phases formed by said polymer.

In some embodiments, the pharmaceutical agent is at least 50% crystalline. In some embodiments, the pharmaceutical agent is at least 75% crystalline. In some embodiments, the pharmaceutical agent is at least 90% crystalline. In some embodiments, the pharmaceutical agent is at least 95% crystalline. In some embodiments, the pharmaceutical agent is at least 99% crystalline.

In some embodiments, the stent has a stent longitudinal length and the coating has a coating outer surface along said stent longitudinal length, wherein said coating comprises pharmaceutical agent in crystalline form present in the coating below said coating outer surface. In some embodiments, the stent has a stent longitudinal length and the coating has a coating outer surface along said stent longitudinal length, wherein said coating comprises pharmaceutical agent in crystalline form present in the coating up to at least 1 µm below said coating outer surface. In some embodiments, the stent has a stent longitudinal length and the coating has a coating outer surface along said stent longitudinal length, wherein said coating comprises pharmaceutical agent in crystalline form present in the coating up to at least 5 µm below said coating outer surface.

In some embodiments, the coating exhibits an X-ray spectrum showing the presence of said pharmaceutical agent in crystalline form. In some embodiments, the coating exhibits a Raman spectrum showing the presence of said pharmaceutical agent in crystalline form. In some embodiments, the coating exhibits a Differential Scanning Calorimetry (DSC) curve showing the presence of said pharmaceutical agent in crystalline form. In some embodiments, said coating exhibits Wide Angle X-ray Scattering (WAXS) spectrum showing the presence of said pharmaceutical agent in crystalline form. In some embodiments, the coating exhibits a wide angle radiation scattering spectrum showing the presence of said pharmaceutical agent in crystalline form. In some embodiments, the coating exhibits an Infra Red (IR) spectrum showing the presence of said pharmaceutical agent in crystalline form

In some embodiments, the stent has a stent longitudinal axis and a stent length along said stent longitudinal axis, wherein said coating is conformal to the stent along substantially said stent length.

In some embodiments, the stent has a stent longitudinal axis and a stent length along said stent longitudinal axis, wherein said coating is conformal to the stent along at least 75% of said stent length. In some embodiments, the stent has a stent longitudinal axis and a stent length along said stent longitudinal axis, wherein said coating is conformal to the stent along at least 85% of said stent length. In some embodiments, the stent has a stent longitudinal axis and a stent length along said stent longitudinal axis, wherein said coating is conformal to the stent along at least 90% of said stent length. In some embodiments, the stent has a stent longitudinal axis and a stent length along said stent longitudinal axis, wherein said coating is conformal to the stent along at least 95% of said stent length. In some embodiments, the stent has a stent longitudinal axis and a stent length along said stent longitudinal axis, wherein said coating is conformal to the stent along at least 99% of said stent length.

In some embodiments, the stent has a stent longitudinal axis and a plurality of struts along said stent longitudinal axis, wherein said coating is conformal to at least 50% of said struts. In some embodiments, the stent has a stent longitudinal axis and a plurality of struts along said stent longitudinal axis, wherein said coating is conformal to at least 75% of said struts. In some embodiments, the stent has a stent longitudinal axis and a plurality of struts along said stent longitudinal axis, wherein said coating is conformal to at least 90% of said struts. In some embodiments, the stent has a stent longitudinal axis and a plurality of struts along said stent longitudinal axis, wherein said coating is conformal to at least 99% of said struts. In some embodiments, the stent has a stent longitudinal axis and a stent length along said stent longitudinal axis, wherein an electron microscopy examination of the device shows said coating is conformal to said stent along at least 90% of said stent length.

In some embodiments, the stent has a stent longitudinal axis and a stent length along said stent longitudinal axis, wherein said coating has a substantially uniform thickness along substantially said stent length.

In some embodiments, the stent has a stent longitudinal axis and a stent length along said stent longitudinal axis, wherein said coating has a substantially uniform thickness along at least 75% of said stent length. In some embodiments, the stent has a stent longitudinal axis and a stent length along said stent longitudinal axis, wherein said coating has a substantially uniform thickness along at least 95% of said stent length.

In some embodiments, the stent has a stent longitudinal axis and a stent length along said stent longitudinal axis, wherein said coating has an average thickness determined by an average calculated from coating thickness values measured at a plurality of points along said stent longitudinal axis; wherein a thickness of the coating measured at any point along stent longitudinal axis is from about 75% to about 125% of said average thickness. In some embodiments, the stent has a stent longitudinal axis and a stent length along said stent longitudinal axis, wherein said coating has an average thickness determined by an average calculated from coating thickness values measured at a plurality of points along said stent longitudinal axis; wherein a thickness of the coating measured at any point along stent longitudinal axis is from about 95% to about 105% of said average thickness. As used herein, the term "about" when referring to a coating thickness means variations of any of 0.5%, 1%, 2%, 5%, 10%, 15%, 20%, 25%, 30%, and 50%, depending on the embodiment. For non-limiting example, a coating thickness at a point along the stent longitudinal axis which is 75% of the average thickness and having a variation of 10% may actually be anywhere from 65% to 85% of the average thickness.

Provided herein is a device comprising: a stent; and a plurality of layers that form a laminate coating on said stent, wherein a first layer comprises a first bioabsorbable polymer, a second layer comprises a pharmaceutical agent, a third layer comprises a second bioabsorbable polymer, a fourth layer comprises the pharmaceutical agent, and a fifth layer comprises a third bioabsorbable polymer, wherein the pharmaceutical agent is selected from rapamycin, a prodrug, a derivative, an analog, a hydrate, an ester, and a salt thereof, and wherein at least a portion of the pharmaceutical agent is in crystalline form.

In some embodiments, at least two of said first bioabsorbable polymer, said second bioabsorbable polymer and said third bioabsorbable polymer are the same polymer. In some embodiments, the first bioabsorbable polymer, the second bioabsorbable polymer and the third bioabsorbable polymer are the same polymer. In some embodiments, at least two of said first bioabsorbable polymer, said second bioabsorbable polymer and said third bioabsorbable polymer are different polymers. In some embodiments, the first bioabsorbable polymer, said second bioabsorbable polymer and said third bioabsorbable polymer are different polymers.

In some embodiments, the third layer has at least one contact point with particles of said pharmaceutical agent in said second layer; and said third layer has at least one contact point with said first layer.

In some embodiments, at least two of the first polymer, the second polymer, and the third polymer are the same polymer, and wherein said same polymer comprises a PLGA copolymer. In some embodiments, the third polymer has an in vitro dissolution rate higher than the in vitro dissolution rate of the first polymer. In some embodiments, the third polymer is PLGA copolymer with a ratio of about 40:60 to about 60:40 and the first polymer is a PLGA copolymer with a ratio of about 70:30 to about 90:10. In some embodiments, the third polymer is PLGA copolymer having a molecular weight of about 10kD (weight average molecular weight) and the second polymer is a PLGA copolymer having a molecular weight of about 19kD (weight average molecular weight). In some embodiments, the first polymer, the second polymer, and the third polymer each comprise a PLGA copolymer having a number average molecular weight of between about 9.5kD and about 25kD. In some embodiments, the first polymer, the second polymer, and the third polymer each comprise a PLGA copolymer having a number average molecular weight of between about 14.5kD and about 15kD. As used herein, the term "about," when referring to a copolymer ratio, means variations of any of 0.5%, 1%, 2%, 5%, 10%, 15%, 20%, 25%, 30%, and 50%, depending on the embodiment. For example, a copolymer ratio of 40:60 having a variation of 10% ranges from 35:65 to 45:55, which is a range of 10% of the total (100) about the target. As used herein, the term "about" when referring to a polymer molecular weight means variations of any of 0.5%, 1%, 2%, 5%, 10%, 15%, 20%, 25%, 30%, and 50%, depending on the embodiment. For example, a polymer molecular weight of 10kD (weight average molecular weight) having a variation of 10% ranges from 9kD to 11kD, which is a range of 10% of the target 10kD on either side of the target 10kD.

In some embodiments, measuring the in vitro dissolution rate of said polymers comprises contacting the device with elution media and determining polymer weight loss at one or more selected time points. In some embodiments, measuring the in vitro dissolution rate of said polymers comprises contacting the device with elution media and determining polymer weight loss at one or more selected time points.

Provided herein is a device, comprising: a stent; and a coating on said stent comprising a first bioabsorbable polymer, a second bioabsorbable polymer; and pharmaceutical agent selected from rapamycin, a prodrug, a derivative, an analog, a hydrate, an ester, and a salt thereof wherein at least a portion of the pharmaceutical agent is in crystalline form, and wherein the first polymer has an in vitro dissolution rate higher than the in vitro dissolution rate of the second polymer.

In some embodiments, the first polymer is PLGA copolymer with a ratio of about 40:60 to about 60:40 and the second polymer is a PLGA copolymer with a ratio of about 70:30 to about 90:10. In some embodiments, the first polymer is PLGA copolymer having a molecular weight of about 10kD (weight average molecular weight) and the second polymer is a PLGA copolymer having a molecular weight of about 19kD (weight average molecular weight). In some embodiments, the coating comprises a PLGA copolymer having a number average molecular weight of between about 9.5kD and about 25kD. In some embodiments, the coating comprises a PLGA copolymer having a number average molecular weight of between about 14.5kD and about 15kD. In some embodiments, measuring the in vitro dissolution rate of said polymers comprises contacting the device with elution media and determining polymer weight loss at one or more selected time points. As used herein, the term "about," when referring to a copolymer ratio, means variations of any of 0.5%, 1%, 2%, 5%, 10%, 15%, 20%, 25%, 30%, and 50%, depending on the embodiment. For example, a copolymer ratio of 40:60 having a variation of 10% ranges from 35:65 to 45:55, which is a range of 10% of the total (100) about the target. As used herein, the term "about" when referring to a polymer molecular weight means variations of any of 0.5%, 1%, 2%, 5%, 10%, 15%, 20%, 25%, 30%, and 50%, depending on the embodiment. For example, a polymer molecular weight of 10kD (weight average molecular weight) having a variation of 10% ranges from 9kD to 11kD, which is a range of 10% of the target 10kD on either side of the target 10kD.

Provided herein is a device comprising a stent; and a plurality of layers that form a laminate coating on said stent; wherein at least one of said layers comprises a first bioabsorbable polymer, at least one of said layers comprises a second bioabsorbable polymer, and at least one of said layers comprises one or more active agents; wherein at least a portion of the active agent is in crystalline form, and wherein the first polymer has an in vitro dissolution rate higher than the in vitro dissolution rate of the second polymer.

Provided herein is a device comprising a stent; and a plurality of layers that form a laminate coating on said stent; wherein at least one of said layers comprises a first bioabsorbable polymer, at least one of said layers comprises a second bioabsorbable polymer, and at least one of said layers comprises a pharmaceutical agent selected from rapamycin, a prodrug, a derivative, an analog, a hydrate, an ester, and a salt thereof; wherein at least a portion of the pharmaceutical agent is in crystalline form and wherein the first polymer has an in vitro dissolution rate higher than the in vitro dissolution rate of the second polymer.

In some embodiments, the first polymer is PLGA copolymer with a ratio of about 40:60 to about 60:40 and the second polymer is a PLGA copolymer with a ratio of about 70:30 to about 90:10. In some embodiments, the first polymer is PLGA copolymer having a molecular weight of about 10kD (weight average molecular weight) and the second polymer is a PLGA copolymer having a molecular weight of about 19kD (weight average molecular weight). In some embodiments, at least one of the first coating and the second coating comprises a PLGA copolymer having a number average molecular weight of between about 9.5kD and about 25kD. In some embodiments, at least one of the first coating and the second coating comprises a PLGA copolymer having a number average molecular weight of between about 14.5kD and about 15kD. In some embodiments, measuring the in vitro dissolution rate comprises contacting the device with elution media and determining polymer weight loss at one or more selected time points. As used herein, the term "about," when referring to a copolymer ratio, means variations of any of 0.5%, 1%, 2%, 5%, 10%, 15%, 20%, 25%, 30%, and 50%, depending on the embodiment. For example, a copolymer ratio of 40:60 having a variation of 10% ranges from 35:65 to 45:55, which is a range of 10% of the total (100) about the target. As used herein, the term "about" when referring to a polymer molecular weight means variations of any of 0.5%, 1%, 2%, 5%, 10%, 15%, 20%, 25%, 30%, and 50%, depending on the embodiment. For example, a polymer molecular weight of 10kD (weight average molecular weight) having a variation of 10% ranges from 9kD to 11kD, which is a range of 10% of the target 10kD on either side of the target 10kD.

Provided herein is a device comprising a stent; and a plurality of layers that form a laminate coating on said stent; wherein at least one of said layers comprises a bioabsorbable polymer, at least one of said layers comprises a first active agent and at least one of said layers comprises a second active agent; wherein at least a portion of first and/or second active agents is in crystalline form.

In some embodiments, the bioabsorbable polymer is selected from the group PLGA, PGA poly(glycolide), LPLA poly(l-lactide), DLPLA poly(dl-lactide), PCL poly(e-caprolactone) PDO, poly(dioxolane) PGA-TMC, 85/15 DLPLGp(dl-lactide-co-glycolide), 75/25 DLPL, 65/35 DLPLG, 50/50 DLPLG, TMC poly(trimethylcarbonate), poly(anhydrides) such as p(CPP:SA) poly(1,3-bis-p-(carboxyphenoxy)propane-co-sebacic acid). In some embodiments, the polymer comprises an intimate mixture of two or more polymers.

In some embodiments, the first and second active agents are independently selected from pharmaceutical agents and active biological agents.

In some embodiments, the stent is formed of stainless steel material. In some embodiments, the stent is formed of a material comprising a cobalt chromium alloy. In some embodiments, the stent is formed from a material comprising the following percentages by weight: about 0.05 to about 0.15 C, about 1.00 to about 2.00 Mn, about 0.04 Si, about 0.03 P, about 0.3 S, about 19.0 to about 21.0 Cr, about 9.0 to about 11.0 Ni, about 14.0 to about 16.00 W, about 3.0 Fe, and Bal. Co. In some embodiments, the stent is formed from a material comprising at most the following percentages by weight: about 0.025 C, about 0.15 Mn, about 0.15 Si, about 0.015 P, about 0.01 S, about 19.0 to about 21.0 Cr, about 33 to about37 Ni, about 9.0 to about 10.5 Mo, about 1.0 Fe, about 1.0 Ti, and Bal. Co. In some embodiments, the stent is formed from a material comprising L605 alloy. In some embodiments, the stent is formed from a material comprising MP35N alloy. In some embodiments, the stent is formed from a material comprising the following percentages by weight: about 35 Ni, about 35Cr, about 20 Co, and about 10 Mo. In some embodiments, the stent is formed from a material comprising a cobalt chromium nickel alloy. In some embodiments, the stent is formed from a material comprising Elgiloy®/Phynox®. In some embodiments, the stent is formed from a material comprising the following percentages by weight: about 39 to about 41 Co, about 19 to about 21 Cr, about 14 to about 16 Ni, about 6 to about 8 Mo, and Balance Fe. In some embodiments, the stent is formed of a material comprising a platinum chromium alloy. In some embodiments, the stent is formed of an alloy as described in U.S. Patent 7,329,383. In some embodiments, the stent is formed of an alloy as described in U.S. Patent Application 11/780,060. In some embodiments, the stent may be formed of a material comprising stainless steel, 316L stainless steel, BioDur ® 108 (UNS S29108), 304L stainless steel, and an alloy including stainless steel and 5-60% by weight of one or more radiopaque elements such as Pt, IR, Au, W, PERSS® as described in U.S. Publication No. 2003/001830, U.S. Publication No. 2002/0144757, and U.S. Publication No. 2003/0077200, nitinol, a nickel-titanium alloy, cobalt alloys, Elgiloy®, L605 alloys, MP35N alloys, titanium, titanium alloys, Ti-6Al-4V, Ti-50Ta, Ti-10Ir, platinum, platinum alloys, niobium, niobium alloys, Nb-lZr, Co-28Cr-6Mo, tantalum, and tantalum alloys. Other examples of materials are described in U.S. Publication No. 2005/0070990, and U.S. Publication No. 2006/0153729. Other materials include elastic biocompatible metal such as superelastic or pseudo-elastic metal alloys, as described, for example in Schetsky, L. McDonald, "Shape Memory Alloys", Encyclopedia of Chemical Technology (3d Ed), John Wiley & Sons 1982, vol. 20 pp. 726-736, and U.S. Publication No. 2004/0143317. As used herein, the term "about," when referring to a weight percentage of stent material, means variations of any of 0.5%, 1%, 2%, 5%, 10%, 15%, 20%, 25%, 30%, and 50% of the total weight percent (i.e. 100%) on either side (+/-) of the weight percentage, depending on the embodiment. For example, a weight percentage of stent material of 3.0 Fe having a variation of 1% ranges from 2.0 to 4.0, which is a range of 1% of the total (100) on either side of the target 3.0.

In some embodiments, the stent has a thickness of from about 50% to about 90% of a total thickness of said device. In some embodiments, the device has a thickness of from about 20 µm to about 500 µm. In some embodiments, the device has a thickness of about 90 µm or less. In some embodiments, the laminate coating has a thickness of from about 5 µm to about 50 µm. In some embodiments, the laminate coating has a thickness of from about 10 µm to about 20 µm. In some embodiments, the stent has a thickness of from about 50 µm to about 80 µm. As used herein, the term "about" when referring to a device thickness or coating thickness or laminate coating thickness means variations of any of 0.5%, 1%, 2%, 5%, 10%, 15%, 20%, 25%, 30%, and 50%, depending on the embodiment. For non-limiting example, a device thickness of 20 µm having a variation of 10% ranges from 18 µm to 22 µm, which is a range of 10% on either side of the target 20 µm. The coating can be conformal around the struts, isolated on the abluminal side, patterned, or otherwise optimized for the particular target tissue.

Provided herein is a device comprising: a stent, wherein the stent is formed from a material comprising the following percentages by weight: 0.05-0.15 C, 1.00-2.00 Mn, 0.040 Si, 0.030 P, 0.3 S, 19.00-21.00 Cr, 9.00-11.00 Ni, 14.00-16.00 W, 3.00 Fe, and Bal. Co; and a plurality of layers that form a laminate coating on said stent, wherein a first layer comprises a first bioabsorbable polymer, a second layer comprises a pharmaceutical agent, a third layer comprises a second bioabsorbable polymer, a fourth layer comprises the pharmaceutical agent, and a fifth layer comprises a third bioabsorbable polymer, wherein the pharmaceutical agent is selected from rapamycin, a prodrug, a derivative, an analog, a hydrate, an ester, and a salt thereof, wherein at least a portion of the pharmaceutical agent is in crystalline form, and wherein at least one of said first polymer, second polymer and third polymer comprises a PLGA copolymer.

In some embodiments, the device has a pharmaceutical agent content of from about 0.5 µg/mm to about 20 µg/mm. In some embodiments, the device has a pharmaceutical agent content of from about 8 µg/mm to about 12 µg/mm. In some embodiments, the device has a pharmaceutical agent content of from about 5 µg to about 500 µg. In some embodiments, the device has a pharmaceutical agent content of from about 100 µg to about 160 µg. As used herein, the term "about" when referring to a active agent content (or pharmaceutical agent content) means variations of any of 0.5%, 1%, 2%, 5%, 10%, 15%, 20%, 25%, 30%, and 50%, depending on the embodiment. For non-limiting example, an active agent content (or pharmaceutical agent content) of 120 µg having a variation of 10% ranges from 108 µg to 132 µg, which is a range of 10% on either side of the target 120 µg. Where content is expressed herein in units of µg/mm, however, this may simply be converted to µg/mm2 or another amount per area (e.g., µg/cm2), or vice versa. Similarly, where content is expressed in terms of µg, this may be simply converted to a per-area or per-length term, or vice versa as needed.

Provided herein is a method of preparing a device comprising a stent and a plurality of layers that form a laminate coating on said stent; said method comprising: (a) providing a stent; (b) forming a plurality of layers on said stent to form said laminate coating on said stent; wherein at least one of said layers comprises a bioabsorbable polymer and at least one of said layers comprises one or more active agents; wherein at least a portion of the active agent is in crystalline form.

Provided herein is a method of preparing a device comprising a stent and a plurality of layers that form a laminate coating on said stent; said method comprising: (a) providing a stent; (b) forming a plurality of layers to form said laminate coating on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein at least a portion of the pharmaceutical agent is in crystalline form.

Provided herein is a method of preparing a device comprising a stent and a plurality of layers that form a laminate coating on said stent; said method comprising: (a) providing a stent; (b) forming a plurality of layers to form said laminate coating on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein at least a portion of the pharmaceutical agent is in crystalline form, wherein said method comprises forming at least one pharmaceutical agent layer defined by a three-dimensional physical space occupied by crystal particles of said pharmaceutical agent and said three dimensional physical space is free of polymer.

Provided herein is a method of preparing a device comprising a stent and a plurality of layers that form a laminate coating on said stent; said method comprising: (a) providing a stent; (b) discharging at least one pharmaceutical agent and/or at least one active biological agent in dry powder form through a first orifice; (c) forming a supercritical or near supercritical fluid solution comprising at least one supercritical fluid solvent and at least one polymer and discharging said supercritical or near supercritical fluid solution through a second orifice under conditions sufficient to form solid particles of the polymer; (d) depositing the polymer and pharmaceutical agent and/or active biological agent particles onto said substrate, wherein an electrical potential is maintained between the substrate and the polymer and pharmaceutical agent and/or active biological agent particles, thereby forming said coating; and (e) sintering said polymer under conditions that do not substantially modify a morphology of said pharmaceutical agent and/or activity of said biological agent.

In some embodiments, step (b) comprises discharging a pharmaceutical agent selected from rapamycin, a prodrug, a derivative, an analog, a hydrate, an ester, and a salt thereof; wherein at least a portion of the pharmaceutical agent is in crystalline form. In some embodiments, step (c) comprises forming solid particles of a bioabsorbable polymer.

In some embodiments, step (e) comprises forming a polymer layer having a length along a horizontal axis of said device wherein said polymer layer has a layer portion along said length, wherein said layer portion is free of pharmaceutical agent.

In some embodiments, step (e) comprises contacting said polymer with a densified fluid. In some embodiments, step (e) comprises contacting said polymer with a densified fluid for a period of time at a temperature of from about 5 °C and 150 °C and a pressure of from about 0.07 MPa (10 psi) to about 3.45 MPa (500 psi). In some embodiments, step (e) comprises contacting said polymer with a densified fluid for a period of time at a temperature of from about 25 °C and 95 °C and a pressure of from about 0.17 MPa (25 psi) to about 0.69 MPa (100 psi). In some embodiments, step (e) comprises contacting said polymer with a densified fluid for a period of time at a temperature of from about 50 °C and 85 °C and a pressure of from about 0.24 MPa (35 psi) to about 0.44 MPa (65 psi). The term "about" when used in reference to a temperature in the coating process means variations of any of 0.5%,
1%, 2%, 5%, 10%, 15%, 20%, 25%, 30%, and 50%, on either side of the target or on a single side of the target, depending on the embodiment. For non-limiting example, for a temperature of 150 °C having a variability of 10% on either side of the target (of 150°C), the temperature would range from 135°C to 165°C. The term "about" when used in reference to a pressure in the coating process means variations of any of 0.5%, 1%, 2%, 5%, 10%, 15%, 20%, 25%, 30%, and 50%, depending on the embodiment. For non-limiting example, for a pressure of 0.69 MPa (100 psi) having a variability of 10% on either side of the target (of 0.69 MPa (100 psi)), the pressure would range from 0.62 MPa (90 psi) to 0.76 MPa (110 psi).

Provided herein is a method of preparing a device comprising a stent and a plurality of layers that form a laminate coating on said stent; said method comprising: (a) providing a stent; (b) forming a supercritical or near supercritical fluid solution comprising at least one supercritical fluid solvent and a first polymer, discharging said supercritical or near supercritical fluid solution under conditions sufficient to form solid particles of said first polymer, depositing said first polymer particles onto said stent, wherein an electrical potential is maintained between the stent and the first polymer, and sintering said first polymer; (c) depositing pharmaceutical agent particles in dry powder form onto said stent, wherein an electrical potential is maintained between the stent and said pharmaceutical agent particles; and (d) forming a supercritical or near supercritical fluid solution comprising at least one supercritical fluid solvent and a second polymer and discharging said supercritical or near supercritical fluid solution under conditions sufficient to form solid particles of said second polymer, wherein an electrical potential is maintained between the stent and the second polymer, and sintering said second polymer.

In some embodiments, step (c) and step (d) are repeated at least once. In some embodiments, steps (c) and step (d) are repeated 2 to 20 times.

In some embodiments, the pharmaceutical agent is selected from rapamycin, a prodrug, a derivative, an analog, a hydrate, an ester, and a salt thereof; wherein at least a portion of the pharmaceutical agent is in crystalline form. In some embodiments, the first and second polymers are bioabsorbable.

In some embodiments, step (d) comprises forming a polymer layer having a length along a horizontal axis of said device wherein said polymer layer has a layer portion along said length, wherein said layer portion is free of pharmaceutical agent.

In some embodiments, sintering said first and/or sintering said second polymer comprises contacting said first and/or second polymer with a densified fluid.

In some embodiments, the contacting step is carried out for a period of from about 1 minute to about 60 minutes. In some embodiments, the contacting step is carried out for a period of from about 10 minutes to about 30 minutes.

In some embodiments, maintaining said electrical potential between said polymer particles and or pharmaceutical agent particles and said stent comprises maintaining a voltage of from about 5 kvolts to about 100 kvolts. In some embodiments, maintaining said electrical potential between said polymer particles and or pharmaceutical agent particles and said stent comprises maintaining a voltage of from about 20 kvolts to about 30 kvolts.

Provided herein is a device prepared by a process comprising a method as described herein.

Provided herein is method of treating a subject comprising delivering a device as described herein in a body lumen of the subject.

Provided herein is a method of treating a subject comprising delivering in the body of the subject a device comprising: a stent, wherein the stent is formed from a material comprising the following percentages by weight: 0.05-0.15 C, 1.00-2.00 Mn, 0.040 Si, 0.030 P, 0.3 S, 19.00-21.00 Cr, 9.00-11.00 Ni, 14.00-16.00 W, 3.00 Fe, and Bal. Co; and a plurality of layers that form a laminate coating on said stent, wherein a first layer comprises a first bioabsorbable polymer, a second layer comprises a pharmaceutical agent, a third layer comprises a second bioabsorbable polymer, a fourth layer comprises the pharmaceutical agent, and a fifth layer comprises a third bioabsorbable polymer, wherein the pharmaceutical agent is selected from rapamycin, a prodrug, a derivative, an analog, a hydrate, an ester, and a salt thereof, wherein at least a portion of the pharmaceutical agent is in crystalline form, and wherein at least one of said first polymer, second polymer and third polymer comprises a PLGA copolymer.

In some embodiments, the device has a pharmaceutical agent content of from about 0.5 µg/mm to about 20 µg/mm. In some embodiments, the device has a pharmaceutical agent content of from about 8 µg/mm to about 12 µg/mm. In some embodiments, the device has a pharmaceutical agent content of from about 100 µg to about 160 µg. In some embodiments, the device has a pharmaceutical agent content of from about 120 µg to about 150 µg. As used herein, the term "about" when referring to a pharmaceutical agent content means variations of any of 0.5%, 1%, 2%, 5%, 10%, 15%, 20%, 25%, 30%, and 50%, depending on the embodiment. For non-limiting example, a pharmaceutical agent content of 120 µg having a variation of 10% ranges from 108 µg to 132 µg, which is a range of 10% on either side of the target 120 µg. Where content is expressed herein in units of µg/mm, however, this may simply be converted to µg/mm2 or another amount per area (e.g., µg/cm2), or vice versa, or converted to a total pharmaceutical content by multiplying by the area or length as needed.

In some embodiments, the device has an initial pharmaceutical agent amount and the amount of pharmaceutical agent delivered by said device to vessel wall tissue of said subject is higher than the amount of pharmaceutical agent delivered by a conventional drug eluting stent having the same initial pharmaceutical agent content as the initial pharmaceutical agent content of said device. In some embodiments, the amount of pharmaceutical agent delivered by said device to vessel wall tissue of said subject is at least 25% more that the amount of pharmaceutical agent delivered to vessel wall tissue of said subject by said conventional drug eluting stent. In some embodiments, the method comprises treating restenosis in a blood vessel of said the subject. In some embodiments, the subject is selected from a pig, a rabbit and a human.

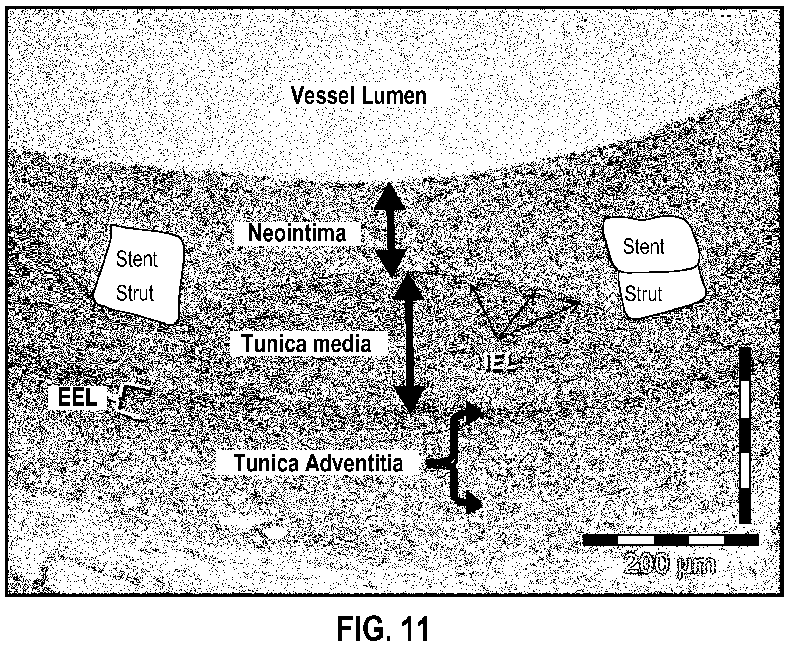

"Vessel wall tissue" as used herein is shown in Figure 11, which depicts the tissue surrounding the lumen of a vessel, including the endothelium, neointima, tunica media, IEL (internal elastic lamina), EEL (external elastic lamina), and the tunica adventitia.

Provided herein is a device comprising: a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows about 5% to about 25% of pharmaceutical agent is eluted one day after the device is contacted with elution media; 15% to about 45% of pharmaceutical agent is eluted 7 days after the device is contacted with elution media; about 25% to about 60% of pharmaceutical agent is eluted 14 days after the device is contacted with elution media; about 35% to about 70% of pharmaceutical agent is eluted 21 days after the device is contacted with elution media; and about 40% to about 100% of pharmaceutical agent is eluted 28 days after the device is contacted with elution media. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the aspect target, depending on the embodiment. For non-limiting example, for an elution of 25% having a variation of 5%, this could mean 25% plus or minus 5%-- equating to a range of 20% to 30%.

Provided herein is a device comprising a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows about 7% to about 15% of pharmaceutical agent is eluted one day after the device is contacted with elution media; 25% to about 35% of pharmaceutical agent is eluted 7 days after the device is contacted with elution media; about 35% to about 55% of pharmaceutical agent is eluted 14 days after the device is contacted with elution media; about 45% to about 60% of pharmaceutical agent is eluted 21 days after the device is contacted with elution media; and about 50% to about 70% of pharmaceutical agent is eluted 28 days after the device is contacted with elution media. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the aspect target, depending on the embodiment. For non-limiting example, for an elution of 25% having a variation of 5%, this could mean 25% plus or minus 5%-- equating to a range of 20% to 30%.

Provided herein is a device comprising a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows at least 5% of pharmaceutical agent is eluted one day after the device is contacted with elution media; at least 15% of pharmaceutical agent is eluted 7 days after the device is contacted with elution media; at least 25% of pharmaceutical agent is eluted 14 days after the device is contacted with elution media; at least 30% of pharmaceutical agent is eluted 21 days after the device is contacted with elution media; at least 40% of pharmaceutical agent is eluted 28 days after the device is contacted with elution media.

Provided herein is a device comprising a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows about 10% of pharmaceutical agent is eluted one day after the device is contacted with elution media; about 30% of pharmaceutical agent is eluted 7 days after the device is contacted with elution media; about 45% of pharmaceutical agent is eluted 14 days after the device is contacted with elution media; about 50% of pharmaceutical agent is eluted 21 days after the device is contacted with elution media; about 60% of pharmaceutical agent is eluted 28 days after the device is contacted with elution media.

Provided herein is a device comprising a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows about 10% to about 75% of pharmaceutical agent is eluted at week 1 after the device is contacted with elution media, about 25% to about 85% of pharmaceutical agent is eluted at week 2 and about 50% to about 100% of pharmaceutical agent is eluted at week 10. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the aspect target, depending on the embodiment. For non-limiting example, for an elution of 25% having a variation of 5%, this could mean 25% plus or minus 5%-- equating to a range of 20% to 30%.

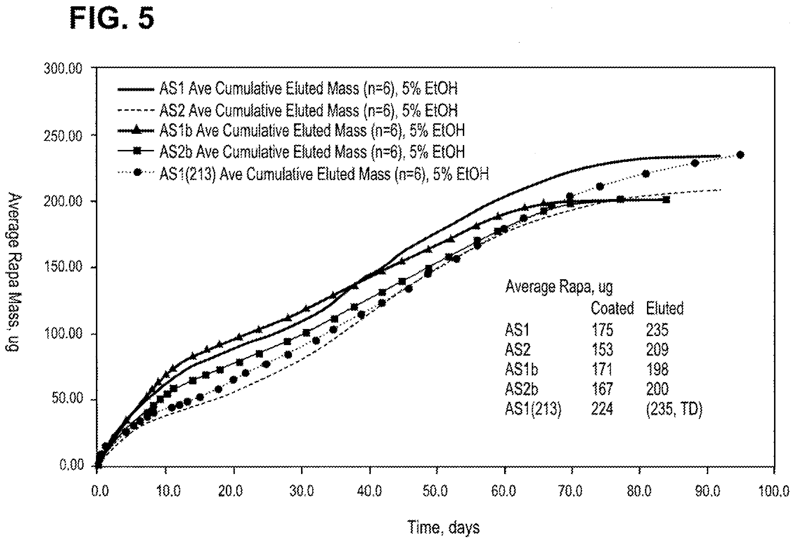

Provided herein is a device comprising: a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile shown in Figure 5.

In some embodiments, the in vitro pharmaceutical agent elution profile is determined by a procedure comprising: (i) contacting the device with an elution media comprising 5% ethanol by volume wherein the pH of the media is about 7.4 and wherein the device is contacted with the elution media at a temperature of about 37°C; (ii) optionally agitating the elution media during the contacting step in (i); (iii) removing the elution media at designated time points; and (iv) assaying the removed elution media to determine pharmaceutical agent content.

In some embodiments, the in vitro pharmaceutical agent elution profile is determined by a procedure comprising: (i) contacting the device with an elution media comprising 5% ethanol by volume, wherein the pH of the media is about 7.4 and wherein the device is contacted with the elution media at a temperature of about 37°C; (ii) optionally agitating the elution media during the contacting step in (i); (iii) removing said device from the elution media at designated time points; and (iv) assaying the elution media to determine pharmaceutical agent content.

In some embodiments, the in vitro pharmaceutical agent elution profile is determined in the absence of agitation.

In some embodiments, the procedure further comprises: (v) determining polymer weight loss by comparing the weight of the device before and after the contacting step and adjusting for the amount of pharmaceutical agent eluted into the elution media as determined in step (iv). In some embodiments, step (v) shows at least 50% of polymer is released into the media after the device is contacted with the media for 90 days or more. In some embodiments, step (v) shows at least 75% of polymer is released into the media after the device is contacted with the media for 90 days or more.

In some embodiments, step (v) shows at least 85% of polymer is released into the media after the device is contacted with the media for 90 days or more. In some embodiments, step (v) shows at least 50% of polymer is released into the media after the device is contacted with the media for about 90 days. In some embodiments, step (v) shows at least 75% of polymer is released into the media after the device is contacted with the media for about 90 days. In some embodiments, step (v) shows at least 85% of polymer is released into the media after the device is contacted with the media for about 90 days. In some embodiments, step (v) shows at least 95% of polymer is released into the media after the device is contacted with the media for about 90 days. In some embodiments, step (v) shows up to 100% of polymer is released into the media after the device is contacted with the media for about 90 days. As used herein, the term "about" when referring to the media contact time can vary up to 1%, 5%, 10%, 20%, 25%, 6 hrs, 12 hrs, 24 hrs, 1 day, 2 days, 3 days, 5 days, or 7 days.

Provided herein is a device comprising: a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows about 1% to about 35% of pharmaceutical agent is eluted one hour after the device is contacted with elution media; 5% to about 45% of pharmaceutical agent is eluted 3 hours after the device is contacted with elution media; about 30% to about 70% of pharmaceutical agent is eluted 1 day after the device is contacted with elution media; about 40% to about 80% of pharmaceutical agent is eluted 3 days after the device is contacted with elution media; about 50% to about 90% of pharmaceutical agent is eluted 10 days after the device is contacted with elution media about 55% to about 95% of pharmaceutical agent is eluted 15 days after the device is contacted with elution media; and about 60% to about 100% of pharmaceutical agent is eluted 20 days after the device is contacted with elution media. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the aspect target, depending on the embodiment. For non-limiting example, for an elution of 25% having a variation of 5%, this could mean 25% plus or minus 5%-equating to a range of 20% to 30%.

Provided herein is a device comprising: a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile wherein said elution profile shows about 5% to about 25% of pharmaceutical agent is eluted one hour after the device is contacted with elution media; 5% to about 35% of pharmaceutical agent is eluted 3 hours after the device is contacted with elution media; about 30% to about 65% of pharmaceutical agent is eluted 1 day after the device is contacted with elution media; about 45% to about 70% of pharmaceutical agent is eluted 3 days after the device is contacted with elution media; about 55% to about 85% of pharmaceutical agent is eluted 10 days after the device is contacted with elution media about 65% to about 85% of pharmaceutical agent is eluted 15 days after the device is contacted with elution media; and about 75% to about 100% of pharmaceutical agent is eluted 20 days after the device is contacted with elution media.

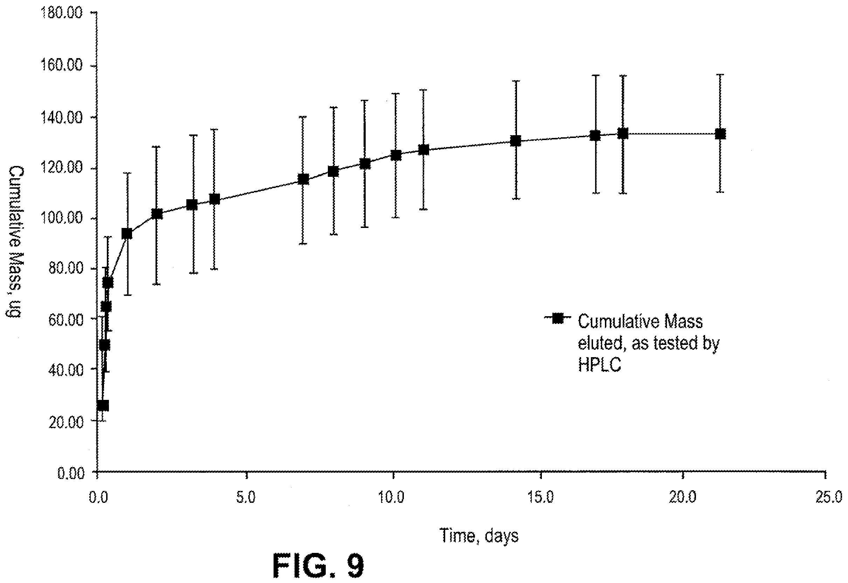

Provided herein is a device comprising: a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof; wherein said device provides an in vitro pharmaceutical agent elution profile shown in Figure 9.

In some embodiments, the in vitro pharmaceutical agent elution profile is determined by a procedure comprising: (i) contacting the device with an elution media comprising ethanol and phosphate buffered saline wherein the pH of the media is about 7.4 and wherein the device is contacted with the elution media at a temperature of about 37°C; (ii) optionally agitating the elution media during the contacting step in (i); (iii) removing the elution media at designated time points; and (iv) assaying the removed elution media to determine pharmaceutical agent content.

In some embodiments, the in vitro pharmaceutical agent elution profile is determined by a procedure comprising: (i) contacting the device with an elution media comprising ethanol and phosphate buffered saline wherein the pH of the media is about 7.4 and wherein the device is contacted with the elution media at a temperature of about 37°C; (ii) optionally agitating the elution media during the contacting step in (i); (iii) removing said device from the elution media at designated time points; and (iv) assaying the elution media to determine pharmaceutical agent content.

In some embodiments, the in vitro pharmaceutical agent elution profile is determined in the absence of agitation.

In some embodiments, the procedure further comprises: (v) determining polymer weight loss by comparing the weight of the device before and after the contacting step and adjusting for the amount of pharmaceutical agent eluted into the elution media as determined in step iv. The device of claim 160 wherein step v shows at least 50% of polymer is released into the media after the device is contacted with the media for 90 days or more.

In some embodiments, step (v) shows at least 75% of polymer is released into the media after the device is contacted with the media for 90 days or more. In some embodiments, step (v) shows at least 85% of polymer is released into the media after the device is contacted with the media for 90 days or more. In some embodiments, step (v) shows at least 50% of polymer is released into the media after the device is contacted with the media for about 90 days. In some embodiments, step (v) shows at least 75% of polymer is released into the media after the device is contacted with the media for about 90 days. In some embodiments, step (v) shows at least 85% of polymer is released into the media after the device is contacted with the media for about 90 days. In some embodiments, step (v) shows at least 95% of polymer is released into the media after the device is contacted with the media for about 90 days. As used herein, the term "about" when referring to the media contact time can vary up to 1%, 5%, 10%, 20%, 25%, 6 hrs, 12 hrs, 24 hrs, 1 day, 2 days, 3 days, 5 days, or 7 days.

Provided herein is a device comprising: a stent; and a coating comprising a pharmaceutical agent selected from rapamycin, a prodrug, a derivative, ester and a salt thereof and a polymer wherein the coating has an initial pharmaceutical agent amount; wherein when said device is delivered in a body lumen of a subject the pharmaceutical agent is delivered in vessel wall tissue of the subject as follows: from about 0.1% to about 35% of the initial pharmaceutical agent amount is delivered in the subject's vessel wall tissue one week after the device is delivered in the subject's body; and from about 0.5% to about 50% of the initial pharmaceutical agent amount is delivered in the subject's vessel wall tissue two weeks after the device is delivered in the subject's body. As used herein, the term "about" when used in reference to percent delivery of the pharmaceutical agent means variations of any of 0.01%, 0.05%, 0.1%, 0.5%, 1%, 2%, 5%, 10%, 15%, 20%, 25%, 30%, and 50% on either side of the percent elution or on a single side of the aspect target, depending on the embodiment. For non-limiting example, for an delivery of 25% having a variation of 5%, this could mean 25% plus or minus 5%-- equating to a range of 20% to 30%.

In some embodiments, the amount delivered to the subject's lumen is obtained by adding pharmaceutical agent present alone in said subject's vessel wall tissue and pharmaceutical agent delivered together with said polymer. In some embodiments, the subject is a human.

In some embodiments, subject is a pig and the amount of pharmaceutical agent delivered in the subject's vessel wall tissue is determined as follows: delivering the device in the pig's blood vessel lumen; euthanizing the pig at predetermined period of time after the device is delivered in the pig's blood vessel lumen and explanting the device; measuring the amount of pharmaceutical agent delivered in the vessel wall tissue. In some embodiments, subject is a rabbit and the amount of pharmaceutical agent delivered in the subject's vessel wall tissue is determined as follows: delivering the device in the rabbit's blood vessel lumen; euthanizing the rabbit at predetermined period of time after the device is delivered in the rabbit's blood vessel lumen and explanting the device; measuring the amount of pharmaceutical agent delivered in the vessel wall tissue.

Provided herein, a device comprising: a stent; and a coating comprising a pharmaceutical agent selected from rapamycin, a prodrug, a derivative, an analog, a hydrate, an ester, and a salt thereof and a bioabsorbable polymer wherein the coating has an initial pharmaceutical agent content of about 1 µg/mm to about 15 µg/mm; wherein said device provides an area under a curve (AUC) for content of pharmaceutical agent delivered in the vessel wall tissue of a subject over time as follows: from about 0.05 (µg/mm)*day to about 1 (µg/mm)*day when AUC is calculated from the time the device is delivered in a subject's body to one day after the device is delivered in the subject's body; from about 5 (µg/mm)*day to about 10 (µg/mm)*day when AUC is calculated starting after the first week the device is delivered in the subject's body through the second week after the device is delivered in the subject's body; from about 10 (µg/mm)*day to about 20 (µg/mm)*day when AUC is calculated starting after the second week the device is delivered in the subject's body through the fourth week after the device is delivered in the subject's body; and an AUClast of from about 40 (µg/mm)*day to about 60 (µg/mm)*day. As used herein, the term "about" when used in reference to AUC means variations of any of 0.01%, 0.05%, 0.1%, 0.5%, 1%, 2%, 5%, 10%, 15%, 20%, 25%, 30%, and 50% on either side of the target, depending on the embodiment.

Provided herein is a device comprising: a stent; and a coating comprising a pharmaceutical agent selected from rapamycin, a prodrug, a derivative, an analog, a hydrate, an ester, and a salt thereof and a bioabsorbable polymer wherein the coating has an initial polymer amount; wherein when said device is delivered in a body lumen of a subject about 75% of polymer is released from the device 90 days or more after the device is delivered in the body lumen of the subject. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the target, depending on the embodiment. For non-limiting example, for an elution of 25% having a variation of 5%, this could mean 25% plus or minus 5%-- equating to a range of 20% to 30%.

Provided herein is a device comprising: a stent; and a coating comprising a pharmaceutical agent selected from rapamycin, a prodrug, a derivative, an analog, a hydrate, an ester, and a salt thereof and a bioabsorbable polymer wherein the coating has an initial polymer amount; wherein when said device is delivered in a body lumen of a subject about 85% of polymer is released from the device about 90 days after the device is delivered in the body lumen of the subject. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the target, depending on the embodiment. For non-limiting example, for an elution of 25% having a variation of 5%, this could mean 25% plus or minus 5%-- equating to a range of 20% to 30%. As used herein, the term "about" when referring to the media contact time can vary up to 1%, 5%, 10%, 20%, 25%, 6 hrs, 12 hrs, 24 hrs, 1 day, 2 days, 3 days, 5 days, or 7 days.

Provided herein is a device comprising: a stent; and a coating comprising a pharmaceutical agent selected from rapamycin, a prodrug, a derivative, an analog, a hydrate, an ester, and a salt thereof and a bioabsorbable polymer wherein the coating has an initial polymer amount; wherein when said device is delivered in a body lumen of a subject at least about 75% of polymer is released from the device about 90 days after the device is delivered in the body lumen of the subject. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the aspect target, depending on the embodiment. For non-limiting example, for an elution of 25% having a variation of 5%, this could mean 25% plus or minus 5%-- equating to a range of 20% to 30%. As used herein, the term "about" when referring to the media contact time can vary up to 1%, 5%, 10%, 20%, 25%, 6 hrs, 12 hrs, 24 hrs, 1 day, 2 days, 3 days, 5 days, or 7 days.

Provided herein is a device comprising: a stent; and a coating comprising a pharmaceutical agent selected from rapamycin, a prodrug, a derivative, an analog, a hydrate, an ester, and a salt thereof and a bioabsorbable polymer wherein the coating has an initial polymer amount; wherein when said device is delivered in a body lumen of a subject about 100% of polymer is released from the device about 90 days after the device is delivered in the body lumen of the subject. As used herein, the term "about" when used in reference to percent elution means variations of any of 0.01%, 0.05%, 0.1%, 0.5%, 1%, 2%, 5%, 10%, 15%, 20%, 25%, 30%, and 50% on either side of the percent elution or on a single side of the target, depending on the embodiment. For non-limiting example, for an elution of 25% having a variation of 5%, this could mean 25% plus or minus 5%-- equating to a range of 20% to 30%. As used herein, the term "about" when referring to the media contact time can vary up to 1%, 5%, 10%, 20%, 25%, 6 hrs, 12 hrs, 24 hrs, 1 day, 2 days, 3 days, 5 days, or 7 days.

In some embodiments, the subject is a human. In some embodiments, the subject is a pig and the amount of polymer released from the device is determined as follows: delivering the device in the pig's blood vessel lumen; euthanizing the pig at predetermined period of time after the device is delivered in the pig's blood vessel lumen and explanting the device; and measuring the amount of polymer released from the device.

In some embodiments, measuring the amount of polymer released from the device comprises LC/MS/MS measurements. In some embodiments, measuring the amount released from the device comprises weight loss measurement. In some embodiments, weight loss measurement comprises measuring an amount of polymer remaining in the device and subtracting said remaining amount from the initial amount present in the device prior to delivering the device to the pig's blood vessel lumen.

Provided herein is a device comprising a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof, wherein the device has an initial pharmaceutical agent content of about 1 µg/mm to about 15 µg/mm; wherein when said device is delivered in a body lumen of a subject said device provides a blood concentration within 60 minutes from delivery of said device to the subject's body lumen that is from about 1% to about 50% of the blood concentration provided by a conventional drug eluting stent delivered to the subject under similar conditions. The term "about" when used in reference to a percent of blood concentration provided by a conventional drug eluting stent means variations of any of 0.01%, 0.05%, 0.1%, 0.5%, 1%, 2%, 5%, 10%, 15%, 20%, and 25% on either side of the percent or on a single side of the percent, depending on the embodiment. For non-limiting example, for a blood concentration that is 50% of the blood concentration provided by a conventional drug eluting stent and having a variability of 5%, the blood concentration may range from 45% to 55%, (i.e. 5% about the target of 50%).

Provided herein is a device comprising a stent; and a plurality of layers on said stent; wherein at least one of said layers comprises a bioabsorbable polymer and at least one of said layers comprises a pharmaceutical agent selected from rapamycin, a prodrug, a derivative, an analog, a hydrate, an ester, and a salt thereof, wherein the device has an initial pharmaceutical agent content of about 1 µg/mm to about 15 µg/mm; wherein when said device is delivered in a body lumen of a subject said device provides a blood concentration within 60 minutes from delivery of said device to the subject's body lumen that is from about 11% to about 20% of the blood concentration provided by a conventional drug eluting stent delivered to the subject under similar conditions.

Provided herein is a device comprising a stent; and coating on said stent; wherein said coating comprises a bioabsorbable polymer and a pharmaceutical agent selected from rapamycin, a prodrug, a derivative, an analog, a hydrate, an ester, and a salt thereof, wherein the device has an initial pharmaceutical agent content of about 1 µg/mm to about 15 µg/mm; wherein when said device is delivered in a body lumen of a subject said device provides about the same blood concentration over the first 72 hours from delivery of said device to the subject's body lumen.

In some embodiments, the blood concentration during the first 72 hours from delivery of said device to the subject's body lumen remains between 75% and 125% of an average blood concentration calculated over the first 72 hours from delivery of said device to the subject's body lumen. In some embodiments, the average blood concentration is from about 0.05 ng/mL to about 0.5 ng/mL. In some embodiments, the device provides an AUC for blood concentration over a period of 72 hours after the device is delivered to the subject's body lumen of from about 2 (ng/mL)*hour to about 20 (ng/mL)*hour.

In some embodiments, the device provides an AUC for blood concentration over a period of 72 hours after the device is delivered to the subject's body lumen of from about 4 (ng/mL)*hour to about 10 (ng/mL)*hour. In some embodiments, at least part of pharmaceutical agent is in crystalline form. In some embodiments, the pharmaceutical agent is provided at a reduced dose compared to a conventional drug eluting stent. In some embodiments, at least one of said layers comprises a PLGA bioabsorbable polymer.

In some embodiments, the pharmaceutical agent in said device has a shelf stability of at least 12 months.

In some embodiments, the device provides an in vitro pharmaceutical agent elution profile comparable to first order kinetics.

In some embodiments, the device provides pharmaceutical agent tissue concentration of at least twice the tissue concentration provided by a conventional stent. In some embodiments, the device provides a pharmaceutical agent tissue concentration of at least 5 times greater than the tissue concentration provided by a conventional stent. In some embodiments, the device provides a pharmaceutical agent tissue concentration of at least 25 times greater than the tissue concentration provided by a conventional stent. In some embodiments, the device provides a pharmaceutical agent tissue concentration of at least 100 times greater than the tissue concentration provided by a conventional stent.

In some embodiments, about 50% of said polymer is resorbed within 45-90 days after an angioplasty procedure wherein said device is delivered in a subject's body. In some embodiments, about 75% of said polymer is resorbed within 45-90 days after an angioplasty procedure wherein said device is delivered in a subject's body. In some embodiments, about 95% of said polymer is resorbed within 45-90 days after an angioplasty procedure wherein said device is delivered in a subject's body. The term "about" when referring to the percent of the polymer resorbed means variations of any of 0.01%, 0.05%, 0.1%, 0.5%, 1%, 2%, 5%, 10%, 15%, 20%, and 25% on either side of the percent or on a single side of the percent.

In some embodiments, 99% of said polymer is resorbed within 45-90 days after an angioplasty procedure wherein said device is delivered in a subject's body. In some embodiments, at least 99% of said polymer is resorbed within 45-90 days after an angioplasty procedure wherein said device is delivered in a subject's body. In some embodiments, at least 95% of said polymer is resorbed within 45-90 days after an angioplasty procedure wherein said device is delivered in a subject's body. In some embodiments, at least 90% of said polymer is resorbed within 45-90 days after an angioplasty procedure wherein said device is delivered in a subject's body. In some embodiments, at least 80% of said polymer is resorbed within 45-90 days after an angioplasty procedure wherein said device is delivered in a subject's body. In some embodiments, at least 75% of said polymer is resorbed within 45-90 days after an angioplasty procedure wherein said device is delivered in a subject's body. In some embodiments, 100% of said polymer is resorbed within 45-90 days after an angioplasty procedure wherein said device is delivered in a subject's body. In some embodiments, at least 99% of said polymer is absorbed within 45-90 days after an angioplasty procedure wherein said device is delivered in a subject's body. In some embodiments, at least 95% of said polymer is absorbed within 45-90 days after an angioplasty procedure wherein said device is delivered in a subject's body. In some embodiments, at least 90% of said polymer is absorbed within 45-90 days after an angioplasty procedure wherein said device is delivered in a subject's body. In some embodiments, at least 80% of said polymer is absorbed within 45-90 days after an angioplasty procedure wherein said device is delivered in a subject's body. In some embodiments, at least 75% of said polymer is absorbed within 45-90 days after an angioplasty procedure wherein said device is delivered in a subject's body. In some embodiments, 100% of said polymer is absorbed within 45-90 days after an angioplasty procedure wherein said device is delivered in a subject's body.

Generally, polymers associated with drug-eluting stents are solvent labile and susceptible to artifactual removal during the histologic processing of implanted arteries. Polymers, present during the formation of the neointima, are a space occupying mass in which the smooth muscle cells must accommodate, and around which the nascent neointima must form. With the removal of the polymer during processing, clear spaces are created and interpreted to be the negative image, or approximate facsimile, of the stent polymer/drug in situ, despite the absence of observable polymer. As such, these clear areas may be used to qualitatively characterize the size, spread, localization and apparent resorption of polymer coating material as a function of implant duration. Bioabsorbable polymers will resorb over time by the body, thus, over time these clear areas will be fewer and smaller, until the polymer is fully absorbed (resorbed). These may be detected by histologic processing of implanted arteries, such as noted in Examples 34-37, at least, and visualized under microscopy as noted therein.

The polymer/drug coating of the coated stents described herein are typically characterized by a larger clear zone intimately surrounding the struts. Lacunae refers to variably sized and shaped clear space(s) located in the peri/extra-strut neointima which appear to have been separated from the polymer intimately associated with the struts. These lacunae were interpreted to represent the deposition/migration of the strut-associated polymer/drug into the surrounding neointima. Since lacunae were not observed in the bare metal stents similarly implanted, their presence in the coated stent tissue samples may be the local effects of neointimal formation inhibition secondary to the presence of the polymer (i.e., space-occupying mass) and/or sirolimus (i.e., smooth muscle cell inhibition).

Neointimal lacunae were only observed only at Day 30 (when evaluated at about day 3, 30, 90, 180, and 365), whether there was a single coated stent implanted, or whether there were two coated stents implanted (overlapping as noted elsewhere herein). The magnitude of extra-strut neointimal lacunae (i.e., polymer/drug) was minimal, and though they were commonly seen on a per plane basis (∼ 70%), within each affected plane the change was generally limited to only one to two foci. Regardless, the presence of neointimal lacunae after 30 days implantation of the coated stents implanted as noted herein did not appear to be associated with any adverse tissue response. Rarely (∼ <5%), lacunae were present in the adventitia, with associated inflammation, and usually the result of mural injury.

In some embodiments, the device provides reduced inflammation over the course of polymer resorbtion compared to a conventional stent.

Provided herein is a method of treating a subject comprising delivering a device as described herein in a body lumen.

Provided herein, is a method of treating a subject comprising delivering in the body of the subject a device comprising: a stent; and a coating comprising a pharmaceutical agent selected from rapamycin, a prodrug, a derivative, an analog, a hydrate, an ester, and a salt thereof and a polymer wherein the coating has an initial pharmaceutical agent amount; wherein said device is delivered in a body lumen of the subject and the pharmaceutical agent is delivered in vessel wall tissue of the subject as follows: i. from about 0.05% to about 35% of the initial pharmaceutical agent amount is delivered in the subject's vessel wall tissue one week after the device is delivered in the subject's body; and ii. from about 0.5% to about 50% of the initial pharmaceutical agent amount is delivered in the subject's vessel wall tissue two weeks after the device is delivered in the subject's body.

In some embodiments, the device provides reduced inflammation over the course of polymer resorbtion.

In some embodiments, the presence of crystallinity is shown by at least one of XRD, Raman Spectroscopy, Infrared analytical methods, and DSC.

In some embodiments, the coating on an abluminal surface of said stent has a greater thickness than coating on a luminal surface of said stent. In some embodiments, the ratio of coating on the abluminal surface to coating on the luminal surface of the device is 80:20. In some embodiments, the ratio of coating on the abluminal surface to coating on the luminal surface of the device is 75:25. In some embodiments, the ratio of coating on the abluminal surface to coating on the luminal surface of the device is 70:30. In some embodiments, the ratio of coating on the abluminal surface to coating on the luminal surface of the device is 60:40.

Provided herein is a device comprising a stent comprising a cobalt-chromium alloy; and a coating on the stent; wherein the coating comprises at least one polymer and at least one active agent; wherein at least one of: quantified neointima, media, percent stenosis, wall injury, and inflammation exhibited at 30 days following implantation of the device in a first artery of an animal is significantly reduced for the device as compared to a bare metal cobalt-chromium stent implanted in a second artery of an animal when both the device and the bare metal cobalt chromium stent are compared in a the study, wherein the study design overlaps two of the devices in the first artery and overlaps two of the bare metal cobalt-chromium stents in the second artery.

In some embodiments, the test performed to determine significant differences between the device and the bare metal cobalt-chromium stent is the Mann-Whitney Rank Sum Test and the p value is less than 0.10. In some embodiments, the test performed to determine significant differences between the device and the bare metal cobalt-chromium stent is the Mann-Whitney Rank Sum Test and the p value is less than 0.05.

In some embodiments, at least one of wall injury, inflammation, neointimal maturation, and adventitial fibrosis of the device tested at day 3 of the animal study is equivalent to the bare metal stent.

In some embodiments, at least one of lumen area, artery area, lumen diameter, IEL diameter, stent diameter, arterial diameter, lumen area/artery area ratio, neointimal area/medial area ratio, EEL/IEL ratio, endothelialization, neointimal maturation, and adventitial fibrosis of the device tested at day 30 of the animal study is equivalent to the bare metal stent.

In some embodiments, at least one of lumen area, artery area, neointimal area, medial area, percent stenosis, wall injury, and inflammation of the device tested at day 30 of the animal study is equivalent to the bare metal stent.

In some embodiments, at least one of lumen area, artery area, neointimal area, medial area, percent stenosis, wall injury, inflammation, endothelialization, neointimal maturation, and adventitial fibrosis of the device tested at day 30 of the animal study is equivalent to the bare metal stent.

In some embodiments, the active agent is at least one of: 50% crystalline, at least 75% crystalline, at least 90% crystalline.

In some embodiments, the polymer comprises a bioabsorbable polymer. In some embodiments, the polymer comprises PLGA. In some embodiments, the polymer comprises PLGA with a ratio of about 40:60 to about 60:40 and further comprises PLGA with a ratio of about 60:40 to about 90:10. In some embodiments, the polymer comprises PLGA having a molecular weight of about 10kD (weight average molecular weight) and wherein the coating further comprises PLGA having a molecular weight of about 19kD (weight average molecular weight). In some embodiments, the polymer is selected from the group: PLGA, a copolymer comprising PLGA (i.e. a PLGA copolymer), a PLGA copolymer with a ratio of about 40:60 to about 60:40, a PLGA copolymer with a ratio of about 70:30 to about 90:10, a PLGA copolymer having a molecular weight of about 10kD (weight average molecular weight), a PLGA copolymer having a molecular weight of about 19kD (weight average molecular weight), a PLGA copolymer having a number average molecular weight of between about 9.5kD and about 25kD, a PLGA copolymer having a number average molecular weight of between about 14.5kD and about 15kD, PGA poly(glycolide), LPLA poly(l-lactide), DLPLA poly(dl-lactide), PCL poly(e-caprolactone) PDO, poly(dioxolane) PGA-TMC, 85/15 DLPLG p(dl-lactide-co-glycolide), 75/25 DLPL, 65/35 DLPLG, 50/50 DLPLG, TMC poly(trimethylcarbonate), poly(anhydrides) such as p(CPP:SA) poly(1,3-bis-p-(carboxyphenoxy)propane-co-sebacic acid), and a combination thereof. As used herein, the term "about," when referring to a copolymer ratio, means variations of any of 0.5%, 1%, 2%, 5%, 10%, 15%, 20%, 25%, 30%, and 50%, depending on the embodiment. For example, a copolymer ratio of 40:60 having a variation of 10% ranges from 35:65 to 45:55, which is a range of 10% of the total (100) about the target. As used herein, the term "about" when referring to a polymer molecular weight means variations of any of 0.5%, 1%, 2%, 5%, 10%, 15%, 20%, 25%, 30%, and 50%, depending on the embodiment. For example, a polymer molecular weight of 10kD (weight average molecular weight) having a variation of 10% ranges from 9kD to 11kD, which is a range of 10% of the target 10kD on either side of the target 10kD.

In some embodiments, the stent is formed from a material comprising the following percentages by weight: about 0.05 to about 0.15 C, about 1.00 to about 2.00 Mn, about 0.04 Si, about 0.03 P, about 0.3 S, about 19.0 to about 21.0 Cr, about 9.0 to about11.0 Ni, about 14.0 to about 16.00 W, about 3.0 Fe, and Bal. Co. In some embodiments, the stent is formed from a material comprising at most the following percentages by weight: about 0.025 C, about 0.15 Mn, about 0.15 Si, about 0.015 P, about 0.01 S, about 19.0 to about 21.0 Cr, about 33 to about 37 Ni, about 9.0 to about 10.5 Mo, about 1.0 Fe, about 1.0 Ti, and Bal. Co. As used herein, the term "about," when referring to a weight percentage of stent material, means variations of any of 0.5%, 1%, 2%, 5%, 1 0%, 15%, 20%, 25%, 30%, and 50% of the total weight percent (i.e. 100%) on either side (+/-) of the weight percentage, depending on the embodiment. For example, a weight percentage of stent material of 3.0 Fe having a variation of 1% ranges from 2.0 to 4.0, which is a range of 1% of the total (100) on either side of the target 3.0.

In some embodiments, the stent has a thickness of from about 50% to about 90% of a total thickness of the device. In some embodiments, the coating has a total thickness of from about 5 µm to about 50 µm. The coating can be conformal around the struts, isolated on the abluminal side, patterned, or otherwise optimized for the target tissue. As used herein, the term "about" when referring to a device thickness or coating thickness means variations of any of 0.5%, 1%, 2%, 5%, 10%, 15%, 20%, 25%, 30%, and 50%, depending on the embodiment. For non-limiting example, a device thickness of 20 µm having a variation of 10% ranges from 18 µm to 22 µm, which is a range of 10% on either side of the target 20 µm. For non-limiting example, a coating thickness of 100 µm having a variation of 10% ranges from 90 µm to 110 µm, which is a range of 10% on either side of the target 100 µm.

In some embodiments, the device has an active agent content of from about 5 µg to about 500 µg. In some embodiments, device has an active agent content of from about 100 µg to about 160 µg. As used herein, the term "about" when referring to a pharmaceutical agent content means variations of any of 0.5%, 1%, 2%, 5%, 10%, 15%, 20%, 25%, 30%, and 50%, depending on the embodiment. For non-limiting example, a pharmaceutical agent content of 120 µg having a variation of 10% ranges from 108 µg to 132 µg, which is a range of 10% on either side of the target 120 µg. Where content is expressed herein in units of µg/mm, however, this may simply be converted to µg/mm2 or another amount per area (e.g., µg/cm2), or vice versa, or converted to a total pharmaceutical content by multiplying by the area or length as needed.

### Examples

The following examples are provided to illustrate selected embodiments. Examples 1 to 38 are not according to the invention.

### Sample Preparation

Generally speaking, coatings on stents, on coupons, or samples prepared for in-vivo models are prepared as below. Nevertheless, modifications for a given analytical method are presented within the examples shown, and/or would be obvious to one having skill in the art. Thus, numerous variations, changes, and substitutions will now occur to those skilled in the art without departing from the invention. It should be understood that various alternatives to the embodiments of the invention described herein and examples provided may be employed in practicing the invention and showing the parameters and/or characteristics described.

### Coatings on Stents

Coated stents as described herein and/or made by a method disclosed herein are prepared. In some examples, the coated stents have a targeted thickness of ∼ 15 microns (which includes a mass fraction and/or weight fraction of active agent that is about 25% to about 30% of the total volume of the coating and/or mass of the coating). In some examples, the coating process is PDPDP (Polymer, sinter, Drug, Polymer, sinter, Drug, Polymer, sinter) using deposition of drug in dry powder form and deposition of polymer particles by RESS methods and equipment described herein. In the illustrations below, resulting coated stents may have a 3-layer coating comprising polymer (for example, PLGA) in the first layer, drug (for example, rapamycin) in a second layer and polymer in the third layer, where a portion of the third layer is substantially drug free (e.g. a sub-layer within the third layer having a thickness equal to a fraction of the thickness of the third layer). As described layer, the middle layer (or drug layer) may be overlapping with one or both first (polymer) and third (polymer) layer. The overlap between the drug layer and the polymer layers is defined by extension of polymer material into physical space largely occupied by the drug. The overlap between the drug and polymer layers may relate to partial packing of the drug particles during the formation of the drug layer. When crystal drug particles are deposited on top of the first polymer layer, voids and or gaps may remain between dry crystal particles. The voids and gaps are available to be occupied by particles deposited during the formation of the third (polymer) layer. Some of the particles from the third (polymer) layer may rest in the vicinity of drug particles in the second (drug) layer. When the sintering step is completed for the third (polymer) layer, the third polymer layer particles fuse to form a continuous film that forms the third (polymer) layer. In some embodiments, the third (polymer) layer however will have a portion along the longitudinal axis of the stent whereby the portion is free of contacts between polymer material and drug particles. The portion of the third layer that is substantially of contact with drug particles can be as thin as 1 nanometer.

Polymer-coated stents having coatings comprising polymer but no drug are made by a method disclosed herein and are prepared having a targeted thickness of, for example,- 5 microns. An example coating process is PPP (PLGA, sinter, PLGA, sinter, PLGA, sinter) using RESS methods and equipment described herein. These polymer-coated stents may be used as control samples in some of the examples herein.

In some examples, the stents are made of a cobalt-chromium alloy and are 5 to 50 mm in length, preferably 9 mm to 30 mm in length, with struts of thickness between 20 and 100 microns, preferably 50-70 microns, measuring from an abluminal surface to a luminal surface, or measuring from a side wall to a side wall. In some examples, the stent may be cut lengthwise and opened to lay flat be visualized and/or assayed using the particular analytical technique provided.

The coating may be removed (for example, for analysis of a coating band and/or coating on a strut, and/or coating on the abluminal surface of a flattened stent) by scraping the coating off using a scalpel, knife or other sharp tool. This coating may be sliced into sections which may be turned 90 degrees and visualized using the surface composition techniques presented herein or other techniques known in the art for surface composition analysis (or other characteristics, such as crystallinity, for example). In this way, what was an analysis of coating composition through a depth when the coating was on the stent or as removed from the stent (i.e. a depth from the abluminal surface of the coating to the surface of the removed coating that once contacted the strut or a portion thereof), becomes a surface analysis of the coating which can, for example, show the layers in the slice of coating, at much higher resolution. Coating removed from the stent may be treated the same way, and assayed, visualized, and/or characterized as presented herein using the techniques described and/or other techniques known to a person of skill in the art.

### Coatings on Coupons

In some examples, samples comprise coupons of glass, metal, e.g. cobalt-chromium, or another substance that are prepared with coatings as described herein, with a plurality of layers as described herein, and/or made by a method disclosed herein. In some examples, the coatings comprise polymer. In some examples, the coatings comprise polymer and active agent. In some examples, the coated coupons are prepared having a targeted thickness of ∼ 10 microns (which includes a mass fraction and/or weight fraction of active agent that is about 25% to about 30% of the total volume of the coating and/or mass of the coating), and have coating layers as described for the coated stent samples, infra.

### Sample Preparation for In-Vivo Models

Devices comprising stents having coatings disclosed herein are implanted in the porcine coronary arteries of pigs (domestic swine, juvenile farm pigs, or Yucatan miniature swine). Porcine coronary stenting is exploited herein since such model yields results that are comparable to other investigations assaying neointimal hyperplasia in human subjects. The stents are expanded to a 1:1.1 balloon:artery ratio. At multiple time points, animals are euthanized (e.g. t = 1 day, 7 days, 14 days, 21 days, and 28 days), the stents are explanted, and assayed.

Devices comprising stents having coatings disclosed herein alternatively are implanted in the common iliac arteries of New Zealand white rabbits. The stents are expanded to a 1:1.1 balloon:artery ratio. At multiple time points, animals are euthanized (e.g., t = 1 day, 7 days, 14 days, 21 days, and 28 days), the stents are explanted, and assayed.

### Example 1.

This example illustrates embodiments that provide a coated coronary stent, comprising: a stent framework and a rapamycin-polymer coating wherein at least part of rapamycin is in crystalline form and the rapamycin-polymer coating comprises one or more resorbable polymers.

In these experiments two different polymers were employed:
Polymer A: - 50:50 PLGA-Ester End Group, MW∼19kD (weight average molecular weight), degradation rate ∼1-2 months
Polymer B: - 50:50 PLGA-Carboxylate End Group, MW∼10kD (weight average molecular weight), degradation rate -28 days

Metal stents were coated as follows:
AS1: Polymer A/Rapamycin/Polymer A/Rapamycin/Polymer A
AS2: Polymer A/Rapamycin/Polymer A/Rapamycin/Polymer B
AS1 (B) or AS 1(213): Polymer B/Rapamycin/Polymer B/Rapamycin/Polymer B
AS1 b: Polymer A/Rapamycin/Polymer A/Rapamycin/Polymer A
AS2b: Polymer A/Rapamycin/Polymer A/Rapamycin/Polymer B

### Example 2. Crystallinity

The presence and or quantification of the Active agent crystallinity can be determined from a number of characterization methods known in the art e.g., XRPD, vibrational spectroscopy (FTIR, NIR, Raman), polarized optical microscopy, calorimetry, thermal analysis and solid-state NMR.

### X-Ray Diffraction to Determine the Presence and/or Quantification of Active Agent Crystallinity

Active agent and polymer coated proxy substrates are prepared using 316L stainless steel coupons for X-ray powder diffraction (XRPD) measurements to determine the presence of crystallinity of the active agent. The coating on the coupons is equivalent to the coating on the stents described herein. Coupons of other materials described herein, such as cobalt-chromium alloys, may be similarly prepared and tested. Likewise, substrates such as stents, or other medical devices described herein may be prepared and tested. Where a coated stent is tested, the stent may be cut lengthwise and opened to lay flat in a sample holder.

For example XRPD analyses are performed using an X-ray powder diffractometer (for example, a Bruker D8 Advance X-ray diffractometer) using Cu Kα radiation. Diffractograms are typically collected between 2 and 40 degrees 2 theta. Where required low background XRPD sample holders are employed to minimize background noise.

The diffractograms of the deposited active agent are compared with diffractograms of known crystallized active agents, for example micronized crystalline sirolimus in powder form. XRPD patterns of crystalline forms show strong diffraction peaks whereas amorphous show diffuse and non-distinct patterns. Crystallinity is shown in arbitrary Intensity units.

A related analytical technique which may also be used to provide crystallinity detection is wide angle scattering of radiation (e.g.; Wide Angle X-ray Scattering or WAXS), for example, as described in F. Unger, et al., "Poly(ethylene carbonate): A thermoelastic and biodegradable biomaterial for drug eluting stent coatings?" Journal of Controlled Release, Volume 117, Issue 3, 312-321 (2007) for which the technique and variations of the technique specific to a particular sample would be obvious to one of skill in the art.

### Raman Spectroscopy

Raman spectroscopy, a vibrational spectroscopy technique, can be useful, for example, in chemical identification, characterization of molecular structures, effects of bonding, identification of solid state form, environment and stress on a sample. Raman spectra can be collected from a very small volume (< 1 µm³); these spectra allow the identification of species present in that volume. Spatially resolved chemical information, by mapping or imaging, terms often used interchangeably, can be achieved by Raman microscopy.

Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), and/or described in Belu et al., "Three-Dimensional Compositional Analysis of Drug Eluting Stent Coatings Using Cluster Secondary Ion Mass Spectroscopy" Anal. Chem. 80: 624-632 (2008) may be used.

For example, to test a sample using Raman microscopy and in particular confocal Raman microscopy, it is understood that to get appropriate Raman high resolution spectra sufficient acquisition time, laser power, laser wavelength, sample step size and microscope objective need to be optimized. For example a sample (a coated stent) is prepared as described herein. Alternatively, a coated coupon could be tested in this method. Maps are taken on the coating using Raman microscopy. A WITec CRM 200 scanning confocal Raman microscope using a Nd:YAG laser at 532 nm is applied in the Raman imaging mode. The laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 seconds of integration time. Each confocal cross-sectional image of the coatings displays a region 70 µm wide by 10 µm deep, and results from the gathering of 6300 spectra with a total imaging time of 32 min.

Multivariate analysis using reference spectra from samples of rapamycin (amorphous and crystalline) and polymer are used to deconvolve the spectral data sets, to provide chemical maps of the distribution.

Raman Spectroscopy may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared
according to the methods described herein, and tested according to the testing methods of Belu- Chemical Imaging.

A WITec CRM 200 scanning confocal Raman microscope (Ulm, Germany) using a NiYAG laser at 532 nm may be applied in Raman imaging mode. The stent sample may be placed upon a piezoelectrically driven table, the laser light focused on the stent coating using a 100x dry objective (Nikon, numerical aperture 0.90), and the finely focused laser spot scanned into the coating. As the laser scans the sample, over each 0.33 micron interval, for example, a Raman spectrum with high signal to noise may be collected using 0.3 s of integration time. Each confocal cross-sectional image of the coatings may display a region 70 micron wide by 10 micron seep, and results from the gathering of 6300 spectra with total imaging time of 32 min. To deconvolute the spectra and obtain separate images of drug (pharmaceutical agent) and polymer, all the spectral data (6300 spectra over the entire spectral region 500-3500 cm-1) may be processed using an augmented classical least squares algorithm (Eigenvector Research, Wenatchee WA) using basis spectra obtained from samples of the drug (e.g. rapamycin amorphous and/or crystalline) and the polymer (e.g. PLGA or other polymer).

For each stent, several areas may be measured by Raman to ensure that the trends are reproducible. Images may be taken on the coatings before elution, and/or at time points following elution. For images taken following elution, stents may be removed from the elution media and dried in a nitrogen stream. A warming step (e.g. 70C for 10 minutes) may be necessary to reduce cloudiness resulting from soaking the coating in the elution media (to reduce and/or avoid light scattering effects when testing by Raman).

### Infrared (IR) Spectroscopy for In-Vitro Testing

Infrared (IR) Spectroscopy such as FTIR and ATR-IR are well utilized techniques that can be applied to show, for example, the quantitative drug content, the distribution of the drug in the sample coating, the quantitative polymer content in the coating, and the distribution of polymer in the coating. Infrared (IR) Spectroscopy such as FTIR and ATR-IR can similarly be used to show, for example, drug crystallinity. The following table (Table 1) lists the typical IR materials for various applications. These IR materials are used for IR windows, diluents or ATR crystals.

**Table 1**

| MATERIAL | NACL | KBR | CSI | AGCL | GE | ZNSE | DIAMOND |
|---|---|---|---|---|---|---|---|
| Transmission range (cm-1) | 40,000 ∼625 | 40,000 ∼400 | 40,000 ∼200 | 25,000 ∼360 | 5,500 ∼625 | 20,000 ∼454 | 40,000 ∼2,500 & 1667-33 |
| Water sol (g/100g, 25C) | 35.7 | 53.5 | 44.4 | Insol. | Insol. | Insol. | Insol. |
| Attacking materials | Wet Solvents | Wet Solvents | Wet Solvents | Ammonium Salts | H2SO4, aqua regin | Acids, strong alkalies, chlorinated solvents | K2Cr2Os, conc. H2SO4 |

In one test, a coupon of crystalline ZnSe is coated by the processes described herein, creating a PDPDP (Polymer, Drug, Polymer, Drug, Polymer) layered coating that is about 10 microns thick. The coated coupon is analyzed using FTIR. The resulting spectrum shows crystalline drug as determined by comparison to the spectrum obtained for the crystalline form of a drug standard (i.e. a reference spectrum).

### Differential Scanning Calorimetry (DSC)

DSC can provide qualitative evidence of the crystallinity of the drug (e.g. rapamycin) using standard DSC techniques obvious to one of skilled in the art. Crystalline melt can be shown using this analytical method (e.g. rapamycin crystalline melting - at about 185 decrees C to 200 degrees C, and having a heat of fusion at or about 46.8 J/g). The heat of fusion decreases with the percent crystallinity. Thus, the degree of crystallinity could be determined relative to a pure sample, or versus a calibration curve created from a sample of amorphous drug spiked and tested by DSC with known amounts of crystalline drug. Presence (at least) of crystalline drug on a stent could be measured by removing (scraping or stripping) some drug from the stent and testing the coating using the DSC equipment for determining the melting temperature and the heat of fusion of the sample as compared to a known standard and/or standard curve.

### Example 3: Determination of Bioabsorbability/Bioresorbability/Dissolution Rate of a Polymer Coating a Device

### Gel Permeation Chromatography In-vivo Weight Loss Determination

Standard methods known in the art can be applied to determine polymer weight loss, for example gel permeation chromatography and other analytical techniques such as described in Jackson et al., "Characterization of perivascular poly(lactic-co-glycolic acid) films containing paclitaxel" Int. J. of Pharmaceutics, 283:97-109 (2004).

For example rabbit *in vivo* models as described above are euthanized at multiple time points (t = 1 day, 2 days, 4 days, 7 days, 14 days, 21 days, 28 days, 35 days n=5 per time point). Alternatively, pig *in vivo* models as described above are euthanized at multiple time points (t =1 day, 2 days, 4 days, 7 days, 14 days, 21 days, 28 days, 35 days n=5 per time point). The stents are explanted, and dried down at 30°C under a stream of gas to complete dryness. A stent that has not been implanted in the animal is used as a control for no loss of polymer.

The remaining polymer on the explanted stents is removed using a solubilizing solvent (for example chloroform). The solutions containing the released polymers for each time point are filtered. Subsequent GPC analysis is used for quantification of the amount of polymer remaining in the stent at each explant time point.. The system, for example, comprises a Shimadzu LC-10 AD HPLC pump, a Shimadzu RID-6A refractive index detector coupled to a 50Å Hewlett Packard Pl-Gel column. The polymer components are detected by refractive index detection and the peak areas are used to determine the amount of polymer remaining in the stents at the explant time point. A calibration graph of log molecular weight versus retention time is established for the 50A Pl-Gel column using polystyrene standards with molecular weights of 300, 600, 1.4k, 9k, 20k, and 30k g/mol. The decreases in the polymer peak areas on the subsequent time points of the study are expressed as weight percentages relative to the 0 day stent.

### Gel Permeation Chromatography In- Vitro testing

Gel Permeation Chromatography (GPC) can also be used to quantify the bioabsorbability/ bioresorbability, dissolution rate, and/or biodegradability of the polymer coating. The *in vitro* assay is a degradation test where the concentration and molecular weights of the polymers can be assessed when released from the stents in an aqueous solution that mimics physiological surroundings. See for example, Jackson et al., "Characterization of perivascular poly(lactic-co-glycolic acid) films containing paclitaxel" Int. J. of Pharmaceutics, 283:97-109 (2004).

For example Stents (n=15) described herein are expanded and then placed in a solution of 1.5 ml solution of phosphate buffered saline (pH = 7.4) with 0.05% wt of Tween20, or in the alternative 10 mM Tris, 0.4 wt.% SDS, pH 7.4, in a 37°C bath with bath rotation at 70 rpm. Alternatively, a coated coupon could be tested in this method. The solution is then collected at the following time points: 0 min., 15 min., 30 min., 1 hr, 2 hr, 4 hr, 6 hr, 8 hr, 12 hr, 16 hr, 20 hr, 24 hr, 30 hr, 36 hr, 48 hr, and daily up to 70 days, for example. The solution is replaced at least at each time point, and/or periodically (e.g. every four hours, daily, weekly, or longer for later time points) to prevent saturation, the removed solution is collected, saved, and assayed. The solutions containing the released polymers for each time point are filtered to reduce clogging the GPC system. For time points over 4 hours, the multiple collected solutions are pooled together for liquid extraction.

1 ml Chloroform is added to the phosphate buffered saline solutions and shaken to extract the released polymers from the aqueous phase. The chloroform phase is then collected for assay via GPC.

The system comprises a Shimadzu LC-10 AD HPLC pump, a Shimadzu RID-6A refractive index (RI) detector coupled to a 50Å Hewlett Packard Pl-Gel column. The mobile phase is chloroform with a flow rate of 1 mL/min. The injection volume of the polymer sample is 100 µL of a polymer concentration. The samples are run for 20 minutes at an ambient temperature.

For determination of the released polymer concentrations at each time point, quantitative calibration graphs are first made using solutions containing known concentrations of each polymer in chloroform. Stock solutions containing each polymer in 0-5mg/ml concentration range are first analyzed by GPC and peak areas are used to create separate calibration curves for each polymer.

For polymer degradation studies, a calibration graph of log molecular weight versus retention time is established for a 50 Å Pl-Gel column (Hewlett Packard) using polystyrene standards with molecular weights of 300, 600, 1.4k, 9k, 20k, and 30k g/mol. In the alternative, a Multi angle light scattering (MALS) detector may be fitted to directly assess the molecular weight of the polymers without the need of polystyrene standards.

To perform an accelerated in-vitro dissolution of the bioresorbable polymers, a protocol is adapted from ISO Standard 13781 "Poly(L-lactide) resides and fabricated an accelerated froms for surgical implants *-in* vitro degradation testing" (1997), incorporated in its entirety herein by reference. Briefly, elution buffer comprising 18% v/v of a stock solution of 0.067 mol/L KH₂PO₄ and 82% v/v of a stock solution of 0.067 mol/L Na₂HPO₄ with a pH of 7.4 is used. Stents described herein are expanded and then placed in 1.5 ml solution of this accelerated elution in a 70°C bath with rotation at 70 rpm. The solutions are then collected at the following time points: 0 min., 15 min., 30 min., 1 hr, 2 hr, 4 hr, 6 hr, 8 hr, 12 hr, 16 hr, 20 hr, 24 hr, 30 hr, 36 hr and 48 hr. Fresh accelerated elution buffer are added periodically every two hours to replace the incubated buffers that are collected and saved in order to prevent saturation. The solutions containing the released polymers for each time point are filtered to reduce clogging the GPC system. For time points over 2 hours, the multiple collected solutions are pooled together for liquid extraction by chloroform. Chloroform extraction and GPC analysis is performed in the manner described above.

### Scanning Electron Microscopy (SEM) with Focused Ion Beam (FIB) Milling In-Vitro Testing

Focused ion beam FIB is a tool that allows precise site-specific sectioning, milling and depositing of materials. FIB can be used in conjunction with SEM, at ambient or cryo conditions, to produce in-situ sectioning followed by high-resolution imaging. FIB -SEM can produce a cross-sectional image of the polymer layers on the stent. The image can be used to quantitate the thickness of the layers to reveal rate of bioresorbability of single or multiple polymers as well as show whether there is uniformity of the layer thickness at manufacture and at time points after stenting (or after in-vitro elution at various time points).

For example, testing is performed at multiple time points. Stents are removed from the elution media and dried, the dried stent is visualized using FIB-SEM for changes in the coating. Alternatively, a coated coupon could be tested in this method.

Stents (n=15) described herein are expanded and then placed in 1.5 ml solution of phosphate buffered saline (pH = 7.4) with 0.05% wt of Tween20 in a 37°C bath with bath rotation at 70 rpm. Alternatively, a coated coupon could be tested in this method. The phosphate buffered saline solution is periodically replaced with fresh solution at each time point and/or every four hours to prevent saturation. The stents are collected at the following time points: 30 min, 1 hr, 2 hr, 4 hr, 6 hr, 8, hr, 12 hr, 16 hr, 20 hr, 24 hr, 30 hr, 36 hr, 48 hr, 60 h and 72 h. The stents are dried down at 30°C under a stream of gas to complete dryness. A stent that not been subjected to these conditions is used as a t = 0 control.

A FEI Dual Beam Strata 235 FIB/SEM system is a combination of a finely focused Ga ion beam (FIB) accelerated by 30 kV with a field emission electron beam in a scanning electron microscope instrument and is used for imaging and sectioning the stents. Both beams focus at the same point of the sample with a probe diameter less than 10nm. The FIB can also produce thinned down sections for TEM analysis.

To prevent damaging the surface of the stent with incident ions, a Pt coating is first deposited via electron beam assisted deposition and ion beam deposition prior to FIB sectioning. For FIB sectioning, the Ga ion beam is accelerated to 30 kV and the sectioning process is about 2 h in duration. Completion of the FIB sectioning allows one to observe and quantify by SEM the thickness of the polymer layers that are left on the stent as they are absorbed.

### Raman Spectroscopy In-Vitro Testing

As discussed in example 2, Raman spectroscopy can be applied to characterize the chemical structure and relative concentrations of drug and polymer coatings. This can also be applied to characterize in-vitro tested polymer coatings on stents or other substrates.

For example, confocal Raman Spectroscopy / microscopy can be used to characterize the relative drug to polymer ratio at the outer ∼ 1µm of the coated surface as a function of time exposed to elution media. In addition confocal Raman x-z or z (maps or line scans) microscopy can be applied to characterize the relative drug to polymer ratio as a function of depth at time t after exposure to elution media.

For example a sample (a coated stent) is prepared as described herein and placed in elution media (e.g., 10 mM tris(hydroxymethyl)aminomethane (Tris), 0.4 wt.% Sodium dodecyl sulphate (SDS), pH 7.4 or 1.5 ml solution of phosphate buffered saline (pH = 7.4) with 0.05% wt of Tween20) in a 37°C bath with bath rotation at 70 rpm. Confocal Raman Images are taken on the coating before elution. At least four elution time points within a 48 day interval, (e.g. 0 min., 15 min., 30 min., 1 hr, 2 hr, 4 hr, 6 hr, 8, hr, 12 hr, 16 hr, 20 hr, 24 hr, 30 hr, 36 hr and 48 hr) the sample is removed from the elution, and dried (for example, in a stream of nitrogen). The dried stent is visualized using Raman Spectroscopy for changes in coating. Alternatively, a coated coupon could be tested in this method. After analysis, each is returned to the buffer for further elution.

Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), and/or described in Belu et al., "Three-Dimensional Compositional Analysis of Drug Eluting Stent Coatings Using Cluster Secondary Ion Mass Spectroscopy" Anal. Chem. 80: 624-632 (2008) may be used.

For example a WITec CRM 200 scanning confocal Raman microscope using a Nd:YAG laser at 532 nm is applied in the Raman imaging mode to generate an x-z map. The sample is placed upon a piezoelectrically driven table, the laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 Seconds of integration time. Each confocal crosssectional image of the coatings displays a region 70 µm wide by 10 µm deep, and results from the gathering of 6300 spectra with a total imaging time of 32 min.

### SEM- In- Vitro Testing

Testing is performed at multiple time points (e.g. 0 min., 15 min., 30 min., 1 hr, 2 hr, 4 hr, 6 hr, 8, hr, 12 hr, 16 hr, 20 hr, 24 hr, 30 hr, 36 hr and 48 hr). Stents are removed from the elution media (described supra) and dried at these time points. The dried stent is visualized using SEM for changes in coating.

For example the samples are observed by SEM using a Hitachi S-4800 with an accelerating voltage of 800V. Various magnifications are used to evaluate the coating integrity, especially at high strain regions. Change in coating over time is evaluated to visualize the bioabsorption of the polymer over time.

### X-rayphotoelectron spectroscopy (XPS)-In- Vitro Testing

XPS can be used to quantitatively determine elemental species and chemical bonding environments at the outer 5-10nm of sample surface. The technique can be operated in spectroscopy or imaging mode. When combined with a sputtering source, XPS can be utilized to give depth profiling chemical characterization.

XPS testing can be used to characterize the drug to polymer ratio at the very surface of the coating of a sample. Additionally XPS testing can be run in time lapse to detect changes in composition. Thus, in one test, samples are tested using XPS at multiple time points (e.g. 0 min., 15 min., 30 min., 1 hr, 2 hr, 4 hr, 6 hr, 8, hr, 12 hr, 16 hr, 20 hr, 24 hr, 30 hr, 36 hr and 48 hr). Stents are removed from the elution media (e.g., 10 mM Tris, 0.4 wt.% SDS, pH 7.4 or 1.5 ml solution of phosphate buffered saline (pH = 7.4) with 0.05% wt of Tween20) in a 37°C bath with rotation at 70 rpm and dried at these time points.

XPS (ESCA) and other analytical techniques such as described in Belu et al., "Three-Dimensional Compositional Analysis of Drug Eluting Stent Coatings Using Cluster Secondary Ion Mass Spectroscopy" Anal. Chem. 80: 624-632 (2008) may be used.

For example, XPS analysis is performed using a Physical Electronics Quantum 2000 Scanning ESCA. The monochromatic Al Kα source is operated at 15 kV with a power of 4.5 W. The analysis is performed at a 45° take off angle. Three measurements are taken along the length of each stent with the analysis area ∼ 20 microns in diameter. Low energy electron and Ar⁺ ion floods are used for charge compensation.

ESCA (among other test methods), may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

ESCA analysis (for surface composition testing) may be done on the coated stents using a Physical Electronics Quantum 2000 Scanning ESCA (e.g. from Chanhassen, MN). The monochromatic AL Ka x-ray source may be operated at 15 kV with a power of 4.5 W. The analysis may be done at a 45degree take-off angle. Three measurements may be taken along the length of each stent with the analysis area about 20 microns in diameter. Low energy electron and Ar+ ion floods may be used for charge compensation. The atomic compositions determined at the surface of the coated stent may be compared to the theoretical compositions of the pure materials to gain insight into the surface composition of the coatings. For example, where the coatings comprise PLGA and Rapamycin, the amount of N detected by this method may be directly correlated to the amount of drug at the surface, whereas the amounts of C and O determined represent contributions from rapamycin, PLGA (and potentially silicone, if there is silicone contamination as there was in Belu- Chemical Imaging). The amount of drug at the surface may be based on a comparison of the detected % N to the pure rapamycin %N. Another way to estimate the amount of drug on the surface may be based on the detected amounts of C and O in ration form %O/%C compared to the amount expected for rapamycin. Another way to estimate the amount of drug on the surface may be based on high resolution spectra obtained by ESCA to gain insight into the chemical state of the C, N, and O species. The C 1 s high resolution spectra gives further insight into the relative amount of polymer and drug at the surface. For both Rapamycin and PLGA (for example), the C 1 s signal can be curve fit with three components: the peaks are about 289.0 eV: 286.9 eV : 284.8 eV, representing O-C=O, C-O and/or C-N, and C-C species, respectively. However, the relative amount of the three C species is different for rapamycin versus PLGA, therefore, the amount of drug at the surface can be estimated based on the relative amount of C species. For each sample, for example, the drug may be quantified by comparing the curve fit area measurements for the coatings containing drug and polymer, to those of control samples of pure drug and pure polymer. The amount of drug may be estimated based on the ratio of O-C=O species to C-C species (e.g. 0.1 for rapamycin versus 1.0 for PLGA).

### Time of Flight Secondary Ion Mass Spectrometry (TOF-SIMS)

TOF-SIMS can be used to determine molecular species at the outer 1-2nm of sample surface when operated under static conditions. The technique can be operated in spectroscopy or imaging mode at high spatial resolution. When operated under dynamic experimental conditions, known in the art, depth profiling chemical characterization can be achieved.

TOF-SIMS testing can be used to characterize the presence of polymer and or drug at uppermost surface of the coating of a sample. Additionally TOF-SIMS testing can be run in time lapse to detect changes in composition. Thus, in one test, samples are tested using TOF-SIMS at multiple time points (e.g., 0 min., 15 min., 30 min., 1 hr, 2 hr, 4 hr, 6 hr, 8, hr, 12 hr, 16 hr, 20 hr, 24 hr, 30 hr, 36 hr and 48 hr). Stents are removed from the elution media (e.g. 10 mM Tris, 0.4 wt.% SDS, pH 7.4 or 1.5 ml solution of phosphate buffered saline (pH = 7.4) with 0.05% wt of Tween20) in a 37°C bath with rotation at 70 rpm and dried at these time points.

For example, to analyze the uppermost surface only, static conditions (for example a ToF-SIMS IV (IonToF, Munster)) using a 25Kv Bi⁺⁺ primary ion source maintained below 10¹² ions per cm² is used. Where necessary a low energy electron flood gun (0.6 nA DC) is used to charge compensate insulating samples.

Cluster Secondary Ion Mass Spectrometry, may be employed for depth profiling as described Belu et al., "Three-Dimensional Compositional Analysis of Drug Eluting Stent Coatings Using Cluster Secondary Ion Mass Spectroscopy" Anal. Chem. 80: 624-632 (2008).

For example, a stent as described herein is obtained. The stent is prepared for SIMS analysis by cutting it longitudinally and opening it up with tweezers. The stent is then pressed into multiple layers of indium foil with the outer diameter facing outward.

TOF-SIMS depth profiling experiments are performed using an Ion-TOF IV instrument equipped with both Bi and SF5+ primary ion beam cluster sources. Sputter depth profiling is performed in the dual-beam mode, while preserving the chemical integrity of the sample. For example, the analysis source is a pulsed, 25-keV bismuth cluster ion source, which bombarded the surface at an incident angle of 45° to the surface normal. The target current is maintained at ∼0.3 pÅ (+10%) pulsed current with a raster size of 200 micron x 200 micron for all experiments. Both positive and negative secondary ions are extracted from the sample into a reflectron-type time-of-flight mass spectrometer. The secondary ions are then detected by a microchannel plate detector with a post-acceleration energy of 10 kV. A low-energy electron flood gun is utilized for charge neutralization in the analysis mode.

The sputter source used is a 5-keV SF5+ cluster source also operated at an incident angle of 45° to the surface normal. For thin model samples on Si, the SF5+ current is maintained at ∼2.7 nÅ with a 750 micron x 750 micron raster. For the thick samples on coupons and for the samples on stents, the current is maintained at 6nA with a 500 micron x 500 micron raster. All primary beam currents are measured with a Faraday cup both prior to and after depth profiling.

All depth profiles are acquired in the noninterlaced mode with a 5-ms pause between sputtering and analysis. Each spectrum is averaged over a 7.37 second time period. The analysis is immediately followed by 15 seconds of SF₅⁺ sputtering. For depth profiles of the surface and subsurface regions only, the sputtering time was decreased to 1 second for the 5% active agent sample and 2 seconds for both the 25% and 50% active agent samples.

Temperature-controlled depth profiles are obtained using a variable-temperature stage with Eurotherm Controls temperature controller and IPSG V3.08 software. Samples are first placed into the analysis chamber at room temperature. The samples are brought to the desired temperature under ultra high-vacuum conditions and are allowed to stabilize for 1 minute prior to analysis. All depth profiling experiments are performed at -100 degrees C and 25 degrees C.

### Infrared (IR) Spectroscopy for In- Vitro Testing

Infrared (IR) Spectroscopy such as FTIR, ATR-IR and micro ATR-IR are well utilized techniques that can be applied to show the quantitative polymer content in the coating, and the distribution of polymer in the coating.

For example using FTIR, a coupon of crystalline ZnSe is coated by the processes described herein, creating a PDPDP (Polymer, Drug, Polymer, Drug, Polymer) layered coating that is about 10 microns thick. At time=0 and at least four elution time points within a 48 day interval (e.g., 0 min., 15 min., 30 min., 1 hr, 2 hr, 4 hr, 6 hr, 8, hr, 12 hr, 16 hr, 20 hr, 24 hr, 30 hr, 36 hr and 48 hr), the sample (coated crystal) was tested by FTIR for polymer content. The sample was placed in an elution media (e.g. 10 mM Tris, 0.4 wt.% SDS, pH 7.4 or 1.5 ml solution of phosphate buffered saline (pH = 7.4) with 0.05% wt of Tween20) in a 37°C bath with bath rotation at 70 rpm and at each time point, the sample is removed from the elution media and dried (e.g. in a stream of nitrogen). FTIR spectrometry was used to quantify the polymer on the sample. After analysis, each is returned to the buffer for further elution.

In another example using FTIR, sample elution media at each time point was tested for polymer content. In this example, a coated stent was prepared that was coated by the processes described herein, creating a PDPDP (Polymer, Drug, Polymer, Drug, Polymer) layered coating that is about 10 microns thick. The coated stent was placed in an elution media (e.g. 10 mM Tris, 0.4 wt.% SDS, pH 7.4 or 1.5 ml solution of phosphate buffered saline (pH = 7.4) with 0.05% wt of Tween20) in a 37°C bath with rotation at 70 rpm. and at each time point (e.g., 0 min., 15 min., 30 min., 1 hr, 2 hr, 4 hr, 6 hr, 8 hr, 12 hr, 16 hr, 20 hr, 24 hr, 30 hr, 36 hr and 48 hr), a sample of the elution media is removed and dried onto a crystalline ZnSe window(e.g. in a stream of nitrogen). At each elution time point, the sample elution media was tested by FTIR for polymer content.

### Atomic Force Microscopy (AFM)

AFM is a high resolution surface characterization technique. AFM is used in the art to provide topographical imaging, in addition when employed in Tapping Mode™ can image material and or chemical properties of the surface. The technique can be used under ambient, solution, humidified or temperature controlled conditions. Other modes of operation are well known and can be readily employed here by those skilled in the art. The AFM topography images can be run in time-lapse to characterize the surface as a function of elution time. Three-dimensionally rendered images show the surface of a coated stent, which can show holes or voids of the coating which may occur as the polymer is absorbed and the drug is eluted over time.

A stent as described herein is obtained. AFM is used to determine the drug polymer distribution. AFM may be employed as described in Ranade et al., "Physical characterization of controlled release of paclitaxel from the TAXUS Express2 drug-eluting stent" J. Biomed. Mater. Res. 71(4):625-634 (2004).

For example a multi-mode AFM (Digital Instruments/Veeco Metrology, Santa Barbara, CA) controlled with Nanoscope IIIa and NanoScope Extender electronics is used. Samples are examined in the dry state using AFM before elution of the drug (e.g. rapamycin). Samples are also examined at select time points through a elution period (e.g. 48 hours) by using an AFM probe-tip and flow-through stage built to permit analysis of wet samples. The wet samples are examined in the presence of the same elution medium used for in-vitro kinetic drug release analysis (e.g. PBS-Tween20, or 10 mM Tris, 0.4 wt.% SDS, pH 7.4). Saturation of the solution is prevented by frequent exchanges of the release medium with several volumes of fresh medium. TappingMode™ AFM imaging may be used to show topography (a real-space projection of the coating surface microstructure) and phase-angle changes of the AFM over the sample area to contrast differences in the material and physical structure.

### Nano X-Ray Computer Tomography

Another technique that may be used to view the physical structure of a device in 3-D is Nano X-Ray Computer Tomography (e.g. such as made by SkyScan), which could be used in an elution test and/or bioabsorbability test, as described herein to show the physical structure of the coating remaining on stents at each time point, as compared to a scan prior to elution/ bioabsorption.

### pH Testing

The bioabsorbability of PLGA of a coated stent can be shown by testing the pH of an elution media (EtOH/PBS, for example) in which the coated stent is placed. Over time, a bioabsorbable PLGA coated stent (with or without the drug) will show a decreased pH until the PLGA is fully bioabsorbed by the elution media.

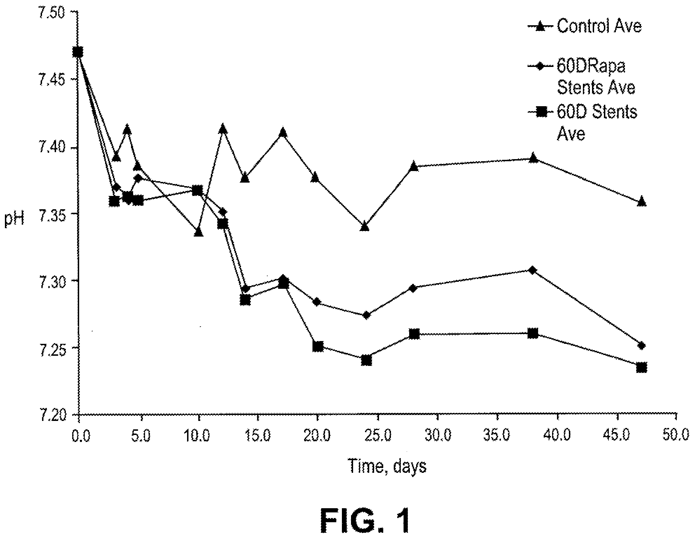

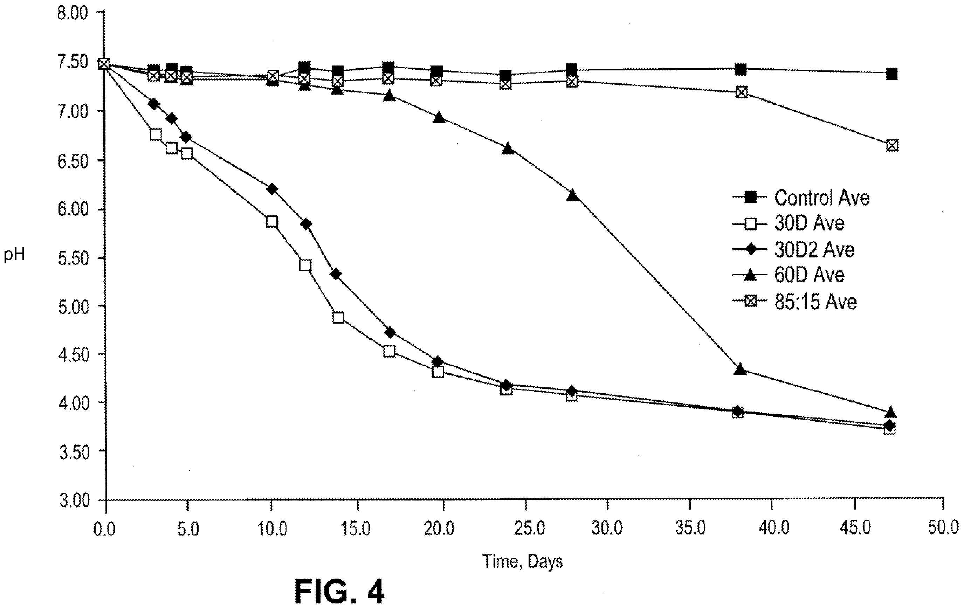

A test was performed using stents coated with PLGA alone, stents coated with PLGA and rapamycin, PLGA films, and PLGA films containing rapamycin. The samples were put in elution media of 20% EtOH/PBS at 37°C. The elution media was tested at multiple intervals from 0 to 48 days. In Figure 1, 2 and 3, stents having coatings as provided herein were tested for pH over time according to this method. Figure 4 shows results of the PLGA films (with and without rapamycin) tested according to this method. Control elution media was run in triplicate alongside the samples, and the results of this pH testing was averaged and is presented as "Control AVE" in each of the Figures 1-4.

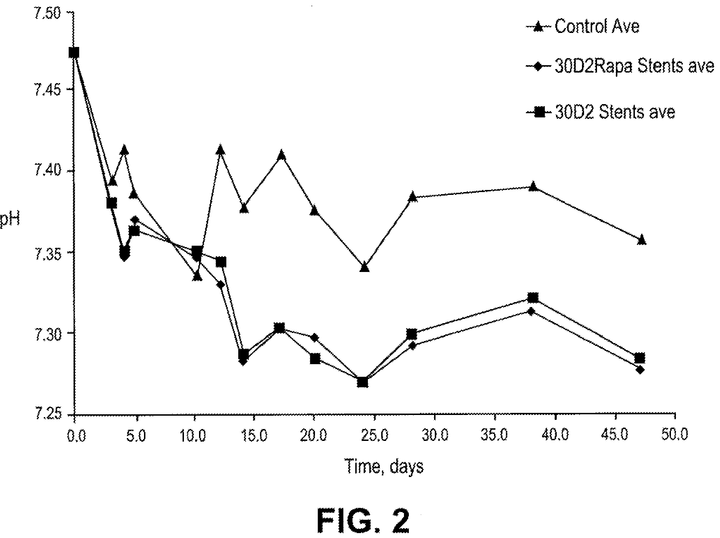

In Figure 2, the "30D2Rapa Stents ave" line represents a stent having coating according to AS1 (213) of Example 1 (PDPDP) with Polymer B (50:50 PLGA-Carboxylate end group, weight average MW ∼10kD) and rapamycin, where the coating was removed from the stent and tested in triplicate for pH changes over time in the elution media, the average of which is presented. The "30D2 Stents ave" line represents a stent having coating of only Polymer B (50:50 PLGA-Carboxylate end group, weight average MW ∼10kD) (no rapamycin), where the coating was removed from the stent and tested in triplicate for pH changes over time in the elution media, the average of which is presented.

In Figure 1, the "60DRapa Stents ave" line represents a stent having coating according to AS1 of Example 1 (PDPDP) with Polymer A (50:50 PLGA-Ester end group, weight average MW ∼19kD) and rapamycin, where the coating was removed from the stent and tested in triplicate for pH changes over time in the elution media, the average of which is presented. The "60D Stents ave" line represents a stent having coating of only Polymer A (50:50 PLGA-Ester end group, weight average MW ∼19kD) (no rapamycin), where the coating was removed from the stent and tested in triplicate for pH changes over time in the elution media, the average of which is presented.

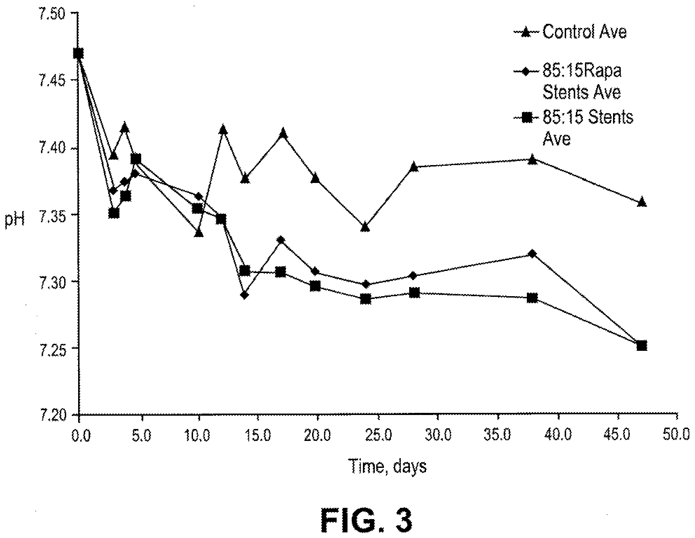

In Figure 3, the "85:15Rapa Stents ave" line represents a stent having coating according to PDPDP with a PLGA comprising 85% lactic acid, 15% glycolic acid, and rapamycin, where the coating was removed from the stent and tested in triplicate for pH changes over time in the elution media, the average of which is presented. The "85:15 Stents ave" line represents a stent having coating of only PLGA comprising 85% lactic acid, 15% glycolic acid (no rapamycin), where the coating was removed from the stent and tested in triplicate for pH changes over time in the elution media, the average of which is presented.

In Figure 4, the "30D Ave" line represents a polymer film comprising Polymer B (50:50 PLGA-Carboxylate end group, weight average MW ∼10kD) (no rapamycin), where the film was tested in triplicate for pH changes over time in the elution media, the average of which is presented. The "30D2 Ave" line also represents a polymer film comprising Polymer B (50:50 PLGA-Carboxylate end group, weight average MW ∼10kD) (no rapamycin), where the film was tested in triplicate for pH changes over time in the elution media, the average of which is presented. The "60D Ave" line represents a polymer film comprising Polymer A (50:50 PLGA-Ester end group, weight average MW ∼19kD) (no rapamycin), where the film was tested in triplicate for pH changes over time in the elution media, the average of which is presented. The "85:15 Ave" line represents a polymer film comprising PLGA comprising 85% lactic acid, 15% glycolic acid (no rapamycin), where the film was tested in triplicate for pH changes over time in the elution media, the average of which is presented. To create the polymer films in Figure 4, the polymers were dissolved in methylene chloride, THF, and ethyl acetate. The films that were tested had the following average thicknesses and masses, 30D - 152.4 um, 12.0mg; 30D2 - 127.0um, 11.9mg; 60D - 50.8um, 12.4mg; 85:15 - 127um, 12.5mg.

### Example 4: Visualization of Polymer/Active Agent Layers Coating a Device

### Raman Spectroscopy

As discussed in example 2, Raman spectroscopy can be applied to characterize the chemical structure and relative concentrations of drug and polymer coatings. For example, confocal Raman Spectroscopy / microscopy can be used to characterize the relative drug to polymer ratio at the outer ∼ 1µm of the coated surface. In addition confocal Raman x-z or z (maps or line scans) microscopy can be applied to characterize the relative drug to polymer ratio as a function of depth. Additionally cross-sectioned samples can be analysed. Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), and/or described in Belu et al., "Three-Dimensional Compositional Analysis of Drug Eluting Stent Coatings Using Cluster Secondary Ion Mass Spectroscopy" Anal. Chem. 80: 624-632 (2008) may be used.

A sample (a coated stent) is prepared as described herein. Images are taken on the coating using Raman Spectroscopy. Alternatively, a coated coupon could be tested in this method. To test a sample using Raman microscopy and in particular confocal Raman microscopy, it is understood that to get appropriate Raman high resolution spectra sufficient acquisition time, laser power, laser wavelength, sample step size and microscope objective need to be optimized.

For example a WITec CRM 200 scanning confocal Raman microscope using a Nd:YAG laser at 532 nm is applied in the Raman imaging mode to give x-z maps. The sample is placed upon a piezoelectrically driven table, the laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 Seconds of integration time. Each confocal cross-sectional image of the coatings displays a region 70 µm wide by 10 µm deep, and results from the gathering of 6300 spectra with a total imaging time of 32 min. Multivariate analysis using reference spectra from samples of rapamycin and polymer are used to deconvolve the spectral data sets, to provide chemical maps of the distribution.

In another test, spectral depth profiles (x-z maps) of samples are performed with a CRM200 microscope system from WITec Instruments Corporation (Savoy, IL). The instrument is equipped with a Nd:YAG frequency doubled laser (532 excitation), a single monochromator (Acton) employing a 600 groove/mm grating and a thermoelectrically cooled 1024 by 128 pixel array CCD camera (Andor Technology). The microscope is equipped with appropriate collection optics that include a holographic laser bandpass rejection filter (Kaiser Optical Systems Inc.) to minimize Rayleigh scatter into the monochromator. The Raman scattered light are collected with a 50 micron optical fiber. Using the "Raman Spectral Imaging" mode of the instrument, spectral images are obtained by scanning the sample in the x, z direction with a piezo driven xyz scan stage and collecting a spectrum at every pixel. Typical integration times are 0.3s per pixel. The spectral images are 4800 total spectra corresponding to a physical scan dimension of 40 by 20 microns. For presentation of the confocal Raman data, images are generated based on unique properties of the spectra (i.e. integration of a Raman band, band height intensity, or band width). The microscope stage is modified with a custom-built sample holder that positioned and rotated the stents around their primary axis. The x direction is defined as the direction running parallel to the length of the stent and the z direction refers to the direction penetrating through the coating from the air-coating to the coating-metal interface. Typical laser power is <10mW on the sample stage. All experiments can be conducted with a plan achromat objective, 100 x N_{A}= 0.9 (Nikon).

Samples (n=5) comprising stents made of L605 (0.05-0.15% C, 1.00-2.00% Mn, maximum 0.040% Si, maximum 0.030% P, maximum 0.3% S, 19.00-21.00% Cr, 9.00-11.00% Ni, 14.00-16.00% W, 3.00% Fe, and Bal. Co) and having coatings as described herein and/or produced by methods described herein can be analyzed. For each sample, three locations are selected along the stent length. The three locations are located within one-third portions of the stents so that the entire length of the stent are represented in the data. The stent is then rotated 180 degrees around the circumference and an additional three locations are sampled along the length. In each case, the data is collected from the strut portion of the stent. Six random spatial locations are also profiled on coated coupon samples made of L605 and having coatings as described herein and/or produced by methods described herein. The Raman spectra of each individual component present in the coatings are also collected for comparison and reference. Using the instrument software, the average spectra from the spectral image data are calculated by selecting the spectral image pixels that are exclusive to each layer. The average spectra are then exported into GRAMS/AI v. 7.02 software (Thermo Galactic) and the appropriate Raman bands are fit to a Voigt function. The band areas and shift positions are recorded.

The pure component spectrum for each component of the coating (e.g. drug, polymer) are also collected at 532 and 785 nm excitation. The 785 nm excitation spectra are collected with a confocal Raman microscope (WITec Instruments Corp. Savoy, IL) equipped with a 785 nm diode laser, appropriate collection optics, and a back-illuminated thermoelectrically cooled 1024 x 128 pixel array CCD camera optimized for visible and infrared wavelengths (Andor Technology).

Raman Spectroscopy may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

A WITec CRM 200 scanning confocal Raman microscope (Ulm, Germany) using a NiYAG laser at 532 nm may be applied in Raman imaging mode. The stent sample may be placed upon a piezoelectrically driven table, the laser light focused on the stent coating using a 100x dry objective (Nikon, numerical aperture 0.90), and the finely focused laser spot scanned into the coating. As the laser scans the sample, over each 0.33 micron interval, for example, a Raman spectrum with high signal to noice may be collected using 0.3 s of integration time. Each confocal cross-sectional image of the coatings may display a region 70 micron wide by 10 micron seep, and results from the gathering of 6300 spectra with total imaging time of 32 min. To deconvolute the spectra and obtain separate images of drug (pharmaceutical agent) and polymer, all the spectral data (6300 spectra over the entire spectral region 500-3500 cm-1) may be processed using an augmented classical least squares algorithm (Eigenvector Research, Wenatchee WA) using basis spectra obtained from samples of the drug (e.g. rapamycin amorphous and/or crystalline) and the polymer (e.g. PLGA or other polymer).

For example, small regions of the stent coating (e.g. 70x 10 microns) imaged in a cross-section perpendicular to the stent may show a dark region above the coating (air), a colored crescent shaped region (coating) and a dark region below the coating (stent). Within the coating region the images may exhibit colors related to the relative Raman signal intensities of the drug (pharmaceutical agent, e.g., or rapamycin, e.g.) and polymer (e.g. PLGA) obtained from deconvolution of the Raman spectrum measured at each image pixel. Overlapping regions may yield various shades of other colors. Color saturation values (threshold values) chosen for visual contrast may show relative changes in signal intensity.

For each stent, several areas may be measured by Raman to ensure that the trends are reproducible. Images may be taken on the coatings before elution, and/or at time points following elution. For images taken following elution, stents may be removed from the elution media and dried in a nitrogen stream. A warming step (e.g. 70C for 10 minutes) may be necessary to reduce cloudiness resulting from soaking the coating in the elution media (to reduce and/or avoid light scattering effects when testing by Raman).

### X-ray photoelectron spectroscopy (XPS)

XPS can be used to quantitatively determine elemental species and chemical bonding environments at the outer 5-10nm of sample surface. The technique can be operated in spectroscopy or imaging mode. When combined with a sputtering source XPS can be utilized to give depth profiling chemical characterization. XPS (ESCA) and other analytical techniques such as described in Belu et al., "Three-Dimensional Compositional Analysis of Drug Eluting Stent Coatings Using Cluster Secondary Ion Mass Spectroscopy" Anal. Chem. 80: 624-632 (2008) may be used.

For example, in one test, a sample comprising a stent coated by methods described herein and/or a device as described herein is obtained. XPS analysis is performed on a sample using a Physical Electronics Quantum 2000 Scanning ESCA. The monochromatic Al Kα source is operated at 15 kV with a power of 4.5 W. The analysis is done at a 45° take off angle. Three measurements are taken along the length of each sample with the analysis area ∼ 20 microns in diameter. Low energy electron and Ar⁺ ion floods are used for charge compensation.

ESCA (among other test methods), may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

ESCA analysis (for surface composition testing) may be done on the coated stents using a Physical Electronics Quantum 2000 Scanning ESCA (e.g. from Chanhassen, MN). The monochromatic AL Ka x-ray source may be operated at 15 kV with a power of 4.5 W. The analysis may be done at a 45degree take-off angle. Three measurements may be taken along the length of each stent with the analysis area about 20 microns in diameter. Low energy electron and Ar+ ion floods may be used for charge compensation. The atomic compositions determined at the surface of the coated stent may be compared to the theoretical compositions of the pure materials to gain insight into the surface composition of the coatings. For example, where the coatings comprise PLGA and Rapamycin, the amount of N detected by this method may be directly correlated to the amount of drug at the surface, whereas the amounts of C and O determined represent contributions from rapamycin, PLGA (and potentially silicone, if there is silicone contamination as there was in Belu- Chemical Imaging). The amount of drug at the surface may be based on a comparison of the detected % N to the pure rapamycin %N. Another way to estimate the amount of drug on the surface may be based on the detected amounts of C and O in ration form %O/%C compared to the amount expected for rapamycin. Another way to estimate the amount of drug on the surface may be based on high resolution spectra obtained by ESCA to gain insight into the chemical state of the C, N, and O species. The C 1 s high resolution spectra gives further insight into the relative amount of polymer and drug at the surface. For both Rapamycin and PLGA (for example), the C 1 s signal can be curve fit with three components: the peaks are about 289.0 eV: 286.9 eV : 284.8 eV, representing O-C=O, C-O and/or C-N, and C-C species, respectively. However, the relative amount of the three C species is different for rapamycin versus PLGA, therefore, the amount of drug at the surface can be estimated based on the relative amount of C species. For each sample, for example, the drug may be quantified by comparing the curve fit area measurements for the coatings containing drug and polymer, to those of control samples of pure drug and pure polymer. The amount of drug may be estimated based on the ratio of O-C=O species to C-C species (e.g. 0.1 for rapamycine versus 1.0 for PLGA).

### Time of Flight Secondary Ion Mass Spectrometry (TOF-SIMS)

TOF-SIMS can be used to determine molecular species (drug and polymer) at the outer 1-2nm of sample surface when operated under static conditions. The technique can be operated in spectroscopy or imaging mode at high spatial resolution. Additionally cross-sectioned samples can be analysed. When operated under dynamic experimental conditions, known in the art, depth profiling chemical characterization can be achieved.

For example, to analyze the uppermost surface only, static conditions (for example a ToF-SIMS IV (IonToF, Munster)) using a 25Kv Bi⁺⁺ primary ion source maintained below 10¹² ions per cm² is used. Where necessary a low energy electron flood gun (0.6 nA DC) is used to charge compensate insulating samples.

Cluster Secondary Ion Mass Spectrometry, may be employed for depth profiling as described Belu et al., "Three-Dimensional Compositional Analysis of Drug Eluting Stent Coatings Using Cluster Secondary Ion Mass Spectroscopy" Anal. Chem. 80: 624-632 (2008).

For example, a stent as described herein is obtained. The stent is prepared for SIMS analysis by cutting it longitudinally and opening it up with tweezers. The stent is then pressed into multiple layers of indium foil with the outer diameter facing outward.

TOF-SIMS depth profiling experiments are performed using an Ion-TOF IV instrument equipped with both Bi and SF5+ primary ion beam cluster sources. Sputter depth profiling is performed in the dual-beam mode, whilst preserving the chemical integrity of the sample. The analysis source is a pulsed, 25-keV bismuth cluster ion source, which bombarded the surface at an incident angle of 45° to the surface normal. The target current is maintained at ∼0.3 pÅ (+10%) pulsed current with a raster size of 200 um x 200 um for all experiments. Both positive and negative secondary ions are extracted from the sample into a reflectron-type time-of-flight mass spectrometer. The secondary ions are then detected by a microchannel plate detector with a post-acceleration energy of 10 kV. A low-energy electron flood gun is utilized for charge neutralization in the analysis mode.

The sputter source used is a 5-keV SF5+ cluster source also operated at an incident angle of 45° to the surface normal. For thin model samples on Si, the SF5+ current is maintained at ∼2.7 nÅ with a 750 um x 750 um raster. For the thick samples on coupons and for the samples on stents, the current is maintained at 6nA with a 500 um x 500 um raster. All primary beam currents are measured with a Faraday cup both prior to and after depth profiling.

All depth profiles are acquired in the noninterlaced mode with a 5-ms pause between sputtering and analysis. Each spectrum is averaged over a 7.37 second time period. The analysis is immediately followed by 15 seconds of SF₅⁺ sputtering. For depth profiles of the surface and subsurface regions only, the sputtering time was decreased to 1 second for the 5% active agent sample and 2 seconds for both the 25% and 50% active agent samples.

Temperature-controlled depth profiles are obtained using a variable-temperature stage with Eurotherm Controls temperature controller and IPSG V3.08 software. Samples are first placed into the analysis chamber at room temperature. The samples are brought to the desired temperature under ultra high-vacuum conditions and are allowed to stabilize for 1 minute prior to analysis. All depth profiling experiments are performed at -100C and 25C.

TOF-SIMS may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

TOF-SIMS depth profiling studies may be performed on an ION-TOF instrument (e.g. Muenster, Germany). The depth profiles may be obtained on coupons and/or stents, to allow development of proper instrumental conditions. The instrument may employ a 5 KeV SF+5 source which is sputtered over a 500 micron x 500 micron area with 6nA continuous current. Initial depth profiles may be obtained using a 25 keV Ga+ analytical source with 2 pA pulsed current. Further experiments may be done using a 25 keV Bi+3 analytical source with 0.3- 0.4 pA pulsed current. The analytical source may be rastered over 200 micron x 200 microns. The depth profiles may be done in the non-interlaced mode. A low energy electron flood gun may be used for charge neutralization. All depth profiled may be done at -100C (an optimum temperature for depth profiling with SF+5). Sputter rates may be determined from thin model films of each formulation (about 200 nm) cast on Si wafers. After sputtering through the film on the substrate, the crater depth may be measured by stylus profilometry (tencor Instruments alpha-step 200 with a 10-mg stylus force, Milpitas, CA). The average sputter rates may be calculated for each formulation. The experiments may need to be performed at low temperatures (e.g. 100C) to maintain the integrity of the drug and/or polymer while eroding through them. Additionally, there may be adjustments needed to account for damage accumulation rates that occur with higher drug concentrations.

### Atomic Force Microscopy (AFM)

AFM is a high resolution surface characterization technique. AFM is used in the art to provide topographical imaging, in addition when employed in Tapping Mode™ can image material and or chemical properties of the surface. Additionally cross-sectioned samples can be analyzed. The technique can be used under ambient, solution, humidified or temperature controlled conditions. Other modes of operation are well known and can be readily employed here by those skilled in the art.

A stent as described herein is obtained. AFM is used to determine the structure of the drug polymer layers. AFM may be employed as described in Ranade et al., "Physical characterization of controlled release of paclitaxel from the TAXUS Express2 drug-eluting stent" J. Biomed. Mater. Res. 71(4):625-634 (2004).

Polymer and drug morphologies, coating composition, at least may be determined using atomic force microscopy (AFM) analysis. A multi-mode AFM (Digital Instruments/Veeco Metrology, Santa Barbara, CA) controlled with Nanoscope IIIa and NanoScope Extender electronics is used. Samples are examined in the dry state using AFM before elution of the drug (e.g. rapamycin). Samples are also examined at select time points through a elution period (e.g. 48 hours) by using an AFM probe-tip and flow-through stage built to permit analysis of wet samples. The wet samples are examined in the presence of the same elution medium used for in-vitro kinetic drug release analysis (e.g. PBS-Tween20, or 10 mM Tris, 0.4 wt.% SDS, pH 7.4). Saturation of the solution is prevented by frequent exchanges of the release medium with several volumes of fresh medium. TappingMode™ AFM imaging may be used to show topography (a real-space projection of the coating surface microstructure) and phase-angle changes of the AFM over the sample area to contrast differences in the materials properties. The AFM topography images can be three-dimensionally rendered to show the surface of a coated stent, which can show holes or voids of the coating which may occur as the polymer is absorbed and the drug is eluted over time, for example.

### Scanning Electron Microscopy (SEM) with Focused Ion Beam (FIB) Milling

Stents as described herein, and or produced by methods described herein are visualized using SEM-FIB. Alternatively, a coated coupon could be tested in this method. Focused ion beam FIB is a tool that allows precise site-specific sectioning, milling and depositing of materials. FIB can be used in conjunction with SEM, at ambient or cryo conditions, to produce in-situ sectioning followed by high-resolution imaging . FIB -SEM can produce a cross-sectional image of the polymer and drug layers on the stent. The image can be used to quantitate the thickness of the layers and uniformity of the layer thickness at manufacture and at time points after stenting (or after in-vitro elution at various time points).

A FEI Dual Beam Strata 235 FIB/SEM system is a combination of a finely focused Ga ion beam (FIB) accelerated by 30 kV with a field emission electron beam in a scanning electron microscope instrument and is used for imaging and sectioning the stents. Both beams focus at the same point of the sample with a probe diameter less than 10nm. The FIB can also produce thinned down sections for TEM analysis.

To prevent damaging the surface of the stent with incident ions, a Pt coating is first deposited via electron beam assisted deposition and ion beam deposition prior to FIB sectioning. For FIB sectioning, the Ga ion beam is accelerated to 30 kV and the sectioning process is about 2 h in duration. Completion of the FIB sectioning allows one to observe and quantify by SEM the thickness of the polymer layers that are, for example, left on the stent as they are absorbed.

### Example 5: Analysis of the Thickness of a Device Coating

Analysis can be determined by either in-situ analysis or from cross-sectioned samples.

### X-ravphotoelectron spectroscopy (XPS)

XPS can be used to quantitatively determine the presence of elemental species and chemical bonding environments at the outer 5-10nm of sample surface. The technique can be operated in spectroscopy or imaging mode. When combined with a sputtering source XPS can be utilized to give depth profiling chemical characterization. XPS (ESCA) and other analytical techniques such as described in Belu et al., "Three-Dimensional Compositional Analysis of Drug Eluting Stent Coatings Using Cluster Secondary Ion Mass Spectroscopy" Anal. Chem. 80: 624-632 (2008).

Thus, in one test, a sample comprising a stent coated by methods described herein and/or a device as described herein is obtained. XPS analysis is done on a sample using a Physical Electronics Quantum 2000 Scanning ESCA. The monochromatic Al Kα source is operated at 15 kV with a power of 4.5 W. The analysis is done at a 45° take off angle. Three measurements are taken along the length of each sample with the analysis area ∼ 20 microns in diameter. Low energy electron and Ar⁺ ion floods are used for charge compensation.

### Time of Flight Secondary Ion Mass Spectrometry

TOF-SIMS can be used to determine molecular species (drug and polymer) at the outer 1-2nm of sample surface when operated under static conditions. The technique can be operated in spectroscopy or imaging mode at high spatial resolution. Additionally cross-sectioned samples can be analysed. When operated under dynamic experimental conditions, known in the art, depth profiling chemical characterization can be achieved.

For example, under static conditions (for example a ToF-SIMS IV (IonToF, Munster)) using a 25Kv Bi⁺⁺ primary ion source maintained below 10¹² ions per cm² is used. Where necessary a low energy electron flood gun (0.6 nA DC) is used to charge compensate insulating samples.

Cluster Secondary Ion Mass Spectrometry, may be employed for depth profiling as described Belu et al., "Three-Dimensional Compositional Analysis of Drug Eluting Stent Coatings Using Cluster Secondary Ion Mass Spectroscopy" Anal. Chem. 80: 624-632 (2008).

A stent as described herein is obtained. The stent is prepared for SIMS analysis by cutting it longitudinally and opening it up with tweezers. The stent is then pressed into multiple layers of iridium foil with the outer diameter facing outward.

TOF-SIMS experiments are performed on an Ion-TOF IV instrument equipped with both Bi and SF5+ primary ion beam cluster sources. Sputter depth profiling is performed in the dual-beam mode. The analysis source is a pulsed, 25-keV bismuth cluster ion source, which bombarded the surface at an incident angle of 45° to the surface normal. The target current is maintained at ∼0.3 pÅ (+10%) pulsed current with a raster size of 200 um x 200 um for all experiments. Both positive and negative secondary ions are extracted from the sample into a reflectron-type time-of-flight mass spectrometer. The secondary ions are then detected by a microchannel plate detector with a post-acceleration energy of 10 kV. A low-energy electron flood gun is utilized for charge neutralization in the analysis mode.

The sputter source used is a 5-keV SF5+ cluster source also operated at an incident angle of 45° to the surface normal. For thin model samples on Si, the SF5+ current is maintained at ∼2.7 nÅ with a 750 um x 750 um raster. For the thick samples on coupons and for the samples on stents, the current is maintained at 6nA with a 500 um x 500 um raster. All primary beam currents are measured with a Faraday cup both prior to and after depth profiling.

All depth profiles are acquired in the noninterlaced mode with a 5-ms pause between sputtering and analysis. Each spectrum is averaged over a 7.37 second time period. The analysis is immediately followed by 15 seconds of SF₅⁺ sputtering. For depth profiles of the surface and subsurface regions only, the sputtering time was decreased to 1 second for the 5% active agent sample and 2 seconds for both the 25% and 50% active agent samples.

Temperature-controlled depth profiles are obtained using a variable-temperature stage with Eurotherm Controls temperature controller and IPSG V3.08 software, samples are first placed into the analysis chamber at room temperature. The samples are brought to the desired temperature under ultra high-vacuum conditions and are allowed to stabilize for 1 minute prior to analysis. All depth profiling experiments are performed at -100C and 25C.

TOF-SIMS may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

TOF-SIMS depth profiling studies may be performed on an ION-TOF instrument (e.g. Muenster, Germany). The depth profiles may be obtained on coupons and/or stents, to allow development of proper instrumental conditions. The instrument may employ a 5 KeV SF+5 source which is sputtered over a 500 micron x 500 micron area with 6nA continuous current. Initial depth profiles may be obtained using a 25 keV Ga+ analytical source with 2 pA pulsed current. Further experiments may be done using a 25 keV Bi+3 analytical source with 0.3- 0.4 pA pulsed current. The analytical source may be rastered over 200 micron x 200 microns. The depth providles may be done in the non-interlaced mode. A low energy electron flood gun may be used for charge neutralization. All depth profiled may be done at -100C (an optimum temperature for depth profiling with SF+5). Sputter rates may be determined from thin model films of each formulation (about 200 nm) cast on Si wafers. After sputtering through the film on the substrate, the crater depth may be measured by stylus profilometry (tencor Instruments alpha-step 200 with a 10-mg stylus force, Milpitas, CA). The average sputter rates may be calculated for each formulation. The experiments may need to be performed at low temperatures (e.g. 100C) to maintain the integrity of the drug and/or polymer while eroding through them. Additionally, there may be adjustments needed to account for damage accumulation rates that occur with higher drug concentrations.

### Atomic Force Microscopy (AFM)

AFM is a high resolution surface characterization technique. AFM is used in the art to provide topographical imaging, in addition when employed in Tapping Mode™ can image material and or chemical properties of the surface. Additionally cross-sectioned samples can be analyzed.

A stent as described herein is obtained. AFM may be alternatively be employed as described in Ranade et al., "Physical characterization of controlled release of paclitaxel from the TAXUS Express2 drug-eluting stent" J. Biomed. Mater. Res. 71(4):625-634 (2004).

Polymer and drug morphologies, coating composition, and cross-sectional thickness at least may be determined using atomic force microscopy (AFM) analysis. A multi-mode AFM (Digital Instruments/Veeco Metrology, Santa Barbara, CA) controlled with Nanoscope IIIa and NanoScope Extender electronics is usedTappingMode™ AFM imaging may be used to show topography (a real-space projection of the coating surface microstructure) and phase-angle changes of the AFM over the sample area to contrast differences in the materials properties. The AFM topography images can be three-dimensionally rendered to show the surface of a coated stent or cross-section.

### Scanning Electron Microscopy (SEM with Focused Ion Beam (FIB)

Stents as described herein, and or produced by methods described herein are visualized using SEM-FIB analysis. Alternatively, a coated coupon could be tested in this method. Focused ion beam FIB is a tool that allows precise site-specific sectioning, milling and depositing of materials. FIB can be used in conjunction with SEM, at ambient or cryo conditions, to produce in-situ sectioning followed by high-resolution imaging . FIB -SEM can produce a cross-sectional image of the polymer layers on the stent. The image can be used to quantitate the thickness of the layers as well as show whether there is uniformity of the layer thickness at manufacture and at time points after stenting (or after in-vitro elution at various time points).

A FEI Dual Beam Strata 235 FIB/SEM system is a combination of a finely focused Ga ion beam (FIB) accelerated by 30 kV with a field emission electron beam in a scanning electron microscope instrument and is used for imaging and sectioning the stents. Both beams focus at the same point of the sample with a probe diameter less than 10nm. The FIB can also produce thinned down sections for TEM analysis.

To prevent damaging the surface of the stent with incident ions, a Pt coating is first deposited via electron beam assisted deposition and ion beam deposition prior to FIB sectioning. For FIB sectioning, the Ga ion beam is accelerated to 30 kV and the sectioning process is about 2 h in duration. Completion of the FIB sectioning allows one to observe and quantify by SEM the thickness of the polymer layers that are, for example, left on the stent as they are absorbed.

### Interferometry

Interferometry may additionally and/or alternatively used to determine the thickness of the coating as noted in Belu et al., "Three-Dimensional Compositional Analysis of Drug Eluting Stent Coatings Using Cluster Secondary Ion Mass Spectroscopy" Anal. Chem. 80: 624-632 (2008) incorporated herein in its entirety by reference may be used.

Interferometry may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

Interferometry may be done to test coating thickness on the coated stents using a Wyco NT1100 instrument from, for example, Veeco Instruments (Santa Barbara, CA) using a 20x objective with 2x zoom. A refractive index (RI) value of 1.4 may be used to determine the coating thicknesses. The RI value is estimated from product literature values for the RI of the particular polymer (e..g. poly lactic acid 1.35-1.45, Natureworks LLC; monomers lactic acid 1.42, glycolic acid 1.41, Sigma-Aldrich Corp.). Data may be obtained over an area of about 50 microns by 300 microns, and the average thickness may be calculated over this area. Measurements may be taken at, for example, 3-5 locations along the length of the stent (end, 1, ¼, ½, ¾, end, for example).

### Ellipsometry

Ellipsometry is sensitive measurement technique for coating analysis on a coupon. It uses polarized light to probe the dielectric properties of a sample. Through an analysis of the state of polarization of the light that is reflected from the sample the technique allows the accurate characterization of the layer thickness and uniformity. Thickness determinations ranging from a few angstroms to tens of microns are possible for single layers or multilayer systems. See, for example, Jewell, et al., "Release of Plasmid DNA from Intravascular Stents Coated with Ultrathin Multilayered Polyelectrolyte Films" Biomacromolecules. 7: 2483-2491 (2006) incorporated herein in its entirety by reference.

### Example 6: Analysis of the Thickness of a Device

### Scanning Electron Microscopy (SEM)

A sample coated stent described herein is obtained. Thickness of the device can be assessed using this analytical technique. The thickness of multiple struts were taken to ensure reproducibility and to characterize the coating and stent. The thickness of the coating was observed by SEM using a Hitachi S-4800 with an accelerating voltage of 800V. Various magnifications are used. SEM can provide top-down and cross-section images at various magnifications.

### Nano X-Rav Computer Tomography

Another technique that may be used to view the physical structure of a device in 3-D is Nano X-Ray Computer Tomography (e.g. such as made by SkyScan).

### Example 7: Determination of the Type or Composition of a Polymer Coating a Device

### Nuclear Magnetic Resonance (NMR)

Composition of the polymer samples before and after elution can be determined by ¹H NMR spectrometry as described in Xu et al., "Biodegradation of poly(1-lactide-co-glycolide tube stents in bile" Polymer Degradation and Stability. 93:811-817 (2008). Compositions of polymer samples are determined for example using a 300M Bruker spectrometer with d-chloroform as solvent at room temperature.

### Raman Spectroscopy

FT- Raman or confocal raman microscopy can be employed to determine composition.

For example, a sample (a coated stent) is prepared as described herein. Images are taken on the coating using Raman Spectroscopy. Alternatively, a coated coupon could be tested in this method. To test a sample using Raman microscopy and in particular confocal Raman microscopy, it is understood that to get appropriate Raman high resolution spectra sufficient acquisition time, laser power, laser wavelength, sample step size and microscope objective need to be optimized. Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), incorporated in its entirety herein by reference, and/or described in Belu et al., "Three-Dimensional Compositional Analysis of Drug Eluting Stent Coatings Using Cluster Secondary Ion Mass Spectroscopy" Anal. Chem. 80: 624-632 (2008) incorporated herein in its entirety by reference may be used.

For example a WITec CRM 200 scanning confocal Raman microscope using a Nd:YAG laser at 532 nm is applied in the Raman imaging mode. The sample is placed upon a piezoelectrically driven table, the laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 Seconds of integration time. Each confocal crosssectional image of the coatings displays a region 70 µm wide by 10 µm deep, and results from the gathering of 6300 spectra with a total imaging time of 32 min. Multivariate analysis using reference spectra from samples of rapamycin (amorphous and crystalline) and polymer references are used to deconvolve the spectral data sets, to provide chemical maps of the distribution.

In another test, spectral depth profiles of samples are performed with a CRM200 microscope system from WITec Instruments Corporation (Savoy, IL). The instrument is equipped with a NdYAG frequency doubled laser (532 excitation), a single monochromator (Acton) employing a 600 groove/mm grating and a thermoelectrically cooled 1024 by 128 pixel array CCD camera (Andor Technology). The microscope is equipped with appropriate collection optics that include a holographic laser bandpass rejection filter (Kaiser Optical Systems Inc.) to minimize Rayleigh scatter into the monochromator. The Raman scattered light are collected with a 50 micron optical fiber. Using the "Raman Spectral Imaging" mode of the instrument, spectral images are obtained by scanning the sample in the x, z direction with a piezo driven xyz scan stage and collecting a spectrum at every pixel. Typical integration times are 0.3s per pixel. The spectral images are 4800 total spectra corresponding to a physical scan dimension of 40 by 20 microns. For presentation of the confocal Raman data, images are generated based on unique properties of the spectra (i.e. integration of a Raman band, band height intensity, or band width). The microscope stage is modified with a custom-built sample holder that positioned and rotated the stents around their primary axis. The x direction is defined as the direction running parallel to the length of the stent and the z direction refers to the direction penetrating through the coating from the air-coating to the coating-metal interface. Typical laser power is <10mW on the sample stage. All experiments can be conducted with a plan achromat objective, 100 x N_{A}= 0.9 (Nikon).

Samples (n=5) comprising stents made of L605 and having coatings as described herein and/or produced by methods described herein can be analyzed. For each sample, three locations are selected along the stent length. The three locations are located within one-third portions of the stents so that the entire length of the stent are represented in the data. The stent is then rotated 180 degrees around the circumference and an additional three locations are sampled along the length. In each case, the data is collected from the strut portion of the stent. Six random spatial locations are also profiled on coated coupon samples made of L605 and having coatings as described herein and/or produced by methods described herein. The Raman spectra of each individual component present in the coatings are also collected for comparison and reference. Using the instrument software, the average spectra from the spectral image data are calculated by selecting the spectral image pixels that are exclusive to each layer. The average spectra are then exported into GRAMS/AI v. 7.02 software (Thermo Galactic) and the appropriate Raman bands are fit to a Voigt function. The band areas and shift positions are recorded.

The pure component spectrum for each component of the coating (e.g. drug, polymer) are also collected at 532 and 785 nm excitation. The 785 nm excitation spectra are collected with a confocal Raman microscope (WITec Instruments Corp. Savoy, IL) equipped with a 785 nm diode laser, appropriate collection optics, and a back-illuminated thermoelectrically cooled 1024 x 128 pixel array CCD camera optimized for visible and infrared wavelengths (Andor Technology).

Raman Spectroscopy may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). The method may be adapted to compare the results of the testing to various known polymers and drugs. Where needed, coated stents and/or coated coupons may be prepared according to the methods described herein, and tested according to the testing methods of Belu- Chemical Imaging.

A WITec CRM 200 scanning confocal Raman microscope (Ulm, Germany) using a NiYAG laser at 532 nm may be applied in Raman imaging mode. The stent sample may be placed upon a piezoelectrically driven table, the laser light focused on the stent coating using a 100x dry objective (Nikon, numerical aperture 0.90), and the finely focused laser spot scanned into the coating. As the laser scans the sample, over each 0.33 micron interval, for example, a Raman spectrum with high signal to noice may be collected using 0.3 s of integration time. Each confocal cross-sectional image of the coatings may display a region 70 micron wide by 10 micron seep, and results from the gathering of 6300 spectra with total imaging time of 32 min. To deconvolute the spectra and obtain separate images of drug (pharmaceutical agent) and polymer, all the spectral data (6300 spectra over the entire spectral region 500-3500 cm-1) may be processed using an augmented classical least squares algorithm (Eigenvector Research, Wenatchee WA) using basis spectra obtained from samples of the drug (e.g. rapamycin amorphous and/or crystalline) and the polymer (e.g. PLGA or other polymer).

For example, small regions of the stent coating (e.g. 70x 10 microns) imaged in a cross-section perpendicular to the stent may show a dark region above the coating (air), a colored crescent shaped region (coating) and a dark region below the coating (stent). Within the coating region the images may exhibit colors related to the relative Raman signal intensities of the drug (pharmaceutical agent, e.g., or rapamycin, e.g.) and polymer (e.g. PLGA) obtained from deconvolution of the Raman spectrum measured at each image pixel. Overlapping regions may yield various shades of other colors. Color saturation values (threshold values) chosen for visual contrast may show relative changes in signal intensity.

For each stent, several areas may be measured by Raman to ensure that the trends are reproducible. Images may be taken on the coatings before elution, and/or at time points following elution. For images taken following elution, stents may be removed from the elution media and dried in a nitrogen stream. A warming step (e.g. 70C for 10 minutes) may be necessary to reduce cloudiness resulting from soaking the coating in the elution media (to reduce and/or avoid light scattering effects when testing by Raman).

### Time of Flight Secondary Ion Mass Spectrometry

TOF-SIMS can be used to determine molecular species (drug and polymer) at the outer 1-2nm of sample surface when operated under static conditions. The technique can be operated in spectroscopy or imaging mode at high spatial resolution. Additionally cross-sectioned samples can be analysed. When operated under dynamic experimental conditions, known in the art, depth profiling chemical characterization can be achieved.

For example, under static conditions (for example a ToF-SIMS IV (IonToF, Munster)) using a 25Kv Bi⁺⁺ primary ion source maintained below 10¹² ions per cm² is used. Where necessary a low energy electron flood gun (0.6 nA DC) is used to charge compensate insulating samples.

Cluster Secondary Ion Mass Spectrometry, may be employed as described Belu et al., "Three-Dimensional Compositional Analysis of Drug Eluting Stent Coatings Using Cluster Secondary Ion Mass Spectroscopy" Anal. Chem. 80: 624-632 (2008).

A stent as described herein is obtained. The stent is prepared for SIMS analysis by cutting it longitudinally and opening it up with tweezers. The stent is then pressed into multiple layers of iridium foil with the outer diameter facing outward.

TOF-SIMS experiments are performed on an Ion-TOF IV instrument equipped with both Bi and SF5+ primary ion beam cluster sources. Sputter depth profiling is performed in the dual-beam mode. The analysis source is a pulsed, 25-keV bismuth cluster ion source, which bombarded the surface at an incident angle of 45° to the surface normal. The target current is maintained at ∼0.3 pÅ (+10%) pulsed current with a raster size of 200 um x 200 um for all experiments. Both positive and negative secondary ions are extracted from the sample into a reflectron-type time-of-flight mass spectrometer. The secondary ions are then detected by a microchannel plate detector with a post-acceleration energy of 10 kV. A low-energy electron flood gun is utilized for charge neutralization in the analysis mode.

The sputter source used is a 5-keV SF5+ cluster source also operated at an incident angle of 45° to the surface normal. For thin model samples on Si, the SF5+ current is maintained at ∼2.7 nÅ with a 750 um x 750 um raster. For the thick samples on coupons and for the samples on stents, the current is maintained at 6nA with a 500 um x 500 um raster. All primary beam currents are measured with a Faraday cup both prior to and after depth profiling.

All depth profiles are acquired in the noninterlaced mode with a 5-ms pause between sputtering and analysis. Each spectrum is averaged over a 7.37 second time period. The analysis is immediately followed by 15 seconds of SF₅⁺ sputtering. For depth profiles of the surface and subsurface regions only, the sputtering time was decreased to 1 second for the 5% active agent sample and 2 seconds for both the 25% and 50% active agent samples.

Temperature-controlled depth profiles are obtained using a variable-temperature stage with Eurotherm Controls temperature controller and IPSG V3.08 software, samples are first placed into the analysis chamber at room temperature. The samples are brought to the desired temperature under ultra high-vacuum conditions and are allowed to stabilize for 1 minute prior to analysis. All depth profiling experiments are performed at -100C and 25C.

TOF-SIMS may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

TOF-SIMS depth profiling studies may be performed on an ION-TOF instrument (e.g. Muenster, Germany). The depth profiles may be obtained on coupons and/or stents, to allow development of proper instrumental conditions. The instrument may employ a 5 KeV SF+5 source which is sputtered over a 500 micron x 500 micron area with 6nA continuous current. Initial depth profiles may be obtained using a 25 keV Ga+ analytical source with 2 pA pulsed current. Further experiments may be done using a 25 keV Bi+3 analytical source with 0.3- 0.4 pA pulsed current. The analytical source may be rastered over 200 micron x 200 microns. The depth providles may be done in the non-interlaced mode. A low energy electron flood gun may be used for charge neutralization. All depth profiled may be done at -100C (an optimum temperature for depth profiling with SF+5). Sputter rates may be determined from thin model films of each formulation (about 200 nm) cast on Si wafers. After sputtering through the film on the substrate, the crater depth may be measured by stylus profilometry (tencor Instruments alpha-step 200 with a 10-mg stylus force, Milpitas, CA). The average sputter rates may be calculated for each formulation. The experiments may need to be performed at low temperatures (e.g. 100C) to maintain the integrity of the drug and/or polymer while eroding through them. Additionally, there may be adjustments needed to account for damage accumulation rates that occur with higher drug concentrations.

### Atomic Force Microscopy (AFM)

AFM is a high resolution surface characterization technique. AFM is used in the art to provide topographical imaging, in addition when employed in Tapping Mode™ can image material and or chemical properties of the surface. Additionally cross-sectioned samples can be analyzed. Coating composition may be determined using Tapping Mode™ atomic force microscopy (AFM) analysis. Other modes of operation are well known and can be employed here by those skilled in the art.

A stent as described herein is obtained. AFM may be employed as described in Ranade et al., "Physical characterization of controlled release of paclitaxel from the TAXUS Express2 drug-eluting stent" J. Biomed. Mater. Res. 71(4):625-634 (2004).

Polymer and drug morphologies, coating composition, at least may be determined using atomic force microscopy (AFM) analysis. A multi-mode AFM (Digital Instruments/Veeco Metrology, Santa Barbara, CA) controlled with Nanoscope IIIa and NanoScope Extender electronics is used. TappingMode™ AFM imaging may be used to show topography (a real-space projection of the coating surface microstructure) and phase-angle changes of the AFM over the sample area to contrast differences in the materials properties.

### Infrared (IR) Spectroscopy for In-Vitro Testing

Infrared (IR) Spectroscopy using FTIR, ATR-IR or micro ATR-IR can be used to identify polymer composition by comparison to standard polymer reference spectra.

### Example 8: Determination of the Bioabsorbability of a Device

In some embodiments of the device the substrate coated itself is made of a bioabsorbable material, such as the bioabsorbable polymers presented herein, or another bioabsorbable material such as magnesium and, thus, the entire device is bioabsorbable. Techniques presented with respect to showing Bioabsorbability of a polymer coating may be used to additionally and/or alternatively show the bioabsorbability of a device, for example, by GPC In-Vivo testing, HPLC In-Vivo Testing, GPC In-Vitro testing, HPLC In-Vitro Testing, SEM-FIB Testing, Raman Spectroscopy, SEM, and XPS as described herein with variations and adjustments which would be obvious to those skilled in the art. Another technique to view the physical structure of a device in 3-D is Nano X-Ray Computer Tomography (e.g. such as made by SkyScan), which could be used in an elution test and/or bioabsorbability test, as described herein to show the physical structure of the coating remaining on stents at each time point, as compared to a scan prior to elution/ bioabsorption.

### Example 9: Determination of Secondary Structures Presence of a Biological Agent

### Raman Spectroscopy

FT- Raman or confocal raman microscopy can be employed to determine secondary structure of a biological Agent. For example fitting of the Amide I, II, or III regions of the Raman spectrum can elucidate secondary structures (e.g. alpha-helices, beta-sheets). See, for example, Iconomidou, et al., "Secondary Structure of Chorion Proteins of the Teleosetan Fish Dentex dentex by ATR FR-IR and FT-Raman Spectroscopy" J. of Structural Biology, 132, 112-122 (2000); Griebenow, et al., "On Protein Denaturation in Aqueous-Organic Mixtures but Not in Pure Organic Solvents" J. Am. Chem. Soc., Vol 118, No. 47, 11695-11700 (1996).

### Infrared (IR) Spectroscopy for In-Vitro Testing

Infrared spectroscopy, for example FTIR, ATR-IR and micro ATR-IR can be employed to determine secondary structure of a biological Agent. For example fitting of the Amide I, II, of III regions of the infrared spectrum can elucidate secondary structures (e.g. alpha-helices, beta-sheets).

### Example 10: Determination of the Microstructure of a Coating on a Medical Device

### Atomic Force Microscopy (AFM)

AFM is a high resolution surface characterization technique. AFM is used in the art to provide topographical imaging, in addition when employed in Tapping Mode™ can image material and or chemical properties of the surface. Additionally cross-sectioned samples can be analyzed. The technique can be used under ambient, solution, humidified or temperature controlled conditions. Other modes of operation are well known and can be readily employed here by those skilled in the art.

A stent as described herein is obtained. AFM is used to determine the microstructure of the coating. A stent as described herein is obtained. AFM may be employed as described in Ranade et al., "Physical characterization of controlled release of paclitaxel from the TAXUS Express2 drug-eluting stent" J. Biomed. Mater. Res. 71(4):625-634 (2004).

For example, polymer and drug morphologies, coating composition, and physical structure may be determined using atomic force microscopy (AFM) analysis. A multi-mode AFM (Digital Instruments/Veeco Metrology, Santa Barbara, CA) controlled with Nanoscope IIIa and NanoScope Extender electronics is used. Samples are examined in the dry state using AFM before elution of the drug (e.g. rapamycin). Samples are also examined at select time points through a elution period (e.g. 48 hours) by using an AFM probe-tip and flow-through stage built to permit analysis of wet samples. The wet samples are examined in the presence of the same elution medium used for in-vitro kinetic drug release analysis (e.g. PBS-Tween20, or 10 mM Tris, 0.4 wt.% SDS, pH 7.4). Saturation of the solution is prevented by frequent exchanges of the release medium with several volumes of fresh medium. TappingMode™ AFM imaging may be used to show topography (a real-space projection of the coating surface microstructure) and phase-angle changes of the AFM over the sample area to contrast differences in the materials properties. The AFM topography images can be three-dimensionally rendered to show the surface of a coated stent, which can show holes or voids of the coating which may occur as the polymer is absorbed and the drug is released from the polymer over time, for example.

### Nano X-Ray Computer Tomography

Another technique that may be used to view the physical structure of a device in 3-D is Nano X-Ray Computer Tomography (e.g. such as made by SkyScan), which could be used in an elution test and/or bioabsorbability test, as described herein to show the physical structure of the coating remaining on stents at each time point, as compared to a scan prior to elution/ bioabsorption.

### Example 11: Determination of an Elution Profile

### In vitro

**Example 11a:** In one method, a stent described herein is obtained. The elution profile is determined as follows: stents are placed in 16mL test tubes and 15 mL of 10mM PBS (pH 7.4) is pipetted on top. The tubes are capped and incubated at 37C with end-over-end rotation at 8 rpm. Solutions are then collected at the designated time points (e.g. 1d, 7d, 14d, 21d, and 28d) (e.g. 1 week, 2 weeks, and 10 weeks) and replenished with fresh 1.5 ml solutions at each time point to prevent saturation. One mL of DCM is added to the collected sample of buffer and the tubes are capped and shaken for one minute and then centrifuged at 200 x G for 2 minutes. The supernatant is discarded and the DCM phase is evaporated to dryness under gentle heat (40°C) and nitrogen gas. The dried DCM is reconstituted in 1 mL of 60:40 acetonitrile:water (v/v) and analyzed by HPLC. HPLC analysis is performed using Waters HPLC system (mobile phase 58:37:5 acetonitrile:water:methanol 1 mL/min, 20uL injection, C18 Novapak Waters column with detection at 232 nm).

**Example 11b:** In another method, the in vitro pharmaceutical agent elution profile is determined by a procedure comprising contacting the device with an elution media comprising ethanol (5%) wherein the pH of the media is about 7.4 and wherein the device is contacted with the elution media at a temperature of about 37°C. The elution media containing the device is optionally agitating the elution media during the contacting step. The device is removed (and/or the elution media is removed) at least at designated time points (e.g. 1h, 3h, 5h, 7h, 1d or 24 hrs, and daily up to 28d) (e.g. 1 week, 2 weeks, and 10 weeks). The elution media is then assayed using a UV-Vis for determination of the pharmaceutical agent content. The elution media is replaced at each time point with fresh elution media to avoid saturation of the elution media. Calibration standards containing known amounts of drug were also held in elution media for the same durations as the samples and used at each time point to determine the amount of drug eluted at that time (in absolute amount and as a cumulative amount eluted).

In one test, devices were coated tested using this method. In these experiments two different polymers were employed: Polymer A: - 50:50 PLGA-Ester End Group, weight average MW∼19kD, degradation rate ∼70 days; Polymer B: - 50:50 PLGA-Carboxylate End Group, weight average MW∼10kD, degradation rate ∼28 days. Metal stents were coated as follows: AS1: (n=6) Polymer A/Rapamycin/Polymer A/Rapamycin/Polymer A; AS2: (n=6) Polymer A/Rapamycin/Polymer A/Rapamycin/Polymer B; AS1 (213): (n=6) Polymer B/Rapamycin/Polymer B/Rapamycin/Polymer B; AS1b: (n=6) Polymer A/Rapamycin/Polymer A/Rapamycin/Polymer A; AS2b: (n=6) Polymer A/Rapamycin/Polymer A/Rapamycin/Polymer B. The in vitro pharmaceutical agent elution profile was determined by contacting each device with an elution media comprising ethanol (5%) wherein the pH of the media is about 7.4 and wherein the device was contacted with the elution media at a temperature of about 37°C. The elution media was removed from device contact at least at 1h, 3h, 5h, 7h, Id, and at additional time points up to 70 days (See Figures 5-8). The elution media was then assayed using a UV-Vis for determination of the pharmaceutical agent content (in absolute amount and cumulative amount eluted). The elution media was replaced at each time point with fresh elution media to avoid saturation of the elution media. Calibration standards containing known amounts of drug were also held in elution media for the same durations as the samples and assayed by UV-Vis at each time point to determine the amount of drug eluted at that time (in absolute amount and as a cumulative amount eluted), compared to a blank comprising Spectroscopic grade ethanol (95%). Elution profiles as shown in Figures 5-8, showing the average amount of rapamycin eluted at each time point (average of all stents tested) in micrograms. Table 2 shows for each set of stents (n=6) in each group (AS1, AS2, AS(213), AS1b, AS2b), the average amount of rapamycin in ug loaded on the stents, the average amount of polymer in ug loaded on the stents, and the total amount of rapamycin and polymer in ug loaded on the stents.

**Table 2**

| **Stent Coating** | **Ave. Rapa, ug** | **Ave. Poly, ug** | **Ave. Total Mass, ug** |
|---|---|---|---|
| AS1 | 175 | 603 | 778 |
| AS2 | 153 | 717 | 870 |
| AS1(213) | 224 | 737 | 961 |
| AS1b | 171 | 322 | 493 |
| AS2b | 167 | 380 | 547 |

Figure 5: Rapamycin Elution Profile of coated stents (PLGA/Rapamycin coatings) where the elution profile was determined by a static elution media of 5% EtOH/water, pH 7.4, 37°C via UV-Vis test method as described in Example 11b of coated stents described therein.

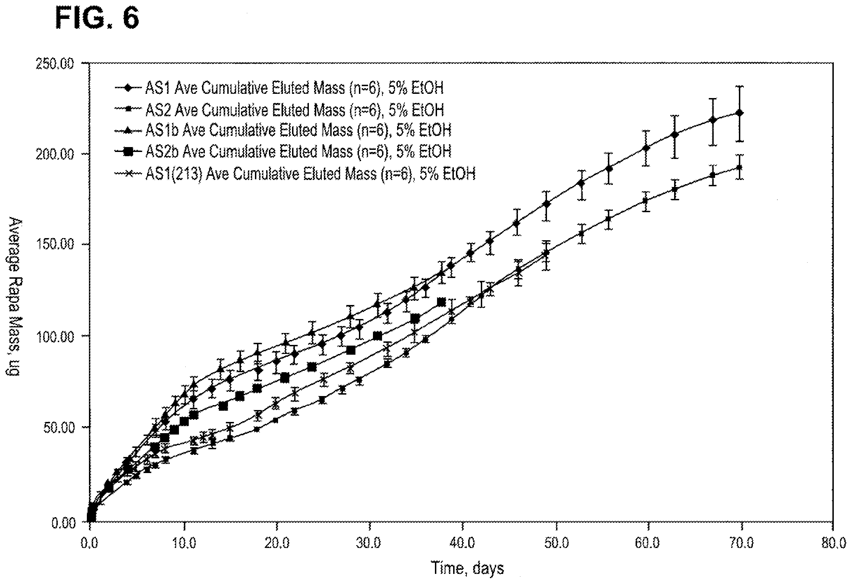

Figure 6: Rapamycin Elution Profile of coated stents (PLGA/Rapamycin coatings) where the elution profile was determined by static elution media of 5% EtOH/water, pH 7.4, 37°C via a UV-Vis test method as described in Example 11b of coated stents described therein. Figure 6 depicts AS1 and AS2 as having statistically different elution profiles; AS2 and AS2b have statistically different profiles; AS 1 and AS1b are not statistically different; and AS2 and AS1(213) begin to converge at 35 days. Figure 6 suggests that the coating thickness does not affect elution rates form 3095 polymer, but does affect elution rates from the 213 polymer.

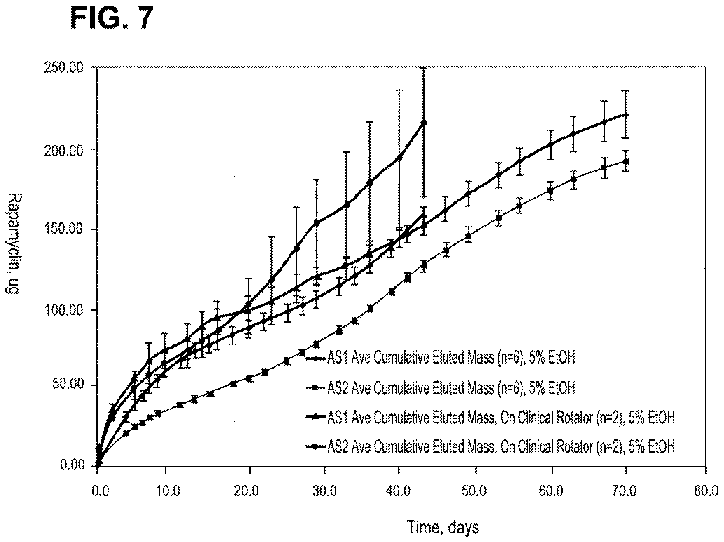

Figure 7: Rapamycin Elution Rates of coated stents (PLGA/Rapamycin coatings) where the static elution profile was compared with agitated elution profile by an elution media of 5% EtOH/water, pH 7.4, 37°C via a UV-Vis test method a UV-Vis test method as described in Example 11b of coated stents described therein. Figure 7 depicts that agitation in elution media increases the rate of elution for AS2 stents, but is not statistically significantly different for AS1 stents. The profiles are based on two stent samples.

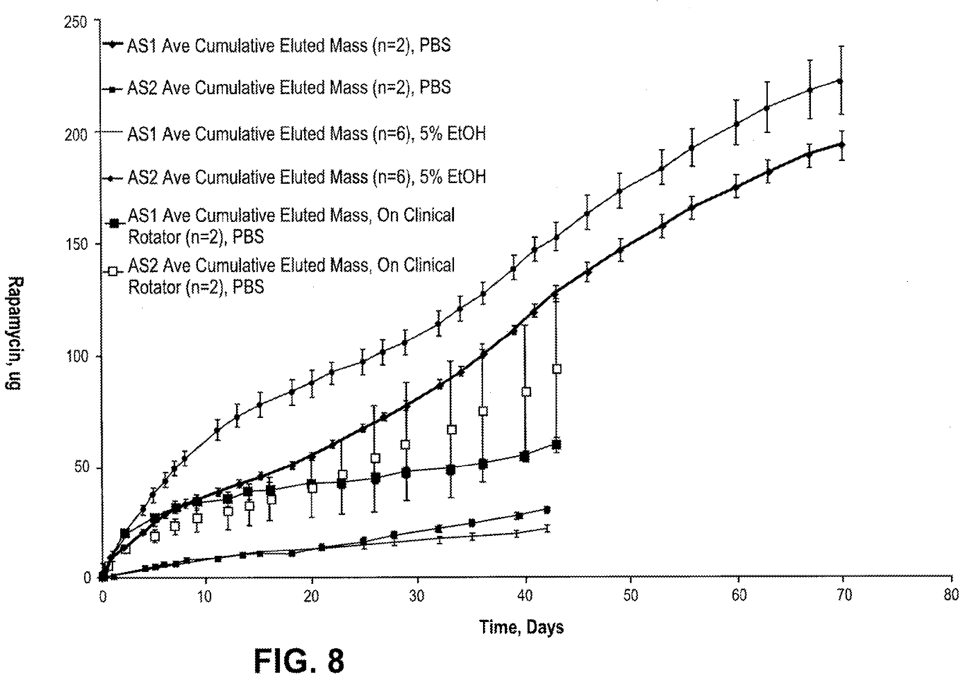

Figure 8 Rapamycin Elution Profile of coated stents (PLGA/Rapamycin coatings) where the elution profile by 5% EtOH/water, pH 7.4, 37°C elution buffer was compare with the elution profile using phosphate buffer saline pH 7.4, 37°C; both profiles were determined by a UV-Vis test method as described in Example 11b of coated stents described therein. Figure 8 depicts that agitating the stent in elution media increases the elution rate in phosphate buffered saline, but the error is much greater.

**Example 11c:** In another method, the in vitro pharmaceutical agent elution profile is determined by a procedure comprising contacting the device with an elution media comprising ethanol (20%) and phosphate buffered saline (80%) wherein the pH of the media is about 7.4 and wherein the device is contacted with the elution media at a temperature of about 37°C. The elution media containing the device is optionally agitating the elution media during the contacting step. The device is removed (and/or the elution media is removed) at least at designated time points (e.g. 1h, 3h, 5h, 7h, 1d, and daily up to 28d) (e.g. 1 week, 2 weeks, and 10 weeks). The elution media is replaced periodically (at least at each time point, and/or daily between later time points) to prevent saturation; the collected media are pooled together for each time point. The elution media is then assayed for determination of the pharmaceutical agent content using HPLC. The elution media is replaced at each time point with fresh elution media to avoid saturation of the elution media. Calibration standards containing known amounts of drug are also held in elution media for the same durations as the samples and used at each time point to determine the amount of drug eluted at that time (in absolute amount and as a cumulative amount eluted). Where the elution method changes the drug over time, resulting in multiple peaks present for the drug when tested, the use of these calibration standards will also show this change, and allows for adding all the peaks to give the amount of drug eluted at that time period (in absolute amount and as a cumulative amount eluted).

In one test, devices (n=9, laminate coated stents) as described herein were coated and tested using this method. In these experiments a single polymer was employed: Polymer A: 50:50 PLGA-Ester End Group, weight average MW∼19kD. The metal (stainless steel) stents were coated as follows: Polymer A/Rapamycin/Polymer A/Rapamycin/Polymer A, and the average amount of rapamycin on each stent was 162 ug (stdev 27ug). The coated stents were contacted with an elution media (5.00 mL) comprising ethanol (20%) and phosphate buffered saline wherein the pH of the media is about 7.4 (adjusted with potassium carbonate solution - 1g/100mL distilled water) and wherein the device is contacted with the elution media at a temperature of about 37°C+/- 0.2°C. The elution media containing the device was agitated in the elution media during the contacting step. The elution media was removed at least at time points of 1h, 3h, 5h, 7h, 1d, and daily up to 28d. The elution media was assayed for determination of the pharmaceutical agent (rapamycin) content using HPLC. The elution media was replaced at each time point with fresh elution media to avoid saturation of the elution media. Calibration standards containing known amounts of drug were also held in elution media for the same durations as the samples and assayed at each time point to determine the amount of drug eluted at that time (in absolute amount and as a cumulative amount eluted). The multiple peaks present for the rapamycin (also present in the calibration standards) were added to give the amount of drug eluted at that time period (in absolute amount and as a cumulative amount eluted). HPLC analysis is performed using Waters HPLC system, set up and run on each sample as provided in the Table 3 below using an injection volume of 100uL.

**Table 3**

| Time point (minutes) | % Acetonitrile | % Ammonium Acetate (0.5%), pH 7.4 | Flow Rate (mL/min) |
|---|---|---|---|
| 0.00 | 10 | 90 | 1.2 |
| 1.00 | 10 | 90 | 1.2 |
| 12.5 | 95 | 5 | 1.2 |
| 13.5 | 100 | 0 | 1.2 |
| 14.0 | 100 | 0 | 3 |
| 16.0 | 100 | 0 | 3 |
| 17.0 | 10 | 90 | 2 |
| 20.0 | 10 | 90 | 0 |

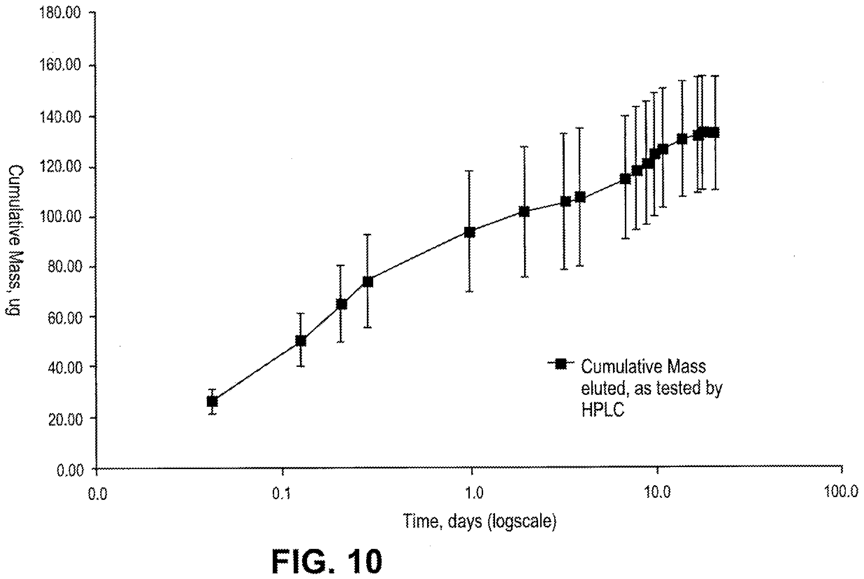

Figure 9 elution profiles resulted, showing the average cumulative amount of rapamycin eluted at each time point (average of n=9 stents tested) in micrograms. Figure 9 depicts Rapamycin Elution Profile of coated stents (PLGA/Rapamycin coatings) where the elution profile was determined by a 20% EtOH/phosphate buffered saline, pH 7.4, 37°C elution buffer and a HPLC test method as described in Example 11c described therein, wherein the elution time (x-axis) is expressed linearly. Figure 10 also expresses the same elution profile, graphed on a logarithmic scale (x-axis is log(time)). Figure 10 depicts Rapamycin Elution Profile of coated stents (PLGA/Rapamycin coatings) where the elution profile was determined by a 20% EtOH/phosphate buffered saline, pH 7.4, 37°C elution buffer and a HPLC test method as described in Example 11c of described therein, , wherein the elution time (x-axis) is expressed in logarithmic scale (i.e., log(time)).

**Example 11d:** To obtain an accelerated *in-vitro* elution profile, an accelerated elution buffer comprising 18% v/v of a stock solution of 0.067 mol/L KH2PO4 and 82% v/v of a stock solution of 0.067 mol/L Na2HPO4 with a pH of 7.4 is used. Stents described herein are expanded and then placed in 1.5 ml solution of this accelerated elution in a 70°C bath with rotation at 70 rpm. The solutions are then collected at the following time points: 0 min., 15 min., 30 min., 1 hr, 2 hr, 4 hr, 6 hr, 8 hr, 12 hr, 16 hr, 20 hr, 24 hr, 30 hr, 36 hr and 48 hr. Fresh accelerated elution buffer are added periodically at least at each time point to replace the incubated buffers that are collected and saved in order to prevent saturation. For time points where multiple elution media are used (refreshed between time points), the multiple collected solutions are pooled together for liquid extraction by dichloromethane. Dichloromethane extraction and HPLC analysis is performed in the manner described previously.

**Example 11e:** In another method, the in vitro pharmaceutical agent elution profile is determined by a procedure comprising contacting the device with an elution media comprising 1:1 spectroscopic grade ethanol (95%) / phosphate buffer saline wherein the pH of the media is about 7.4 and wherein the device is contacted with the elution media at a temperature of about 37°C. The elution media containing the device is optionally agitating the elution media during the contacting step. The device is removed (and/or the elution media is removed) at least at designated time points, e.g. 1h (day 0), 24 hrs (day 1.0), and optionally daily up to 28d, or other time points, as desired. The elution media is then assayed using a UV-Vis at 278 nm by a diode array spectrometer or determination of the pharmaceutical agent content. The elution media is replaced at each time point with fresh elution media to avoid saturation of the elution media. Calibration standards containing known amounts of drug were also held in elution media for the same durations as the samples and used at each time point to determine the amount of drug eluted at that time (in absolute amount and as a cumulative amount eluted).

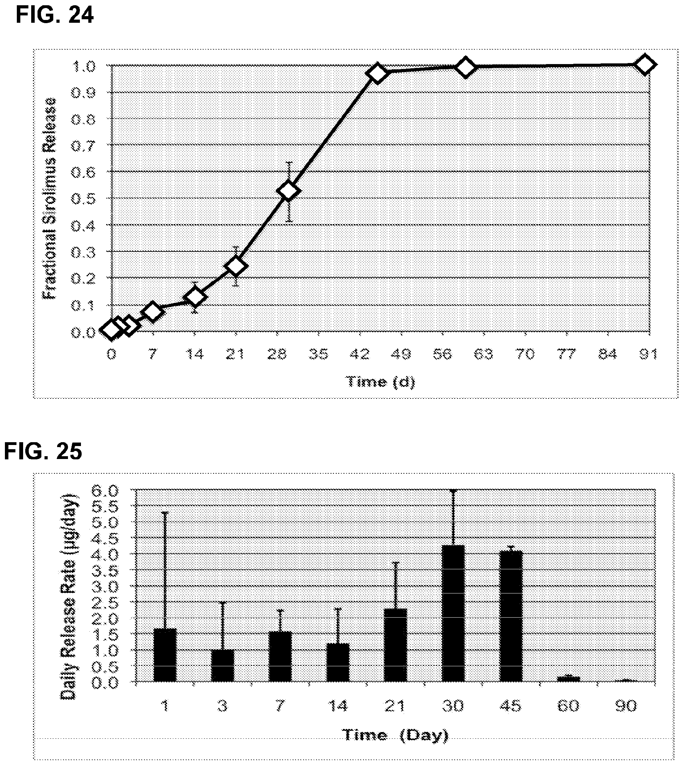

This test method was used to test stents coated as described in Examples 26, 27, and 28, results for which are depicted in Figures 24, 25, and 26, respectively.

### In vivo

**Example 11f:** Rabbit in vivo models as described above are euthanized at multiple time points. Stents are explanted from the rabbits. The explanted stents are placed in 16mL test tubes and 15 mL of 10mM PBS (pH 7.4) is pipette on top. One mL of DCM is added to the buffer and the tubes are capped and shaken for one minute and then centrifuged at 200 x G for 2 minutes. The supernatant is discarded and the DCM phase is evaporated to dryness under gentle heat (40°C) and nitrogen gas. The dried DCM is reconstituted in 1 mL of 60:40 acetonitrile:water (v/v) and analyzed by HPLC. HPLC analysis is performed using Waters HPLC system (mobile phase 58:37:5 acetonitrile:water:methanol 1 mL/min, 20uL injection, C18 Novapak Waters column with detection at 232 nm).

### Example 12: Determination of the Conformability (Conformality) of a Device Coating

The ability to uniformly coat arterial stents with controlled composition and thickness using electrostatic capture in a rapid expansion of supercritical solution (RESS) experimental series has been demonstrated.

### Scanning Electron Microscopy (SEM)

Stents are observed by SEM using a Hitachi S-4800 with an accelerating voltage of 800V. Various magnifications are used to evaluate the integrity, especially at high strain regions. SEM can provide top-down and cross-section images at various magnifications. Coating uniformity and thickness can also be assessed using this analytical technique.

Pre- and post-expansions stents are observed by SEM using a Hitachi S-4800 with an accelerating voltage of 800V. Various magnifications are used to evaluate the integrity of the layers, especially at high strain regions.

### Scanning Electron Microscopy (SEM) with Focused Ion Beam (FIB)

Stents as described herein, and/or produced by methods described herein, are visualized using SEM-FIB analysis. Alternatively, a coated coupon could be tested in this method. Focused ion beam FIB is a tool that allows precise site-specific sectioning, milling and depositing of materials. FIB can be used in conjunction with SEM, at ambient or cryo conditions, to produce in-situ sectioning followed by high-resolution imaging .Cross-sectional FIB images may be acquired, for example, at 7000x and/or at 20000x magnification. An even coating of consistent thickness is visible.

### Optical Microscopy

An Optical microscope may be used to create and inspect the stents and to empirically survey the coating of the substrate (e.g. coating uniformity). Nanoparticles of the drug and/or the polymer can be seen on the surfaces of the substrate using this analytical method. Following sintering, the coatings can be see using this method to view the coating conformality and for evidence of crystallinity of the drug.

### Example 13: Determination of the Total Content of the Active Agent

Determination of the total content of the active agent in a coated stent may be tested using techniques described herein as well as other techniques obvious to one of skill in the art, for example using GPC and HPLC techniques to extract the drug from the coated stent and determine the total content of drug in the sample.

UV-VIS can be used to quantitatively determine the mass of rapamycin coated onto the stents. A UV-Vis spectrum of Rapamycin can be shown and a Rapamycin calibration curve can be obtained, (e.g. λ @ 277nm in ethanol). Rapamycin is then dissolved from the coated stent in ethanol, and the drug concentration and mass calculated.

In one test, the total amount of rapamycin present in units of micrograms per stent is determined by reverse phase high performance liquid chromatography with UV detection (RP-HPLC-UV). The analysis is performed with modifications of literature-based HPLC methods for rapamycin that would be obvious to a person of skill in the art. The average drug content of samples (n=10) from devices comprising stents and coatings as described herein, and/or methods described herein are tested.

### Example 14: Determination of the Extent of Aggregation of an Active Agent

### Raman Spectroscopy

Confocal Raman microscopy can be used to characterize the drug aggregation by mapping in the x-y or x-z direction. Additionally cross-sectioned samples can be analysed. Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), and/or described in Belu et al., "Three-Dimensional Compositional Analysis of Drug Eluting Stent Coatings Using Cluster Secondary Ion Mass Spectroscopy" Anal. Chem. 80: 624-632 (2008) may be used.

A sample (a coated stent) is prepared as described herein. Images are taken on the coating using Raman Spectroscopy. Alternatively, a coated coupon could be tested in this method. A WITec CRM 200 scanning confocal Raman microscope using a NiYAG laser at 532 nm is applied in the Raman imaging mode. The sample is place upon a piezoelectrically driven table, the laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 Seconds of integration time. Each confocal crosssectional image of the coatings displays a region 70 µm wide by 10 µm deep, and results from the gathering of 6300 spectra with a total imaging time of 32 min. To deconvolute the spectra and obtain separate images of the active agent and the polymer, all the spectral data (6300 spectra over the entire spectral region 500-3500 cm-1) are processed using an augmented classical least squares algorithm (Eigenvector Research, Wenatchee WA) using basis spectra obtained from samples of rapamycin (amorphous and crystalline) and polymer. For each sample, several areas are measured by Raman to ensure that results are reproducible, and to show layering of drug and polymer through the coating. Confocal Raman Spectroscopy can profile down micron by micron, can show the composition of the coating through the thickness of the coating.

Raman Spectroscopy may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

A WITec CRM 200 scanning confocal Raman microscope (Ulm, Germany) using a NiYAG laser at 532 nm may be applied in Raman imaging mode. The stent sample may be placed upon a piezoelectrically driven table, the laser light focused on the stent coating using a 100x dry objective (Nikon, numerical aperture 0.90), and the finely focused laser spot scanned into the coating. As the laser scans the sample, over each 0.33 micron interval, for example, a Raman spectrum with high signal to noice may be collected using 0.3 s of integration time. Each confocal cross-sectional image of the coatings may display a region 70 micron wide by 10 micron seep, and results from the gathering of 6300 spectra with total imaging time of 32 min. To deconvolute the spectra and obtain separate images of drug (pharmaceutical agent) and polymer, all the spectral data (6300 spectra over the entire spectral region 500-3500 cm-1) may be processed using an augmented classical least squares algorithm (Eigenvector Research, Wenatchee WA) using basis spectra obtained from samples of the drug (e.g. rapamycin amorphous and/or crystalline) and the polymer (e.g. PLGA or other polymer).

For example, small regions of the stent coating (e.g. 70x 10 microns) imaged in a cross-section perpendicular to the stent may show a dark region above the coating (air), a colored crescent shaped region (coating) and a dark region below the coating (stent). Within the coating region the images may exhibit colors related to the relative Raman signal intensities of the drug (pharmaceutical agent, e.g., or rapamycin, e.g.) and polymer (e.g. PLGA) obtained from deconvolution of the Raman spectrum measured at each image pixel. Overlapping regions may yield various shades of other colors. Color saturation values (threshold values) chosen for visual contrast may show relative changes in signal intensity.

For each stent, several areas may be measured by Raman to ensure that the trends are reproducible. Images may be taken on the coatings before elution, and/or at time points following elution. For images taken following elution, stents may be removed from the elution media and dried in a nitrogen stream. A warming step (e.g. 70C for 10 minutes) may be necessary to reduce cloudiness resulting from soaking the coating in the elution media (to reduce and/or avoid light scattering effects when testing by Raman).

### Time of Flight Secondary Ion Mass Spectrometry

TOF-SIMS can be used to determine drug aggregation at the outer 1-2nm of sample surface when operated under static conditions. The technique can be operated in spectroscopy or imaging mode at high spatial resolution. Additionally cross-sectioned samples can be analysed. When operated under dynamic experimental conditions, known in the art, depth profiling chemical characterization can be achieved.

For example, under static conditions (for example a ToF-SIMS IV (IonToF, Munster)) using a 25Kv Bi⁺⁺ primary ion source maintained below 10¹² ions per cm² is used.. Where necessary a low energy electron flood gun (0.6 nA DC) is used to charge compensate insulating samples.

Cluster Secondary Ion Mass Spectrometry, may be employed as described in Belu et al., "Three-Dimensional Compositional Analysis of Drug Eluting Stent Coatings Using Cluster Secondary Ion Mass Spectroscopy" Anal. Chem. 80: 624-632 (2008).

A stent as described herein is obtained. The stent is prepared for SIMS analysis by cutting it longitudinally and opening it up with tweezers. The stent is then pressed into multiple layers of iridium foil with the outer diameter facing outward.

For example TOF-SIMS experiments are performed on an Ion-TOF IV instrument equipped with both Bi and SF5+ primary ion beam cluster sources. Sputter depth profiling is performed in the dual-beam mode. The analysis source is a pulsed, 25-keV bismuth cluster ion source, which bombarded the surface at an incident angle of 45° to the surface normal. The target current is maintained at ∼0.3 pÅ (+10%) pulsed current with a raster size of 200 um x 200 um for all experiments. Both positive and negative secondary ions are extracted from the sample into a reflectron-type time-of-flight mass spectrometer. The secondary ions are then detected by a microchannel plate detector with a post-acceleration energy of 10 kV. A low-energy electron flood gun is utilized for charge neutralization in the analysis mode.

The sputter source used is a 5-keV SF5+ cluster source also operated at an incident angle of 45° to the surface normal. For thin model samples on Si, the SF5+ current is maintained at ∼2.7 nÅ with a 750 um x 750 um raster. For the thick samples on coupons and for the samples on stents, the current is maintained at 6nA with a 500 um x 500 um raster. All primary beam currents are measured with a Faraday cup both prior to and after depth profiling.

All depth profiles are acquired in the noninterlaced mode with a 5-ms pause between sputtering and analysis. Each spectrum is averaged over a 7.37 second time period. The analysis is immediately followed by 15 seconds of SF5+ sputtering. For depth profiles of the surface and subsurface regions only, the sputtering time was decreased to 1 second for the 5% active agent sample and 2 seconds for both the 25% and 50% active agent samples.

Temperature-controlled depth profiles are obtained using a variable-temperature stage with Eurotherm Controls temperature controller and IPSG V3.08 software, samples are first placed into the analysis chamber at room temperature. The samples are brought to the desired temperature under ultra high-vacuum conditions and are allowed to stabilize for 1 minute prior to analysis. All depth profiling experiments are performed at -100C and 25C.

TOF-SIMS may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

TOF-SIMS depth profiling studies may be performed on an ION-TOF instrument (e.g. Muenster, Germany). The depth profiles may be obtained on coupons and/or stents, to allow development of proper instrumental conditions. The instrument may employ a 5 KeV SF+5 source which is sputtered over a 500 micron x 500 micron area with 6nA continuous current. Initial depth profiles may be obtained using a 25 keV Ga+ analytical source with 2 pA pulsed current. Further experiments may be done using a 25 keV Bi+3 analytical source with 0.3- 0.4 pA pulsed current. The analytical source may be rastered over 200 micron x 200 microns. The depth providles may be done in the non-interlaced mode. A low energy electron flood gun may be used for charge neutralization. All depth profiled may be done at -100C (an optimum temperature for depth profiling with SF+5). Sputter rates may be determined from thin model films of each formulation (about 200 nm) cast on Si wafers. After sputtering through the film on the substrate, the crater depth may be measured by stylus profilometry (tencor Instruments alpha-step 200 with a 10-mg stylus force, Milpitas, CA). The average sputter rates may be calculated for each formulation. The experiments may need to be performed at low temperatures (e.g. 100C) to maintain the integrity of the drug and/or polymer while eroding through them. Additionally, there may be adjustments needed to account for damage accumulation rates that occur with higher drug concentrations.

### Atomic Force Microscopy (AFM)

AFM is a high resolution surface characterization technique. AFM is used in the art to provide topographical imaging, in addition when employed in Tapping Mode™ can image material and or chemical properties for example imaging drug in an aggregated state. Additionally cross-sectioned samples can be analyzed.

A stent as described herein is obtained. AFM may be employed as described in Ranade et al., "Physical characterization of controlled release of paclitaxel from the TAXUS Express2 drug-eluting stent" J. Biomed. Mater. Res. 71(4):625-634 (2004).

Polymer and drug morphologies, coating composition, at least may be determined using atomic force microscopy (AFM) analysis. A multi-mode AFM (Digital Instruments/Veeco Metrology, Santa Barbara, CA) controlled with Nanoscope IIIa and NanoScope Extender electronics is used. TappingMode™ AFM imaging may be used to show topography (a real-space projection of the coating surface microstructure) and phase-angle changes of the AFM over the sample area to contrast differences in the materials properties.

### Example 15: Determination of the Blood Concentration of an Active Agent

This assay can be used to demonstrate the relative efficacy of a therapeutic compound delivered from a device to not enter the blood stream and may be used in conjunction with a drug penetration assay (such as is described in PCT/US2006/010700). At predetermined time points (e.g. 1d, 7d, 14d, 21d, and 28d, or e.g. 6hrs, 12hrs, 24hrs, 36hrs, 2d, 3d, 5d, 7d, 8d, 14d, 28d, 30d, and 60d), blood samples from the subjects that have devices that have been implanted are collected by any art-accepted method, including venipuncture. Blood concentrations of the loaded therapeutic compounds are determined using any art-accepted method of detection, including immunoassay, chromatography (including liquid/liquid extraction HPLC tandem mass spectrometric method (LC-MS/MS), and activity assays. See, for example, Ji, et al., "96-Well liquid-liquid extraction liquid chromatography-tandem mass spectrometry method for the quantitative determination of ABT-578 in human blood samples" Journal of Chromatography B. 805:67-75 (2004).

In one test, blood samples are collected by venipuncture into evacuated collection tubes containing editic acid (EDTA) (n=4). Blood concentrations of the active agent (e.g. rapamycin) are determined using a validated liquid/liquid extraction HPLC tandem pass mass spectormetric method (LC-MS/MS) (Ji *et al.,* et al., 2004). The data are averaged, and plotted with time on the x-axis and blood concentration of the drug is represented on the y-axis in ng/ml.

### Example 16. Preparation of supercritical solution comprising poly(lactic-co-glycolic acid) (PLGA) in hexafluropropane.

A view cell at room temperature (with no applied heat) is pressurized with filtered 1,1,1,2,3,3-Hexafluoropropane until it is full and the pressure reaches 31 MPa (4500 psi). Poly(lactic-co-glycolic acid) (PLGA) is added to the cell for a final concentration of 2mg/ml. The polymer is stirred to dissolve for one hour. The polymer is fully dissolved when the solution is clear and there are no solids on the walls or windows of the cell.

### Example 17. Dry powder rapamycin coating on an electrically charged L605 cobalt chromium metal coupon.

A 1cm x 2cm L605 cobalt chromium metal coupon serving as a target substrate for rapamycin coating is placed in a vessel and attached to a high voltage electrode. Alternatively, the substrate may be a stent or another biomedical device as described herein, for example. The vessel (V), of approximately 1500cm³ volume, is equipped with two separate nozzles through which rapamycin or polymers could be selectively introduced into the vessel. Both nozzles are grounded. Additionally, the vessel (V) is equipped with a separate port was available for purging the vessel. Upstream of one nozzle (D) is a small pressure vessel (PV) approximately 5cm³ in volume with three ports to be used as inlets and outlets. Each port is equipped with a valve which could be actuated opened or closed. One port, port (1) used as an inlet, is an addition port for the dry powdered rapamycin. Port (2), also an inlet is used to feed pressurized gas, liquid, or supercritical fluid into PV. Port (3), used as an outlet, is used to connect the pressure vessel (PV) with nozzle (D) contained in the primary vessel (V) with the target coupon.

Dry powdered Rapamycin obtained from LC Laboratories in a predominantly crystalline solid state, 50mg milled to an average particle size of approximately 3 microns, is loaded into (PV) through port (1) then port (1) is actuated to the closed position. The metal coupon is then charged to +7.5kV using a Glassman Series EL high-voltage power source. The drug nozzle on port has a voltage setting of - 7.5kV. After approximately 60-seconds, the drug is injected and the voltage is eliminated. Upon visual inspection of the coupon using an optical microscope, the entire surface area of the coupon is examined for relatively even distribution of powdered material. X-ray diffraction (XRD) is performed as described herein to confirm that the powdered material is largely crystalline in nature as deposited on the metal coupon. UV-Vis and FTIR spectroscopy is performed as describe herein to confirm that the material deposited on the coupon is rapamycin.

### Example 18. Polymer coating on an electrically charged L605 coupon using rapid expansion from a liquefied gas.

A coating apparatus as described in example 17 above is used in the foregoing example. In this example the second nozzle, nozzle (P), is used to feed precipitated polymer particles into vessel (V) to coat a L605 coupon. Alternatively, the substrate may be a stent or another biomedical device as described herein, for example. Nozzle (P) is equipped with a heater and controller to minimize heat loss due to the expansion of liquefied gases. Upstream of nozzle (P) is a pressure vessel, (PV2), with approximately 25-cm3 internal volume. The pressure vessel (PV2) is equipped with multiple ports to be used for inlets, outlets, thermocouples, and pressure transducers. Additionally, (PV2) is equipped with a heater and a temperature controller. Each port is connected to the appropriate valves, metering valves, pressure regulators, or plugs to ensure adequate control of material into and out of the pressure vessel (PV2). One outlet from (PV2) is connected to a metering valve through pressure rated tubing which was then connected to nozzle (P) located in vessel (V). In the experiment, 150 mg of poly(lactic-co-glycolic acid) (PLGA) is added to pressure vessel (PV2). 1,1,1,2,3,3-hexafluropropane is added to the pressure vessel (PV2) through a valve and inlet. Pressure vessel (PV2) is set at room temperature with no applied heat and the pressure is 31 MPa (4500 psi). Nozzle (P) is heated to 150°C. A 1-cm x 2-cm L605 coupon is placed into vessel (V), attached to an electrical lead and heated via a heat block 110°C. Nozzle (P) is attached to ground. The voltage is set on the polymer spray nozzle and an emitter=pair beaker to a achieve a current greater than or equal to 0.02 mAmps using a Glassman high-voltage power source at which point the metering valve is opened between (PV2) and nozzle (P) in pressure vessel (PV). Polymer dissolved in liquefied gas and is fed at a constant pressure of 1.48 MPa (200 psig) into vessel (V) maintained at atmospheric pressure through nozzle (P) at an approximate rate of 3.0 cm³/min. After approximately 5 seconds, the metering valve is closed discontinuing the polymer-solvent feed. Vessel (V) is Nitrogen gas for 30 seconds to displace the fluorocarbon. After approximately 30 seconds, the metering valve is again opened for a period of approximately 5 seconds and then closed. This cycle is repeated about 4 times. After an additional 1-minute the applied voltage to the coupon was discontinued and the coupon was removed from pressure vessel (V). Upon inspection by optical microscope, a polymer coating is examined for even distribution on all non-masked surfaces of the coupon.

### Example 19. Dual coating of a metal coupon with crystalline rapamycin and poly(lactic-co-glycolic acid) (PLGA).

An apparatus described in example 17 and further described in example 18 is used in the foregoing example. In preparation for the coating experiment, 25 mg of crystalline powdered rapamycin with an average particle size of 3-microns is added to (PV) through port (1), then port (1) was closed. Next, 150 mg of poly(lactic-co-glycolic acid) (PLGA) is added to pressure vessel (PV2). 1,1,1,2,3,3-hexafluropropane is added to the pressure vessel (PV2) through a valve and inlet. Pressure vessel (PV2) is kept at room temperature with no applied heat with the pressure inside the isolated vessel (PV2) approximately 31 MPa (4500 psi). Nozzle (P) is heated to 150°CA 1-cm x 2-cm L605 coupon is added to vessel (V) and connected to a high-voltage power lead. Both nozzles (D) and (P) are grounded. To begin, the coupon is charged to +7.5kV after which port (3) connecting (PV) containing rapamycin to nozzle (D) charged at -7.5 kV is opened allowing ejection of rapamycin into vessel (V) maintained at ambient pressure. Alternatively, the substrate may be a stent or another biomedical device as described herein, for example. After closing port (3) and approximately 60-seconds, the metering valve connecting (PV2) with nozzle (P) inside vessel (V) is opened allowing for expansion of liquefied gas to a gas phase and
introduction of precipitated polymer particles into vessel (V) while maintaining vessel (V) at ambient pressure. After approximately 15 seconds at a feed rate of approximately 3cm³/min., the metering valve s closed while the coupon remained charged. The sequential addition of drug followed by polymer as described above is optionally repeated to increase the number of drug-polymer layers after which the applied potential is removed from the coupon and the coupon was removed from the vessel. The coupon is then examined using an optical microscope to determine whether a consistent coating is visible on all surfaces of the coupon except where the coupon was masked by the electrical lead.

### Example 20. Dual coating of a metal coupon with crystalline rapamycin and poly(lactic-co-glycolic acid) (PLGA) followed by Supercritical Hexafluropropane Sintering.

After inspection of the coupon created in example 19, the coated coupon (or other coated substrate, e.g. coated stent) is carefully placed in a sintering vessel that is at a temperature of 75°C. 1,1,1,2,3,3-hexafluropropane in a separate vessel at 0.52 MPa (75 psi) is slowly added to the sintering chamber to achieve a pressure of 0.16 MPa to 0.19 (23 to 27 psi). This hexafluropropane sintering process is done to enhance the physical properties of the film on the coupon. The coupon remains in the vessel under these conditions for approximately 10 min after which the supercritical hexafluropropane is slowly vented from the pressure vessel and then the coupon was removed and reexamined under an optical microscope. The coating is observed in conformal, consistent, and semi-transparent properties as opposed to the coating observed and reported in example 19 without dense hexafluropropane treatment. The coated coupon is then submitted for x-ray diffraction (XRD) analysis, for example, as described herein to confirm the presence of crystalline rapamycin in the polymer.

### Example 21. Coating of a metal cardiovascular stent with crystalline rapamycin and poly(lactic-co-glycolic acid) (PLGA)

The apparatus described in examples 17, 18 and 20 is used in the foregoing example. The metal stent used is made from cobalt chromium alloy of a nominal size of 18 mm in length with struts of 63 microns in thickness measuring from an abluminal surface to a luminal surface, or measuring from a side wall to a side wall. The stent is coated in an alternating fashion whereby the first coating layer of drug is followed by a layer of polymer. These two steps, called a drug/polymer cycle, are repeated twice so there are six layers in an orientation of drug-polymer-drug-polymer-drug-polymer. After completion of each polymer coating step and prior the application of the next drug coating step, the stent is first removed from the vessel (V) and placed in a small pressure vessel where it is exposed to supercritical hexafluropropane as described above in example 20.

### Example 22. Layered coating of a cardiovascular stent with an anti-restenosis therapeutic and polymer in layers to control drug elution characteristics.

A cardiovascular stent is coated using the methods described in examples 10 and 11 above. The stent is coated in such as way that the drug and polymer are in alternating layers. The first application to the bare stent is a thin layer of a non-resorbing polymer, approximately 2-microns thick. The second layer is a therapeutic agent with anti-restenosis indication. Approximately 35 micrograms are added in this second layer. A third layer of polymer is added at approximately 2-microns thick, followed by a fourth drug layer which is composed of about 25 micrograms of the anti-restenosis agent. A fifth polymer layer, approximately 1- micron thick is added to stent, followed by the sixth layer that includes the therapeutic agent of approximately 15-micrograms. Finally, a last polymer layer is added to a thickness of about 2-microns. After the coating procedure, the stent is annealed using carbon dioxide as described in example 16 above. In this example a drug eluting stent (DES) is described with low initial drug "burst" properties by virtue of a "sequestered drug layering" process, not possible in conventional solvent-based coating processes. Additionally, by virtue of a higher concentration of drug at the stent 'inter-layer' the elution profile is expected to reach as sustained therapeutic release over a longer period of time.

### Example 23. Layered coating of a cardiovascular stent with an anti-restenosis therapeutic and an anti-thrombotic therapeutic in a polymer.

A cardiovascular stent is coated as described in example 11 above. In this example, after a first polymer layer of approximately 2-microns thick, a drug with anti-thrombotic indication is added in a layer of less than 2-microns in thickness. A third layer consisting of the non-resorbing polymer is added to a thickness of about 4-microns. Next another drug layer is added, a different therapeutic, with an anti-restenosis indication. This layer contains approximately 100 micrograms of the anti-restenosis agent. Finally, a polymer layer approximately 2-microns in thickness is added to the stent. After coating the stent is treated as described in example 20 to sinter the coating using hexafluropropane.

### Example 24. Coating of stent with Rapamycin and poly(lactic-co-glycolic acid) (PLGA)

Micronized Rapamycin is purchased from LC Laboratories. 50:50 PLGA (Mw = ∼90) are purchased from Aldrich Chemicals. Eurocor CoCr (7cell) stents are used. The stents are coated by dry electrostatic capture followed by supercritical fluid sintering, using 3 stents/coating run and 3 runs/data set. Analysis of the coated stents is performed by multiple techniques on both stents and coupons with relevant control experiments described herein.

In this example, PLGA is dissolved in 1,1,1,2,3,3-Hexafluoropropane with the following conditions: a) room temperature, with no applied heat; b) 31 MPa (4500 psi); and c) at 2mg/ml concentration. The spray line is set at 31 MPa (4500 psi), 150°C and nozzle temperature at 150°C. The solvent (Hexafluoropropane) is rapidly vaporized when coming out of the nozzle (at 150°C). A negative voltage is set on the polymer spray nozzle to achieve a current of greater than or equal to 0.02 mAmps. The stent is loaded and polymer is sprayed for 15 seconds to create a first polymer coating.

The stent is then transferred to a sintering chamber that is at 75°C. The solvent, in this example 1,1,2,3,3-hexafluropropane, slowly enters the sintering chamber to create a pressure at 0.16 MPa to 0.19 (23 to 27 psi). Stents are sintered at this pressure for 10 minutes.

11.5 mg Rapamycin is loaded into the Drug injection port. The injection pressure is set at 1.9 MPa (280 psi) with +7.5 kV for the stent holder and -7.5 kV for the drug injection nozzle. After the voltage is set for 60 s, the drug is injected into the chamber to create a first drug coating.

A second polymer coating is applied with two 15 second sprays of dissolved polymer with the above first polymer coating conditions. The second coating is also subsequently sintered in the same manner.

A second drug coating is applied with the same parameters as the first drug coating. Lastly, the outer polymer layer is applied with three 15 second sprays of dissolved polymer with the above polymer coating conditions and subsequently sintered.

### Example 25. Histology of in vivo stented porcine models and preparation for pharmacokinetics studies

Coronary stenting was applied to porcine animal models as described previously. An angiography was perform on each animal prior to euthanasia. After prenecropsy angiography, each animal was euthanized via an overdose of euthanasia solution or potassium chloride solution, IV in accordance to the Test Facility's Standard Operating Procedure and was performed in accordance with accepted American Veterinary Medical Association's "AVMA Guidelines on Euthanasia" (June 2007; accessed at http:/./www.avma.org/issues/animal_welfare/euthansia.pdf).

A limited necropsy consisting of examination of the heart was performed on all animals. Observations of macroscopic findings were recorded. Any evidence of macroscopic findings, were processed for histological examination. Regardless, all hearts were collected for histologic processing and assessment.

The hearts were perfusion fixed at ∼100 mmHg with Lactated Ringer's Solution until cleared of blood followed by 10% neutral buffered formalin (NBF). The fixed hearts were placed in a NBF filled container and labeled as appropriate.

Whole heart radiographs were taken to document stent location and morphology in situ. In addition, each explanted stent was radiographed in two views (perpendicular or orthogonal incidences) along its longitudinal plane to assist in the assessment of expansion morphology, damage and/or areas of stent discontinuity (e.g., strut fractures).

Fixed stented vessels were carefully dissected from the myocardium, leaving sufficient vessel both proximal and distal to the stented portion. Unless otherwise stated or required, all tissues/sections were processed according procedures typical for such activity and known to one of skill in the art. In particular, transverse sections of unstented vessel were obtained within approximately 1-3 mm of the proximal and distal ends of the stent (i.e., unstented vessel) and from the proximal, middle and distal regions of the stented vessel. All vessel sections were stained with hematoxylin and eosin and a tissue elastin stain (e.g., Verhoeff's).

The remaining myocardium was then transversely sectioned (i.e., "bread-loafed") from apex to base (∼1 cm apart) to further assess for evidence of adverse reactions (e.g., infarction). If gross findings were present they were collected and processed for light microscopy. Remaining myocardial tissue were stored until finalization of the study at which time, it was disposed of according to Test Facility standard operating procedures, shipped to Sponsor, or archived at Sponsor's request and expense.

Quantitative morphometric analysis was performed on the histological sections from each stented artery. For each histological section, the parameters listed in Table 4 were directly measured using standard light microscopy and computer-assisted image measurement systems.

**Table 4**

| Morphometry Parameters | | | |
|---|---|---|---|
| Parameter | Abbreviation | Calculation | Unit |
| Lumen Area | Lₐ | directly measured | mm² |
| Internal Elastic Layer (IEL) Bounded Area | IELₐ | directly measured | mm² |
| Stent Area | Sₐ | directly measured | mm² |
| External Elastic Layer (EEL) Bounded Area | EELₐ | directly measured | mm² |

From these direct measurements, all other histomorphological parameters were calculated. Measured and calculated parameters, formulae, and units of measure are in Table 5.

**Table 5**

| Calculated Morphometry Parameters and Units of Measure | | | |
|---|---|---|---|
| Parameter | Abbreviation | Calculation | Unit |
| **Area Measurements** | | | |
| Neointimal Area | Nₐ | IELₐ - Lₐ | mm² |
| Medial Area | Mₐ | EELₐ - IELₐ | mm² |
| Artery Area | Aₐ | Lₐ + Nₐ + Mₐ | mm² |

| **Length Measurements** | | | |
|---|---|---|---|
| Lumen Diameter | L_{d} | 2 x √(Lₐ/π) | mm |
| IEL Diameter | IEL_{d} | 2 x √( Lₐ + Na)/□π | mm |
| Stent Diameter | S_{d} | 2 x √(Sₐ/□π) | mm |
| Arterial Diameter | A_{d} | 2 x √(Aₐ/□π) | mm |

| **Ratios** | | | |
|---|---|---|---|
| Lumen / Artery Areas | L:A | Lₐ/Aₐ | NA |
| Neointima / Media Areas | N:M | Nₐ / Mₐ | NA |
| EEL / IEL Areas | EELₐ: IELₐ | Aₐ / (Lₐ+Nₐ) | NA |
| IEL/ Stent Areas | IELₐ:Sₐ | IELₐ/Sₐ | NA |

| **Restenosis Parameters** | | | |
|---|---|---|---|
| % Area Occlusions) | % AO | Nₐ/(Nₐ+La) x 100% | % |
| Neointima Thickness | N_{µm} | Nₘₘ x 1000(µm/mm) | µm |
| Neointima Thickness | Nₘₘ | (IEL_{d}-L_{d})/2 | mm |

### Histopathology - Stented & Adjacent Non-Stented Vessels

Histopathological scoring via light microscopy was also used to grade various parameters that reflect the degree and extent of the host response/repair process to treatment. These parameters included, but were not limited to, injury, inflammation, endothelialization, and fibrin deposition. When a microscopic endpoint listed below is not present/observed, the score 0 was given.

The scoring of the arterial cross-sections was carried out as follows: Injury score for stented arterial segments is dependent on that portion of the arterial wall which is disrupted by the stent and/or associated tissue response. Injury was scored on a per-strut basis and the median and average calculated per plane (i.e., proximal, middle, distal) and stent. The scoring polymer for injury at each strut is listed in Table 6.

**Table 6**

| Injury Score | |
|---|---|
| Score | Value |
| 0 | IEL intact |
| 1 | Disruption of IEL |
| 2 | Disruption of tunica media |
| 3 | Disruption of tunica adventitia |

Inflammation score depends on the degree of inflammation and extent of inflammation on a per-strut basis as outlined in Table 7. Inflammation was scored on a per strut basis and the average was calculated per plane and stent.

**Table 7**

| Inflammation Score | |
|---|---|
| Score | Value |
| 0 | Absent |
| 1 | Scattered cellular infiltrates associated with strut |
| 2 | Notable cellular infiltrates associated with strut |
| 3 | Cellular infiltrates circumscribing strut |

Neointimal fibrin score depends on the degree of fibrin deposition in the neointima as outlined in Table 8.

**Table 8**

| Neointimal Fibrin Score | |
|---|---|
| Score | Value |
| 0 | Absent |
| 1 | Infrequent spotting of fibrin |
| 2 | Heavier deposition of fibrin |
| 3 | Heavy deposition of fibrin that spans between struts |

Endothelialization score depends on the extent of the circumference of the artery lumen showing coverage with endothelial cells as outlined in Table 9.

**Table 9**

| Endothelialization Score | |
|---|---|
| Score | Value |
| 0 | Absent |
| 1 | < 25% |
| 2 | 25% to 75% |
| 3 | >75% |
| 4 | 100%, confluent |

Adventitial fibrosis score depends on the severity of response and circumference of artery affected as outlined in Table 10.

**Table 10**

| Adventitial Fibrosis Score Polymer | |
|---|---|
| Score | Observation |
| 0 | Absent |
| 1 | Minimal presence of fibrous tissue |
| 2 | Notable fibrous tissue in 25%-50% of artery circumference |
| 3 | Notable fibrous tissue in ≥ 50% of artery circumference |

Neointimal maturation depends on the cellularity and organization of the neointima as outlined in Table 11.

**Table 11**

| Neointimal Maturation Score Polymer | |
|---|---|
| Score | Observation |
| 0 | Absent |
| 1 | Immature, predominantly fibrino-vascular tissue |
| 2 | Transitional, predominantly organizing smooth muscle |
| 3 | Mature, generalized organized smooth muscle |

The histologic section of the artery was also examined for other histologic parameters including hemorrhage, necrosis, medial fibrosis, type and relative amounts of inflammatory cell infiltrates (e.g., neutrophils, histiocytes, lymphocytes, multinucleated giant cells), mineralization, strut malapposition, thrombosis and/or neointimal vascularity, or others as deemed appropriate by the pathologist. Unless otherwise stated in the pathology data/report, additional findings were graded as follow: 0= Absent; 1 = Present, but minimal feature; 2 = Notable feature; 3 = Overwhelming feature.

Sections of the non-stented proximal and distal portions of the stented arteries, were similarly assessed and scored for histologic parameters as above (excluding neointimal fibrin) but were assessed for histomorphometry.

One histology study according to the description above was performed using the groups and coated stents (test articles) as noted in Table 12 which were coated according to the methods provided herein, and/or devices having coatings as described herein (for example, at AS1, AS2, or another coating combination as described herein) as compared to a control bare metal stent (BMS, AS3) The animals were Yucatan pigs, which were given an anticoagulation regimen of Day 1: ASA 650mg + Plavix 300mg, maintenance of: ASA 81mg + Plavix75, and Procedural: ACT ∼ 250 sec. Oversizing was ∼10-20%.

**Table 12**

| Group | Test Article | Number of Test Devices | Necropsy Time Point |
|---|---|---|---|
| 1 | AS1 | N=6 | Day 28 |
| | | N=6 | Day 90 |
| 2 | AS2 | N=6 | Day 28 |
| | | N=6 | Day 90 |
| 3 | AS3 (Bare metal Stent) | N=6 | Day 28 |
| | | N=6 | Day 90 |

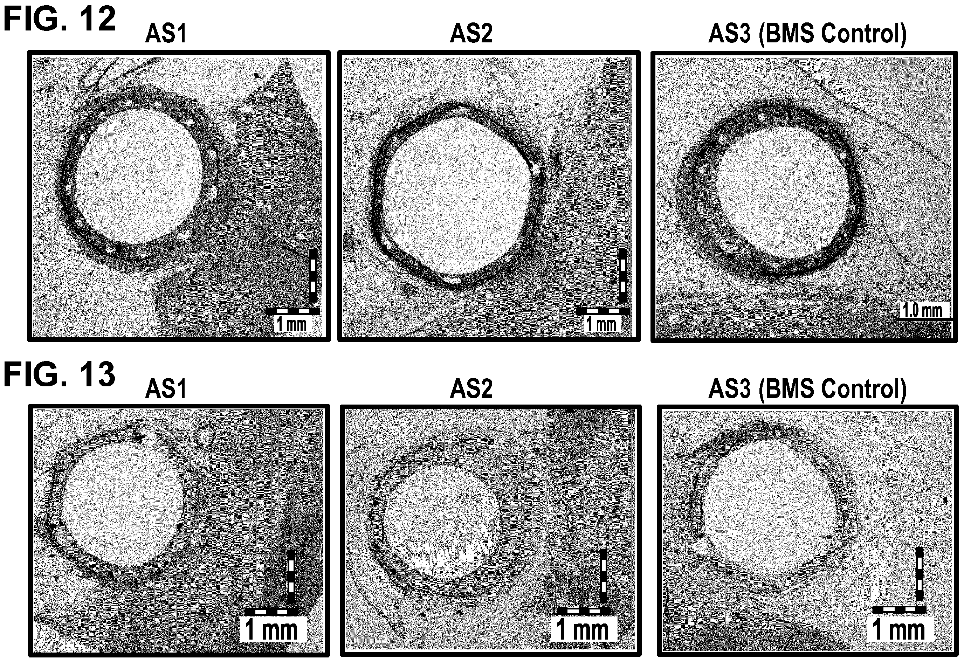

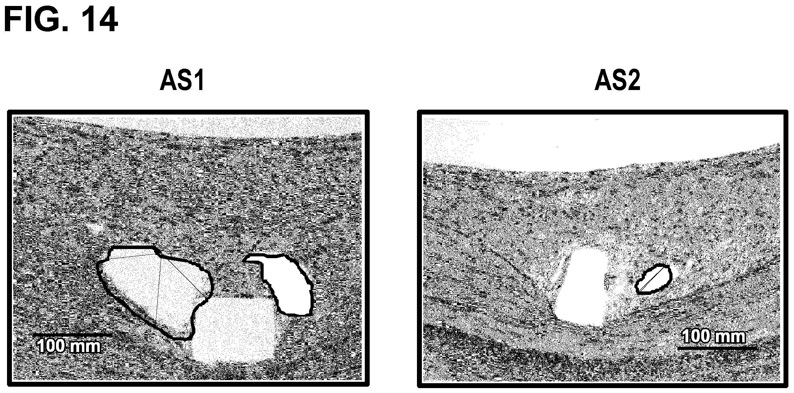

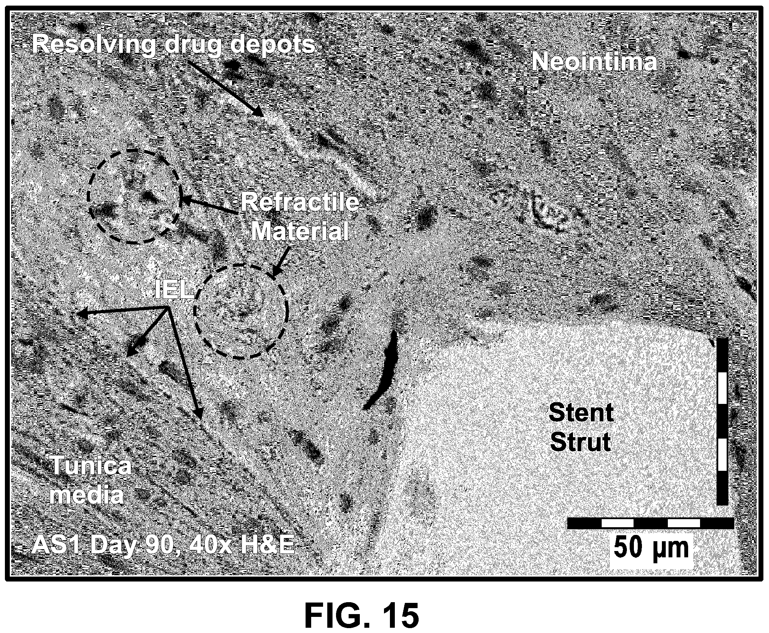

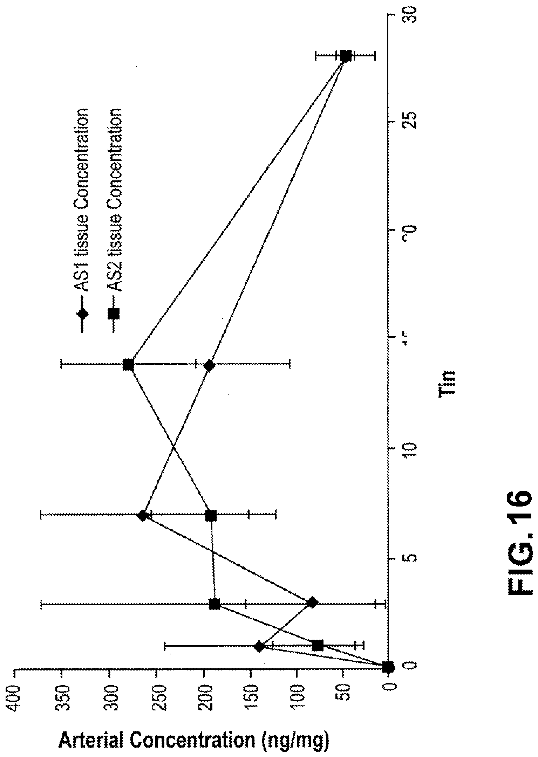

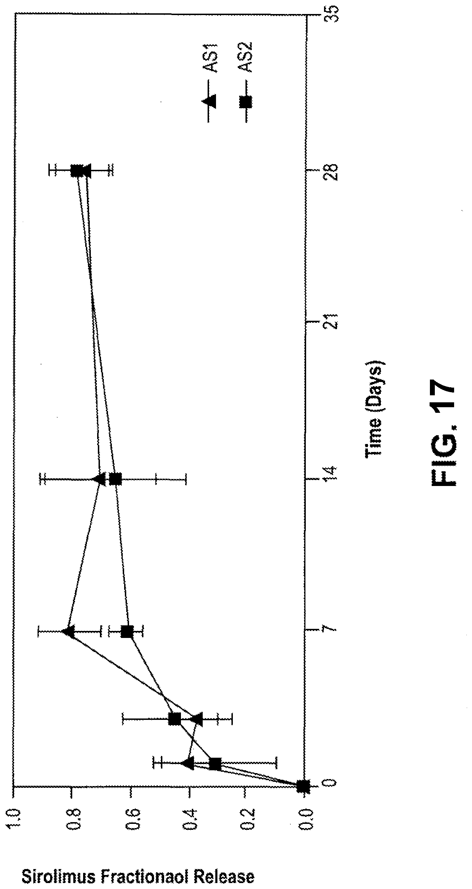

Results of histology studies performed according to the methods described above are presented in Figures 12-23. Figures 12 and 13 depict low-magnification cross-sections of porcine coronary artery stent implants (AS1, AS2 and Bare-metal stent control) at 28 days and 90 days post-implantation. Figures 14 and 15 show drug depots in low-magnification cross-sections of porcine coronary artery stent implants. Figure 16 shows arterial tissue concentrations (y-axis) versus time (x-axis) for AS1 and AS2 stents implantations in swine coronary arteries expressed as absolute tissue level (y-axis) versus time (x-axis). Figure 17 is Fractional Sirolimus Release (y-axis) versus time (x-axis) in Arterial Tissue for AS 1 and AS2 Stents. Pigs were implanted with coated stents as described above. Blood was drawn at predetermined times and assayed to determine rapamycin concentration. The assays were based on technology known to one of ordinary skill in the art.

### Example 26: Normalized % Elution of Rapamycin Where Test Group has Sintering between the 2d and 3d polymer application in the 3d polymer layer

In this example, 12 coated stents (3.0 mm diameter x 15 mm length) were produced, 6 control coated stents and 6 test coated stents. The control stents and the test stents were produced according to methods described herein, with the test stents receiving a sintering step between the second and the third polymer application in the third polymer layer. Each layer of some embodiments of coated stents described herein comprise a series of sprays. In this example, the stents were coated with PDPDP layers (i.e. Polymer Drug Polymer Drug Polymer), having a sinter step after each "P" (or polymer) layer, wherein the polymer is 50:50 PLGA. The "D" (i.e. active agent, also called "drug" herein) was sirolimus in this Example. The third polymer layer comprised a series of polymer sprays (3 polymer spray steps). In the control stents, the third polymer layer was sintered only after the final polymer spray step, and in the test stents there was a sinter step (100°C/1.03 MPa (150 psi)/10 min) between the second and third spray of
polymer in the final (third) polymer layer, as well as a sinter step after the final spray step of the final (third) polymer layer.

Following coating and sintering, SEM testing of one stent from each of the control stents and the test stents was performed according to the test methods noted herein. The SEM images that resulted show more active agent on the surface of the coating in the control stent than in the test stent.

Total Drug Content of one stent from each of the control stents and the test stents was performed according to the test methods noted herein. The total drug mass (pharmaceutical agent total content) of the control stent was determined to be 138 micrograms. The total drug mass of the control stent was determined to be 140 micrograms.

Total Mass of the coating was determined for each stent in both the control stents and the test stents. The total coating mass of the control stents was determined to be 660 µg, 658 µg, 670 µg, 642 µg, 666 µg, and 670µg. The total coating mass of the test stents was determined to be 714 µg, 684µg, 676 µg, 676 µg, 682µg, and 712µg.

Elution testing following coating and sintering was performed as described herein and in Example 11e, in 50% Ethanol/Phosphate Buffered Saline (1:1 spectroscopic grade ethanol (95%) / phosphate buffer saline), pH 7.4, 37C. The elution media was agitated media during the contacting step. The device was removed (and the elution media was removed and replaced) at three time points, 1h (day 0), 24 hrs (day 1.0), and 2 days. The elution media was assayed using a UV-Vis at 278 nm by a diode array spectrometer or determination of the pharmaceutical agent (rapamycin) content. Calibration standards containing known amounts of drug were also held in elution media for the same durations as the samples and used at each time point to determine the amount of drug eluted at that time (in absolute amount and as a cumulative amount eluted).

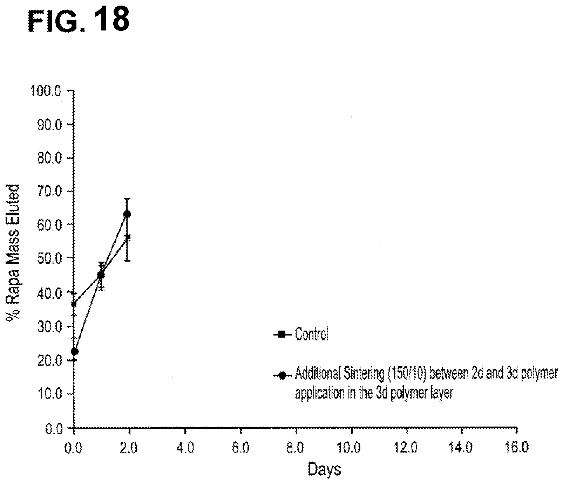

Elution results for the coated stents (4 control, 4 test) are depicted in Figure 18. Results were normalized by the total content of the stents, and expressed as % rapamycin total mass eluted (y-axis) at each time point (x-axis). The test group (bottom line at day 0) is shown in Figure 18 having a lower burst with lesser surface available drug than the control stents (top line at day 0).

### Example 27: Normalized % Elution of Rapamycin Where Test Group has an Additional 15 Second Spray after Final Sinter Step of Normal Process (control) Followed by a Sinter Step

In this example, 12 coated stents (3.0 mm diameter x 15 mm length) were produced, 6 control coated stents and 6 test coated stents. The control stents and the test stents were produced according to methods described herein, with the test stents receiving an additional 15 second polymer spray after final sinter step of normal process (control) followed by a sinter step (100°C/1.03 MPa (150 psi)/10 min). In this example, the stents were coated with PDPDP layers (i.e. Polymer Drug Polymer Drug Polymer), having a sinter step after each P (polymer) layer, wherein the polymer is 50:50 PLGA. The "D" (i.e. active agent, also called "drug" herein) was sirolimus in this Example. In the test stents (but not in the control stents) following the final sintering step, the coated stents received an additional 15 second polymer spray and sinter (100°C/1.03 MPa (150 psi)/10 min).

Following coating and sintering, SEM testing of one stent from each of the control stents and the test stents was performed according to the test methods noted herein. The SEM images that resulted show more active agent on the surface of the coating in the control stent than in the test stent.

Total Drug Content of one stent from each of the control stents and the test stents was performed according to the test methods noted herein. The total drug mass of the control stent was determined to be 143 micrograms (µg). The total drug mass of the control stent was determined to be 143 micrograms.

Total Mass of the coating was determined for each stent in both the control stents and the test stents. The total coating mass of the control stents was determined to be 646 µg, 600µg, 604µg, 616µg, 612µg, and 600µg. The total coating mass of the test stents was determined to be 726 µg, 694µg, 696 µg, 690 µg, 696µg, and 696µg.

Elution testing following coating and sintering was performed as described herein and in Example 11e, in 50% Ethanol/Phosphate Buffered Saline (1:1 spectroscopic grade ethanol (95%) / phosphate buffer saline), pH 7.4, 37C. The elution media was agitated media during the contacting step. The device was removed (and the elution media was removed and replaced) at three time points, 1h (day 0), 24 hrs (day 1.0), and 2 days. The removed elution media was assayed using a UV-Vis at 278 nm by a diode array spectrometer or determination of the pharmaceutical agent (rapamycin) content. Calibration standards containing known amounts of drug were also held in elution media for the same durations as the samples and used at each time point to determine the amount of drug eluted at that time (in absolute amount and as a cumulative amount eluted).

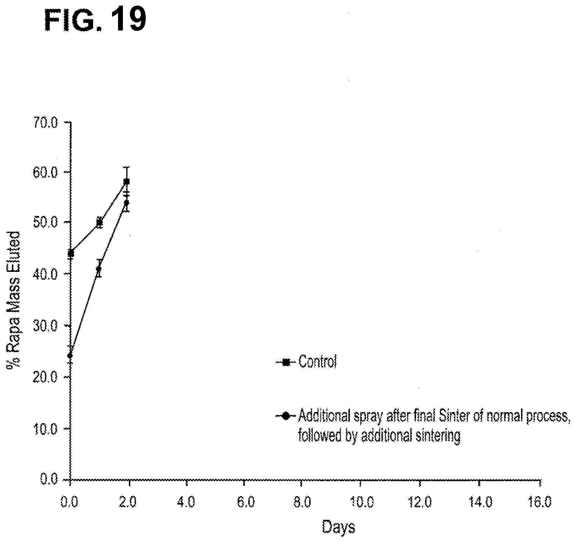

Elution results for the coated stents (4 control, 4 test) are depicted in Figure 19. Results were normalized by the total content of the stents, and expressed as % rapamycin total mass eluted (y-axis) at each time point (x-axis). The test group (bottom line) is shown in Figure 19 having a lower burst with lesser surface available drug than the control stents (top line).

### Example 28: Normalized % Elution of Rapamycin Where Test Group has Less polymer in all powder coats of final layer (1 second less for each of 3 sprays), then Sintering, and an Additional polymer spray (3 seconds) and Sintering

In this example, 12 coated stents (3.0 mm diameter x 15 mm length) were produced, 6 control coated stents and 6 test coated stents. The control stents and the test stents were produced according to methods described herein, with both groups receiving a series of polymer sprays in the final polymer layer. Each layer of some embodiments of coated stents described herein comprise a series of sprays. In this example, the stents (of both groups) were coated with PDPDP layers (i.e. Polymer Drug Polymer Drug Polymer), having a sinter step after each "P" (or polymer) layer, wherein the polymer is 50:50 PLGA. The "D" (i.e. active agent, also called "drug" herein) was sirolimus in this Example. The third polymer layer comprised a series of polymer sprays. In the control stents, the third polymer layer was sintered (100°C/1.03 MPa (150 psi)/10 min) after the final polymer spray step of 3 polymer sprays in the final layer. In the test stents four spray steps were used in the final polymer layer. Each of the first three spray steps was shortened by 1 second (i.e. 3 seconds total less polymer spray time), and after the third polymer
spray there was a sinter step (100°C/1.03 MPa (150 psi)/10 min). Following this, a fourth spray step (3 seconds) was performed followed by a sinter step (100°C/1.03 MPa (150 psi)/10 min).

Following coating and sintering, SEM testing of one stent from each of the control stents and the test stents was performed according to the test methods noted herein. The SEM images that resulted show more active agent on the surface of the coating in the control stent than in the test stent.

Total Drug Content of one stent from each of the control stents and the test stents was performed according to the test methods noted herein. The total drug mass of the control stent was determined to be 136 micrograms (µg). The total drug mass of the control stent was determined to be 139 micrograms.

Total Mass of the coating was determined for each stent in both the control stents and the test stents. The total coating mass of the control stents was determined to be 606µg, 594 µg, 594 µg, 622µg, 632 µg, and 620µg. The total coating mass of the test stents was determined to be 634 µg, 638 µg, 640 µg, 644 µg, 636µg, and 664µg.

Elution testing following coating and sintering was performed as described herein and in Example 11e, in 50% Ethanol/Phosphate Buffered Saline (1:1 spectroscopic grade ethanol (95%) / phosphate buffer saline), pH 7.4, 37C. The elution media was agitated media during the contacting step. The device was removed (and the elution media was removed and replaced) at three time points, 1h (day 0), 24 hrs (day 1.0), and 2 days. The removed elution media was assayed using a UV-Vis at 278 nm by a diode array spectrometer or determination of the pharmaceutical agent (rapamycin) content. Calibration standards containing known amounts of drug were also held in elution media for the same durations as the samples and used at each time point to determine the amount of drug eluted at that time (in absolute amount and as a cumulative amount eluted).

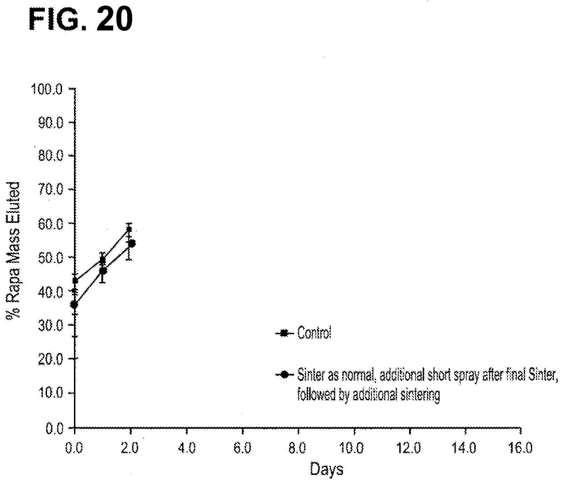

Elution results for the coated stents (4 control, 4 test) are depicted in Figure 20. Results were normalized by the total content of the stents, and expressed as % rapamycin total mass eluted (y-axis) at each time point (x-axis). The test group (bottom line) is shown in Figure 20 having a slightly lower burst with lesser surface available drug than the control stents (top line).

### Example 29: Determination of Surface Composition of a Coated Stent

ESCA (among other test methods), may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated stents and/or coated coupons may be prepared according to the methods described herein, and tested according to the testing methods of Belu- Chemical Imaging.

ESCA analysis (for surface composition testing) may be done on the coated stents using a Physical Electronics Quantum 2000 Scanning ESCA (e.g. from Chanhassen, MN). The monochromatic AL Ka x-ray source may be operated at 15 kV with a power of 4.5 W. The analysis may be done at a 45degree take-off angle. Three measurements may be taken along the length of each stent with the analysis area about 20 microns in diameter. Low energy electron and Ar+ ion floods may be used for charge compensation. The atomic compositions determined at the surface of the coated stent may be compared to the theoretical compositions of the pure materials to gain insight into the surface composition of the coatings. For example, where the coatings comprise PLGA and Rapamycin, the amount of N detected by this method may be directly correlated to the amount of drug at the surface, whereas the amounts of C and O determined represent contributions from rapamycin, PLGA (and potentially silicone, if there is silicone contamination as there was in Belu- Chemical Imaging). The amount of drug at the surface may be based on a comparison of the detected % N to the pure rapamycin %N. Another way to estimate the amount of drug on the surface may be based on the detected amounts of C and O in ration form %O/%C compared to the amount expected for rapamycin. Another way to estimate the amount of drug on the surface may be based on high resolution spectra obtained by ESCA to gain insight into the chemical state of the C, N, and O species. The C 1 s high resolution spectra gives further insight into the relative amount of polymer and drug at the surface. For both Rapamycin and PLGA (for example), the C 1 s signal can be curve fit with three components: the peaks are about 289.0 eV: 286.9 eV : 284.8 eV, representing O-C=O, C-O and/or C-N, and C-C species, respectively. However, the relative amount of the three C species is different for rapamycin versus PLGA, therefore, the amount of drug at the surface can be estimated based on the relative amount of C species. For each sample, for example, the drug may be quantified by comparing the curve fit area measurements for the coatings containing drug and polymer, to those of control samples of pure drug and pure polymer. The amount of drug may be estimated based on the ratio of O-C=O species to C-C species (e.g. 0.1 for rapamycine versus 1.0 for PLGA).

### Example 30: % Elution of Rapamycin

In this example, 148 coated stents (3.0 mm diameter x 15 mm length) were produced according to methods described herein. The stents were coated with PDPDP layers (i.e. Polymer Drug Polymer Drug Polymer), having a sinter step (100°C/1.03 MPa (150 psi)/10 min) after each "P" (or polymer) layer, wherein the polymer is 50:50 PLGA. The "D" (i.e. active agent, also called "drug" herein) was sirolimus in this Example. Twenty-two (22) stents were removed from the testing results since there was contamination detected in the coating process and coating. Additionally, a single statistical outlier stent was removed from testing results.

Elution testing following coating and sintering was performed as described herein and in Example 11e, in 50% Ethanol/Phosphate Buffered Saline (1:1 spectroscopic grade ethanol (95%) / phosphate buffer saline), pH 7.4, 37C. The elution media was agitated media during the contacting step. The devices were removed (and the elution media was removed and replaced) at multiple time points, 1h (day 0), 1 day, 2 days, 5 days, 4 days, 5 days, 7 days, 9 days, 11 days, and 15 days. Not all stents were tested at all time points (see Table 13) since testing results were calculated prior to all stents completing the full 15 days of elution testing. The removed elution media was assayed using a UV-Vis at 278 nm by a diode array spectrometer or determination of the active agent (rapamycin) content. Calibration standards containing known amounts of drug were also held in elution media for the same durations as the samples and used at each time point to determine the amount of drug eluted at that time (in absolute amount and as a cumulative amount eluted).

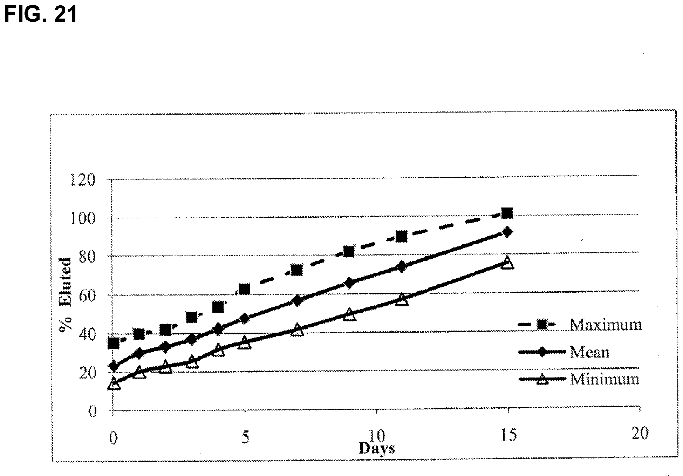

Elution results for the coated stents are depicted in Figure 21. This figure shows the average (or mean) percent elution of all the tested stents at each time point (middle line), expressed as % rapamycin total mass eluted (y-axis) at each time point (x-axis). The minimum (bottom line) and maximum (top line) % eluted at each time point is also shown in Figure 21. The data for Figure 21 is also provided in Table 13.

**Table 13: % rapamycin eluted by in-vitro testing**

| **Days** | **Time** | **Mean** | **Samples** | **Stdev** | **Min** | **Max** |
|---|---|---|---|---|---|---|
| 0 | 1h | 23.1 | 125 | 4.9 | 35.2 | 14.3 |
| 1 | Id | 29.7 | 125 | 4.0 | 39.7 | 20.1 |
| 2 | 2d | 33.0 | 125 | 4.0 | 41.9 | 22.9 |
| 3 | 3d | 37.0 | 125 | 4.4 | 48.2 | 25.5 |
| 4 | 4d | 42.1 | 113 | 4.5 | 53.6 | 31.5 |
| 5 | 5d | 47.4 | 108 | 5.5 | 62.7 | 35.3 |
| 7 | 7d | 56.6 | 98 | 6.4 | 72.3 | 41.7 |
| 9 | 9d | 65.5 | 98 | 7.1 | 81.8 | 49.5 |
| 11 | 11d | 73.8 | 87 | 7.2 | 89.4 | 57.1 |
| 15 | 15d | 91.2 | 75 | 6.8 | 101.1 | 75.6 |

### Example 31:

The acute performance and tissue response associated with use of sirolimus eluting stent systems processed and created as described herein was evaluated in both single implant and overlapping stent implant configurations and compared to a standard marketed bare metal stent (BMS), the Vision BMS stent (Abbott Vascular). Comparison between two groups of sirolimus eluting stent systems manufactured using two different coating instruments (different coating tool platforms), denoted for this study as "Process 1" or "Automated" and "Process 2" or "Manual". Process 1 had an automated mechanism to move materials and fixtures through the coating process while Process 2 required manual operation. All other aspects of the coating procedure were the same.

The sirolimus eluting stent systems (Sirolimus DES and systems) were built according to methods described herein, and the coated stents comprised sirolimus and PLGA. The process for making the Sirolimus DES included supercritical fluid deposition which allowed the drug/polymer coating to be applied to a bare metal stent. The absorbable drug/polymer formulation controls drug elution and the duration of polymer exposure. As a result, the coating delivers a therapeutic solution for coronary artery disease with the potential to avoid the long-term safety concerns associated with current drug-eluting coronary stents that use non-absorbing or very slowly absorbing polymers. The sirolimus eluting stents (Sirolimus DES) comprised 3.0 x 15 mm CoCr stents, having a nominal drug dose per stent of 135 micrograms of sirolimus. Sirolimus DES stents were coated as follows: PDPDP layers (i.e. Polymer Drug Polymer Drug Polymer), having a sinter step (100°C/1.03 MPa (150 psi)/10 min) after each "P" (or polymer) layer, wherein the polymer is 50:50 PLGA. There was 135 micrograms +/- 15% sirolimus on each coated stent in this study. The coating was about 5-15 micrometers thick on each stent, and comprised a thicker coating on the abluminal surface (coating bias). The coating encapsuled each of the stents.

The objectives of this study were to evaluate the sirolimus drug eluting stent (Sirolimus DES) produced as described herein, in porcine coronary arteries with respect to: 1) Safety via the assessment of the tissue response at 3, 30, 90, 180, and 270 days post-implantation utilizing standard histological processing for coronary artery stents and light microscopy; 2) Comparison at 30 days following implantation of Sirolimus DESs manufactured using two different sirolimus/polymer coating processes (Manual and Automated); and 3) Overall acute performance characteristics of the stent and stent delivery system (SDS). A marketed bare metal stent (Vision BMS, Abbott Vascular) was used as a control. The control stents were 3.0 x 15 mm CoCr Vision (Abbott Vascular) bare metal stents. Table 14 describes the study design. Results discussed herein are from the Day 3 and Day 30, the only groups completed and analyzed thus far.

**Table 14**

| Group | Test/Control Articles | Number of Test/Control Articles | Implantation Scheme | Necropsy Time Point |
|---|---|---|---|---|
| 1 | Sirolimus DES (Process 1) | n = 7 - 8 per time point | Up to 3 vessels were implanted per animal (RCA, LAD, LCX or branches thereof) | **Groups 1V, 3V:** |
| 2 | Sirolimus DES (Process 2) | n = minimum of 8 per time point | | Days 3, 30, 90, and, 180 (± 5%) |
| 3 | Vision BMS (control) | n = minimum of 8 per time point | | **Groups 1, 3:** |
| IV (overlapped) | Sirolimus DES (Process 1) | n = minimum of 8 pairs per time point | | Days 3, 30, 90, 180, and 270 (± 5%) |
| | | | | **Group 2:** |
| 3V (overlapped) | Vision BMS (control) | n = minimum of 8 pairs per time point | | Day 30 only (± 5%) |

This study enrolled 86 Yucatan pigs (3 and 30 day data from 36 animals are presented herein). Animals underwent a single interventional procedure on Day 0 in which stents were implanted in up to 3 coronary arteries. For Groups 1, 2, and 3: Stents were introduced into the coronary arteries by advancing the stent delivery system (SDS) through the guide catheter and over the guide wire to the deployment site within the coronary artery. The balloon was then inflated at a steady rate to a pressure sufficient to target a visually assessed balloon-artery ratio of 1.05:1-1.15:1. Confirmation of this balloon-artery ratio was made when the angiographic images were quantitatively assessed. After the target balloon-artery ratio was achieved, vacuum was applied to the inflation device in order to deflate the balloon. Complete balloon deflation was verified with fluoroscopy. While maintaining guide wire position, the delivery system was slowly removed. Contrast injections were used to determine device patency and each stent/SDS was evaluated for acute performance characteristics. For Groups IV, and 3V: Two overlapping stents of the same type were implanted. Each SDS was advanced over the guide wire to the deployment site. The balloon was then inflated at a steady rate to a pressure to target a balloon-artery ratio of 1.05:1-1.15:1. Confirmation of this balloon-artery ratio was made when the angiographic images were quantitatively assessed. After the target balloon-artery ratio was achieved, vacuum was applied to the inflation device in order to deflate the balloon. Complete balloon deflation was verified with fluoroscopy. The delivery system was slowly removed. Any resistance during delivery or removal of the stent delivery system was noted. The second stent of the overlapped pair was advanced to achieve an approximately 50% overlap. Contrast injections were used to determine device patency and each stent/SDS was evaluated for acute performance characteristics. These processes were repeated until stents were deployed in up to 3 vessels.

There were no differences between the Sirolimus DES and Vision BMS controls with respect to device delivery and deployment. Sirolimus DES graded slightly better for trackability. There were few challenges in the swine coronary artery model; however, the proximal Vision BMS in the overlapping stent groups often resisted tracking into the distal stent, which was not observed in the Sirolimus DES groups even with the use of a floppy guidewire. Accuracy of deployment was better with the Sirolimus DES than with the Vision BMS as shown when the Vision BMS would, on occasion, deploy slightly more distal than the target. This was not observed with the Sirolimus DES.

Angiography was performed and recorded on Day 0 (before stent placement, during balloon expansion, and after stent implant) and prior to necropsy. On Day 3 and 30 animals were euthanized and subjected to a comprehensive necropsy and the hearts were collected.

Balloon to artery ratios (ratio of balloon diameter size during peak inflation pressure to the vessel diameter size before stent placement) were calculated from the Quantitative Coronary Angiography (QCA) measurements by dividing the baseline vessel diameter size into the balloon diameter size. Percent stenosis was calculated by subtracting the prenecropsy minimum lumen diameter from the post-implant reference diameter and dividing that value by the post-implant reference diameter. For vessels containing overlapped stents, the proximal and distal stents were averaged to obtain values per vessel. Baseline vessel diameters were similar for all groups of stents at each time point. Average balloon to artery ratios (B:A ratios) for the single Sirolimus DES and single Vision BMS were similar and the overlapping stents were also similar in comparison in both time points. They ranged from approximately 1.09:1 to 1.15:1 which reduces injury to the artery wall and minimizes risk of malapposition. Angiographic evaluation showed there was no difference in mean percent stenosis between any stent groups at either time point. Overall acute performance characteristics and handling of the sirolimus eluting stent & stent systems during implant were comparable to the Vision BMS. Although stent migration did occur, it was infrequent and involved both Sirolimus DES and Vision BMS and always occurred in the proximal LAD where vessel tapering and limited angiographic angles can sometimes affect the accuracy of QCA.

Histomorphometry results revealed that with regard to lumen area, neointimal area, medial area, artery area, lumen diameter, IEL diameter, stent diameter, arterial diameter, lumen area/artery area ratio, neointimal area/medial area ratio, EEL/IEL ratio, area percent stenosis and neointimal thickness, there was no difference at day 30 or 90 between the Sirolimus DES and the Vision BMS device when nonoverlapping stents were implanted (i.e. single stent per vessel). Histomorphometry results revealed that with regard to lumen area, artery area, lumen diameter, IEL diameter, stent diameter, arterial diameter, lumen area/artery area ratio, neointimal area/medial area ratio, and EEL/IEL ratio, there was no difference at day 30 or 90 between the Sirolimus DES and the Vision BMS device when overlapping stents were implanted. Definitions of the various parameters listed in Table 15 are provided in Example 25.

Histomorphometry results revealed that with regard to neointimal area, medial area, area percent stenosis and neointimal thickness, there was a statistically significant difference at day 30 between the Sirolimus DES and the Vision BMS device when overlapping stents were implanted. For neointimal area at day 30 between the Sirolimus DES and the Vision BMS device when overlapping stents were implanted, the neointimal area of the vessel having overlapping Sirolimus DES implanted therein (1.38 mm² ± 0.44 mm²) was significantly lower than neointimal area of the vessel having overlapping Vision BMS implanted therein (2.26 mm² ± 0.82 mm²). For medial area at day 30 between the Sirolimus DES and the Vision BMS device when overlapping stents were implanted, the medial area of the vessel having overlapping Sirolimus DES implanted therein (1.15 mm²± 0.27 mm²) was significantly lower than medial area of the vessel having overlapping Vision BMS implanted therein (1.88 mm²± 0.83 mm²). For area percent stenosis at day 30 between the Sirolimus DES and the Vision BMS device when overlapping stents were implanted, the area percent stenosis of the vessel having overlapping Sirolimus DES implanted therein (22% ± 9%) was significantly lower than area percent stenosis of the vessel having overlapping Vision BMS implanted therein (35% ± 12%). For neointimal thickness at day 30 between the Sirolimus DES and the Vision BMS device when overlapping stents were implanted, the neointimal thickness of the vessel having overlapping Sirolimus DES implanted therein (0.17 mm ± 0.07 mm) was significantly lower than neointimal thickness of the vessel having overlapping Vision BMS implanted therein (0.28 mm ± 0.11 mm). Quantified neointima, media, and percent stenosis were significantly reduced in the overlapping Process 1 stents at 30 days compared to Vision overlapping BMS. Other histomorphological endpoints were similar. Sirolimus DES therefore resulted in similar or better vessel wall morphology compared to the control.

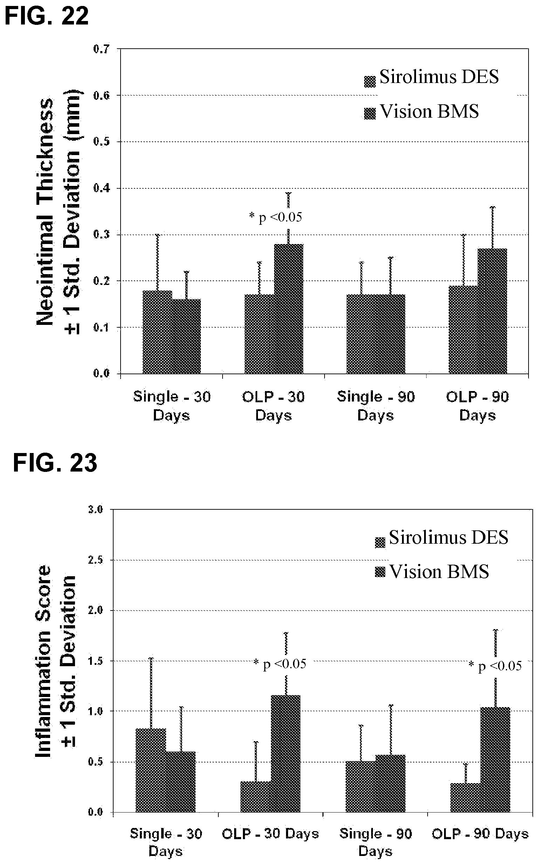

Furthermore, for neointimal thickness at day 90 between the Sirolimus DES and the Vision BMS device when overlapping stents were implanted, the neointimal thickness of the vessel having overlapping Sirolimus DES implanted therein was significantly lower than neointimal thickness of the vessel having overlapping Vision BMS implanted therein. Figure 22 shows the neointimal thickness score and standard deviation recorded at each of 30 days and 90 days in both a single and overlapping (OLP) Sirolimus DES and Vision BMS stent implantation in a porcine model as described in this example. In Figure 22, the Sirolimus DES average neointimal thickness is shown at each time point as the first bar, and the Vision BMS control average neointimal thickness is shown as the second bar at each time point.

The results presented in this example show for all of these parameters, whether at 30 days or at 90 days, that the Sirolimus DES was at least as good as the Vision BMS from a histomorphometric point of view. For certain of the parameters noted above, these results show that whether at 30 days or at 90 days, the Sirolimus DES was statistically better than the Vision BMS from a histomorphometric point of view.

Summary of histopathology results is presented in Figure 23 (for inflammation scores) and described herein. Scores for each parameter are provided according to the definitions provided in Example 25' supra, except as provided in Tables 16 and Table 17, which further explain the scores for the parameters noted therein.

**Table 16**

| **Score** | **Inflammation Score Matrix- Observation** |
|---|---|
| 0 | No cells present |
| 1 | Fewer than ∼ 20 cells associated with stent strut |
| 2 | Greater than ∼ 20 cells associated with stent strut, with or without tissue effacement and little to no impact on tissue function |
| 3 | Greater than 20 cells associated with stent strut, with effacement of adjacent vascular tissue and adverse impact on tissue function |

**Table 17**

| **Score** | **Injury Score Matrix- Observation** |
|---|---|
| 0 | No injury; Internal elastic lamina (IEL) intact |
| 1 | Disruption of IEL |
| 2 | Disruption of tunica media |
| 3 | Disruption of external elastic lamina (EEL)/tunica adventitia |

Sections of the non-stented proximal and distal portions of the stented arteries were similarly assessed and scored for histologic parameters as above (excluding neointimal fibrin and neointimal maturation) but were not assessed for histomorphometry.

Implantation of either Sirolimus DES or Vision BMS for 3, 30, or 90 days resulted in negligible vessel wall injury and no significant differences between the two types of stents with exception of the 30 day overlapping stents; the Sirolimus DES values were significantly lower than Vision BMS (p<0.05). Examination of injury score frequency demonstrated Grade 2 (mild to moderate) and/or Grade 3 (severe) injury was generally rare (∼ <10%, per strut incidence), regardless of time point; there was a slight increase in incidence with single Sirolimus DES (18%) vs control and overlapped Vision (15%) BMS vs overlapped Sirolimus DES at Day 30.

Inflammation was also similar for both types of stents at both time points with the exception of overlapping stents at 30 days and at 90 days where Sirolimus DES inflammation was significantly reduced compared to Vision BMS (p<0.05). Figure 23 shows the average inflammation score and standard deviation recorded at each of 30 days and 90 days in both a single and overlapping (OLP) Sirolimus DES and Vision BMS stent implantation in a porcine model as described in this example. In Figure 23, the Sirolimus DES average inflammation score is shown at each time point as the first bar, and the Vision BMS control average inflammation score is shown as the second bar at each time point.

Regardless of the variability in the inflammatory response between the different configurations (i.e., single vs. overlap), the overall magnitude, incidence and nature of the strut-associated foreign body response to the Sirolimus DES groups was comparable (single stent) or significantly decreased (overlapped stent) compared to that of the Vision BMS control. Comparable inflammatory response between the Sirolimus DES and the BMS control may be interpreted as meaning that the Sirolimus DES inflammation response is at least as good as the BMS control inflammation response. Comparable inflammatory response between the Sirolimus DES and the BMS control may be interpreted as meaning that the Sirolimus DES inflammation response is equivalent to the BMS control inflammation response. Comparable inflammatory response between the Sirolimus DES and the BMS control may be interpreted as meaning that the Sirolimus DES inflammation response is no worse than the BMS control inflammation response.

Histopathological assessment demonstrated the sirolimus eluting stent had no adverse effects on the stented arteries with a tissue response which was, with few exceptions, comparable to the Vision BMS control. Quantified neointima, media, and percent stenosis were significantly reduced in the Sirolimus DES overlapping stents at 30 days compared to Vision overlapping BMS. All other histomorphological endpoints were similar. The polymer/drug coating of the sirolimus eluting stent was interpreted to have favorable biocompatibility, regardless of the coating process, and elicited little to no associated inflammatory response. Neointimal fibrin was significantly increased in the sirolimus eluting stent compared to Vision BMS (whether single or overlapping) consistent with the presence of sirolimus. Unfavorable/adverse observations such as granulomatous inflammation, myocardial fibrosis (i.e. "infarction) were uncommon and generally observed in both the sirolimus eluting stented and Vision BMS stented arteries. Regardless of the device involved, such observations were interpreted to be consistent with the low incidence of occurrence and/or idiosyncratic responses encountered in this model. The polymer/drug coating of the sirolimus eluting stent was typically intimately associated with the strut with occasional observation of small amounts of polymer in the neointima adjacent to its strut; however, this extra-strut deposition of polymer/drug appeared to have no adverse effect on the tissue. Refractile foreign material with associated granulomatous inflammation was rarely observed in the myocardium of both sirolimus eluting stented and the Vision BMS stented arteries. Although the exact source of the foreign material was not apparent, it is interpreted to be related to the interventional procedure and not the stent itself. The sirolimus eluting stent systems therefore resulted in similar or better vessel wall morphology compared to the control (Vision BMS).

Results of this study demonstrate that following 3, 30, and/or 90 days of implantation in porcine coronary arteries, the sirolimus eluting stent described herein (Sirolimus DES) showed acceptable vascular healing and produced a minimal tissue response which was equivalent or favorable to that observed with Vision BMS.

Provided herein is a device comprising a stent comprising a cobalt-chromium alloy; and a coating on the stent; wherein the coating comprises at least one polymer and at least one active agent; wherein at least one of: quantified neointima, media, percent stenosis, wall injury, and inflammation exhibited at 30 days following implantation of the device in a first artery of an animal is significantly reduced for the device as compared to a bare metal cobalt-chromium stent implanted in a second artery of an animal when both the device and the bare metal cobalt chromium stent are compared in a the study, wherein the study design overlaps two of the devices in the first artery and overlaps two of the bare metal cobalt-chromium stents in the second artery.

In some embodiments, the test performed to determine significant differences between the device and the bare metal cobalt-chromium stent is the Mann-Whitney Rank Sum Test and the p value is less than 0.10.

In some embodiments, the test performed to determine significant differences between the device and the bare metal cobalt-chromium stent is the Mann-Whitney Rank Sum Test and the p value is less than 0.05.

In some embodiments, at least one of wall injury, inflammation, neointimal maturation, and adventitial fibrosis of the device tested at day 3 of the animal study is equivalent to the bare metal stent.

In some embodiments, at least one of lumen area, artery area, lumen diameter, IEL diameter, stent diameter, arterial diameter, lumen area/artery area ratio, neointimal area/medial area ratio, EEL/IEL ratio, endothelialization, neointimal maturation, and adventitial fibrosis of the device tested at day 30 of the animal study is equivalent to the bare metal stent.

In some embodiments, at least one of lumen area, artery area, neointimal area, medial area, percent stenosis, wall injury, and inflammation of the device tested at day 30 of the animal study is equivalent to the bare metal stent.

In some embodiments, at least one of lumen area, artery area, neointimal area, medial area, percent stenosis, wall injury, inflammation, endothelialization, neointimal maturation, and adventitial fibrosis of the device tested at day 30 of the animal study is equivalent to the bare metal stent.

Provided herein is a device comprising a stent comprising a cobalt-chromium alloy; and a coating on the stent; wherein the coating comprises at least one polymer and at least one active agent; wherein at least one of: neointimal thickness exhibited at 90 days following implantation of the device in a first artery of an animal and inflammation exhibited at 90 days following implantation of the device in a first artery of an animal is significantly reduced for the device as compared to a bare metal cobalt-chromium stent implanted in a second artery of an animal when both the device and the bare metal cobalt chromium stent are compared in a study, wherein the study comprises overlapping two of the devices in the first artery and overlapping two of the bare metal cobalt-chromium stents in the second artery.

In some embodiments, the test performed to determine significant differences between the device and the bare metal cobalt-chromium stent is the Mann-Whitney Rank Sum Test and the p value is less than 0.10.

In some embodiments, the test performed to determine significant differences between the device and the bare metal cobalt-chromium stent is the Mann-Whitney Rank Sum Test and the p value is less than 0.05.

Provided herein is a method comprising providing a coated stent comprising a stent comprising a cobalt-chromium alloy; and a coating on the stent; wherein the coating comprises at least one polymer and at least one active agent, wherein at least one of: quantified neointima, media, percent stenosis, wall injury, and inflammation exhibited at 30 days following implantation of the device in a first artery of an animal is significantly reduced for the device as compared to a bare metal cobalt-chromium stent implanted in a second artery of an animal when both the device and the bare metal cobalt chromium stent are compared in a study, wherein the study comprises overlapping two of the devices in the first artery and overlapping two of the bare metal cobalt-chromium stents in the second artery.

Provided herein is a method comprising providing a coated stent comprising a stent comprising a cobalt-chromium alloy; and a coating on the stent; wherein the coating comprises at least one polymer and at least one active, wherein at least one of: neointimal thickness exhibited at 90 days following implantation of the device in a first artery of an animal and inflammation exhibited at 90 days following implantation of the device in a first artery of an animal is significantly reduced for the coated stent as compared to a bare metal cobalt-chromium stent implanted in a second artery of an animal when both the device and the bare metal cobalt chromium stent are compared in a study, wherein the study comprises overlapping two of the coated stents in the first artery and overlapping two of the bare metal cobalt-chromium stents in the second artery..

In some embodiments, the test performed to determine significant differences between the device and the bare metal cobalt-chromium stent is the Mann-Whitney Rank Sum Test and the p value is less than 0.10. In some embodiments, the test performed to determine significant differences between the device and the bare metal cobalt-chromium stent is the Mann-Whitney Rank Sum Test and the p value is less than 0.05.

In some embodiments, at least one of wall injury, inflammation, neointimal maturation, and adventitial fibrosis of the device tested at day 3 of the study is equivalent to the bare metal stent.

In some embodiments, at least one of lumen area, artery area, lumen diameter, IEL diameter, stent diameter, arterial diameter, lumen area/artery area ratio, neointimal area/medial area ratio, EEL/IEL ratio, endothelialization, neointimal maturation, and adventitial fibrosis of the device tested at day 30 of the study is equivalent to the bare metal stent.

In some embodiments, at least one of lumen area, artery area, neointimal area, medial area, percent stenosis, wall injury, and inflammation of the device tested at day 30 of the animal study is equivalent to the bare metal stent.

In some embodiments, at least one of lumen area, artery area, neointimal area, medial area, percent stenosis, wall injury, inflammation, endothelialization, neointimal maturation, and adventitial fibrosis of the device tested at day 30 of the animal study is equivalent to the bare metal stent.

### Example 32:

Blood serum levels of porcine subjects having coated stents implanted were tested at multiple time points. The coated stents implanted were prepared as follows: coated stents for the study comprise a coating that was deposited on the stent by deposition of rapamycin in dry powder form by RESS methods and equipment described herein and deposition of polymer particles by RESS methods and equipment described herein. A PDPDP (Polymer, sinter, Drug, Polymer, sinter, Drug, Polymer, sinter) coating sequence was used wherein the polymer was 50:50 PLGA, and the drug was rapamycin. The sinter step was performed at 100°C/1.03 MPa (150 psi)/10 min after each "P" (or polymer) layer. There was 135 micrograms +/- 15% sirolimus on each coated stent in this study. The coating was about 5-15 micrometers thick on each stent, and comprised a thicker coating on the abluminal surface (coating bias). The coating encapsuled each of the stents.

Multiple batches of coated stents were created, implanted in the porcine subjects, one coated stent per subject, and each subject's blood serum levels were tested at multiple time points. Stents were introduced into the coronary arteries by advancing the stent delivery system through the guide catheter and over the guide wire to the deployment site within the coronary artery. The balloon was then inflated at a steady rate to a pressure sufficient to target a visually assessed balloon-artery ratio of 1.1:1-1.2:1. Confirmation of this balloon-artery ratio was made when the angiographic images were quantitatively assessed. After the target balloon-artery ratio was achieved, vacuum was applied to the inflation device in order to deflate the balloon. Complete balloon deflation was verified with fluoroscopy. While maintaining guide wire position, the delivery system was then slowly removed. Contrast injections were used to determine device patency and acute deployment characteristics.

Nine subjects' blood serum levels were tested at each of the following target time points: Time 0 (before implantation), 5 minutes (following implantation), 15 minutes, 30 minutes, 1 hour, 2 hours, 4 hours, 6 hours, 24 hours, day 2, day 3, day 4, day 6, day 8, day 14, day 21, day 30, day 60, and day 90. Six of the subjects' blood serum levels were tested at day 180. Each sample was drawn at times which were +/- 5% of each target time point. The whole blood samples were placed in K2 EDTA tubes and then transferred to cryovials for storage in a ≤-80°C freezer. Samples were collected from any vascular source. Telazol® (2-4 mg/kg IM) and/or isoflurane inhalant was administered as needed for chemical restraint.

The whole blood sample was tested for rapamycin concentration using LC-MS/MS. Samples were quantified for rapamycin using ascomycin as the internal standard. The method of testing can be summarized as follows: The matrix and anticoagulant used was porcine K2 EDTA whole blood, the extraction procedure included protein precipitation followed by solid phase extraction (SPE), the sample volume was 0.200 mL, the analysis was by LC-MS/MS using positive Turbolonspray ionization mode while operating the instrument in the multiple-reaction-monitoring (MRM) mode, the calibration curve and weighting was linear 1/(x^2), the standard curve range was 0.100ng/mL through 10ng/mL, and the QC concentrations were 0.300ng/mL for QC-Low, 0.750ng/mL for QC-mid, and 8.00ng/mL for QC-high.

For every time point, and for every subject, the concentration of rapamycin (ng/mL) was determined to be below quantifiable limit (BQL), except for the following justified exceptions: one subject had no sample taken at time 0 but all other readings for this subject were taken and were BQL, one subject had a low internal standard area at day 2 which indicated test method setup or processing error but all other readings for this subject were BQL, one subject's day 60 sample was replaced with a replacement subject's day 60 sample, this reading for the replacement subject, and all other readings for the original subject were BQL. The quantification limit in this study is <0.100ng/mL. That is, the test could not detect an amount of rapamycin in systemic whole blood that was below 0.100ng/mL concentration. The concentration of rapamycin is expressed in ng rapamycin per mL of whole blood.

Provided herein is a method comprising providing a coated stent comprising a stent and a coating thereon, wherein the coating comprises at least one polymer and rapamycin; implanting the coated stent in a subject, determining an amount of rapamycin in the subject systemically by using a detection test of whole blood of the subject for active agent at any two or more time points during which elution of rapamycin from the coated stent is occurring in the subject, wherein there is less than 0.100 ng of rapamycin per mL of whole blood of the subject at the time points tested in the determining step.

In some embodiments, the detection test is conducted at any two or more of the following time points: 5 minutes after implantation of the coated stent, 15 minutes after implantation of the coated stent, 30 minutes after implantation of the coated stent, 1 hour after implantation of the coated stent, 2 hours after implantation of the coated stent, 4 hours after implantation of the coated stent, 6 hours after implantation of the coated stent, 24 hours after implantation of the coated stent, day 2 after implantation of the coated stent, day 3 after implantation of the coated stent, day 4 after implantation of the coated stent, day 6 after implantation of the coated stent, day 8 after implantation of the coated stent, day 14 after implantation of the coated stent, day 21 after implantation of the coated stent, day 30 after implantation of the coated stent, day 60 after implantation of the coated stent, and day 90 after implantation of the coated stent.

In some embodiments, the detection test is conducted at any three or more of the time points. In some embodiments, the detection test is conducted at any four or more of the time points. In some embodiments, the detection test is conducted at any five or more of the time points. In some embodiments, the detection test is conducted at any six or more of the time points.

In some embodiments, one of the time points at which the detection test is conducted is any of: 14 days after implantation of the coated stent in a subject, 21 days after implantation of the coated stent in a subject, 30 days after implantation of the coated stent in a subject, and 60 days after implantation of the coated stent in a subject. In some embodiments, one of the time points is 180 days after implantation of the coated stent in a subject.

In some embodiments, the quantifiable limit of the detection test of 0.100 ng of active agent per mL of whole blood. In some embodiments, the detection test comprises using LC-MS/MS. In some embodiments, timing of testing for amount of active agent is based on a theoretical elution of active agent from the coated stent. In some embodiments, theoretical elution of active agent from the coated stent is based on one of in-vitro and in-vivo tests of elution rates and timing.

### Example 33:

Systemic levels of pharmaceutical agent in whole blood of subjects having coated stents implanted may be tested at multiple time points. The coated stents implanted may be prepared as follows: coated stents for the study comprise a coating deposited on the stent by deposition of a pharmaceutical agent in dry powder form by RESS methods and equipment described herein and deposition of polymer particles by RESS methods and equipment described herein. A PDPDP (Polymer, sinter, Drug, Polymer, sinter, Drug, Polymer, sinter) coating sequence may be used wherein the polymer is at least one of a bioabsorbable polymer and a durable polymer, and the drug is a pharmaceutical agent as described elsewhere herein. In some embodiments, the pharmaceutical agent is a macrolide immunosuppressive agent as described herein. In some embodiments, the pharmaceutical agent is at least in part crystalline.

The implantation into the subjects may be conducted as noted with respect to Example 32 for porcine, or in another appropriate manner as known to one of skill in the art, for example, when the subject is a human.

Subjects' levels of pharmaceutical agent may be tested at any one or multiple of the following target time points: Time 0 (before implantation), 5 minutes (following implantation), 15 minutes, 30 minutes, 1 hour, 2 hours, 4 hours, 6 hours, 24 hours, day 2, day 3, day 4, day 6, day 8, day 14, day 21, day 30, day 60, day 90, and day 180. Each sample may be drawn at times that are +/- 5% of each target time point. The whole blood samples may be placed in K2 EDTA tubes and then transferred to cryovials for storage in a ≤-80°C freezer. Samples may be collected from any vascular source. Telazol® (2-4 mg/kg IM) and/or isoflurane inhalant may be administered as needed for chemical restraint. Test method and setup may be according to Example 32, or according to another appropriate setup and method known to one of skill in the art.

The whole blood sample may be tested for pharmaceutical agent concentration. It is expected that for any of the target time points, or multiple of the target time points, and for every subject, the concentration of the pharmaceutical agent (ng/mL) will be below quantifiable limit (BQL), except for justified exceptions. The quantification limit in this study is dependent upon the particular pharmaceutical agent tested. However, for macrolide immunosuppressive agents, as noted herein, a quantifiable limit may be, for non-limiting example, <0.100ng/mL. That is, the test does not detect an amount of macrolide immunosuppressive agents in systemic whole blood that is below 0.100ng/mL concentration. The concentration of the macrolide immunosuppressive agent is expressed in ng macrolide immunosuppressive agent per mL of whole blood.

It is expected that the pharmaceutical agent will not be found in amounts greater than or equal to the quantifiable limit in systemic whole blood samples when coated stents are prepared and implanted as noted herein. This is another way of saying that the pharmaceutical agent from coated stents prepared and implanted herein does not wash away into the system of the subject in quantifiable amounts. Moreover, even if some agent from coated substrates prepared and implanted does wash away into the system of the subject (for example, if the agent were different, the coating were different, the target tissue were different, the substrate were different, and/or the agent could be detected in smaller amounts), that amount can be controlled by adjusting the parameters of the coating or the materials of the coating as noted herein to effectively control the amount of agent that is washed into the system. Thus, the coated stent prepared as noted herein efficiently transfers the pharmaceutical agent (e.g. rapamycin) to the target tissue (i.e. the artery), and does not deliver the agent systemically when formulated as designed in this example, at least. This characteristic can be translated to other substrates coated according to processes noted herein, and to other agents and polymers for that matter, despite the specific reference to stents and coatings thereon, and despite description herein regarding the target tissue being an artery.

Provided herein is a method comprising providing a coated stent comprising a stent and a coating thereon, wherein the coating comprises at least one polymer and an active agent; implanting the coated stent in a subject, determining an amount of active agent in the subject systemically by using a detection test of whole blood of the subject for active agent at any two or more time points during which elution of active agent from the coated stent is occurring in the subject, wherein there is less than 0.100 ng of active agent per mL of whole blood of the subject at the time points tested in the determining step.

In some embodiments, the detection test is conducted at any two or more of the following time points: 5 minutes after implantation of the coated stent, 15 minutes after implantation of the coated stent, 30 minutes after implantation of the coated stent, 1 hour after implantation of the coated stent, 2 hours after implantation of the coated stent, 4 hours after implantation of the coated stent, 6 hours after implantation of the coated stent, 24 hours after implantation of the coated stent, day 2 after implantation of the coated stent, day 3 after implantation of the coated stent, day 4 after implantation of the coated stent, day 6 after implantation of the coated stent, day 8 after implantation of the coated stent, day 14 after implantation of the coated stent, day 21 after implantation of the coated stent, day 30 after implantation of the coated stent, day 60 after implantation of the coated stent, and day 90 after implantation of the coated stent.

In some embodiments, the detection test is conducted at any three or more of the time points. In some embodiments, the detection test is conducted at any four or more of the time points. In some embodiments, the detection test is conducted at any five or more of the time points. In some embodiments, the detection test is conducted at any six or more of the time points.

In some embodiments, one of the time points at which the detection test is conducted is any of: 14 days after implantation of the coated stent in a subject, 21 days after implantation of the coated stent in a subject, 30 days after implantation of the coated stent in a subject, and 60 days after implantation of the coated stent in a subject. In some embodiments, one of the time points is 180 days after implantation of the coated stent in a subject.

In some embodiments, the quantifiable limit of the detection test of 0.100 ng of active agent per mL of whole blood. In some embodiments, the detection test comprises using LC-MS/MS. In some embodiments, timing of testing for amount of active agent is based on a theoretical elution of active agent from the coated stent. In some embodiments, theoretical elution of active agent from the coated stent is based on one of in-vitro and in-vivo tests of elution rates and timing.

### Example 34:

Pharmacokinetic studies were performed on coated stents prepared and implanted as noted in Example 32. As noted therein, the coated stents implanted were prepared as follows: coated stents for the study comprise a coating that was deposited on the stent by deposition of rapamycin in dry powder form by RESS methods and equipment described herein and deposition of polymer particles by RESS methods and equipment described herein. A PDPDP (Polymer, sinter, Drug, Polymer, sinter, Drug, Polymer, sinter) coating sequence was used wherein the polymer was 50:50 PLGA, and the drug was rapamycin. The sinter step was performed at 100°C/1.03 MPa (150 psi)/10 min after each "P" (or polymer) layer. There was 135 micrograms +/- 15% sirolimus on each coated stent in this study. The coating was about 5-15 micrometers thick on each stent, and comprised a thicker coating on the abluminal surface (coating bias). The coating encapsuled each of the stents.

Multiple batches of coated stents were created, implanted in the porcine subjects as noted in Table 20a. Stents were introduced into the coronary arteries by advancing the stent delivery system through the guide catheter and over the guide wire to the deployment site within the coronary artery. The balloon was then inflated at a steady rate to a pressure sufficient to target a visually assessed balloon-artery ratio of 1.1:1-1.2:1. Confirmation of this balloon-artery ratio was made when the angiographic images were quantitatively assessed. After the target balloon-artery ratio was achieved, vacuum was applied to the inflation device in order to deflate the balloon. Complete balloon deflation was verified with fluoroscopy. While maintaining guide wire position, the delivery system was then slowly removed. Contrast injections were used to determine device patency and acute deployment characteristics. One stent or two overlapping stents of the same type were placed in each of up to 3 coronary arteries. In groups where there were overlapping stents implanted, the second stent of the overlapped pair was advanced to achieve an approximate 50% overlap (placed proximally to the first-implanted stent). One animal implanted with 1 DES died early and was replaced.

Forty-eight Yucatan pigs underwent a single interventional procedure on Day 0 in which stents were implanted in up to three coronary arteries, as shown in Table 20a. Quantitative Coronary Angiography (QCA) was performed and recorded on Day 0 (before stent placement, during stent deployment (balloon), and after stent deployment) and prior to euthanasia on days 30, 45, 60, 90, 180, and 365. Activated clotting times were monitored during the interventional procedures. Clinical observations were performed daily and body weights were recorded before implant, approximately 2 weeks post-implant, and monthly thereafter, including the day of necropsy (except day 1, 3, and 7). Animals were euthanized, subjected to necropsy and hearts collected for PK analysis (Groups 1 and 2) on Days 1, 3, 7, 14, 21, 30, 45, 60, 90, 180, and 365 (± 5%) or for SEM analysis on day 90 (Groups 3, 4) or day 30 (Groups 3S, 4S, 3V and 4V). Serial analysis of blood drug levels were conducted on Groups 2, 3 and 4 at Time 0 (prior to implant), 5, 15, 30 minutes, 1, 2, 4, 6, 24 hours, 2, 3, 4, 6, 8, 14, 21, 30, 60, 90, and 180 days (± 5% for all time points).

**Table 20a: Study Design**

| Group Number | Test/Control Articles | Number of Test/Control Articles | Sirolimus Blood Analysis Time Points | Necropsy Time Point |
|---|---|---|---|---|
| 1 (PK) | DES | n = 6 per time point | NA | 1, 3, 7, 14, 21, 30, 45, 60, 90, and 365 days (± 5%) |
| 2 (PK) | DES | n = 6 | Time 0 (prior to implant), 5, 15, 30 minutes, 1, 2, 4, 6, 24 hours, 2, 3, 4, 6, 8, 14, 21, 30, 60, 90, 180 days (±5%) | 180 days (± 5%) |
| 3 (SEM) | DES | n = 3 | 0 (prior to implant), 5, 15, 30 minutes and 1, 2, 4, 6, 24 hours and 2, 3, 4, 6, 8, 14, 21, 30, 60, 90 days (±5%) | 90 days (± 5%) |
| 4 (SEM) | Bare Metal Stent (BMS) | n = 3 | | |
| 3S (SEM) | DES | n = 3 | NA | 30 days (± 5%) |
| 4S (SEM) | Bare Metal Stent (BMS) | n = 3 | | |
| 3V (overlapped SEM) | DES | n = 3 pairs per time point | | 30, 90 days (± 5%) |
| 4V (overlapped SEM) | Bare Metal Stent (BMS) | n = 3 pairs per time point | | |

Balloon to artery ratios (ratio of balloon diameter size during peak inflation pressure to the vessel diameter size before stent placement) were calculated from the QCA measurements by dividing the baseline vessel diameter size into the balloon diameter size. Percent stenosis was calculated by subtracting the prenecropsy minimum lumen diameter from the post-implant reference diameter and dividing that value by the post-implant reference diameter. For vessels containing overlapped stents, the proximal and distal stents were averaged to obtain values per vessel. The means and standard deviations for the balloon to artery ratios are summarized in Table 20b.

**Table 20b: Calculated Vascular Angiography Values**

| Time Point (+/- 5%) | | Baseline Diameter (mm) | | Balloon to Artery Ratio | | % Stenosis | |
|---|---|---|---|---|---|---|---|
| | n= | Mean | sd | Mean | sd | Mean | sd |
| Day 1 | 6 | 2.81 | 0.15 | 1.10 | 0.05 | | |
| Day 3 | 6 | 2.75 | 0.17 | 1.15 | 0.05 | | |
| Day 7 | 6 | 2.85 | 0.18 | 1.13 | 0.03 | | |
| Day 14 | 6 | 2.82 | 0.09 | 1.15 | 0.03 | | |
| Day 21 | 6 | 2.84 | 0.14 | 1.14 | 0.05 | | |
| Day 30 Single Stents (MiStent) | 9 | 2.83 | 0.13 | 1.12 | 0.03 | 28 | 15 |
| Day 30 Single Stents (BMS) | 3 | 2.76 | 0.10 | 1.15 | 0.05 | 15 | 7 |
| Day 30 Overlapping Stents (MiStent) | 3 | 2.77 | 0.32 | 1.14 | 0.06 | 20 | 11 |
| Day 30 Overlapping Stents (BMS) | 3 | 2.63 | 0.28 | 1.16 | 0.04 | 17 | 7 |
| Day 45 | 6 | 2.76 | 0.12 | 1.14 | 0.05 | 23 | 17 |
| Day 60 | 6 | 2.77 | 0.17 | 1.15 | 0.03 | 21 | 7 |
| Day 90 Single Stents (MiStent) | 6 | 2.69 | 0.16 | 1.16 | 0.04 | 25 | 15 |
| Day 90 Single Stents (BMS) | 3 | 2.76 | 0.13 | 1.14 | 0.03 | 12 | 9 |
| Day 90 Overlapping Stents (MiStent) | 3 | 2.74 | 0.23 | 1.15 | 0.02 | 28 | 3 |
| Day 90 Overlapping Stents (BMS) | 3 | 2.63 | 0.06 | 1.19 | 0.02 | 26 | 2 |
| Day 180 | 6 | 2.72 | 0.14 | 1.11 | 0.06 | 19 | 13 |
| Day 365 | 6 | 2.82 | 0.08 | 1.14 | 0.03 | 7 | 7 |

Overlapping means are the average of proximal and distal stents. Average balloon to artery ratios ranged by timepoint from 1.1:1 to 1.16:1 which ensures good stent apposition while minimizing injury to the arterial wall. Mean percent stenosis was less than 28% at 30 days and reduced over time.

Subjects were euthanized at the testing time points, and the tissue adjacent the stent (for non-limiting example, tissue surrounding the stent-referred to generally as arterial tissue) was extracted for analysis of the amount of pharmaceutical agent therein. Stents were also analyzed for the amount of pharmaceutical agent remaining on the coated stent at each time point. Testing methods as noted elsewhere herein were used to determine the amount of pharmaceutical agent remaining on the stent or in the tissue, and/or are methods that would be known to one of skill in the art.

All systemic (blood level) sirolimus concentration values were below the quantification limit (BQL) (< 0.100 ng/mL) at all time points.

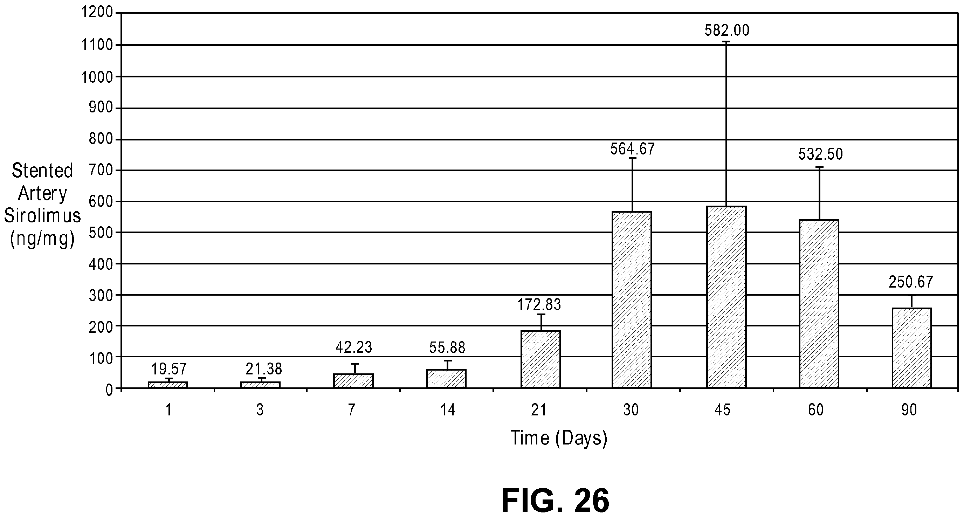

Summary µg sirolimus remaining on the stent and released (calculated based on the amount remaining on the stent) is shown in Table 20c and fractional sirolimus release over 90 days can be found in Figure 24. Incremental sirolimus release rate (µg/day) can be found in Figure 25. Stented artery sirolimus concentration data are summarized in Figure 26.

**Table 20c: Summary Sirolimus Remaining (measured) ± standard deviation and Released (calculated from Day 0 baseline measurement) ± standard deviation**

| **Summary Stent-Associated Sirolimus** | | | | | |
|---|---|---|---|---|---|
| Day | Drug Remaining (µg) | | Drug Released (µg) | | n |
| | Mean | ± | Mean | ± | |
| 0 | 137.00 | 6.245 | 0 | 0 | 3 |
| 1 | 137.00 | 4.980 | 1.67 | 3.615 | 6 |
| 3 | 135.83 | 5.492 | 2.83 | 3.656 | 6 |
| 7 | 127.83 | 2.563 | 9.17 | 2.563 | 6 |
| 14 | 119.67 | 7.789 | 17.33 | 7.789 | 6 |
| 21 | 103.68 | 10.077 | 33.32 | 10.077 | 6 |
| 30 | 65.25 | 15.198 | 71.75 | 15.198 | 6 |
| 45 | 3.89 | 1.991 | 133.12 | 1.991 | 6 |
| 60 | 1.59 | 0.560 | 135.41 | 0.560 | 6 |
| 90 | 0.11 | 0.082 | 136.89 | 0.082 | 6 |
| 180 | BQL | n/a | BQL | n/a | 6 |
| 365 | BQL | n/a | BQL | n/a | 6 |

| | | | | | |
|---|---|---|---|---|---|
| BQL: Below quantification limit (50.0ng/mL in stents after dissolution with 10mL of methanol thus <0.5 µg rapamycin per stent) | | | | | |

Figure 24 shows Release of sirolimus from the Sirolimus DES appeared slower over the initial 14 days following implant compared to release from 14 to 45 days after implant. Average fractional release was 0.127±0.057 (approximately 13%) at Day 14, By 30 days, 0.524±0.111 (approximately 52%) of the initial sirolimus content was no longer associated with the stent, and release was nearly complete, 0.972±0.015 (approximately 97%), by Day 45 with minimal additional release from that point through 90 days (97 - 100%). Stent-associated sirolimus was BQL at Days 180 and 365.

Figure 25 depicts the incremental Stent Sirolimus Loss (Release) Rate from 1 to 90 Days. Average incremental elution rate (alternatively called release rate) was variable and appeared to peak around day 30 (4.27±1.69 µg/day). Incremental release rate was similar between days 30 and 45 then declined thereafter when measured at days 60 and 90 (0.15±0.04 µg/day at Day 60 and 0.05±0.00 µg/day at Day 90). Stent-associated sirolimus was BQL on Days 180 and 365 so not included in release calculation. Average incremental release rate ranged between approximately 1 and 4 µg/day for the first 45 days after implant with no value greater than 6 µg/day. Incremental release rate appeared to peak between days 30 and 45 then declined to a very low rate measured at days 60 and 90 (0.15±0.04 µg/day at Day 60 and 0.05±0.00 µg/day at Day 90).

Stented artery sirolimus concentration (in ng/mg of Tissue within Stented Segments) data are summarized in Figure 26, which shows that peak drug levels are concurrent with a period of maximum absorption of the coating and clearance of the coating and drug from the stent (from around 30 to around 45 days). According to Figure 26: at day 1, 19.57 ng of rapamycin were detected per mg tissue; at day 3, 21.38ng of rapamycin were detected per mg tissue; at day 7, 42.23ng of rapamycin were detected per mg tissue; at day 14, 55.88 ng of rapamycin were detected per mg tissue; at day 21, 172.83 ng of rapamycin were detected per mg tissue; at day 30, 564.67 ng of rapamycin were detected per mg tissue; at day 45, 582.00 ng of rapamycin were detected per mg tissue; at day 60, 532.50 ng (nanograms) of rapamycin were detected per mg tissue; at day 90, 250.67 ng of rapamycin were detected per mg tissue. Further testing detected 2.96 ng of rapamycin per mg tissue at 180 days and 0.396 ng of rapamycin per mg tissue at 365 days.

The total amount of rapamycin in the stented artery at each testing timepoint was as follows (average given): Day1: 0.68 µg, Day 3: 0.88 µg, Day 7: 2.24 µg, Day 14: 4.36 µg, Day 21: 14.72 µg, Day 30: 42.70 µg, Day 45: 35.28 µg, Day 60: 45.48 µg, Day 90: 15.40 µg, Day 180: 0.185 µg, Day 365: 0.036 µg.

In some embodiments, the drug is present in the vessel at about 90 days following implantation, at about 180 days following implantation, and/or at about 365 days following implantation. In some embodiments, the drug is present in the vessel at 90 days following implantation. In some embodiments, the drug is present in the vessel at 180 days following implantation. In some embodiments, the drug is present in the vessel at 365 days following implantation.

Rapamycin concentrations in non-stented proximal and distal artery segments adjacent to the stented segments are summarized in Table 20d. Samples that had no detectable peak or had the calculated concentration below the lower quantification limit are reported as BQL (< 0.500 ng/mL or < 0.0500 ng/mg).

**Table 20d: Sirolimus Concentration in Proximal and Distal Non-Stented Artery Segments**

| Day | Location/Amount | *Proximal* | | *Distal* | |
|---|---|---|---|---|---|
| | | *ng*/*mg* | *total µg* | *ng*/*mg* | *total µg* |
| 1 | *Mean* | BQL | BQL | 0.17 | 0.003 |
| | *sd* | n/a | n/a | 0.22 | 0.003 |
| | *n* | 0 | 0 | 5 | 5 |
| 3 | *Mean* | BQL | BQL | 0.11 | 0.002 |
| | *sd* | n/a | n/a | 0.03 | 0.001 |
| | *n* | 0 | 0 | 6 | 6 |
| 7 | *Mean* | 0.06 | 0.00 | 3.01 | 0.06 |
| | *sd* | n/a | n/a | 6.42 | 0.13 |
| | *n* | 1 | 1 | 5 | 5 |
| 14 | *Mean* | 0.71 | 0.02 | 0.98 | 0.02 |
| | *sd* | 1.09 | 0.02 | 1.50 | 0.03 |
| | *n* | 6 | 6 | 5 | 5 |
| 21 | *Mean* | 1.05 | 0.03 | 1.00 | 0.03 |
| | *sd* | 1.53 | 0.05 | 1.08 | 0.03 |
| | *n* | 6 | 6 | 6 | 6 |
| 30 | *Mean* | 4.14 | 0.11 | 2.27 | 0.06 |
| | *sd* | 4.72 | 0.11 | 1.81 | 0.04 |
| | *n* | 6 | 6 | 6 | 6 |
| 45 | *Mean* | 0.58 | 0.01 | 0.54 | 0.01 |
| | *sd* | 0.42 | 0.01 | 0.27 | 0.00 |
| | *n* | 6 | 6 | 6 | 6 |
| 60 | *Mean* | 0.46 | 0.01 | 0.44 | 0.01 |
| | *sd* | 0.22 | 0.01 | 0.24 | 0.00 |
| | *n* | 6 | 6 | 6 | 6 |
| 90 | *Mean* | 0.35 | 0.01 | 0.43 | 0.01 |
| | *sd* | 0.24 | 0.01 | 0.24 | 0.00 |
| | *n* | 6 | 6 | 6 | 6 |
| 180 | *Mean* | BQL | BQL | 0.06 | 0.001 |
| | *sd* | n/a | n/a | 0.01 | 0.00 |
| | *n* | 6 | 6 | 3 | 3 |
| 365 | *Mean* | BQL | BQL | BQL | BQL |
| | *sd* | n/a | n/a | n/a | n/a |
| | *n* | 6 | 6 | 6 | 6 |

| | | | | | |
|---|---|---|---|---|---|
| Note: number of analyzed vessels was 6 for all time points except Day 7 Proximal where n=5. "n" in table represents number of vessels with measurable sirolimus concentration n/a = not applicable; BQL = Below the quantification limit | | | | | |

Sirolimus concentrations in arterial tissue surrounding the stent increased for the first 30 days, plateaued until Day 60 and then declined at Day 90. By 180 days, levels had declined to <3.0 ng/mg of tissue and were very low at <0.4 ng/mg of tissue by 365 days. The drug measured in the tissue is in the form of either drug eluted from the coating or drug still trapped in coating that has dissociated from the stent.

Drug concentrations in proximal and distal artery tissue adjacent to the stented segment were variable. Sirolimus concentration was below the level of quantification in most proximal tissue segments during the first week following implant. Drug concentration was measureable in most of the corresponding distal non-stented tissue segments and the peak concentration for the duration of the study in distal tissue occurred at 7 days (3.01±6.42 ng/mg). At 14 days and subsequent time points, sirolimus was measurable in both proximal and distal segments with increased concentrations and increased variability at successive time points. Proximal non-stented segment sirolimus concentration increased to a peak value of 4.14±4.72 ng/mg at 30 days post implant and then declined to a range of approximately 0.4 - 0.6 ng/mg between 45 and 90 days. Distal non-stented segment sirolimus concentration declined following the 7 day peak to approximately 1 ng/mg between days 14 and 21 and then increased to 2.27±1.81 ng/mg at day 30. After day 30, distal non-stented segment sirolimus concentration also declined to approximately 0.4 - 0.5 ng/mg between days 45 and 90. The proximal tissue was BQL at 180 and 365 days and distal tissue was barely measureable at 180 days and BQL at 365 days. At all time points the sirolimus concentration in proximal and distal artery adjacent to the stented segments was less than was observed in the stented artery segments.

Samples of this Example including implanted devices produced as described herein (DES) and as control (BMS) were evaluated by SEM. Table 20d summarized the SEM scores.

Stented artery segments and adjacent segments (approximately 10 mm of proximal nonstented tissue and approximately 10 mm of distal nonstented tissue) were dissected from hearts fixed with glutaraldehyde (GTH) and stored refrigerated in GTH for additional fixation until SEM processing.

Radiographs were used to determine stent location then the vessel was bisected longitudinally into hemi-sections and proximal direction was marked. Specimens were rinsed with 0.1M sodium cacodylate buffer then post-fixed in 1% osmium tetroxide in 0.1M sodium cacodylate buffer, rinsed in 0.1M sodium cacodylate buffer and deionized water. Subsequently, the stented arteries were pinned onto a mountable surface to expose the luminal surface, dehydrated in a graded series of alcohol solutions, then dried using a critical point dryer (CPD), mounted onto metallic disks and ion sputter coated with metal.

The exposed luminal surface of sputter-coated SEM hemisection samples were systematically imaged and digitally photographed with a scanning electron microscope (Hitachi Model S-3400N-II) at low magnification (approximately 14-15x). Image processing software (e.g., Adobe Photoshop vCS) was used to assemble the low magnification digital images into a single montage image depicting the luminal surface topography of the entire hemisection samples. In addition, digitally photographed high magnification images (e.g., 200x) were taken within non-stented proximal, in-stent proximal, mid, distal, and non-stented distal regions to exhibit the cellular and tissue morphology and extent of endothelial coverage. Additional SEM images were taken to document any morphological observations located along the luminal surface.

SEM images of the luminal surface were scored for en face endothelial cell coverage at the stented regions using the semi-quantitative scoring scale below to document the endothelial luminal coverage. Higher magnification SEM images were used to morphologically identify endothelial cells (typically characterized as a confluent or closely aggregating monolayer of squamous spindle to polygonal shaped cells) and scored for extent of confluence. In addition, the presence of spherical-shaped inflammatory cells and thrombus were scored. The following parameters were used for the scoring of the SEM images along the luminal surface of the stented coronary arteries: For SEM Endothelial Coverage Score Matrix: 0 means Endothelial cells absent in SEM imaged area of lumen, 1 means <25% endothelial surface coverage in SEM imaged area, 2 means 25-75% endothelial surface coverage in SEM imaged area, 3 means >75% endothelial surface coverage in SEM imaged area, 4 means 100% confluent endothelial cell coverage in SEM imaged area. For SEM Endothelial Confluence Score Matrix: 0 means No intercellular spacing between endothelial cells, 1 means Minimal intercellular spacing between endothelial cells, 2 means Notable intercellular spacing between endothelial cells, 3 means Overwhelming intercellular spacing between endothelial cells. For SEM Inflammation Score Matrix: 0 means No inflammatory cells observed per area examined, 1 means Minimal adhered inflammatory cells per area examined, 2 means Notable adhered inflammatory cells per area examined, 3 means Overwhelming adhered inflammatory cells per area examined. For SEM Thrombus Score Matrix: 0 means No thrombus observed per area examined, 1 means Light adherence/deposition of thrombus material per area examined, 2 means Notable adherence/deposition of thrombus material per area examined, 3 means Overwhelming adherence/deposition of thrombus material per area examined

**Table 20e SEM Scoring Summary**

| | | Endothelial Coverage Score | | | Endothelial Confluence | | | Thrombus Score | | |
|---|---|---|---|---|---|---|---|---|---|---|
| Time Point | Treatment Group | Mean | ± | sd | Mean ± | ± | sd | Mean | ± | sd |
| 30 Days | 3S DES Single | 4.00 | ± | 0.00 | 0.56 | ± | 0.51 | 0.00 | ± | 0.00 |
| | 4S BMS Single | 4.00 | ± | 0.00 | 0.13 | ± | 0.18 | 0.00 | ± | 0.00 |
| | 3V DES Overlap | 4.00 | ± | 0.00 | 0.17 | ± | 0.29 | 0.00 | ± | 0.00 |
| | 4V BMS Overlap | 4.00 | ± | 0.00 | 0.67 | ± | 0.58 | 0.00 | ± | 0.00 |
| 90 Days | 3S DES Single | 4.00 | ± | 0.00 | 0.00 | ± | 0.00 | 0.00 | ± | 0.00 |
| | 4S BMS Single | 3.89 | ± | 0.19 | 0.00 | ± | 0.00 | 0.00 | ± | 0.00 |
| | 3V DES Overlap | 3.94 | ± | 0.10 | 0.33 | ± | 0.58 | 0.00 | ± | 0.00 |
| | 4V BMS Overlap | 4.00 | ± | 0.00 | 0.00 | ± | 0.00 | 0.00 | ± | 0.00 |

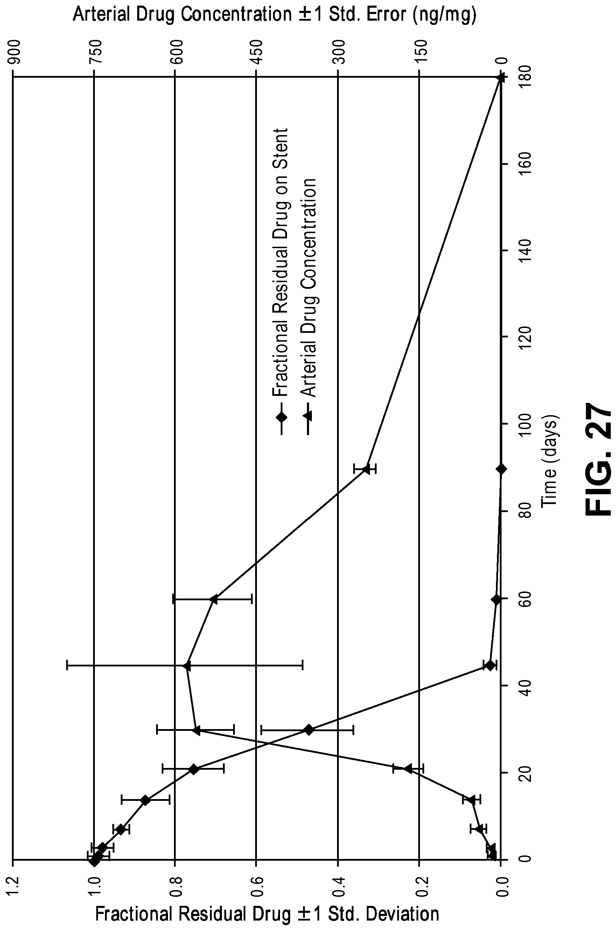

Results from Figure 26 and Figure 27 indicated that about 40% of pharmaceutical agent (rapamycin in this example) released from the coated stent is recovered in the analyzed arterial tissue between days 3 and 60. This percentage of pharmaceutical agent between days 3 and 60 is based on 135 micrograms on the original stent at time 0. Results also indicated that about 60% of the pharmaceutical agent (rapamycin in this example) released from the coated stent was recovered in the analyzed arterial tissue at day 30. Again, this percentage of pharmaceutical agent between days 3 and 60 is based on 135 micrograms on the original stent at time 0. These results may be described as the efficiency of transfer of the pharmaceutical agent into the tissue of the subject. As used herein, the term "about" when used with respect to the amount of active agent recovered in the analyzed tissue, means variability of 5%, 10%, 20%, 25%, and 30% on either side of the target (and is not a percent of the percent). That is, for example, about 40% can mean, in some embodiments, a target of 40% having a variability of 25% and thus would range from 15% to 65%.

The above-noted efficiency of the transfer of the pharmaceutical agent into the tissue of the subject adjacent to the stent may be attributed to a low initial burst of the pharmaceutical agent into the arterial tissue since the pharmaceutical agent does not migrate to the abluminal surface or layer of the coating during coating deposition and/or coating methods. Solvent-based coating processes are subject to migration of pharmaceutical agent to abluminal layers or surfaces of the coating during solvent drying processes, and therefore are subject to initial bursts of pharmaceutical agent released into the arterial tissue. The coating processes disclosed and used herein are not subject to migration of pharmaceutical agent to abluminal layers or surfaces of the coating during coating processes. This initial burst of pharmaceutical agent may exist even in topcoat-based coatings which are solvent-based.

Figure 27 shows in graphical form the fractional residual drug remaining on the stent at various time points (top line at time 0) using the scale on the left y-axis, and the measured arterial drug concentration (bottom line at time 0) measured at various time points using the scale on the right y-axis. These results also show that the crystalline form of the drug extends drug residence time in tissue beyond the elimination of polymer in the tissue, as the polymer appears eliminated from tissue at 90 days at most, while the drug is detectable even at 90 days and there appears some drug still remaining in the arterial tissue according to Figures 26 and 27 beyond the 90d time point of testing.

The above-noted efficiency of the transfer of the pharmaceutical agent into the tissue of the subject adjacent to the stent may be attributed additionally or alternatively to a steady-state release the pharmaceutical agent into the blood of the subject, at levels that are systemically below the quantifiable limit of pharmaceutical agent concentration, as noted in Example 32.

The above-noted efficiency of the transfer of the pharmaceutical agent into the tissue of the subject adjacent to the stent may be attributed additionally or alternatively to slow release the pharmaceutical agent into the tissue of the subject.

The above-noted efficiency of the transfer of the pharmaceutical agent into the tissue may be a result of at least one of: crystalline pharmaceutical agent that creates micro depots for slow pharmaceutical agent release, PLGA that facilitates bulk transfer to tissue and resists washout, and low tissue reaction that promotes healing and pharmaceutical agent entrapment.

As a result of the study showing efficient transfer of the pharmaceutical agent into tissue using the coating materials and/or processes noted in this example, a resulting coated device as thus prepared can be optimized using adjustment to the materials and process which are standard adjustments known to one of skill in the art to provide controlled drug-delivery performance in situations where at least one of the following is desired: 1) effective active agent transfer from a tissue surface, 2) prolonged maintenance of locally high tissue active agent levels, and sustained therapeutic active agent delivery from a relatively small device or depot. The substrates coated thereby may be beneficial in situations where the delivery site is a vessel, duct (e.g. a prostrate, ear, biliary), or other type of tube, for non-limiting example. These substrate coated thereby may be beneficial in situations where there are enclosed spaces or chambers (e.g. anterior/posterior chamber of the eye, nasal sinus, spine) for non-limiting example.

In some embodiments, a bare metal stent is achieved by 90 days at most, as demonstrated by full absorption of the polymer by the arterial tissue. Full absorption may be at least 75% absorption, at least 80% absorption, at least 90% absorption, at least 95% absorption, or 100% absorption as measured according to polymer detection methods described herein or known to one of skill in the art.

In some embodiments, a bare metal stent is achieved by 45 days at most, as demonstrated by clearance of the coating from the stent, such as by measuring the amount of drug on the stent. In some embodiments, a bare metal stent is achieved by 45-60 days at most, as demonstrated by clearance of the coating from the stent, such as by measuring the amount of drug on the stent. In some embodiments, a bare metal stent is achieved by 90 days at most, as demonstrated by clearance of the coating from the stent, such as by measuring the amount of drug on the stent. In some embodiments, a bare metal stent is achieved within 45-90 days, as demonstrated by clearance of the coating from the stent, such as by measuring the amount of drug on the stent. In some embodiments, a bare metal stent is achieved within 45-60 days, as demonstrated by clearance of the coating from the stent, such as by measuring the amount of drug on the stent. Clearance of the coating from the stent may be when over 52% of the sirolimus is no longer associated with the stent thus over 52% of the coating is released from the stent, at least 75% of the sirolimus is no longer associated with the stent thus at least 75% of the coating is released from the stent, at least 80% of the sirolimus is no longer associated with the stent thus at least 80% of the coating is released from the stent, at least 90% of the sirolimus is no longer associated with the stent thus at least 90% of the coating is released from the stent, at least 95% of the sirolimus is no longer associated with the stent thus at least 95% of the coating is released from the stent, at least 97% of the sirolimus is no longer associated with the stent thus at least 97% of the coating is released from the stent as measured according to detection methods described herein (e.g. by measuring the amount of drug on the stent) or known to one of skill in the art.

Provided herein is a method comprising providing a coated stent comprising a stent and a coating thereon, wherein the coating comprises at least one polymer and at least one active agent wherein the active agent is present in crystalline form; implanting the coated stent in a subject, wherein about 40% of active agent released from the device is in tissue adjacent the coated stent at any time point between day 3 after implantation and day 60 after implantation.

In some embodiments, 15% to 65% of active agent released from the device is in tissue adjacent the coated stent at any time point between day 3 after implantation and day 60 after implantation. In some embodiments, 20% to 60% of active agent released from the device is in tissue adjacent the coated stent at any time point between day 3 after implantation and day 60 after implantation. In some embodiments, 30% to 50% of active agent released from the device is in tissue adjacent the coated stent at any time point between day 3 after implantation and day 60 after implantation.

Provided herein is a method comprising providing a coated stent comprising a stent and a coating thereon, wherein the coating comprises at least one polymer and at least one active agent wherein the active agent is present in crystalline form; implanting the coated stent in a subject, wherein about 60% of the active agent released from the coated stent is in tissue adjacent the coated stent at day 30 after implantation.

In some embodiments, 20% to 100% of the active agent released from the coated stent is in tissue adjacent the coated stent at day 30 after implantation. In some embodiments, 30% to 90% of the active agent released from the coated stent is in tissue adjacent the coated stent at day 30 after implantation. In some embodiments, 40% to 80% of the active agent released from the coated stent is in tissue adjacent the coated stent at day 30 after implantation. In some embodiments, 50% to 70% of the active agent released from the coated stent is in tissue adjacent the coated stent at day 30 after implantation. In some embodiments, the amount of active agent in the tissue is determined in an animal pharmacokinetic study.

The data of this example, at least, or when combined with data of similarly produced devices as described elsewhere herein, show a relatively consistent release of sirolimus from the devices described herein that ranges from approximately 1 to 4 µg/day over the first 45 days after implant with no initial burst of drug release. Approximately 50% of drug has been released from the stent at 30 days and by day 45, the drug has almost completely disassociated from the stent. At time points evaluated beyond 90 days, Sirolimus is no longer detectable on the stent. Sirolimus accumulated gradually in the tissue over time, peaking between 30 - 60 days then declining substantially at 90 days. Although Sirolimus can still be detected in the tissue surrounding the stent at 365 days, it is in very low quantity. The accumulation of drug in the tissue represents both amorphous drug eluted from the coating as well as crystalline drug still embedded in coating deposits. There are no detectable levels of sirolimus in the blood at any time. SEM showed that the MiStent DES is comparable to the BMS control with endothelialization in MiStent DES complete or near complete at 30 and 90 days with no thrombus accumulation.

### Example 35:

Coated stents were prepared as follows: coated stents comprise a coating deposited on the stent by deposition of rapamycin in dry powder form by RESS methods and equipment described herein and deposition of polymer particles by RESS methods and equipment described herein. A PDPDP (Polymer, sinter, Drug, Polymer, sinter, Drug, Polymer, sinter) coating sequence was used wherein the polymer was 50:50 PLGA, and the drug was rapamycin. The sinter step was performed at 100°C/1.03 MPa (150 psi)/10 min after each "P" (or polymer) layer. There was 135 micrograms +/- 15% sirolimus on each coated stent in this study. The coating was about 5-15 micrometers thick on each stent, and comprised a thicker coating on the abluminal surface (coating bias). The coating encapsuled each of the stents.

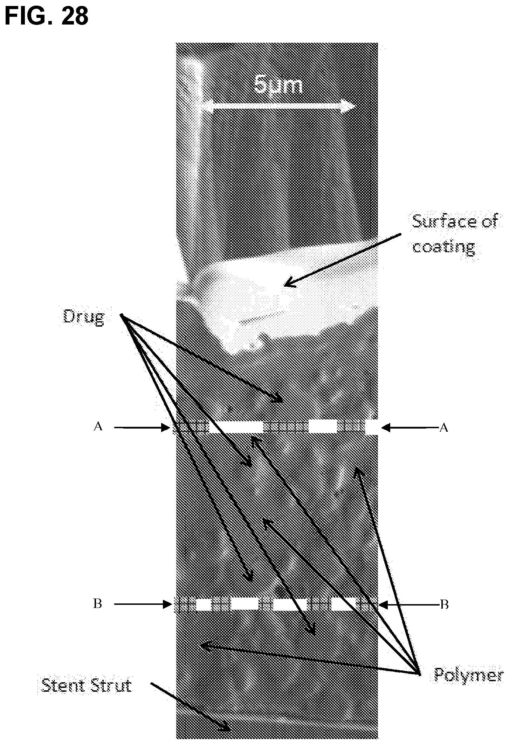

The resulting coated stent was cross-sectioned and visualized by SEM. Figure 28 shows a segment of coating on a stent strut wherein the coated stent is prepared as described herein and wherein the pharmaceutical agent is at least in part crystalline within the polymer of the coating. The surface of the coating is shown, as is crystalline drug (crystalline rapamycin in this case) which appears as pock-marks in the polymer of the cross-sectioned coating.

Despite being laid down in a layering process, the cross-section of Figure 28 shows little or no evidence of layering, and the pharmaceutical agent is interspersed throughout the entire depth of the coating. The resulting coated stent comprises a coating having a active agent density that is the same throughout the coating depth. For example, in some embodiments, the coated stent comprises a coating comprising a density of active agent that is about X at 20% of the total coating depth and is also about X at 80% of the coating depth. The actual density number (X) can be tailored using the processes and materials noted herein to be any target density. The density needn't be expressed as a mass per volume, although it may be. In some examples the density could be expressed as a fraction i.e. active agent/total coating, and expressed in any number of units (e.g. as a distance, depth, etc) or be unitless. For example, the density could be in some embodiments a fraction comprising active agent along a particular line of coating over the total length of the line of coating. That is, in Figure 28, along line A-A there is 3.5microns of active agent (shown as a cross-hatched line) and 3.0 microns of polymer (shown as a white line) resulting in 6.5 microns total of coating and thus the density of active agent along line A-A could be expressed as 3.5/6.5 = 0.54, whereas along line B-B there is also 3.5 microns of active agent (shown as a cross-hatched line) and 3.0 microns of polymer (shown as a white line) resulting in 6.5 microns total of coating, thus the density of active agent along line B-B could also be expressed as 3.5/6.5 = 0.54. In this example, line A-A is about 1/3 of the way from the surface of the coating (as measured from the surface to the stent strut), and line B-B is about 2/3 of the way from the surface of the coating (also as measured from the surface of the stent strut). Thus, thus the density of active agent is the same at a 1/3 depth as it is at a 2/3 depth of coating.

Provided here is a coated stent comprising a stent and a coating thereon wherein the coating comprises at least one polymer and at least one active agent wherein the active agent is present in crystalline form; implanting the coated stent in a subject, wherein the active agent is evenly distributed through the depth of the coating as shown by comparison of a density of active agent in the coating at a first and a second depth.

Provided herein is a method comprising providing a coated stent comprising a stent and a coating thereon, wherein the coating comprises at least one polymer and at least one active agent wherein the active agent is present in crystalline form; implanting the coated stent in a subject, wherein the active agent is evenly distributed through the depth of the coating as shown by comparison of a density of active agent in the coating at a first and a second depth.

In some embodiments, the first depth is about 1/3 of the way from the stent strut to the stent coating surface, and wherein the second depth is about 2/3 of the way from the stent strut to the stent coating surface. In some embodiments, the first depth is about 1/4 of the way from the stent strut to the stent coating surface, and wherein the second depth is about 3/4 of the way from the stent strut to the stent coating surface. In some embodiments, the first depth is any of 1/8 of the way from the stent strut to the stent coating surface, 1/6 of the way from the stent strut to the stent coating surface, ¼ of the way from the stent strut to the stent coating surface, 1/3 of the way from the stent strut to the stent coating surface, 3/8 of the way from the stent strut to the stent coating surface, ½ of the way from the stent strut to the stent coating surface, 5/8 of the way from the stent strut to the stent coating surface, 2/3 of the way from the stent strut to the stent coating surface, ¾ of the way from the stent strut to the stent coating surface, and 7/8 of the way from the stent strut to the stent coating surface. In some embodiments, the second depth is any of 1/8 of the way from the stent strut to the stent coating surface, 1/6 of the way from the stent strut to the stent coating surface, ¼ of the way from the stent strut to the stent coating surface, 1/3 of the way from the stent strut to the stent coating surface, 3/8 of the way from the stent strut to the stent coating surface, ½ of the way from the stent strut to the stent coating surface, 5/8 of the way from the stent strut to the stent coating surface, 2/3 of the way from the stent strut to the stent coating surface, ¾ of the way from the stent strut to the stent coating surface, and 7/8 of the way from the stent strut to the stent coating surface. In some embodiments, the second depth is not the same as the first depth.

### Example 36

Delayed healing, stent thrombosis, late-catch-up and neo-atherosclerosis are unfavorable late-term outcomes associated in part with permanent polymers of current drug-eluting stents (DES). Certain devices as described and produced according to methods herein were used in this example and were designed for improved safety while maintaining strong efficacy. The devices used in this example comprised crystalline sirolimus and sirolimus release at a controlled, linear rate, with coating off the stent in 45-60 days and with full absorption of the polymer by 90 days. The coatings are lubricious and hydrophilic. The device comprised a thin strut (∼64um) cobalt-chromium stent.

A human clinical trial was performed as a prospective, single-arm study at five sites evaluating the device as described herein and according to methods described herein for preliminary safety and efficacy. Patients with discrete de novo lesions (2.5-3.5 mm diameter and ≤20 mm length) in native coronary arteries were enrolled. The Patient selection criteria included stable or unstable angina pectoris (Class I, II, III, or IV), documented ischemia, or documented silent ischemia. Patients could not have recent Q wave MI (<72hrs) and no elevated cardiac biomarkers. The target lesions included planned single, de novo, types A, B1 or B2 coronary lesions (according to the ACC/AHA classification) in the native coronary artery with >50% diameter stenosis. Vessel diameters were to be 2.5 to 3.5mm and a maximum of a 23mm long stent was used and indicated. Lesions were to be excluded if they were highly calcified, tortuous, thrombus present, or proximal angulation. Lesions were to be excluded if they were located at size branch over 2.5mm, at an ostial location, or at a previously treated vessel. Non-target lesions were allowed to be treated if critical in another vessel prior to treating the target lesion.

DAPT (Dual Antiplatelet Therapy) recommendations were in accordance with ACC/AHA/SCAI PCI Practice guidelines. 100% of the patients were found to be taking aspirin daily at 8 months; 80% of the patients remained on daily Plavix of 75mg at 8 months; 20% discontinued Plavix at 6 months.

Table 21 shows the Clinical and Lesion Characteristics of patients in this study (n=30).

**Table 21: Baseline Clinical & Lesion Characteristics**

| Clinical Characteristics | | Actual | Percentages |
|---|---|---|---|
| Demographics | Age (mean, range) | 62.3, 44-86 | NA |
| | Gender (m/f) | 22/8 | 73%/27% |
| Risk Factors | MI | 5 | 17% |
| | PCI | 9 | 30% |
| | CVA | 1 | 3% |
| | Hypertension | 23 | 77% |
| | Hypercholesterolemia | 26 | 87% |
| | Diabetes | 7 | 23% |

| Lesion Characteristics | | Actual | Percentages |
|---|---|---|---|
| Baseline | Length | 13.15 +/- 3.77 | NA |
| Angiography | RVD | 2.86 +/-0.32 | NA |
| Location | RCA | 12 | 40% |
| | LAD | 11 | 37% |
| | LCx | 7 | 23% |
| Classiciation | A | 16 | 53% |
| | B1 | 10 | 44% |
| | B2 | 4 | 13% |
| Tortuous | Moderate | 2 | 7% |
| Calcium | Moderate | 7 | 23% |
| | Severe | 1 | 3% |

Following the device placement, patients were evenly assigned to 3 groups for follow-up imaging (angiography, intravascular ultrasound evaluation (IVUS) and optical coherence tomography (OCT)) at 4-months, 6-months or 8-months to determine in-stent late lumen loss (LLL), vessel healing, and stent coverage. Clinical safety was conducted for all 30 patients at 8-months.

The initial 10 patients evaluated at 4-months demonstrated an in-stent late lumen loss (LLL) of 0.01 ± 0.12 mm assessed by core laboratory quantitative angiography. Imaging with OCT demonstrated thin, homogenous coverage with high rates of stent strut coverage (70% of the patients having >90% strut coverage and 90% having >80% strut coverage) with a low rate of stent strut malapposition. The IVUS findings supported minimal neointimal hyperplasia with a neointimal obstruction of 5.2%. Clinical outcomes and angiographic, OCT and IVUS analyses at 4, 6 and 8-months for the entire study cohort will be presented.

The device as described and tested according to this example, developed with a predictably absorbed polymer encompassing a new morphology of sirolimus, may address current DES concerns while being very effective. Interim imaging with angiography, OCT and IVUS suggest effective inhibition of neointimal hyperplasia with a high rate of strut coverage.

The coated stent as described and tested according to this example is unique as compared to other DES. As the coating migrates off the stent and is reabsorbed into the surrounding tissue, the crystalline sirolimus is deposited into the tissue and drug is released at a controlled rate. The coating is off the stent in 45-60 days leaving a bare metal stent (BMS) with complete absorption of the polymer by 90 days leaving. This methodology allows for managed drug delivery to the treated artery to reduce the extent of restenosis while allowing progressive stent strut coverage which can mean a reduced rate of late stent thrombosis as compared to other DES.

In some embodiments, a bare metal stent is achieved by 90 days at most, as demonstrated by full absorption of the polymer by the arterial tissue. In some embodiments, a bare metal stent is achieved by 45-60 days at most, as demonstrated by clearance of the polymer from the struts of the stent. Full absorption may be at least 75% absorption, at least 80% absorption, at least 90% absorption, at least 95% absorption, at least 99%, or 100% absorption as measured according to polymer detection methods described herein or known to one of skill in the art, such as the methods of Examples 34-37, at least for histological evaluation in porcine models and evaluation by microscopy.

Absorption may also be referred to herein as resorption. Absorption (or resorption) of the polymer by arterial tissue (or by the vessel, or by the tissue surrounding the stent) may occur not only be actual absorption by such tissue, but may alternatively or additionally occur by resorbtion over time by the body in general, which can include metabolization and/or excretion of any part of the polymer or product of the polymer absorption process. For example in the case of PLGA absorption occurs by, hydrolysis of the PLGA to a low molecular weight whereby the degraded PLGA oligomers are soluble in body fluids, diffuse into the surrounding tissues and/or blood stream and are then either metabolized or excreted. Depending on the polymer used, other methods of absorption or resorption may exist and are considered covered by the idea of absorption or resorption of the polymer by arterial tissue, by the body, by the vessel, by the tissue surrounding the stent, or any variation thereof.

In order to determine whether absorption or resorption has occurred, and to what degree, it is generally understood that polymers associated with drug-eluting stents are solvent labile and susceptible to artifactual removal during the histologic processing of implanted arteries. Polymers, present during the formation of the neointima, are a space occupying mass in which the smooth muscle cells must accommodate, and around which the nascent neointima must form. With the removal of the polymer during processing, clear spaces are created and interpreted to be the negative image, or approximate facsimile, of the stent polymer/drug in situ, despite the absence of observable polymer. As such, these clear areas may be used to qualitatively characterize the size, spread, localization and apparent resorption of polymer coating material as a function of implant duration. Bioabsorbable polymers will resorb over time by the body, thus, over time these clear areas will be fewer and smaller, until the polymer is fully absorbed (resorbed). These may be detected by histologic processing of implanted arteries, such as noted in Examples 34-37, at least, and visualized under microscopy as noted therein.

The polymer and drug coating of the coated stents described herein are typically characterized by a larger clear zone intimately surrounding the struts. Lacunae refers to variably sized and shaped clear space(s) located in the peri/extra-strut neointima which appear to have been separated from the polymer intimately associated with the struts. These lacunae were interpreted to represent the deposition/migration of the strut-associated polymer/drug into the surrounding neointima. Since lacunae were not observed in the bare metal stents similarly implanted, their presence in the coated stent tissue samples may be the local effects of neointimal formation inhibition secondary to the presence of the polymer (i.e., space-occupying mass) and/or sirolimus (i.e., smooth muscle cell inhibition).

Neointimal lacunae were only observed only at Day 30 (when evaluated at about days 3, 30, 90, 180, and 365), whether there was a single coated stent implanted, or whether there were two coated stents implanted (overlapping as noted elsewhere herein). Thus, the polymer was resorbed (or absorbed) by day 90, at the latest. In some embodiments the polymer is resorbed (or absorbed) between day 30 and day 90. The magnitude of extra-strut neointimal lacunae (i.e., polymer/drug) was minimal, and though they were commonly seen on a per plane basis (∼ 70%), within each affected plane the change was generally limited to only one to two foci. Regardless, the presence of neointimal lacunae after 30 days implantation of the coated stents implanted as noted herein did not appear to be associated with any adverse tissue response. Rarely (∼ <5%), lacunae were present in the adventitia, with associated inflammation, and usually the result of mural injury.

Full absorption (by the arterial tissue, but the vessel, or by the body, for example) exists when at least 75%, at least 80% at least 90%, at least 95%, at least 99%, or 100% of the polymer is not visible in histology tissue sections tested by Histological evaluation methods of Examples 34-37, and visualized according to methods also in Examples 34-37, at least, or by other evaluation methods known to one of skill in the art. In some embodiments, this may mean that an evaluation of artifacts of the polymer is evaluated, rather than finding polymer itself in the sample. Artifacts may include the clear spaces in the histological samples attributable to polymer (and not the stent struts).

In some embodiments, a bare metal stent is achieved by 45 days at most, as demonstrated by clearance of the coating from the stent, such as by measuring the amount of drug on the stent. In some embodiments, a bare metal stent is achieved by 45-60 days at most, as demonstrated by clearance of the coating from the stent, such as by measuring the amount of drug on the stent. In some embodiments, a bare metal stent is achieved by 90 days at most, as demonstrated by clearance of the coating from the stent, such as by measuring the amount of drug on the stent. In some embodiments, a bare metal stent is achieved within 45-90 days, as demonstrated by clearance of the coating from the stent, such as by measuring the amount of drug on the stent. In some embodiments, a bare metal stent is achieved within 45-60 days, as demonstrated by clearance of the coating from the stent, such as by measuring the amount of drug on the stent. Clearance of the coating from the stent may be when over 52% of the sirolimus is no longer associated with the stent thus over 52% of the coating is released from the stent, at least 75% of the sirolimus is no longer associated with the stent thus at least 75% of the coating is released from the stent, at least 80% of the sirolimus is no longer associated with the stent thus at least 80% of the coating is released from the stent, at least 90% of the sirolimus is no longer associated with the stent thus at least 90% of the coating is released from the stent, at least 95% of the sirolimus is no longer associated with the stent thus at least 95% of the coating is released from the stent, at least 97% of the sirolimus is no longer associated with the stent thus at least 97% of the coating is released from the stent as measured according to detection methods described herein (e.g. by measuring the amount of drug on the stent) or known to one of skill in the art.

In this example a thin strut (64µm) Co Cr GENIUS® Magic coronary Stent & Rx Catheter was used. The stent was coated according to RESS methods described herein using a PDPDP sequence of steps to produce the coated stent. In this example, the PDPDP sequence of steps comprises Polymer single spray, sinter, Drug spray, Polymer double spray, sinter, Drug spray, Polymer triple spray, sinter. In some embodiments, the PDPDP sequence of steps comprises a first Polymer spray, sinter, Drug spray, a second Polymer spray that is about twice as long as the first Polymer spray, sinter, Drug spray, third Polymer spray that is about three times as long as the first Polymer spray, sinter. In some embodiments, the PDPDP sequence of steps comprises a first Polymer spray, sinter, Drug spray, a second Polymer spray that deposits about twice as much Polymer as the first Polymer spray, sinter, Drug spray, third Polymer spray deposits about three times as much Polymer as the first Polymer spray, sinter.

The Polymer was PLGA 50:50 having a number average molecular weight of about 15kD. The drug was sirolimus in crystalline form (or at least partially crystalline). The resulting coated stent comprised crystalline sirolimus having a controlled elution profile similar to or equivalent to the profile as shown and described with respect to Figure 24. The nominal target drug loading on the stents used was according to Table 22. The actual drug loading on a stent, depending on the embodiment for a particular device, may be at least one of: the target +/- 5%, the target +/- 10%, the target +/- 15%, the target +/- 20%, the target +/- 25%, the target +/- 30%, the target +/- 35%, the target +/- 40%, the target +/- 45%, the target +/- 50%, at least 50% of the target, at least 75% of the target, at least 80% of the target, at least 85% of the target, at least 90% of the target, at least 95% of the target, at most 105% of the target, at most 110% of the target, at most 115% of the target, at most 120% of the target, at most 125% of the target, at most 130% of the target, at most 140% of the target, and at most 150% of the target.

**Table 22: Nominal Drug Target loading (in micrograms Sirolimus)**

| **Target Loading** | **9 mm length** | **15 mm length** | **19 mm length** | **23 mm length** | **30 mm length** |
|---|---|---|---|---|---|
| **7 cell stents** | 83µg | 135µg | 175µg | 214µg | 280µg |
| **9 cell stents** | 107µg | 165µg | 210µg | 253µg | NA |

Enrollment in the trial was designed to be 30 patients at 5 sites - all implanted with the coated stent as described in this example. Efficacy was evaluated by reviewing in-stent late lumen loss (LLL) by QCA at each time point. There were exclusive groups of 10 patients at 4, 6 and 8 months follow-up. The Safety evaluation was conducted by reviewing MACE (death, MI and TVR). Mechanistic studies were performed using IVUS and OCT to understand the timeline for vessel healing at each time point. Additionally, angiographic evaluation was conducted at the follow-up time points. Furthermore, patients are to be followed for 5 years (data not available at the time of drafting).

The results were as follows. All 30 patients returned for 4, 6, and 8 months imaging follow up visits. Regarding the MACE Safety endpoint evaluation results are presented in Table 23. All events were adjudicated by CEC.

**Table 23: Safety-MACE**

| n=30 | In- Hospital | <30 Days | <8 months |
|---|---|---|---|
| MACE | 1 | 1 | 2 |
| Death | 0 | 0 | 0 |
| Q wave MI | 0 | 0 | 0 |
| Non Q wave MI | 0 | 0 | 1** |
| Peri-procedural MI | 1* | - | - |
| TVR | 0 | 0 | 0 |
| Stent Thrombosis | 0 | 0 | 0 |

| | | | |
|---|---|---|---|
| *due to elevation of cardiac enzymes only- with no elevation of CK, CK-MB 3.5xULN at 8-hours i.e. CK-MB elevation only post-procedure, 5.75xULN at 24 hours **non-TL, non-Q wave MI: increased trooping post diagnostic angiogram at 44 days post-procedure | | | |

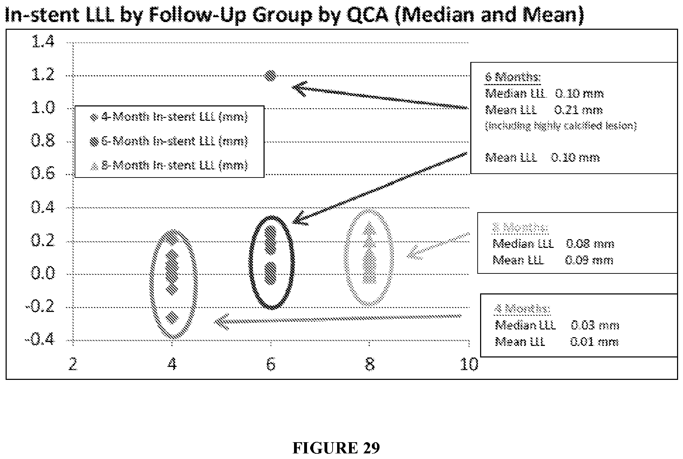

Figure 29 shows the Patient level in-stent LLL by follow-up group, indicating no binary restenosis and having a linear regression indicating minimal change in LLL between 4 and 8 months. The x-axis is depicted in months, while the y-axis is the amount of in-stent LLL given in mm. The mean LLL at 4 months was determined to be 0.01mm with a standard deviation of 0.12mm; the median LLL at 4 months was determined to be 0.03mm. The mean LLL at 6 months was determined to be 0.21mm with a standard deviation of 0.36mm; the median LLL was 0.10mm. Note that there appeared to be a single statistical outlier in this 6 month group, which was attributed to the lesion of this patient being highly calcified and resulting in under-expansion of the stent, in contrast to the other lesions of the other patients. If this outlier is removed, the mean LLL at 6 months was 0.10 mm. The LLL at 8 months was determined to be 0.09mm with a standard deviation of 0.10mm; the median LLL was 0.08mm. The regression line of the data (excluding the single outlier in the 6 month group) is y=0.019x-0.0098 (or - 0.0098 +0.0192*time). The QCA results as shown in Figure 29 and in Table 23, at least, demonstrate a sustained and effectively suppressed neointimal hyperplasia. The results also show no binary restenosis, and linear regression of the data shown indicates minimal change in late lumen loss between 4 and 8 months.

Table 24 shows the Angiography results (QCA) for each of the 4 month, 6 month, and 8 month group. The median, range, and mean and standard deviation is provided for each group regarding in stent LLL. This is the same data that is presented graphically in Figure 29.

**Table 24: Angiography Results (QCA)**

| In-stent LLL (mm) | 4-month group (n=10) | 6-month group (n=10) | 8-month group (n=10) |
|---|---|---|---|
| Median | 0.03 | 0.10 | 0.08 |
| Range | -0.27 to 0.21 | -0.03 to 1.20 | -0.02 to 0.28 |
| Mean +/- SD | 0.01 +/- 0.12 | 0.21 +/- 0.36 | 0.09 +/- 0.10 |

In cross sectional analysis, vessel, stent and lumen borders were manually traced and neointimal area was obtained as stent area minus lumen area. To analyze the complete stented segment, volume parameters were generated using Simpson's method/rule : Volume equals the sum of multiple segments (S1 through Sn) i.e. Sum of Segment 1, Segment 2, Segment 3... Segment n-1, Segment n. The volumes include vessel, lumen, stent, and neointima. To adjust for different stent lengths, the volume data were divided by stent length, and this volume index was shown as volume data. (Volume Index= Volume/length (mm^3/mm)). To evaluate the overall magnitude of neointimal suppression of DES, the percent neointimal volume (% Neointimal volume Obstruction (%NIV)) was defined as neointimal volume divided by the volume and expressed as a percent. Cross-sectional narrowing percentage was defined as neointimal area divided by stent area (x 100, i.e. expressed as a percent) to assess the most severe impact of neointima on luminal encroachment. To assess gross coverage of struts, neointima-free frame ratio (the ratio of frames without neointima) was also calculated and expressed as a percent. In each group certain results were not interpretable, and only interpretable results are presented with sample sizes as indicated (e.g. 1 case at 4 months, 3 cases at 6 months, and 2 cases at 8 months were not interpretable).

Table 25 shows the IVUS (3-D) results for each of the 4 month, 6 month, and 8 month group with respect to neointimal obstraction (%), neointimal volume index (mm^3/mm), and late area loss (mm^2). The entire stent and adjacent reference segment up to 5 mm were analyzed by IVUS. IVUS results demonstrate low neointimal hyperplasia.

**Table 25: IVUS results**

| Parameter | 4-month group Mean (SD) (n=9) | 6-month group Mean (SD) (n=7) | 8-month group Mean (SD) (n=8) |
|---|---|---|---|
| neointimal obstraction (%) | 5.2 (+/-3.2) | 8.0 (+/-3.1) | 10.9 (+/-4.6) |
| neointimal volume index (mm^3/mm) | 0.3 (+/-0.1) | 0.7 (+/-0.4) | 0.8 (+/-0.5) |
| late area loss (mm^2) | 0.4 (+/-0.6) | 0.7 (+/-0.8) | 0.8 (+/-0.8) |

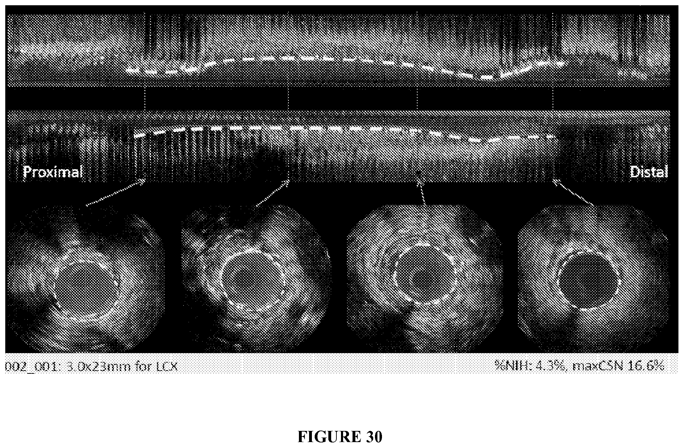

Figure 30 shows a target artery and lesion of a single patient from the study in this example viewed by IVUS at 8 months follow up.

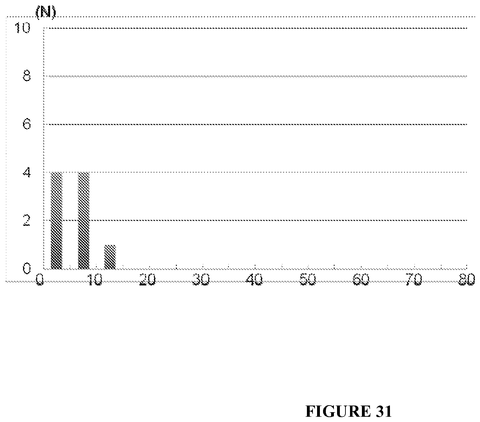

Figure 31 shows a histogram of Neointimal obstruction of devices of this example at 4 months follow up as tested and analyzed using IVUS. The majority of the patient's neointimal obstruction was under 10%. The x-axis shows the percent neointimal obstruction, while the x-axis is sample size (N). the Average percent neointimal obstruction at 4 months was 5.2% +/-3.2%, while the median was 5.3%, the 25% Quartile was 3.0% and the 75% Quartile was 5.85.

The average percent of maximum cross sectional narrowing was detected and determined by IVUS at 4 months to be 11.9 %+/- 4.6% (n=9), and there was no case in which there was lumen encroachment (which would be an encroachment over 50%). The neointima free frame ratio at 4 months was determined to be 20% +/- 17%. Late area Loss was 0.4 +/- 0.6mm^2, and the minimum lumen area in the stented segment appeared to trend to decrease between baseline (at implantation) which had a minimum lumen area in the stented segment of 5.9+/-1.4 mm^2 to 4 month follow up which had a minimum lumen area in the stented segment determined to be 5.5+/- 1.1mm^2, but the decrease was not statistically significant (p=0.122).

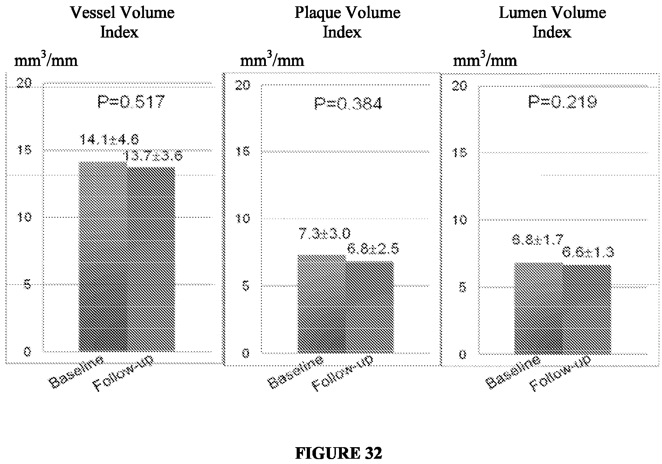

Figure 32 shows Vessel Response in this study, which shows Vessel Volume Index, Plaque Volume Index, and Lumen Volume Index at baseline (at implantation) and at 4 months follow up. No significant changes were observed in any of these Indexes.

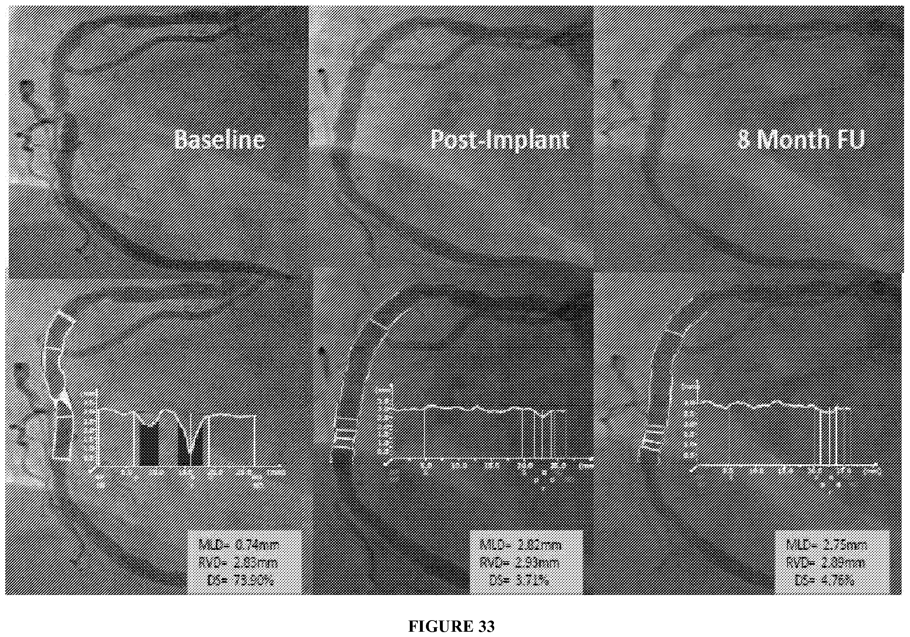

Figure 33 shows the target artery and lesion of a single patient from the study in this example viewed under fluoroscopy prior to implantation of the device from this study (first images top and bottom labeled "Baseline"), just after implantation of the device (middle images top and bottom labeled "Post-Implant") and at 8 months follow up (last top and bottom images labeled "8 Month FU").

In the qualitative analysis using IVUS at 4 month follow up, both tissue prolapse in one case and stent edge dissection (proximal end) in another case was observed. In the serial IVUS analysis, both resolved incomplete stent apposition (edge) in one case and late acquired incomplete stent apposition (body) in another were observed. Table 26 shows the OCT results with respect to strut coverage (%) for each of the 4 month, 6 month, and 8 month groups. The OCT results show a high rate of stent strut coverage at all time points, indicating safety of the device.

**Table 26: OCT results**

| Strut Coverage (%) | 4-month group | 6-month group | 8-month group |
|---|---|---|---|
| Median | 90% | 97% | 96% |
| Mean | 85% | 93% | 96% |

Results of this example study indicate that the device as designed according to methods described herein and having the features noted herein results in a complete absorption of the polymer in 90 days leaving a bare metal stent. The device as shown and described herein has rapid, uniform neointimal coverage with no adverse vessel reaction at four months follow up, at least. The late lumen loss and percent (%) obstruction show good inhibition of neointimal hyperplasia. This is demonstrated at least by: in stent LLL at 8 months was 0.09mm; the percent neointimal obstruction at 8 months was 10.9%; and/or there were no incidences of binary restenosis or revascularizations. In the 4 month follow up analysis, no significant changes were observed in vessel volume index, plaque volume index, or lumen volume index. Neointimal obstruction at 4 months was minimal, and no case showed significant lumen encroachment. Neointima-free frame ratio was 20.2 +/- 16.6%, indicating the large part of the stented segments covered with IVUS- detectable neointima even at 4 months follow up. Late acquired incomplete apposition was observed in one case. Furthermore, OCT demonstrates good strut coverage at all time points. Good strut coverage is demonstrated by OCT evaluation of strut coverage as noted herein and shows that at least 80% of the struts are covered on average at each of 4 months, 6 months and 8 months following implantation of the device. Good strut coverage is demonstrated by OCT evaluation of strut coverage as noted herein and shows that at least 80% of the struts are covered on average at 4 months, and at least 90% of the struts are covered on average at 6 months and at 8 months following implantation of the device. Good strut coverage is demonstrated by OCT evaluation of strut coverage as noted herein and shows that at least 80% of the struts are covered on average at 4 months, at least 90% of the struts are covered on average at 6 months, and at least 95% of the struts are covered on average at 8 months following implantation of the device. Good strut coverage is demonstrated by OCT evaluation of strut coverage as noted herein and shows that at least 85% of the struts are covered (median) at each of 4 months, 6 months and 8 months following implantation of the device. Good strut coverage is demonstrated by OCT evaluation of strut coverage as noted herein and shows that at least 85% of the struts are covered (median) at 4 months, and at least 95% of the struts are covered (median) at 6 months and at 8 months following implantation of the device.

The devices exhibit good efficacy and safety through 8 months follow up and improved safety profile as compared to current DES made by other methods such as using solvent based coating methods wherein the drug is amorphous in form. The devices as described herein and/or as described in this study provide controlled, continuous, sustained release of drug over 6 months, without an initial drug burst into the tissue surrounding the device or into the blood stream (as exists in current DES devices). Efficacy appears comparable or improved as compared to current DES devices. Absence of polymer coating in the tissue (after 90d), or on the stent (once cleared from the stent at 45-60 days) may mitigate hypersensitivity, impaired healing, and abnormal vasomotor function. The devices as described herein can thus reduce risks of DAPT non-compliance and/or interruption. They can be used on high-risk patients. They can reduce or eliminate risks of permanent coating such as long term thrombosis risks. No stent thrombosis was detected in any patient in the study as tested through 8 months.

### Example 37: OCT Testing

Optical Coherence Tomography was performed by sequential evaluation of implants in a porcine coronary model at 1 month (28-30 days), 90 days, and 180 days following implantation with a device as described herein. The devices used in this study were the same as the devices as described in Example 34, and produced accordingly as described therein. The OCT evaluation showed complete strut coverage as early as 1 month; low intimal hyperplasia was shown for the duration of the 180 day study and there was no evidence of late catch up. No stent malapposition was detected through 90 days, and there was no late acquired malapposition detected.

A total of six stents (three, 3 x 15 mm drug eluting stents (target of 135µg sirolimus per stent) and three, 3 x 15 mm EuroCor BMS stents) were implanted in 3 Yucatan Mini swine. These animals were subjected to certain OCT analyses at baseline, 3, 28, 90 and 180 days after implantation. Histopathologic and morphometric analyses were performed on the tissue following necropsy at the 180 time point (refer to Table 27).

**Table 27: Study Design**

| Stent Group Number | Test/Control Articles | Number of Devices | Implantation Scheme | OCT Time Points | Necropsy Time Point |
|---|---|---|---|---|---|
| 1 | DES (Test) | n = 3 | Up to 2 vessels were implanted per animal (RCA, LAD, LCX or branches thereof) | 0, 3, 28, 90, and 180 Day | 180 Day (± 5%) |
| 2 | BMS (Control) | n = 3 | | | |

Hearts were pressure perfused (∼100 mm Hg) ex vivo with lactated Ringer's solution until cleared of blood, and then pressure fixed with 10% neutral buffered formalin (NBF). The fixed hearts were placed in labeled 10% NBF-filled containers pending histologic processing and assessment.

Post-fixation, whole heart ex vivo radiographs were obtained to document stent location and morphology in situ. In addition, each explanted stent, pre and post embedment, was radiographed in two views (two roughly perpendicular or orthogonal incidences) along its longitudinal plane to assist in the assessment of expansion morphology, damage and/or areas of stent discontinuity (e.g., strut fractures).

Coronary Arteries: Formalin-fixed stented vessels were carefully dissected from the heart, leaving sufficient vessel both proximal and distal to the stented portion. Transverse sections of vessel adjacent to the stented segment were obtained within approximately 5 mm of the proximal and distal ends of the stent. All vessel sections were stained with hematoxylin and eosin (H&E) and a Verhoeff's tissue elastin stain.

Quantitative morphometric analysis was performed on histological sections from each stented artery, including the parameters as noted and described in Table 4 above. For each histological section, the parameters of Table 4 were directly measured using standard light microscopy and computer-assisted image measurement systems (Olympus Micro Suite Biological Suite). Measured and calculated parameters, formulae and units were as listed in Table 5 above.

Self-calibrating OCT, Light Lab model C7 XR, imaging was performed at the time of stent deployment and on Day 3 (or Day 4), Day 28, Day 90 and Day 180 post implantation, in part, as a measure of characterizing radial force requirements of the stent and to evaluate stent recoil over time. The same OCT operator performed the imaging and analysis of the images in all animals at all evaluated time points.

An OCT catheter was positioned over a guidewire, distal to the stent. An automated pullback was performed to image the stent from the distal edge to the proximal edge. Lumen diameter measurements were made based on two axes minimum. At each time point, four OCT measurements were taken as follows: one measurement toward the distal end of the stent, one near the middle of the stent, one near the proximal end of the stent, and one in a segment of artery adjacent to the stented segment. The mean measurement for each stent was calculated among the three stent measurements taken.

Neointimal proliferation is a normal physiologic response to stent induced artery injury. It may be used to assess efficacy (neointimal inhibition) and safety (inflammation-induced neointimal proliferation) when other factors (e.g., procedure-induced artery injury or idiosyncratic healing characteristics) are absent. Neointimal thickness and area data at 180 days are summarized in Table 28 and Table 29. Data for these parameters was not evaluated at other time points. The data show that the neointimal response was moderately decreased in the coated stent relative to the uncoated stent, however, there was no statistically significant decrease observed. There was a mean increase with the uncoated stent associated with increased injury in one stent. After 180 days in the porcine coronary artery model, the coated stent had no adverse effect with respect to the neointimal response, which was decreased relative to the uncoated stent.

**Table 28: Neointimal Thickness at 180 Days (in mm)**

| **Group** | **Mean (mm) +/- SD (mm)** |
|---|---|
| Coated Stent | 0.14 +/- 0.05 |
| Uncoated Stent | 0.22 +/- 0.10 |

**Table 29: Neointimal Area at 180 Days (in mm^2)**

| **Group** | **Mean (mm^2) +/- SD (mm^2)** |
|---|---|
| Coated Stent | 1.13 +/- 0.35 |
| Uncoated Stent | 1.77 +/- 0.75 |

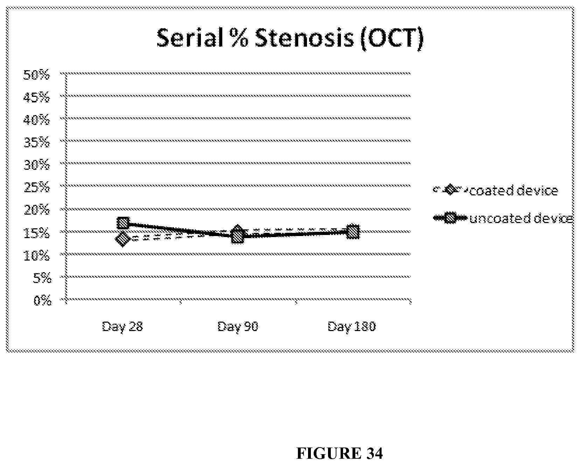

Low neointimal hyperplasia and no late catch-up was found in this study of the devices as described herein. This is evidenced in Figure 34, which shows the average percent stenosis analyzed over the timeframe of the study. Serial percent stenosis for the Day 3, 28, 90, and 180 time points was calculated by dividing the Day 0 stent diameter minus the time point lumen diameter by the Day 0 stent diameter. Percent stenosis was slightly higher for the EuroCor BMS group compared to the DES group on Day 28 but by Day 180 they were equivalent, which is an expected vascular healing observation.

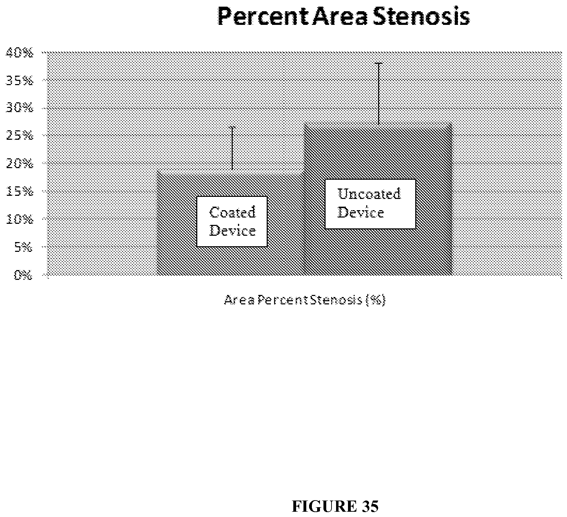

Low neointimal hyperplasia and no late catch-up is also evidenced in Figure 35, which shows the percent area occlusion over the course of the study.

Complete strut coverage even as early as 28 days was observed and is shown, at least, in Table 30, which depicts the average neointima analyzed over the timeframe of the study. Proximal, mid, and distal area measurements were averaged to obtain a single value (area in mm²) for each stent for the Day 28, 90, and 180 time point. Serial neointima for the Day 28, 90, and 180 time points was calculated by subtracting the average luminal area from the average stent area for each time point. Neointimal growth was slightly higher for the EuroCor BMS group compared to the DES group on Day 28 but by Day 180 they were equivalent, which is an expected vascular healing observation.

**Table 30: Serial Neointimal Growth**

| Serial Neointima (by OCT) | 28 days | 90 days | 180 days |
|---|---|---|---|
| Coated device | 1.28 +/- 0.52 mm^2 | 1.39 +/- 0.65 mm^2 | 1.29 +/- 0.61 mm^2 |
| Uncoated device (BMS) | 1.69 +/- 0.68 mm^2 | 1.31 +/- 0.32 mm^2 | 1.17 +/- 0.49 mm^2 |

OCT demonstrated that the stents (coated and uncoated) demonstrated adequate radial force allowing for continuous apposition of the both stents to the vessel wall. This apposition was visually evident from an examination of the OCT images at each time point. This demonstrates there was no stent malapposition through 90 days and no late acquired malapposition (at 180 days) Also, serial OCT was able to determine the extent of neointimal growth during the course of the study but was unable to identify the specific makeup of the neointima.

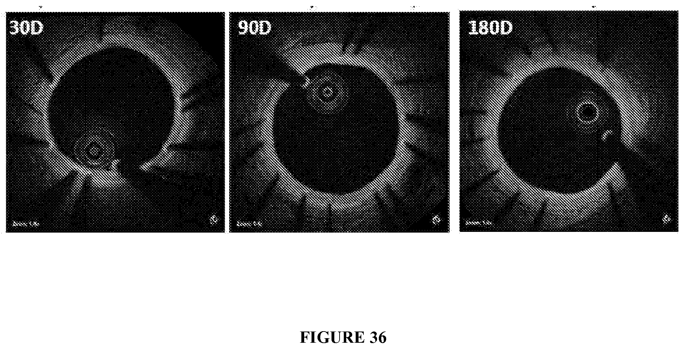

Figure 36 shows example target arteries having an embodiment device implanted therein at each of 30 days, 90 days and 180 days after implantation of the device, and showing, at least, thin homogeneous tissue coverage of the stent struts at each time point.

### Example 38: OCT and Histological Evaluation

Novel vascular scaffolds have been developed aiming at equipoise between safety and efficacy. Intravascular optical coherence tomography (OCT) allows in-vivo serial assessment of stent-vessel interactions with high resolution (∼10µm) and frequent sampling (∼0.2mm intervals) and may complement histology assessment of new technologies. Vascular response to a sirolimus-eluting stent (DES) comprising a coating as described herein, in particularly as described and/or referenced in Example 37 and/or Example 34, was evaluated by means of serial OCT and histology in a porcine model.

One DES and one bare-metal stent (BMS) were implanted in separate epicardial coronary arteries in each of three Yucatan mini-swine. Serial OCT imaging was performed at post procedure, 3, 28, 90 and 180 days follow-up. Normalized optical density (NOD) was used for the assessment of tissue response in the stented arterial segments over time. Histological evaluation was performed at 180-day post-procedure.

A total of 5,064 stent struts in 595 cross-sections were analyzed. OCT revealed 100% of struts covered at 28 days, and a significant difference in NOD from 3 to 28 days (0.64 ± 0.07 vs. 0.71 ± 0.05, respectively, p<0.001) in DES group. Progressive maturation of neointima was observed until 180 days in both groups. Neointimal thickness (NIT) was 0.14±0.08mm, 0.17±0.11 mm, and 0.16±0.09 mm in the DES and 0.18±0.10mm, 0.14±0.09mm, and 0.10±0.08mm in BMS group, while extent of uncovered struts were 0%, 0%, and 3.1% in the DES and 1.4%, 7.8%, and 21.5% in BMS group respectively at 28, 90, and 180 days. No significant changes in the rates of malapposition were observed over time in the DES group and no abnormal intraluminal tissue was revealed. Minimal, non-granulomatous inflammation and a mature endothelialization were demonstrated in both groups by histology. Fibrin deposition was minimal in the entire population at 180 days. There was excellent correlation in stent and vessel areas and diameters between OCT and histomorphometry.

OCT examination provided serial assessment of vascular response to stent implantation over time, and suggested neointimal hyperplasia (NIH) maturation 28 days following DES implantation in pigs. These findings coupled with histological demonstration of low inflammation scores and complete endothelial coverage as measured at 180 days suggest a satisfactory healing response to DES.

Drug-eluting stents reduce the rates of restenosis and repeat revascularization procedures when compared with bare-metal stents (BMS). Nevertheless, arterial healing impairment due to the inhibition of cellular proliferation has been linked with increased risk of late thrombosis due to conventional drug eluting stents (not DES having the coatings as described herein according to methods herein). Indeed, a morphometric predictor of late stent thrombosis according to a post-mortem pathology study was the ratio of uncovered (non-endothelialized) struts to total struts per section. Whether these pathological findings reflect continued vascular reaction to durable polymer, which covers the entire surface of most conventional drug eluting stents, or cell cycle inhibitory effects of the drugs is difficult to determine. Therefore, attempts to improve conventional drug eluting stent safety have focused both on optimizing drug delivery and reducing vessel exposure to polymers. The DES used in this Example uses the coating technology described herein and improves and increases control of drug release as compared to conventional drug eluting stents and comprises an absorbable polymer that is eliminated within 90 days after implantation.

In the present Example, intravascular optical coherence tomography (OCT) was used to explore the vascular tissue response to DES implantation in a porcine model. OCT is a light-based imaging modality that provides high-resolution (∼10µm) imaging, enabling assessment of important morphometric parameters such as stent strut coverage and apposition. OCT has the potential to complement standard histology to assess drug-eluting stents platforms by allowing in vivo serial evaluation of stent vessel interactions at a micron-scale level and frequent sampling (up to 0.2mm intervals) without the need for tissue preparation. In this study, both serial OCT and terminal histological assessment were used to provide a more comprehensive evaluation of the tissue response following stent implantation.

The DES used in this Example comprises a balloon-expandable, laser-cut, cobalt chromium alloy stent with 63.5µm-thick struts. The stent coating comprises poly lactide-co-glycolic acid (PLGA), which is a well-known and characterized biodegradable and biocompatible polymer. The coating also comprises sirolimus in crystalline form; sirolimus is an antiproliferative drug. The coating was approximately 5µm thick on the luminal and 15 µm thick on the abluminal stent surfaces. DES used in this Example comprised a dry powder electrostatic coating process for coating the absorbable polymer and drug components onto the stent. The polymer is completely metabolized to carbon dioxide and water, and the drug is fully released from the stent in a period of 3 months post-implant, leaving an inert BMS within the coronary artery after this period. The Eurocor BMS (EuroCor GmbH, Bonn, Germany) was used as a control in this study as it is the underlying stent platform of the DES. As previously noted, the stent was coated in alignment with the methods of, and resulted in a coated stent as described in, Example 34 or Example 37.

Porcine coronary artery implants and subsequent histopathology and histomorphometry analyses were performed using laboratory standard operating procedures in compliance with the Animal Welfare Act and its amendments. These studies also adhered to the guidelines described in the Guide for the Care and Use of Laboratory Animals. Three Yucatan mini-swine were implanted with DES and/or BMS for 180 days. Each pig received one DES and one BMS in separate coronary arteries. Coronary angiography, as well as OCT were performed at baseline, 3, 28, 90, and 180 days after stent implantation. At 180 days, the animals were euthanized with an overdose of pentobarbital under deep anesthesia and submitted to necropsy and subsequent histological analysis.

In a separate pharmacokinetic study, Yucatan mini-swine were implanted with DES for up to 180 days. At various time intervals after implantation (1, 3, 7, 14, 21, 30, 45, 60, 90 and 180 days) hearts were removed and the stented vessels were dissected from the myocardium including vessel proximal and distal to the stented segment. The stent was cut longitudinally and tissue was removed from the stent. Drug content was assessed separately from the stent and the tissue surrounding the stent. During manual separation of the stent from the arterial tissue, tissue embedded coating deposits were retained with the tissue fraction and account for additive drug in the measured tissue concentrations seen during analysis of drug content. Blood levels were also measured from a separate set of pigs and include additional time points ranging from minutes to hours after implant. Concentration of drug in tissue, blood and on stents was determined using a GLP validated LC-MS/MS method.

Quantitative coronary angiograms at baseline, immediately after PCI, and at follow-up 3, 28, 90 and 180 days after implant were performed in at least two orthogonal views after administration of 50-200 µg intracoronary nitroglycerin. Digital coronary angiograms were analyzed offline at the core laboratory with a validated automated edge detection system (CAAS II, PIE Medical, Maastricht, the Netherlands). Angiographic measurements were made in the same two projections at post-PCI, and follow-up. The stented segment plus 5-mm distal and proximal edges were selected for analysis. Reference vessel diameter was obtained. Late lumen loss (LL) was calculated as the change in minimal luminal diameter (MLD) from post-procedure to follow-up. Binary angiographic restenosis was defined as diameter stenosis ≥50% at follow-up.

OCT images were acquired with a commercially available system (C7-XRTM OCT Intravascular Imaging System, St. Jude Medical, St. Paul, Minnesota) after intracoronary administration of 50-200 µg of nitroglycerin through conventional guiding-catheters. A 0.014-mm guidewire was positioned distally and the OCT catheter (C7 DragonflyTM, St. Jude Medical, St. Paul, Minnesota) was advanced to the distal end of the stent. The entire length of the region of interest was scanned using the integrated automated pullback device at 20 mm/s. During image acquisition, coronary blood flow was replaced by continuous flushing of contrast media in order to create a virtually blood-free environment. All images were digitally stored and submitted to core laboratory offline evaluation and subsequent analysis using proprietary software. The images were analyzed by two experienced OCT analysts blinded to group allocation, and reviewed by a third reader. All cross-sectional images (frames) were initially screened for quality assessment and excluded from analysis if any portion of the image was out of the screen, a side branch occupied > 45° of the cross-section, or the image had poor quality caused by residual blood or sew-up artifact. At 3 day follow-up, a qualitative binary evaluation for coverage (i.e., fibrin) of stent struts was performed at 0.6-mm interval; a strut was considered covered when tissue was visible over its entire circumference. Strut-level analysis was performed considering every three frames (0.6-mm intervals) along the entire target segment. Lumen, stent, and NIH areas and volumes were calculated in a similar fashion for baseline, 28, 90 and 180 days. A strut was considered suitable for analysis only if it had: 1) well-defined bright "blooming" appearance; and 2) characteristic shadow perpendicular to the light source. The inner and outer contours of each strut reflection (blooming) were delineated semi-automatically. The center of the luminal surface of the strut blooming was determined for each strut and its distance to the lumen contour was calculated automatically to determine strut-level intimal thickness (SIT). Struts covered by tissue had positive SIT values, whereas protruding uncovered struts or malapposed struts had negative SIT values. Data were stored in an integrated database system, which corrects for strut thickness of different stent types once the study is completed and data are locked, thus allowing for blinding of the readers. Strut malapposition was determined when the negative value of SIT was higher than the strut thickness, according to the stent manufacturer's specifications (90µm), with the addition of a compensation factor of 20 µm to correct for strut blooming. The blooming compensation factor was determined based on analysis of 2,250 struts. Highly reproducible measurements for strut apposition and coverage using the described methodology have been reported (16). Qualitative imaging assessment was performed in every frame at all the time points for the presence of abnormal intra-stent tissue (AIT). AIT was defined as any mass protruding beyond the stent struts into the lumen, with irregular surface and a sharp intensity gap between mass and neointimal tissue.

Based on OCT imaging properties, the pixel intensity (optical density) of stent strut covering tissue (ODT) localized in the inner side of the struts was evaluated and normalized for the optical density of the stent struts (ODS). The correlation between ODT/ODS (named normalized optical density (NOD)) and morphologic information provided by both light and electron microscopy were evaluated for randomly chosen stent struts at all time points to demonstrate longitudinal changes in NOD of stent strut coverage as assessed by OCT. A region of interest was manually drawn by experienced OCT analysts and the values of ODT and ODS were obtained automatically using computer-assisted analysis software. NOD at 3 and 180 days served as references, assuming that the tissue covering stent struts was mostly fibrin in the former. Conversely, the coverage of the stents at 180 days was essentially composed of neointima as shown by histology in the present study. Thus, the present study NOD ranges for fibrin and neointimal tissue were established based on these results.

An experienced pathologist who was blinded to the groups performed all histomorphometric and histological analysis. Hearts were pressure perfused (∼ 100 mmHg) ex vivo with lactated Ringer's solution until cleared of blood, and then pressure fixed with 10% neutral buffered formalin (NBF). The fixed hearts were placed in labeled 10% NBF-filled containers pending histologic processing and assessment. Post-fixation, whole heart ex vivo radiographs were obtained to document stent location and morphology in situ. In addition, each explanted stent was radiographed in two views (two roughly perpendicular or orthogonal incidences) along its longitudinal plane to assist in the assessment of expansion morphology, damage and/or areas of stent discontinuity (i.e., strut fractures).

Formalin-fixed stented coronary arteries were carefully dissected from the heart, leaving sufficient vessel both proximal and distal to the stented portion. Transverse sections of non-stented vessel were obtained within approximately 5 mm of the proximal and distal ends of the stent. All vessel sections were stained with hematoxylin and eosin (H&E) and a tissue elastin stain (e.g., Verhoeff's), utilizing previously published methods (21). Distal, middle, and proximal sections from each of the stented coronary arterial segments were evaluated. For each histological section, lumen area and diameters, internal (IEL) and external elastic layer (EEL) bounded area, and stent area were directly measured using standard light microscopy and computer-assisted image measurement systems (Olympus Micro Suite Biological Suite). Neointimal thickness [(IEL diameter-lumen diameter)/2] and percent area stenosis [neointimal area/ (lumen area + neointimal area) X 100] were calculated.

The inflammation score was determined by the degree and extent of inflammation on a per-strut basis and the average was calculated per plane (i.e., proximal, middle, and distal) and stent. The score was graded as follows: 0 when there were no cells present; 1 for fewer than 20 cells associated with stent strut; 2 when there were greater than 20 cells associated with stent strut, with or without tissue effacement and little to no impact on tissue function; 3 for >20 cells associated with stent strut with effacement of adjacent vascular tissue and adverse impact on tissue function. The injury score matrix was calculated in a similar fashion and was graded according to the following scores: 0 represented no injury with IEL intact; 1, disruption of IEL; 2, disruption of tunica media, and 3, disruption of EEL/adventitia. Endothelialization, adventitial fibrosis, and neointimal maturation were scored as detailed in Table 31 below.

**Table 31: Histological scores for neointimal maturation, adventitial fibrosis and endothelialization.**

| **Score** | **Neointimal Maturation Score Matrix** | **Adventitial Fibrosis Score Matrix** | **Endothelialization Score Matrix** |
|---|---|---|---|
| **0** | Absent | Absent | Absent |
| **1** | Immature* | Minimal fibrous tissue | <25% |
| **2** | Transitional† | 25-50% fibrous tissue | 25-75% |
| **3** | Mature‡ | > 50% fibrous tissue | >75% |
| **4** | - | - | 100%, confluent |

| | | | |
|---|---|---|---|
| *predominantly fibrino-vascular tissue; †predominantly organizing smooth muscle cells; ‡generalized organized smooth muscle cells. | | | |

All statistical analyses were performed using SAS (v9.2) software (SAS Institute, Cary, NC) and statistical significance was assessed at the 0.05 level. Continuous variables are expressed as mean ± SD, and categorical variables are expressed as counts and percentages. Given the hierarchical nature of the data (stent struts nested within frame nested within lesion nested within pig), multilevel mixed models which can address random effects at lesion and subject levels were used for comparisons of binary and continuous outcomes. Mixed effects model was used to estimate correlation coefficient between measurements from histomorphometry and OCT with repeated observations.

Six stents (three DES and three BMS) were successfully implanted in different porcine epicardial coronary arteries. No complications were identified either during stent deployment or at follow-up assessments. Following stent placement, OCT was successfully performed in all the animals at baseline, 3, 28, 90 and 180 days of follow-up, except for one animal, which was evaluated at day 4 rather than at day 3. Blood samples were taken as part of the pharmacokinetic analyses. All the animals survived until the last follow-up time point of the study (180 days), when they were euthanized.

Elution of drug from the DES coatings containing crystalline sirolimus was characterized by a relatively constant rate of drug delivery throughout the entire period of drug release regardless of the amount of drug remaining in the coating. In vivo release of drug from the stent was complete within 45-60 days after implantation into porcine coronary arteries (Figure 27, top line at day 0 to 20, at least). The average release rate amounts to ∼3 µg/day of sirolimus from a 3.0 x 15 mm stent. Control over sirolimus release results in efficient drug transfer to and deposition within the arterial tissue (Figure 27, bottom line at days 0 to 20, at least). As drug leaves the stent it accumulates in tissue with peak arterial levels occurring 30-60 days after stent implantation. The apparently high value of the sirolimus concentration in tissue is an artifact of the inability to separate embedded coating deposits from surrounding neointima. Thus the concentration of sirolimus in tissue is a composite of drug still contained in a crystalline structure within the coating and drug that has eluted from that coating. Because sirolimus demonstrates high affinity tissue binding even after elution from the coating it is retained in the tissue and cleared relatively slowly over the course of several months.

Quantitative coronary angiography results obtained post procedure and at 180 days are represented in Table 32. The effectiveness parameters evaluated (i.e., late lumen loss, binary restenosis and percentage diameter stenosis) were equivalent between the groups, in concordance with OCT findings.

**Table 32: Quantitative coronary angiography.**

| | **Control BMS** | **Treatment DES** | **p Value** |
|---|---|---|---|
| **Post-procedure** | | | |
| **In-stent MLD, mm** | 2.09 ± 0.25 | 2.32 ± 0.15 | 0.149 |
| **In-lesion MLD mm** | 1.80 ± 0.17 | 2.07 ± 0.26 | 0.129 |
| **In-stent DS,** % | 3.67 ± 0.58 | 4.00 ± 3.00 | 0.885 |
| **In-lesion DS, %** | 12.33 ± 6.11 | 11.67 ± 2.89 | 0.904 |

| **Follow-up 180 Days** | | | |
|---|---|---|---|
| **In-stent MLD, mm** | 1.94 ± 0.22 | 2.11 ± 0.40 | 0.448 |
| **In-lesionMLD, mm** | 1.85 ± 0.26 | 2.03 ± 0.31 | 0.090 |
| **In-stent DS, %** | 13.00 ± 3.46 | 14.00 ± 3.61 | 0.785 |
| **In-lesion DS, %** | 15.33 ± 1.53 | 16.00 ± 4.36 | 0.808 |
| **In-stentlate loss, mm** | 0.15 ± 0.11 | 0.21 ± 0.26 | 0.780 |
| **In-lesion late loss, mm** | -0.05 ± 0.22 | 0.04 ± 0.06 | 0.529 |
| **In-stent binary restenosis, n (%)** | 0 (0.00) | 0 (0.00) | NA |
| **In-lesion binary restenosis, n (%)** | 0 (0.00) | 0 (0.00) | NA |

A total of 5,064 stent struts in 595 cross-sections were analyzed. Only 7.9% of the frames, equally distributed between the groups, were considered not suitable for analysis. Baseline OCT results were equivalent between the groups and are reported in Table 33.

**Table 33: Baseline intravascular optical coherence tomography data.**

| | **Control BMS** | **Treatment DES** | **p Value** |
|---|---|---|---|
| **Lesions** | 3 | 3 | -- |
| **Total length, mm** | 43.6 | 43.2 | -- |
| **Total Frames** | 74 | 74 | -- |
| **Analyzed struts, n** | 631 | 643 | -- |
| **Malapposed struts, n (%)** | 5.7 (36/631) | 2.5 (16/643) | 0.280 |
| **Lumen area, mm²** | 6.49 ± 0.79 | 7.04 ± 1.05 | 0.589 |
| **Stent area, mm²** | 6.10 ± 0.73 | 6.62 ± 1.03 | 0.596 |

Longitudinal OCT assessments are summarized in Table 34. At 3 days, 22% of the stent struts in the control group and 17.8% in the treatment group (p=0.917) were covered by low intensity, irregular tissue, suggestive of fibrin. Stent strut coverage was completed by 28 days in DES group and remained unchanged through 180 days. Furthermore, no stent strut malapposition was identified at 28 and 90 days following DES implantation. At 180 days newly acquired stent malapposition was observed in both groups. While the 2.1% rate of malapposition observed in the DES group was not statistically different from prior assessments (p=0.685), there were 17.4% of struts malapposed in the control group, reflecting a significant increase in malapposition rates from 28-days post implant. A progressive increase in the rates of uncovered struts over time was observed in the control group. One possible mechanism for these findings is a significant enlargement of the luminal area exhibited by one of the pigs (6.15 ± 0.53mm2, 6.39 ± 0.36mm2, and 7.65 ± 0.52mm2, respectively for 28, 90 and 180 days (p<0.001 for the comparisons between 28 and 180 days, and 90 and 180 days)). There was no AIT identified at any time points.

**Table 24: Longitudinal optical coherence tomography assessment.**

| | **Day 28** | **Day 90** | **Day 180** | **p* Value** |
|---|---|---|---|---|
| **Lesions, n** | | | | |
| **CONTROL BMS** | 3 | 3 | 3 | NA |
| **TREATMENT DES** | 3 | 3 | 3 | NA |

| **Total Analyzed Length (mm)** | | | | |
|---|---|---|---|---|
| **CONTROL BMS** | 46.2 | 39.8 | 44.0 | NA |
| **TREATMENT DES** | 45.0 | 42.5 | 42.2 | NA |

| **Total Frames, n** | | | | |
|---|---|---|---|---|
| **CONTROL BMS** | 81 | 69 | 75 | NA |
| **TREATMENT DES** | 77 | 73 | 72 | NA |

| **Analyzed Struts, n** | | | | |
|---|---|---|---|---|
| **CONTROL BMS** | 708 | 525 | 539 | NA |
| **TREATMENT DES** | 729 | 673 | 616 | NA |

| **Uncovered Struts, n (%)** | | | | |
|---|---|---|---|---|
| **CONTROL BMS** | 1.4 (10/708) | 7.8 (41/525) | 21.5 (116/539) | 0.001 |
| **TREATMENT DES** | 0.0 (0/729) | 0.0 (0/673) | 3.1 (19/616) | 0.671 |

| **Frames with >30% uncovered struts, %** | | | | |
|---|---|---|---|---|
| **CONTROL BMS** | 0.0 (0/74) | 15.9 (10/63) | 33.9 (20/59) | 0.423 |
| **TREATMENT DES** | 0.0 (0/75) | 0.0 (0/72) | 4.4 (3/68) | 0.463 |

| **Malapposed Struts, n (%)** | | | | |
|---|---|---|---|---|
| **CONTROL BMS** | 0.1 (1/708) | 2.1 (11/525) | 17.4 (94/539) | 0.002 |
| **TREATMENT DES** | 0.0 (0/729) | 0.0 (0/673) | 2.1 (13/616) | 0.685 |

| **Neointimal thickness of Covered Struts, mm** | | | | |
|---|---|---|---|---|
| **CONTROL BMS** | 0.18 ± 0.10 | 0.14 ± 0.09 | 0.10 ± 0.08 | 0.016 |
| **TREATMENT DES** | 0.14 ± 0.08 | 0.17 ± 0.11 | 0.16 ± 0.09 | 0.370 |

| **Lumen area (mm²)** | | | | |
|---|---|---|---|---|
| **CONTROL BMS** | 4.95 ± 1.02 | 5.22 ± 1.18 | 5.99 ± 1.39 | 0.048 |
| **TREATMENT DES** | 5.40 ± 1.48 | 5.37 ± 1.62 | 5.60 ± 1.74 | 0.577 |

| **Stent area (mm²)** | | | | |
|---|---|---|---|---|
| **CONTROL BMS** | 6.37 ± 0.83 | 6.19 ± 0.91 | 6.32 ± 0.64 | 0.406 |
| **TREATMENT DES** | 6.34 ± 1.10 | 6.57 ± 1.06 | 6.58 ± 1.10 | 0.168 |

| **NIH area (mm²)** | | | | |
|---|---|---|---|---|
| **CONTROL BMS** | 1.42 ± 0.53 | 1.02 ± 0.39 | 0.70 ± 0.48 | 0.018 |
| **TREATMENT DES** | 0.95 ± 0.42 | 1.20 ± 0.61 | 1.08 ± 0.61 | 0.363 |

| **Stenosis (%)** | | | | |
|---|---|---|---|---|
| **CONTROL BMS** | 22.79 ± 8.72 | 17.25 ± 7.85 | 11.61 ± 8.15 | 0.011 |
| **TREATMENT DES** | 16.52 ± 9.91 | 20.31 ± 13.68 | 18.17 ± 12.19 | 0.364 |

| | | | | |
|---|---|---|---|---|
| *: **Longitudinal comparison.** | | | | |

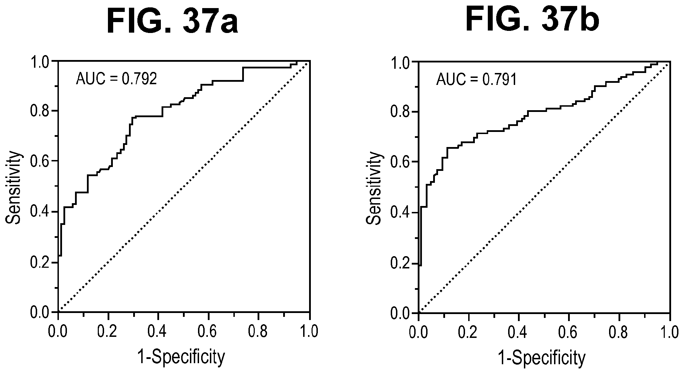

There were 911 struts completely covered by tissue at the 3, 28, 90, or 180 days analyzed. A significant difference in NOD from 3 to 28 days was observed in DES group (0.64 ± 0.07 vs. 0.71 ± 0.05, respectively, p<0.001). There were no differences in NOD between 3 and 28 days (0.66 ± 0.06 vs. 0.68 ± 0.06, respectively, p=NS) in the BMS group, reaching statistical significance at 90 days (0.70 ± 0.05, p=0.007 vs. 3 days). The diagnostic accuracy of NOD was assessed by receiver-operating characteristic (ROC) curve (Figure 37) for the differentiation between fibrin-rich tissue (3-day) and neointimal coverage (180-day) in control (AUC = 0.792) and treatment (AUC = 0.791) groups. Figures 37a and 37b provide an assessment of normalized optical density of stent strut coverage by intravascular optical coherence tomography - including differences between fibrin and neointima. Figures 37a and 37b show receiver-operating characteristic curves showing sensitivity and specificity of normalized optical density to detect fibrin in control (Fig. 37a) and treatment (Fig. 37b) groups, respectively. In this Example, the treatment group is the DES group. For the control group, the best cut-off value to identify fibrin was ≤ 0.700 (sensitivity 72.7%, specificity 71.4%, accuracy 72.1%); for the treatment group the corresponding cut-off value was ≤ 0.685 (sensitivity 72.8%, specificity 72.3%, accuracy 72.6%). The negative and positive predictive values were 71.4% and 72.7% for the control group and 70.8% and 74.3% for the treatment group.

At day 90, the maturation of the tissue covering stent struts as assessed by NOD was equivalent between groups. Percentage of fibrin was 45.5% and 54.5% while percentage of neointimal tissue was 45.9% and 54.1%, respectively for the control and treatment groups (p=NS). At 180 days, the same evaluation revealed reduction of the presence of fibrin-rich tissue in control and treatment groups to 28.5% and 27.7%, respectively (p=0.899).

No animals were found dead or terminated early for this study. There were no macroscopic observations noted at necropsy. Six stented arteries in each group (3 with DES and 3 with BMS) were evaluated. The inflammation score was low and equivalent between groups (0.24±0.23 and 0.54±0.14, respectively for DES and BMS groups, p=non-significant); the inflammatory cells were composed primarily of histiocytes and multinucleated giant cells, regardless of the group. No granulomatous inflammation was identified. Overall incidence and magnitude of injury was slightly decreased in DES group (<10%; limited to grade 2) when compared to BMS group (24%; grades 1-3), although not statistically significant (p= non-significant). Neointimal maturation score matrix was complete with DES and BMS (3.0 ± 0.00 in both groups). Fibrin was virtually absent and comparable between the groups; moreover, adventitial fibrosis, which is characterized by collagen bundles intermixed with fibroblasts, was minimal and no differences were revealed (1.11 ± 0.84 vs. 1.00 ± 1.00, respectively in DES and BMS groups, p= non-significant). Endothelialization score was equivalent and less than complete in both groups, although slightly higher in DES when compared to BMS group (3.22 ± 0.38 vs. 2.67 ± 0.58, respectively, p= non-significant). Neointimal vascularization was rare (33%) and occurred exclusively in BMS group.

At 180 days, NIT was not statistically different between DES and BMS (0.14 ± 0.05mm vs.0.22 ± 0.10mm, respectively, p= non-significant). Percent area stenosis and neointimal area revealed the same tendency (19 ± 8% vs. 27 ± 11%, and 1.13 ± 0.35 mm2 vs. 1.77 ± 0.75 mm2, respectively, p= non-significant for both comparisons). No differences were established regarding tunica media area and lumen:artery ratio between the groups. The correlation between histomorphometry and OCT measurements is demonstrated in Table 35. Correlation was demonstrated between histomorphometry and OCT measurements regarding lumen area (r=0.911) and diameter (r=0.897), stent area (r=0.948) and diameter (r=0.952), as well as in minimal luminal area (r=0.973) and maximum percentage stenosis (r=0.858). The correlation coefficient was not as strong when measuring other aspects using histomorphometry versus OCT.

**Table 35: Comparison between histomorphometry and optical coherence tomography measurements at 180 days.**

| | **Histomorphometry** | **OCT measurement** | **Diff (OCT - histomorphometry)** | **p** | **Correlation coefficient** |
|---|---|---|---|---|---|
| **Lumen Area, mm²** | 4.93 ± 1.27 | 5.86 ± 1.63 | 0.93 ± 0.71 | 0.104 | 0.911 |
| **Minimum Lumen Area, mm²** | 4.51 ± 1.42 | 5.09 ± 1.55 | 0.58 ± 0.37 | 0.110 | 0.973 |
| **Stent Area, mm²** | 6.43 ± 0.92 | 6.48 ± 0.91 | 0.05 ± 0.30 | 0.585 | 0.948 |
| **NIH Area, mm²** | 1.45 ± 0.83 | 0.86 ± 0.58 | -0.59 ± 0.84 | 0.178 | 0.330 |
| **Lumen Diameter, mm** | 2.49 ± 0.33 | 2.70 ± 0.39 | 0.22 ± 0.17 | 0.082 | 0.897 |
| **Stent Diameter, mm** | 2.85 ± 0.21 | 2.86 ± 0.21 | 0.01 ± 0.06 | 0.619 | 0.952 |
| **Stenosis, %** | 23.06 ± 12.29 | 14.49 ± 10.95 | -8.57 ± 12.13 | 0.174 | 0.460 |
| **Maximum Stenosis, %** | 30.50 ± 16.37 | 21.21 ± 14.39 | -9.29 ± 8.40 | 0.119 | 0.858 |
| **NIH thickness, mm** | 0.18 ± 0.11 | 0.11 ± 0.08 | -0.07 ± 0.11 | 0.198 | 0.361 |

This Example evaluated coronary arterial response following the implantation of the DES combining serial imaging assessment using OCT with standard histology. The serial evaluations of arterial response to DES from the time of stent implantation through 180 days of follow-up provided the following observations: 1) the majority of the proliferative response in this porcine non-diseased model depicted by the magnitude of neointimal proliferation and strut coverage occurred in the first 28 days after DES implantation; 2) thereafter, no changes were revealed in the proportion of strut coverage and amount of neointimal hyperplasia at 90 and 180 days; 3) 100% of the post-procedure malapposition resolved by 28-day follow-up.

The present study illustrates the complimentary role of in vivo and ex vivo high-resolution imaging strategies to assess the impact of novel endovascular technologies. Histology remains a standard for tissue characterization and can provide unique information regarding the type and maturation of the tissue covering stent struts, presence of associated inflammation, fibrin or necrosis. OCT enables serial in vivo assessments of stent-vessel interactions at a micron-scale level (∼10µm) without the limitations associated with tissue preparation (i.e. tissue shrinkage). Hence, morphometric parameters, such as stent strut coverage as well as lumen and stent areas and diameters can be followed serially with low intra and inter observer variability and high accuracy as shown herein.

The ability to differentiate fibrin from NIH is important in the evaluation of the vascular response after stent implantation, since the presence of residual fibrin has been associated with delayed healing and stent thrombosis. This Example provides an observation of changes in optical properties of the tissue covering stent struts using a commercially available Fourier Domain OCT system. Others had shown a difference in NOD between fibrin and NIH using a different OCT system (Terumo OFDI system, Terumo R&D Center, Kanagawa, Japan) compared with electron microscopy as standard. This Example applied similar methodology and demonstrated significant differences in NOD of tissue covering DES between 3 and 28 days, suggestive of NIH maturation. Furthermore, serial OCT imaging suggested progressive reduction of fibrin content and its replacement by neointimal tissue that was shown to be equivalent in both groups at 90 and 180 days. This suggests a similar vascular response pattern between DES as configured and produced herein and BMS.

Although a rare clinical condition, stent thrombosis raises a great concern due to its high associated morbidity and mortality. Incomplete stent strut coverage was identified in post-mortem studies as a powerful predictor of late thrombosis in conventional drug eluting stents (not evaluated or shown in DES devices as provided herein). Moreover, stent strut malapposition (markedly the late acquired type), which is more common after conventional drug eluting stent implantation than in BMS implantation, has also been associated with this phenomenon. In this Example, early, complete "healing" has been observed after DES implantation, demonstrated by 100% of stent strut coverage coupled with no malapposition at 28 days. These results were maintained through 180 days. Taken together, the present data suggest a desirable safety profile for the DES technology and support further evaluation. Conversely, in the BMS group, a significant increase of luminal area in one of the pigs over time led to a statistically significant increase in the rates of uncovered and malapposed struts. No signs of exacerbated inflammation were identified by histology in this animal.

This Example demonstrated a mature neointimal tissue with generalized organized smooth muscle cells with minimal presence of fibrin in both groups at 180 days. A postmortem pathological study previously observed persistent fibrin deposition and poor endothelialization of stents deployed in patients who died from late stent thrombosis. Endotheliazation was similar between BMS and DES in the present study and the amount of fibrin was reduced overtime in both groups as demonstrated by OCT.

Sustained inhibition of NIH by DES with no evidence of late "catch-up" was observed in the present study, suggesting long-term efficacy in NIH inhibition.

There are limitations which should be taken into account when interpreting the findings in this Example. Inherent to most pre-clinical device investigations is the lack of direct correlation between vascular response in non-diseased animal models and human diseased coronary arteries. Moreover, a time difference in the healing process (5 to 6 times faster in the porcine model) between humans and swine models should be considered. Nevertheless, the porcine model has been considered the standard pre-clinical model for the evaluation of novel DES, since the stages of healing are comparable to those found in human.

This Example assumed that the coverage of stent struts at the 3-day time point was composed mostly by fibrin-rich tissue based on previous pre-clinical data, as histology at this time frame was not performed in the present study. In spite of longitudinal analysis of 5,064 stent struts utilizing high-resolution imaging by both OCT and histology, the study sample size was small, which limits more definitive conclusions.

Longitudinal examination by means of OCT reveals early coverage, sustained NIH inhibition, and progressive NIH maturation following DES implantation. These findings coupled with low inflammation scores and a mature endothelial coverage at 180 days suggest an satisfactory vascular response to DES. OCT plays a complementary role to histology, allowing serial longitudinal assessments in pre-clinical models.

Provided herein is a device comprising a stent; and a coating on the stent; wherein the coating comprises at least one polymer and a macrolide immunosuppressive (limus) drug, wherein at least a portion of the macrolide immunosuppressive (limus) drug is in crystalline form; wherein a majority of the proliferative response depicted by the magnitude of neointimal proliferation and strut coverage occurs in the first 28 days after implantation.

In some embodiments, after the first 28 days following implantation, no statistically significant changes occur in the proportion of strut coverage and amount of neointimal hyperplasia at 90 and 180 days. In some embodiments, substantially all post-procedure malapposition resolves by 28-day follow-up. In some embodiments, OCT analysis is used to evaluate the device and tissue characteristics following implantation. In some embodiments, a satisfactory healing response to the implantation of the device is shown by histologically demonstrating low inflammation scores and complete endothelial coverage at 180 days in combination with the neointimal maturation at 28 days following implantation shown by OCT analysis.

Provided herein is a method comprising providing a coated stent comprising a stent; and a coating on the stent; wherein the coating comprises at least one polymer and at least one macrolide immunosuppressive (limus) drug; and implanting the coated stent in a subject, wherein at least a portion of the macrolide immunosuppressive (limus) drug is in crystalline form; and determining that the majority of the proliferative response depicted by the magnitude of neointimal proliferation and strut coverage occurs in the first 28 days after implantation.

In some embodiments, the method comprises determining that, after the first 28 days following implantation, no statistically significant changes occur in the proportion of strut coverage and amount of neointimal hyperplasia at 90 and 180 days. In some embodiments, the method comprises determining that substantially all post-procedure malapposition resolves by 28-day follow-up. In some embodiments, the method comprises determining that there is neointimal maturation 28 days following implantation. In some embodiments, the determining step is performed by OCT analysis. In some embodiments, the method comprises showing a satisfactory healing response to the implantation of the device by histologically demonstrating low inflammation scores and complete endothelial coverage at 180 days in combination with the neointimal maturation at 28 days following implantation by OCT analysis.

### Example 39

Stents, mounted on holders, supported on a carousel may be introduced into a coating chamber. The process comprises providing a cloud of charged particles to the stents that are orbiting through the cloud. For the polymer coating steps, this is accomplished by the rapid expansion of the pressurized solution of polymer in densified 1,1,1,2,3,3-hexafluoropropane (FC-236EA) through a small diameter stainless steel orifice. Heat is applied to the orifice to overcome Joule-Thompson cooling and to ensure that the compressed gas is fully vaporized on expansion from the orifice. Further control of the charged polymer cloud is obtained through controlling the polymer solution concentration and flow rate. Flow rate is controlled implicitly by the pressure drop across the nozzle from a constant pressure polymer solution provided by an automated syringe pump.

The solution concentration is controlled by the mass of polymer added to the dissolving chamber and the volume filled within the automated syringe pump. The concentration may be 2 w/v%, 4 w/v%, about 2 w/v%, about 4 w/v%, about 2 w/v% to about 4 w/v%, 2 w/v% to 4 w/v%, 2 w/v% +/- 0.5 w/v%, 2 w/v% +/- 0.25 w/v%, 2 w/v% +/- 0.1 w/v%, 4 w/v% +/- 0. 5 w/v%, 4 w/v% +/- 0.25 w/v%, 4 w/v% +/- 0.1 w/v%, at least 1 w/v%, at least 1.5 w/v%, at least 2 w/v%, at least 3 w/v%, at least 4 w/v%, at most 4 w/v%, at most 5 w/v%, at most 6 w/v%, at most 7 w/v%, at most 8 w/v%, at most 9 w/v%, at most 10 w/v%, at most 11 w/v%, at most 12 w/v%, at most 13 w/v%, at most 14 w/v% ,or at most 15 w/v%. depending on the embodiment. An increase in polymer concentration may coincide with in an attendant decrease in polymer spray time (at fixed flow rate) and greater capacity to coat multiple carousels from a single polymer solution - increasing throughput. Both 2% and 4% have been tested, as examples, and provide a similar polymer particle cloud to the stents. Concentrations of up to 5% have been used yet the upper bound of concentration usable in the coating process has not been determined. In addition, because the sintering steps following application of the polymer particles change the primary polymer particle morphology (but not that of the active agent - whether it be a pharmaceutical agent or biologic agent), implementing the increased solution concentration results in a coating with the same content and performance properties as those coated from 2 w/v% solutions. The limiting effect of concentration is to be low enough to provide particles, instead of fibers, at the exit of the nozzle, wherein particulate may be defined by an aspect ratio of less than 2:1.

Multiple polymer sprays may be incorporated in the coating process (e.g. 2x sprays in the second application of polymer, 3x sprays in the final application of polymer) to provide a consistent cloud and electrostatic environment for all polymer applications, while providing more polymer in the subsequent layers.

A contributing factor to stent coating is the creation of a uniform and reproducible electric potential between the particles (active agent or PLGA), stent and surrounding components (carousel, platform, stainless steel covers, etc.). The process is based on the electrical principle of opposites attract & likes repel. In the process, opposite polarities on the stents vs. particles are established to create an electric field that attracts the particles to the stent. Additionally, the particles are polarized the same as the internal surfaces of the coating chamber leading to enhanced deposition on the stent. Increasing the voltage difference between the stents and the coating chamber increases coating efficiency. However, as voltage increases, the stent struts and fine wires of the stent holder can generate corona discharge disturbing the electric field that can result in poor drug coating consistency. A variety of potentials may be used, however in the present example potentials of ±1.5 kV are conservatively used to minimize the risk of corona discharge and its deleterious effects on the coating. In other embodiments, example potentials include, but are not limited to: ±1.0 kV, ±1.2 kV, ±1.3 kV, ±1.4 kV, ±1.5 kV, ±1.6 kV, ±1.7 kV, ±1.8 kV, ±1.9 kV, ±2 kV, ±3 kV, ±3.5 kV, ±4 kV, ±5 kV, from ±1.0 kV to ±2.0 kV, from ±1.2 kV to ±1.8 kV, from ±1.4 kV to ±1.6 kV, from ±0.5 kV to ±5 kV, or about ±1.5 kV.

After application of each polymer coating step, the stents may be sintered to coalesce the powder coating into a smooth film encapsulating the stent's struts. To accomplish this sintering, the individual stents mounted on stent holders may be moved from the coating carousel into an isothermal sintering chamber set at >40C in the instance of PLGA, or any temperature appropriate for the polymer and drug in question-i.e. at or around the Tg (glass transition temperature) of the polymer, but below the temperature at which the drug (active agent) would change its morphology and/or change its activity. The Tg of PLGA used in this example (50:50), is about 45C, thus, a setting of >40C is sufficient to sinter. A temperature setting at or near the Tg may require longer sintering time. In some embodiments, the sintering temperature is 100C, or about 100C.

The transfer of the stents from the coating carousel to the isothermal sintering chamber is performed touching only the stent holder wire-form because the coating prior to sintering is a powder held only by electrostatic image charge. The sintering may be accomplished by exposing the stent to conditions sufficient to coalesce the powder, yet not aggressive enough to alter the crystalline particle morphology of the drug (or activity of the biologic agent) or cause degradation of the polymer or active agent. For example the setting may be >40C in the instance of PLGA, or any temperature appropriate for the polymer and drug in question-i.e. at or around the Tg (glass transition temperature) of the polymer, but below the temperature at which the drug (active agent) would change its morphology and/or change its activity. The Tg of PLGA used in this example (50:50), is about 45C, thus, a setting of >40C is sufficient to sinter. A temperature setting at or near the Tg may require longer sintering time. In some embodiments, the sintering temperature is 100C, or about 100C. Conditions that would degrade the coating are, for example, solvents, solvent vapors or temperatures > 150°C (in the instance of PLGA and rapamycin). Other temperatures for other polymers and drugs would be appropriate based on the principles noted herein to retain the morphology and/or activity of the agents, and yet to meet or exceed the approximate Tg of the polymer.

Use of compressed gases provides benign conditions for the sintering of polymer powders on surfaces. This may include the use of certain gas in the sintering process, or merely elevated temperature and/or pressure. FC-236EA gas pressure may be provided at each sintering step, or only certain sintering steps. Use of the FC-236EA prolongs the coating process cycle time as compared to elevated temperature and/or pressure only. In certain embodiments, use of the FC-236EA pressurized gas in the sintering process is only at the final sintering step. Inadequate sintering conditions (temperature and/or pressure) can result in inconsistent stent topography and in vitro drug release kinetics.

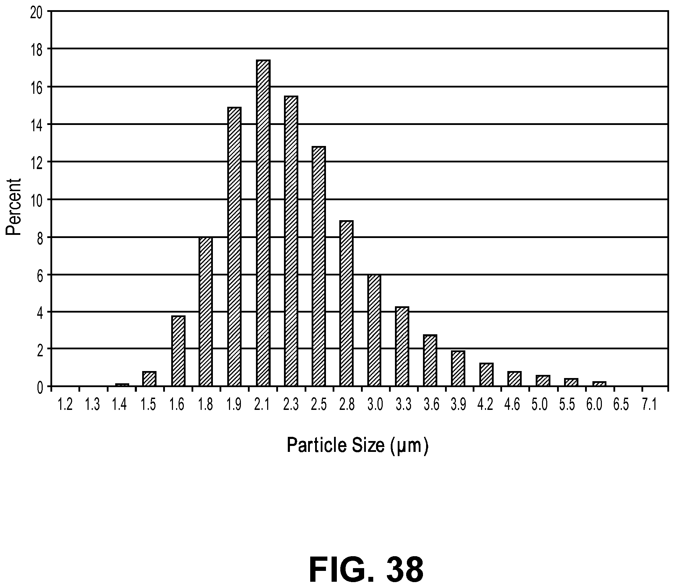

Sirolimus (or any powder form active agent, as noted herein), may be deposited on the stents by an electrostatic dry-powder process, but is distinguished from the polymer coating (e.g. PLGA) process in that the drug is never dissolved in a compressed gas or other solvent In some embodiments, the active agent is micronized prior to deposition on the stent. For example, raw sirolimus may be first micronized to achieve a particle distribution such that at least 99% by volume of the particles are less than 10 microns with the distribution centered at 2.75 +/- 0.5 microns. In other examples, the active agent may be first micronized to achieve a particle distribution such that 80%, 85%, 90%, 95%, 99%, at least 50%, at least 75%, at least 80%, at least 85%, at least 90%, at least 95%, at least 99%, at least about 50%, at least about 75%, at least about 80%, at least about 85%, at least about 90%, at least about 95%, or at least about 99% by volume of the particles are less than 3 microns, less than 5 microns, less than 7.5 microns, less than 10 microns, less than 20 microns, less than 25 microns, less than 30 microns, less than 40 microns, less than 50 microns, less than 75 microns, less than about 10 microns, less than about 15 microns, or less than about 7.5 microns, with the distribution centered at 1.0 +/- 0.5 microns, 1.25 +/- 0.5 microns, 1.5 +/- 0.5 microns, 1.75 +/- 0.5 microns, 2.0 +/- 0.5 microns, 2.25 +/- 0.5 microns, 2.5 +/- 0.5 microns, 2.75 +/- 0.5 microns, 3.0 +/- 0.5 microns, 3.25 +/- 0.5 microns, 3.5 +/- 0.5 microns, 3.75 +/- 0.5 microns, 4.0 +/- 0.5 microns, 4.25 +/- 0.5 microns, 4.5 +/- 0.5 microns, 4.75 +/- 0.5 microns, 5 +/- 0.5 microns, 5.5 +/- 0.5 microns, 6 +/- 0.5 microns, 6.5 +/- 0.5 microns, 7 +/- 0.5 microns, 7.5 +/- 0.5 microns, 8 +/- 0.5 microns, 8.5 +/- 0.5 microns, 9 +/- 0.5 microns, 10 +/- 0.5 microns, 15 +/- 0.5 microns, 20 +/- 0.5 microns, 25 +/- 0.5 microns, 30 +/- 0.5 microns, 35 +/- 0.5 microns, 40 +/- 0.5 microns, 45 +/-0.5 microns, 50 +/- 0.5 microns, about 1.0 microns, about 1.5 microns, about 2.0 microns, about 2.5 microns, about 2.75 microns, about 3.0 microns, about 3.5 microns, about 4.0 microns, about 4.5 microns, about 5 microns, about 6 microns, about 7 microns, about 8 microns, about 9 microns, about 10 microns, about 15 microns, about 20 microns, about 25 microns, about 30 microns, about 35 microns, about 40 microns, about 45 microns, or about 50 microns. Figure 38 depicts an embodiment of micronized sirolimus used in a spray coating process described in this Example, having a particle distribution such that at least 99% by volume of the particles are less than 10 microns with the distribution centered at 2.75 +/- 0.5 microns.

To provide a well dispersed cloud of the fine pharmaceutical agent particles, the drug may be pulsed in to the chamber using a fixed volume of nitrogen pressurized at 2.07 MPa (300 psi) as the propellant. Depending on the embodiment, other propellants may be used, and other pressures. For example, any compatible non-reactive propellant may be used based on the active agent and/or the polymer being used, including to air, one or more noble gas (e.g. argon, nitrogen, helium), or any combination thereof. A variety of pulse pressures may be used, depending on the embodiment. For example, an operating pressure of at least 0.34 MPa (50 psi), at least 0.52 MPa (75 psi), at least 0.69 MPa (100 psi), at least 1.03 MPa (150 psi), at least 1.38 MPa (200 psi), at least 1.72 MPa (250 psi), at least 2.07 MPa (300 psi), about 0.34 MPa (50 psi), about 0.52 MPa (75 psi), about 0.69 MPa (100 psi), about 1.03 MPa (150 psi), about 1.38 MPa (200 psi), about 1.72 MPa (250 psi), about 2.07 MPa (300 psi), about 2.41 MPa (350 psi), about 2.76 MPa (400 psi), about 3.10 MPa (450 psi), about 3.45 MPa (500 psi), about 3.79 MPa (550 psi), about 4.14 MPa (600 psi), 0.34 MPa (50 psi) to 3.45 MPa (500 psi), 1.38 MPa (200 psi) to 2.76 MPa (400 psi), 1.72 MPa (250 psi) to 2.41 MPa (350 psi), 0.34 MPa (50 psi), 0.52 MPa (75 psi), 0.69 MPa (100 psi), 1.03 MPa (150 psi), 1.38 MPa (200 psi), 1.72 MPa (250 psi), 2.07 MPa (300 psi), 2.41 MPa (350 psi), 2.76 MPa (400 psi), 3.10 MPa (450 psi), 3.45 MPa (500 psi), 3.79 MPa (550 psi), or 4.14 MPa (600 psi), may be used. In the instance of sirolimus sprayed as noted herein the operating pressure of 2.07 MPa (300 psi) met the need to deliver a finely dispersed particle cloud into the coating chamber without adding complexity to the coating equipment.

While the stents are sintering, the coating carousel and chambers may be wiped with an acetone-moistened clean room cloth to prevent polymer or drug from building up over multiple cycles on the surfaces exposed to the electrostatic environment.

After the completion of the entire coating sequence: Polymer (1 spray) - Sinter (100°C, ambient pressure) - Drug - Polymer (2 sprays) - Sinter (100°C, ambient pressure) - Drug - Polymer (3 sprays) - Sinter (100°C, 1.03 MPa (150 psi) pressurization with gaseous FC236ea), the stents may be removed from the stent holders for analysis and mounting on a catheter.

The parameters of active agent amount and polymer spray time are dependent on the size of stent (or other device) to be coated. During the course of production, total mass and agent content may be monitored.

## Claims

1. A method of coating a stent comprising:
mounting a stent on a holder in a coating chamber that imparts a charge to the stent,
providing a first cloud of charged particles of polymer to the stents by rapidly expanding a pressurized solution of the polymer in densified 1,1,1,2,3,3-hexafluoropropane through a first orifice, wherein the polymer comprises PLGA, wherein a first polymer layer of the polymer particles is formed on the stent by electrostatic deposition,
sintering the first polymer layer at >40°C in ambient pressure,
providing a first cloud of charged sirolimus particles to the stents having an opposite charge than the charge of the stent by pulsing sirolimus particles into the chamber using a propellant in order to deposit a first agent layer on the stent, wherein at least a portion of the sirolimus particles is in crystalline form,
providing a second cloud of charged particles of the polymer and a third cloud of charged particles of the polymer to the stents by sequentially rapidly expanding the pressurized solution through the first orifice, wherein the particles have an opposite charge than the charge of the stent, wherein a second polymer layer of the polymer particles is formed on the stent by electrostatic deposition,
sintering the second polymer layer at >40°C in ambient pressure,
providing a second cloud of charged sirolimus particles to the stents having an opposite charge than the charge of the stent by pulsing the sirolimus particles into the chamber using a propellant in order to deposit a second agent layer on the stent, wherein at least a portion of the sirolimus particles is in crystalline form,
providing a fourth cloud of charged particles of the polymer, a fifth cloud of charged particles of the polymer, and a sixth cloud of charged particles of the polymer to the stents by sequentially rapidly expanding a pressurized solution through the first orifice, wherein the particles have an opposite charge than the charge of the stent, wherein a third polymer layer of the polymer particles is formed on the stent by electrostatic deposition, and
sintering the third polymer layer at >40°C, at 1.03 MPa (150 psi) pressurization, and with gaseous 1,1,1,2,3,3-hexafluoropropane, wherein the crystalline form sirolimus particles in the first agent layer and second agent layer remain in crystalline form throughout all steps in the method.

2. The method of Claim 1, wherein the stent on the holder is orbiting through any of the first, second third, fourth, fifth, or sixth clouds of charged polymer particles, or through any of the first or second clouds of charged sirolimus particles.

3. The method of Claim 1, wherein the first orifice is heated sufficiently to ensure that the compressed gas is fully vaporized on expansion from the orifice.

4. The method of Claim 1, wherein the concentration of the solution is any of 2 w/v% (weight or mass of polymer per total volume), 4 w/v%, 2 w/v% to 4 w/v%, 2 w/v% to 4 w/v%, 2 w/v% +/- 0.5 w/v%, 2 w/v% +/- 0.25 w/v%, 2 w/v% +/- 0.1 w/v%, 4 w/v% +/- 0. 5 w/v%, 4 w/v% +/- 0.25 w/v%, 4 w/v% +/- 0.1 w/v%, at least 1 w/v%, at least 1.5 w/v%, at least 2 w/v%, at least 3 w/v%, at least 4 w/v%, at most 4 w/v%, at most 5 w/v%, at most 6 w/v%, at most 7 w/v%, at most 8 w/v%, at most 9 w/v%, at most 10 w/v%, at most 11 w/v%, at most 12 w/v%, at most 13 w/v%, at most 14 w/v%, or at most 15 w/v%.

5. The method of Claim 1, wherein the sirolimus particles comprise a particle distribution such that at least 99% by volume of the sirolimus particles are less than 10 microns with the distribution centered at 2.75 +/- 0.5 microns.

6. The method of Claim 1, wherein the sirolimus particles comprise a particle distribution such that 80%, 85%, 90%, 95%, 99%, at least 50%, at least 75%, at least 80%, at least 85%, at least 90%, at least 95%, at least 99% by volume of the particles are less than 10 microns.

7. The method of Claim 1, wherein the sintering is performed at about 100°C.

8. The method of Claim 1, wherein the sirolimus particles comprise a particle distribution such that at least 50% by volume of the particles are less than 3 microns, less than 5 microns, less than 7.5 microns, less than 10 microns, less than 20 microns, less than 25 microns, less than 30 microns, less than 40 microns, less than 50 microns, less than 75 microns.

9. The method of Claim 1, wherein the sirolimus particles have a distribution centered at 1.0 +/- 0.5 microns, 1.25 +/- 0.5 microns, 1.5 +/- 0.5 microns, 1.75 +/- 0.5 microns, 2.0 +/- 0.5 microns, 2.25 +/- 0.5 microns, 2.5 +/- 0.5 microns, 2.75 +/- 0.5 microns, 3.0 +/- 0.5 microns, 3.25 +/- 0.5 microns, 3.5 +/- 0.5 microns, 3.75 +/- 0.5 microns, 4.0 +/- 0.5 microns, 4.25 +/- 0.5 microns, 4.5 +/- 0.5 microns, 4.75 +/- 0.5 microns, 5 +/- 0.5 microns, 5.5 +/- 0.5 microns, 6 +/- 0.5 microns, 6.5 +/- 0.5 microns, 7 +/- 0.5 microns, 7.5 +/- 0.5 microns, 8 +/- 0.5 microns, 8.5 +/- 0.5 microns, 9 +/- 0.5 microns, 10 +/- 0.5 microns, 15 +/-0.5 microns, 20 +/- 0.5 microns, 25 +/- 0.5 microns, 30 +/- 0.5 microns, 35 +/- 0.5 microns, 40 +/- 0.5 microns, 45 +/- 0.5 microns, 50 +/- 0.5 microns.

10. The method of Claim 1, wherein the propellant comprises a noble gas.

11. The method of Claim 10, wherein the noble gas comprises argon, nitrogen or helium.

12. The method of Claim 1, wherein the propellant is pressurized to at least 0.34 MPa (50 psi), at least 0.52 MPa (75 psi), at least 0.69 MPa (100 psi), at least 1.03 MPa (150 psi), at least 1.38 MPa (200 psi), at least 1.72 (250 psi), at least 2.07 MPa (300 psi), about 0.34 MPa (50 psi), about 0.52 MPa (75 psi), about 0.69 MPa (100 psi), about 1.04 MPa (150 psi), about 1.38 MPa (200 psi), about 1.72 MPa (250 psi), about 2.07 MPa (300 psi), about 2.41 MPa (350 psi), about 2.76 MPa (400 psi), about 3.10 MPa (450 psi), about 3.45 MPa (500 psi), about 3.79 MPa (550 psi), about 4.14 MPa (600 psi), 0.34 MPa to 3.45 MPa (50 psi to 500 psi), 1.38 MPa to 2.76 MPa (200 psi to 400 psi), 1.72 MPa to 2.41 MPa (250 psi to 350 psi), 0.34 MPa (50 psi), 0.52 MPa (75 psi), 0.69 MPa (100 psi), 1.04 MPa (150 psi), 1.38 MPa (200 psi), 1.72 MPa (250 psi), 2.07 MPa (300 psi), 2.41 MPa (350 psi), 2.76 MPa (400 psi), 3.10 MPa (450 psi), 3.45 MPa (500 psi), 3.79 MPa (550 psi), or 4.14 MPa (600 psi).

13. The method of Claim 1, wherein the polymer comprises PLGA with a ratio of 40:60 to 60:40.

14. The method of Claim 1, wherein the polymer comprises PLGA 50:50 having a number average molecular weight of 15 kD.

## Patentansprüche

1. Verfahren zum Beschichten eines Stents, umfassend:
Befestigen eines Stents auf einer Halterung in einer Beschichtungskammer, die dem Stent eine Ladung verleiht,
Bereitstellen einer ersten Wolke an geladenen Polymerpartikeln an die Stents durch schnelles Expandieren einer unter Druck stehenden Lösung des Polymers in verdichtetem 1,1,1,2,3,3-Hexafluorpropan durch eine erste Öffnung, wobei das Polymer PLGA umfasst, wobei eine erste Polymerschicht der Polymerpartikel auf dem Stent durch elektrostatische Ablagerung gebildet wird,
Sintern der ersten Polymerschicht bei >40ºC unter Umgebungsdruck,
Bereitstellen einer ersten Wolke an geladenen Sirolimuspartikeln an die Stents mit einer entgegengesetzten Ladung als die Ladung des Stents durch Pulsieren von Sirolimuspartikeln in die Kammer unter Verwendung eines Treibmittels, um eine erste Wirkstoffschicht auf dem Stent abzulagern, wobei zumindest ein Teil der Sirolimuspartikel in kristalliner Form vorliegt,
Bereitstellen einer zweiten Wolke an geladenen Partikeln des Polymers und einer dritten Wolke an geladenen Partikeln des Polymers an die Stents durch aufeinanderfolgendes schnelles Expandieren der unter Druck stehenden Lösung durch die erste Öffnung, wobei die Partikel eine entgegengesetzte Ladung als die Ladung des Stents aufweisen, wobei eine zweite Polymerschicht der Polymerpartikel auf dem Stent durch elektrostatische Ablagerung gebildet wird,
Sintern der zweiten Polymerschicht bei >40ºC unter Umgebungsdruck,
Bereitstellen einer zweiten Wolke an geladenen Sirolimuspartikeln an die Stents mit einer entgegengesetzten Ladung als die Ladung des Stents durch Pulsieren von Sirolimuspartikeln in die Kammer unter Verwendung eines Treibmittels, um eine zweite Wirkstoffschicht auf dem Stent abzulagern, wobei zumindest ein Teil der Sirolimuspartikel in kristalliner Form vorliegt,
Bereitstellen einer vierten Wolke an geladenen Partikeln des Polymers, einer fünften Wolke an geladenen Partikeln des Polymers, und einer sechsten Wolke an geladenen Partikeln des Polymers an die Stents durch aufeinanderfolgendes schnelles Expandieren einer unter Druck stehenden Lösung durch die erste Öffnung, wobei die Partikel eine entgegengesetzte Ladung als die Ladung des Stents aufweisen, wobei eine dritte Polymerschicht der Polymerpartikel auf dem Stent durch elektrostatische Ablagerung gebildet wird, und
Sintern der dritten Polymerschicht bei >40ºC bei 1,03 MPa (150 psi) Druckbeaufschlagung und mit gasförmigem 1,1,1,2,3,3-Hexafluorpropan, wobei die kristallförmigen Sirolimuspartikel in der ersten Wirkstoffschicht und der zweiten Wirkstoffschicht über alle Schritte im Verfahren in kristalliner Form bleiben.

2. Verfahren nach Anspruch 1, wobei der Stent auf der Halterung durch eine beliebige der ersten, zweiten, dritten, vierten, fünften oder sechsten Wolken an geladenen Polymerpartikeln, oder durch eine beliebige der ersten oder zweiten Wolken an geladenen Sirolimuspartikeln kreist.

3. Verfahren nach Anspruch 1, wobei die erste Öffnung ausreichend erhitzt ist, um sicherzustellen, dass das komprimierte Gas bei der Expansion aus der Öffnung vollständig verdampft ist.

4. Verfahren nach Anspruch 1, wobei die Konzentration der Lösung eine beliebige von 2 Gew./Vol.-% (Gewicht oder Masse des Polymers pro Gesamtvolumen), 4 Gew./Vol.-%, 2 Gew./Vol.-% bis 4 Gew./Vol.-%, 2 Gew./Vol.-% bis 4 Gew./Vol.-%, 2 Gew./Vol.-% +/- 0,5 Gew./Vol.-%, 2 Gew./Vol.-% +/- 0,25 Gew./Vol.-%, 2 Gew./Vol.-% +/- 0,1 Gew./Vol.-%, 4 Gew./Vol.-% +/- 0,5 Gew./Vol.-%, 4 Gew./Vol.-% +/- 0,25 Gew./Vol.-%, 4 Gew./Vol.-% +/- 0,1 Gew./Vol.-%, mindestens 1 Gew./Vol.-%, mindestens 1,5 Gew./Vol.-%, mindestens 2 Gew./Vol.-%, mindestens 3 Gew./Vol.-%, mindestens 4 Gew./Vol.-%, höchstens 4 Gew./Vol.-%, höchstens 5 Gew./Vol.-%, höchstens 6 Gew./Vol.-%, höchstens 7 Gew./Vol.-%, höchstens 8 Gew./Vol.-%, höchstens 9 Gew./Vol.-%, höchstens 10 Gew./Vol.-%, höchstens 11 Gew./Vol.-%, höchstens 12 Gew./Vol.-%, höchstens 13 Gew./Vol.-%, höchstens 14 Gew./Vol.-%, oder höchstens 15 Gew./Vol.-% beträgt.

5. Verfahren nach Anspruch 1, wobei die Sirolimuspartikel eine Partikelverteilung umfassen, so dass mindestens 99% bezogen auf das Volumen der Sirolimuspartikel weniger als 10 Mikrometer betragen, wobei die Verteilung bei 2,75 +/- 0,5 Mikrometern zentriert ist.

6. Verfahren nach Anspruch 1, wobei die Sirolimuspartikel eine Partikelverteilung umfassen, so dass 80%, 85%, 90%, 95%, 99%, mindestens 50%, mindestens 75%, mindestens 80%, mindestens 85%, mindestens 90%, mindestens 95%, mindestens 99% bezogen auf das Volumen der Partikel weniger als 10 Mikrometer betragen.

7. Verfahren nach Anspruch 1, wobei das Sintern bei etwa 100ºC durchgeführt wird.

8. Verfahren nach Anspruch 1, wobei die Sirolimuspartikel eine Partikelverteilung umfassen, so dass mindestens 50% bezogen auf das Volumen der Partikel weniger als 3 Mikrometer, weniger als 5 Mikrometer, weniger als 7,5 Mikrometer, weniger als 10 Mikrometer, weniger als 20 Mikrometer, weniger als 25 Mikrometer, weniger als 30 Mikrometer, weniger als 40 Mikrometer, weniger als 50 Mikrometer, weniger als 75 Mikrometer betragen.

9. Verfahren nach Anspruch 1, wobei die Sirolimuspartikel eine Verteilung aufweisen, die bei 1,0 +/- 0,5 Mikrometern, 1,25 +/- 0,5 Mikrometern, 1,5 +/- 0,5 Mikrometern, 1,75 +/- 0,5 Mikrometern, 2,0 +/- 0,5 Mikrometern, 2,25 +/- 0,5 Mikrometern, 2,5 +/- 0,5 Mikrometern, 2,75 +/- 0,5 Mikrometern, 3,0 +/- 0,5 Mikrometern, 3,25 +/- 0,5 Mikrometern, 3,5 +/- 0,5 Mikrometern, 3,75 +/- 0,5 Mikrometern, 4,0 +/- 0,5 Mikrometern, 4,25 +/- 0,5 Mikrometern, 4,5 +/- 0,5 Mikrometern, 4,75 +/- 0,5 Mikrometern, 5 +/- 0,5 Mikrometern, 5,5 +/- 0,5 Mikrometern, 6 +/- 0,5 Mikrometern, 6,5 +/- 0,5 Mikrometern, 7 +/- 0,5 Mikrometern, 7,5 +/- 0,5 Mikrometern, 8 +/- 0,5 Mikrometern, 8,5 +/- 0,5 Mikrometern, 9 +/- 0,5 Mikrometern, 10 +/- 0,5 Mikrometern, 15 +/- 0,5 Mikrometern, 20 +/- 0,5 Mikrometern, 25 +/- 0,5 Mikrometern, 30 +/- 0,5 Mikrometern, 35 +/- 0,5 Mikrometern, 40 +/- 0,5 Mikrometern, 45 +/- 0,5 Mikrometern, 50 +/- 0,5 Mikrometern zentriert ist.

10. Verfahren nach Anspruch 1, wobei das Treibmittel ein Edelgas umfasst.

11. Verfahren nach Anspruch 10, wobei das Edelgas Argon, Stickstoff oder Helium umfasst.

12. Verfahren nach Anspruch 1, wobei das Treibmittel bis mindestens 0,34 MPa (50 psi), mindestens 0,52 MPa (75 psi), mindestens 0,69 MPa (100 psi), mindestens 1,03 MPa (150 psi), mindestens 1,38 MPa (200 psi), mindestens 1,72 (250 psi), mindestens 2,07 MPa (300 psi), etwa 0,34 MPa (50 psi), etwa 0,52 MPa (75 psi), etwa 0,69 MPa (100 psi), etwa 1,04 MPa (150 psi), etwa 1,38 MPa (200 psi), etwa 1,72 MPa (250 psi), etwa 2,07 MPa (300 psi), etwa 2,41 MPa (350 psi), etwa 2,76 MPa (400 psi), etwa 3,10 MPa (450 psi), etwa 3,45 MPa (500 psi), etwa 3,79 MPa (550 psi), etwa 4,14 MPa (600 psi), 0,34 MPa bis 3,45 MPa (50 psi bis 500 psi), 1,38 MPa bis 2,76 MPa (200 psi bis 400 psi), 1,72 MPa bis 2,41 MPa (250 psi bis 350 psi), 0,34 MPa (50 psi), 0,52 MPa (75 psi), 0,69 MPa (100 psi), 1,04 MPa (150 psi), 1,38 MPa (200 psi), 1,72 MPa (250 psi), 2,07 MPa (300 psi), 2,41 MPa (350 psi), 2,76 MPa (400 psi), 3,10 MPa (450 psi), 3,45 MPa (500 psi), 3,79 MPa (550 psi), oder 4,14 MPa (600 psi) unter Druck steht.

13. Verfahren nach Anspruch 1, wobei das Polymer PLGA in einem Verhältnis von 40:60 bis 60:40 umfasst.

14. Verfahren nach Anspruch 1, wobei das Polymer PLGA 50:50 mit einem zahlengemittelten Molekulargewicht von 15kD umfasst.

## Revendications

1. Procédé de revêtement d'une endoprothèse comprenant :
le montage d'une endoprothèse sur un support dans une chambre de revêtement qui confère une charge à l'endoprothèse,
la fourniture aux endoprothèses d'un premier nuage de particules de polymère chargées par dilatation rapide d'une solution sous pression du polymère dans du 1,1,1,2,3,3-hexafluoropropane densifié à travers un premier orifice, dans laquelle le polymère comprend du PLGA, dans laquelle une première couche de polymère des particules de polymère est formée sur l'endoprothèse par dépôt électrostatique,
le frittage de la première couche de polymère à > 40 °C à la pression ambiante,
la fourniture aux endoprothèses d'un premier nuage de particules de sirolimus chargées ayant une charge opposée à la charge de l'endoprothèse en pulsant des particules de sirolimus dans la chambre à l'aide d'un agent propulseur afin de déposer une première couche d'agent sur l'endoprothèse, dans laquelle au moins une partie des particules de sirolimus sont sous forme cristalline,
la fourniture aux endoprothèses d'un deuxième nuage de particules chargées du polymère et d'un troisième nuage de particules chargées du polymère par dilatation rapide et séquentielle de la solution sous pression à travers le premier orifice, dans laquelle les particules ont une charge opposée à la charge de l'endoprothèse, dans laquelle une deuxième couche de polymère des particules de polymère est formée sur l'endoprothèse par dépôt électrostatique,
le frittage de la deuxième couche de polymère à > 40 °C à la pression ambiante,
la fourniture aux endoprothèses d'un deuxième nuage de particules de sirolimus chargées ayant une charge opposée à la charge de l'endoprothèse en pulsant les particules de sirolimus dans la chambre à l'aide d'un agent propulseur afin de déposer une deuxième couche d'agent sur l'endoprothèse, dans laquelle au moins une partie des particules de sirolimus sont sous forme cristalline,
la fourniture aux endoprothèses d'un quatrième nuage de particules chargées du polymère, d'un cinquième nuage de particules chargées du polymère et d'un sixième nuage de particules chargées du polymère par dilatation rapide et séquentielle d'une solution sous pression à travers le premier orifice, dans laquelle les particules ayant une charge opposée à la charge de l'endoprothèse, dans laquelle une troisième couche de polymère des particules de polymère est formée sur l'endoprothèse par dépôt électrostatique, et
le frittage de la troisième couche de polymère à > 40 °C, à une pressurisation de 1,03 MPa (150 psi), et avec du 1,1,1,2,3,3-hexafluoropropane gazeux, dans lequel les particules de sirolimus sous forme cristalline dans la première couche d'agent et dans la deuxième couche d'agent restent sous forme cristalline tout au long de toutes les étapes du procédé.

2. Procédé selon la revendication 1, dans lequel l'endoprothèse sur le support est en orbite à travers l'un quelconque des premier, deuxième, troisième, quatrième, cinquième ou sixième nuages de particules de polymère chargées, ou à travers l'un quelconque des premier ou deuxième nuages de particules de sirolimus chargées.

3. Procédé selon la revendication 1, dans lequel le premier orifice est suffisamment chauffé pour assurer que le gaz comprimé est entièrement vaporisé lors de la dilatation à partir de l'orifice.

4. Procédé selon la revendication 1, dans lequel la concentration de la solution est l'une quelconque de 2 % en p/v (poids ou masse de polymère par volume total), de 4 % en p/v, de 2 % en p/v à 4 % en p/v, de 2 % en p/v à 4 % en p/v, de 2 % en p/v +/- 0,5 % en p/v, de 2 % en p/v +/- 0,25 % en p/v, de 2 % en p/v +/-0,1 % en p/v, de 4 % en p/v +/- 0,5 % en p/v, de 4 % en p/v +/- 0,25 % en p/v, de 4 % en p/v +/- 0,1 % en p/v, d'au moins 1 % en p/v, d'au moins 1,5 % en p/v, d'au moins 2 % en p/v, d'au moins 3 % en p/v, d'au moins 4 % en p/v, d'au plus 4 % en p/v, d'au plus 5 % en p/v, d'au plus 6 % en p/v, d'au plus 7 % en p/v, d'au plus 8 % en p/v, d'au plus 9 % en p/v, d'au plus 10 % en p/v, d'au plus 11 % en p/v, d'au plus 12 % en p/v, d'au plus 13 % en p/v, d'au plus 14 % en p/v ou d'au plus 15 % en p/v.

5. Procédé selon la revendication 1, dans lequel les particules de sirolimus comprennent une distribution de particules telle qu'au moins 99 % en volume des particules de sirolimus sont inférieures à 10 microns, la distribution étant centrée à 2,75 +/- 0,5 microns.

6. Procédé selon la revendication 1, dans lequel les particules de sirolimus comprennent une distribution de particules telle que 80 %, 85 %, 90 %, 95 %, 99 %, au moins 50 %, au moins 75 %, au moins 80 %, au moins 85 %, au moins 90 %, au moins 95 %, au moins 99 % en volume des particules sont inférieures à 10 microns.

7. Procédé selon la revendication 1, dans lequel le frittage est effectué à environ 100 °C.

8. Procédé selon la revendication 1, dans lequel les particules de sirolimus comprennent une distribution de particules telle qu'au moins 50 % en volume des particules sont inférieures à 3 microns, inférieures à 5 microns, inférieures à 7,5 microns, inférieures à 10 microns, inférieures à 20 microns, inférieures à 25 microns, inférieures à 30 microns, inférieures à 40 microns, inférieures à 50 microns, inférieures à 75 microns.

9. Procédé selon la revendication 1, dans lequel les particules de sirolimus ont une distribution centrée à 1,0 +/- 0,5 microns, 1,25 +/- 0,5 microns, 1,5 +/- 0,5 microns, 1,75 +/- 0,5 microns, 2,0 +/- 0,5 microns, 2,25 +/- 0,5 microns, 2,5 +/- 0,5 microns, 2,75 +/- 0,5 microns, 3,0 +/- 0,5 microns, 3,25 +/- 0,5 microns, 3,5 +/- 0,5 microns, 3,75 +/- 0,5 microns, 4,0 +/- 0,5 microns, 4,25 +/- 0,5 microns, 4,5 +/- 0,5 microns, 4,75 +/- 0,5 microns, 5 +/- 0,5 microns, 5,5 +/- 0,5 microns, 6 +/- 0,5 microns, 6,5 +/- 0,5 microns, 7 +/- 0,5 microns, 7,5 +/- 0,5 microns, 8 +/- 0,5 microns, 8,5 +/- 0,5 microns, 9 +/- 0,5 microns, 10 +/- 0,5 microns, 15 +/- 0,5 microns, 20 +/- 0,5 microns, 25 +/- 0,5 microns, 30 +/- 0,5 microns, 35 +/- 0,5 microns, 40 +/- 0,5 microns, 45 +/- 0,5 microns, 50 +/- 0,5 microns.

10. Procédé selon la revendication 1, dans lequel l'agent propulseur comprend un gaz rare.

11. Procédé selon la revendication 10, dans lequel le gaz rare comprend de l'argon, de l'azote ou de l'hélium.

12. Procédé selon la revendication 1, dans lequel l'agent propulseur est pressurisé à au moins 0,34 MPa (50 psi), au moins 0,52 MPa (75 psi), au moins 0,69 MPa (100 psi), au moins 1,03 MPa (150 psi), au moins 1,38 MPa (200 psi), au moins 1,72 MPa (250 psi), au moins 2,07 MPa (300 psi), environ 0,34 MPa (50 psi), environ 0,52 MPa (75 psi), environ 0,69 MPa (100 psi), environ 1,04 MPa (150 psi), environ 1,38 MPa (200 psi), environ 1,72 MPa (250 psi), environ 2,07 MPa (300 psi), environ 2,41 MPa (350 psi), environ 2,76 MPa (400 psi), environ 3,10 MPa (450 psi), environ 3,45 MPa (500 psi), environ 3,79 MPa (550 psi), environ 4,14 MPa (600 psi), 0,34 MPa à 3,45 MPa (50 psi à 500 psi), 1,38 MPa à 2,76 MPa (200 psi à 400 psi), 1,72 MPa à 2,41 MPa (250 psi à 350 psi), 0,34 MPa (50 psi), 0,52 MPa (75 psi), 0,69 MPa (100 psi), 1,04 MPa (150 psi), 1,38 MPa (200 psi), 1,72 MPa (250 psi), 2,07 MPa (300 psi), 2,41 MPa (350 psi), 2,76 MPa (400 psi), 3,10 MPa (450 psi), 3,45 MPa (500 psi), 3,79 MPa (550 psi) ou 4,14 MPa (600 psi).

13. Procédé selon la revendication 1, dans lequel le polymère comprend du PLGA selon un rapport compris entre 40:60 et 60:40.

14. Procédé selon la revendication 1, dans lequel le polymère comprend du PLGA 50:50 ayant un poids moléculaire moyen en nombre de 15 kD.
